# EUROPEAN PATENT APPLICATION

(11) **EP 3 822 350 A1**
(43) Date of publication of application: **19.05.2021**
(21) Application number: 19810129.7
(22) Date of filing: 31.05.2019
(51) Int. Cl.: C12N 15/113, C12Q 1/6876

(54) **RNA INTERFERENCE-INDUCING NUCLEIC ACID INHIBITING NONCANONICAL TARGETS OF MICRO RNA, AND USE FOR SAME**

(30) Priority: 31.05.2018 KR 20180063054; 31.05.2018 KR 20180063055
(71) Applicant: Korea University Research and Business Foundation, Seoul 02841 (KR)
(72) Inventor: CHI, Sung Wook, Seoul 06362 (KR); JANG, Eun-Sook, Seoul 06362 (KR); SEOK, Hee Young, Seoul 06337 (KR)
(74) Representative: Dehns
(86) International application number: PCT/KR2019/006603
(87) International publication number: WO 2019/231288

(57) **Abstract**

The present invention relates to RNA interference-inducing nucleic acid that inhibits noncanonical target genes of micro RNA, in which part of the sequence of a specific micro RNA has been modified, and by using the RNA interference-inducing nucleic acid of the present invention, the biological function micro RNA exhibits by inhibiting noncanonical target genes is effectively increased or there is the benefit of selectively exhibiting only one of the biological functions of conventional micro RNA, i.e., the function of inhibiting noncanonical target genes, and the interference-inducing nucleic acid of the present invention enables cell cycling, differentiation, dedifferentiation, formation, movement, splitting, proliferation or death adjustment, and it is expected that the invention can be used in various fields such as drugs and cosmetics.

## Description

### [Technical Field]

The present invention relates to an RNA interference-inducing nucleic acid which inhibits gene expression and a use thereof, and particularly, to an interference-inducing nucleic acid having a useful effect exhibited by selectively suppressing a noncanonical target of microRNA and a use thereof.

This application claims priority to and the benefit of Korean Patent Application No. 10-2018-0063054, filed on May 31, 2018, Korean Patent Application No. 10-2018-0063055, filed on May 31, 2018, Korean Patent Application No.10-2019-0064333, filed on May 31, 2019, Korean Patent Application No.10-2019-0064334, filed on May 31, 2019, Korean Patent Application No. 10-2019-0064335, filed on May 31, 2019, and Korean Patent Application No. 10-2019-0064386, filed on May 31, 2019, the disclosures of which are incorporated herein by reference in their entirety.

### [Background Art]

RNA interference is a phenomenon of inhibiting gene expression at the post-transcription level. The RNA interference phenomenon naturally occurring is caused by microRNA (miRNA). MiRNA consists of 18 to 25 nucleotides, and most miRNAs are small RNA consisting of approximately 21 nucleotides and have base sequences complementary to messenger RNAs (mRNAs) of a target gene by Argonaute protein. Animal miRNAs are associated with the Argonaute protein to have partial base pairing with target mRNAs. In this case, when at least 6 consecutive bases are paired in a seed region defined by nucleotides from positions 1 to 8 based on the 5' end of miRNA, this sequence is recognized as a target, and most significantly, when at least 6 bases in positions 2 to 7 from the 5' end consecutively pair with target mRNA, the expression of the mRNA is suppressed by sufficiently degrading the corresponding target mRNA or inhibiting translation (Lewis BP, et.al, 2003, Cell, 115 (7), 787-98). Since the miRNA recognizes mRNA of a target gene through pair base pairing, one miRNA may usually affect the expression of hundreds to thousands of genes.

The suppressive action of miRNA on target gene expression is one of the major mechanisms of gene expression, is involved in differentiation and growth of cells under normal circumstances, and causes cancer, a degenerative disease or diabetes when there is a functional abnormality, and therefore, miRNA attracts attention as a key to life. Accordingly, to artificially induce the gene expression suppressive action of miRNA, an RNA interference material (siRNA or shRNA) having a seed region of miRNA is designed to be transfected into cells, thereby artificially differentiating cells or changing their functions, and in some cases, being used as a therapeutic for diseases. Therefore, complementary base pairing in the miRNA seed region which recognizes target mRNA by Argonaute is important for exhibiting a proper function of an RNA interference material such as miRNA. Particularly, to apply such an RNA interference material, it is required to identify each function of miRNA. Here, the function of miRNA is determined according to which target gene is suppressed, and thus analysis for all target genes (target) of miRNA is required.

At the transcriptome level, research on a miRNA target complex was first conducted through Ago HITS-CLIP (or called CLIP-Seq) by the inventors. Ago HITS CLIP assay is a method of forming a complex by covalent bonds between RNA and Argonaute protein (Ago) in cells through UV irradiation of cells or a tissue sample, isolating the RNA-Ago complex through immunoprecipitation using an Ago-specific recognition antibody, and analyzing the isolated RNA through next-generation sequencing. Accordingly, Ago-bound miRNA, a target mRNA group complementarily pairing therewith and its position can be exactly analyzed (Chi S et al, Nature, 2009, 460 (7254): 479-86).

As a result, the inventors identified that miRNA may bind to target mRNA although not binding with exact complementarity with a miRNA seed region. More specifically, binding of the miRNA seed region defined by a base sequence in positions 1 to 8 from the 5' end of miRNA with perfect pairing with at least 6 consecutive bases, and particularly, most significantly by base pairing in position 2 to 7 from the 5' end, with mRNA is referred to as canonical target recognition. Although not with consecutive and exact complementarity with the miRNA seed region, which deviates from the above-described rule, recognition as a target of miRNA and binding to miRNA is referred to as non-canonical target recognition. According to the result of Ago HITS-CLIP assay, it can be seen that the frequency of non-canonical target recognition through a seed region by miRNA is approximately 50% of a canonical recognition frequency.

Accordingly, it can be seen that the conventional RNA interference material (e. g., siRNA or shRNA) designed to include the sequence of the miRNA seed region has a biological function by suppressing several non-canonical target genes in addition to a canonical target gene.

### [Disclosure]

### [Technical Problem]

Therefore, the present invention is directed to solving a limitation of the conventional RNA interference material designed to include the intact sequence of miRNA seed region and improving is efficiency.

More specifically, the conventional RNA interference material designed to include the intact sequence of the miRNA seed region suppresses both a canonical target gene and a non-canonical target gene, and the canonical target gene is strongly suppressed, but the non-canonical target gene is very weakly suppressed. Therefore, the present invention is directed to providing an RNA interference-inducing nucleic acid having an effect of efficiently improving biological functions exhibited by suppressing a non-canonical target gene by conventional miRNA, or selectively exhibiting one of the biological functions, that is, only biological functions exhibited by suppressing a non-canonical target gene by conventional miRNA.

### [Technical Solution]

To solve the above-described problems, the present invention provides an RNA interference-inducing nucleic acid which suppresses a non-canonical target gene of miRNA by modifying a partial sequence of specific miRNA.

More specifically,
the present invention provides an RNA interference-inducing nucleic acid, which suppresses a non-canonical target gene of miRNA by modifying a partial sequence of specific miRNA in one or more single strands of the double strands of the nucleic acid inducing RNA interference,
wherein the RNA interference-inducing nucleic acid has a base sequence in positions 2 to 7 from the 5' end of specific miRNA, which is the most significant site involving in pairing with target mRNA, the base sequence having the sequence of four bases in positions 2 to 5 from the 5' end and the 6^{th} and 7^{th} bases that are the same and complementary to a base capable of pairing with the 6^{th} base of the specific miRNA, including all complementary bases including G:A and G:U wobble pairs, to allow non-canonical target base pairs bound to a bulge generated in the target gene between positions 5 and 6 of the miRNA to be a consecutive base pair by the disappearance of the bulge, or
the RNA interference-inducing nucleic acid has a modified base sequence in which at least one guanine base is substituted with uracil or adenine, preferably, in a sequence in positions 2 to 7 based on the 5' end of a base sequence between 1 to 9 bases from the 5' end of miRNA, such that a G:A or G:U wobble pair at the corresponding site becomes a canonical base pair of U:A or A:U.

Preferably, the specific miRNA has the same seed sequence selected from the group consisting of miR-124, miR-155, miR-122, miR-1, let-7, miR-133, miR-302 and miR-372, and consists of 18 to 24 bases.

Preferably, the RNA interference-inducing nucleic acid has any one or more of the sequences of bases in positions 2 to 7 from the 5' end:
an RNA interference-inducing nucleic acid having a base sequence of 5'-AA GGC C-3' (miR-124-BS) as an RNA interference-inducing nucleic acid suppressing a non-canonical target gene of miR-124 (SEQ ID NO: 1);
an RNA interference-inducing nucleic acid having a base sequence of 5'-GG AGU U-3' (miR-122-BS) as an RNA interference-inducing nucleic acid suppressing a non-canonical target gene of miR-122 (SEQ ID NO: 2);
an RNA interference-inducing nucleic acid having a base sequence of 5'-UA AUG G-3' (miR-155-BS) as an RNA interference-inducing nucleic acid suppressing a non-canonical target gene of miR-155 (SEQ ID NO: 3); or
an RNA interference-inducing nucleic acid having a base sequence of 5'-GG AAU U-3' (miR-1-BS) as an RNA interference-inducing nucleic acid suppressing a non-canonical target gene of miR-1 (SEQ ID NO: 4).

Preferably, the base sequence of the RNA interference-inducing nucleic acid is represented by any one or more as follows:
an RNA interference-inducing nucleic acid having a base sequence of 5'-UAA GGC CAC GCG GUG AAU GCC-3' (miR-124-BS) as an RNA interference-inducing nucleic acid suppressing a non-canonical target gene of miR-124 (SEQ ID NO: 5);
an RNA interference-inducing nucleic acid having a base sequence of 5'-UGG AGU UGU GAC AAU GGU GUU-3' (miR-122-BS) as an RNA interference-inducing nucleic acid suppressing a non-canonical target gene of miR-122 (SEQ ID NO: 6);
an RNA interference-inducing nucleic acid having a base sequence of 5'-UUA AUG GCU AAU CGU GAU AGG-3' (miR-155-BS) as an RNA interference-inducing nucleic acid suppressing a non-canonical target gene of miR-155 (SEQ ID NO: 7); or
an RNA interference-inducing nucleic acid having a base sequence of 5'-UGG AAU UGU AAA GAA GUA UGU-3' (miR-1-BS) as an RNA interference-inducing nucleic acid suppressing a non-canonical target gene of miR-1 (SEQ ID NO: 8).

Preferably, the RNA interference-inducing nucleic acid is any one or more of the base sequences between the 1^{st} to 9^{th} bases from the 5' end:
an RNA interference-inducing nucleic acid preferably having a modified base sequence in which at least one guanine is substituted with uracil among the sequence in positions 2 to 7 from the 5' end, such as a base sequence of 5'-UAA UGC ACG-3' (miR-124-G4U) (SEQ ID NO: 9), 5'-UAA GUC ACG-3' (miR-124-G5U) (SEQ ID NO: 10) or 5'-UAA UUC ACG-3' (miR-124-G4,5U) (SEQ ID NO: 11), as the RNA interference-inducing nucleic acid suppressing the non-canonical target gene of miR-124;
an RNA interference-inducing nucleic acid preferably having a modified base sequence in which at least one guanine is substituted with uracil among the sequence in positions 2 to 7 from the 5' end, such as a base sequence of 5'-UUG AAU GUA-3' (miR-1-G2U) (SEQ ID NO: 12), 5'-UGU AAU GUA-3' (miR-1-G3U) (SEQ ID NO: 13), 5'-UGG AAU UUA-3' (miR-1-G7U) (SEQ ID NO: 14), 5'-UUU AAU GUA-3' (miR-1-G2,3U) (SEQ ID NO: 15), 5'-UGU AAU UUA-3' (miR-1-G3,7U) (SEQ ID NO: 16), 5'-UUG AAU UUA-3' (miR-1-G2,7U) (SEQ ID NO: 17) or 5'-UUU AAU UUA-3' (miR-1-G2,3,7U) (SEQ ID NO: 18), as the RNA interference-inducing nucleic acid suppressing the non-canonical target gene of miR-1;
as an RNA interference-inducing nucleic acid suppressing the non-canonical target gene of miR-122,
an RNA interference-inducing nucleic acid having a base sequence of 5'-UUG AGU GUG-3' (miR-122-G2U) (SEQ ID NO: 19), 5'-UGU AGU GUG-3' (miR-122-G3U) (SEQ ID NO: 20), 5'-UGG AUU GUG-3' (miR-122-G5U) (SEQ ID NO: 21), 5'-UGG AGU UUG-3' (miR-122-G7U) (SEQ ID NO: 22), 5'-UGG AGU GUU-3' (miR-122-G9U) (SEQ ID NO: 23), 5'-UUU AGU GUG-3' (miR-122-G2,3U) (SEQ ID NO: 24), 5'-UUG AUU GUG-3' (miR-122-G2,5U) (SEQ ID NO: 25), 5'-UUG AGU UUG-3' (miR-122-G2,7U) (SEQ ID NO: 26), 5'-UUG AGU GUU-3' (miR-122-G2,9U) (SEQ ID NO: 27), 5'-UGU AUU GUG-3' (miR-122-G3,5U) (SEQ ID NO: 28), 5'-UGU AGU UUG-3' (miR-122-G3,7U) (SEQ ID NO: 29), 5'-UGU AGU GUU-3' (miR-122-G3,9U) (SEQ ID NO: 30), 5'-UGG AUU UUG-3' (miR-122-G5,7U) (SEQ ID NO: 31), 5'-UGG AUU GUU-3' (miR-122-G5,9U) (SEQ ID NO: 32), or 5'-UGG AGU UUU-3 (miR-122-G7,9U) (SEQ ID NO: 33), and preferably, having a modified base sequence in which at least one guanine is substituted with uracil among the sequence in positions 2 to 7 from the 5' end, such as 5'-UUG AGU GUG-3' (miR-122-G2U) (SEQ ID NO: 19), 5'-UGU AGU GUG-3' (miR-122-G3U) (SEQ ID NO: 20), 5'-UGG AUU GUG-3' (miR-122-G5U) (SEQ ID NO: 21), 5'-UGG AGU UUG-3' (miR-122-G7U) (SEQ ID NO: 22), 5'-UUU AGU GUG-3' (miR-122-G2,3U) (SEQ ID NO: 24), 5'-UUG AUU GUG-3' (miR-122-G2,5U) (SEQ ID NO: 25), 5'-UUG AGU UUG-3' (miR-122-G2,7U) (SEQ ID NO: 26), 5'-UGU AUU GUG-3' (miR-122-G3,5U) (SEQ ID NO: 28), 5'-UGU AGU UUG-3' (miR-122-G3,7U) (SEQ ID NO: 29) or 5'-UGG AUU UUG-3' (miR-122-G5,7U) (SEQ ID NO: 31);
an RNA interference-inducing nucleic acid having a modified base sequence in which at least one guanine is substituted with uracil in the sequence in positions 2 to 7 from the 5' end, such as a base sequence of 5'-UUU UGU CCC-3' (miR-133-G4U) (SEQ ID NO: 34), 5'-UUU GUU CCC-3' (miR-133-G5U) (SEQ ID NO: 35) or 5'-UUU UUU CCC-3'(miR-133-G4,5U) (SEQ ID NO: 36) as an RNA interference-inducing nucleic acid suppressing the non-canonical target gene of miR-133;
an RNA interference-inducing nucleic acid having a base sequence of 5'-UUA GGU AGU-3' (let-7-G2U) (SEQ ID NO: 37), 5'-UGA UGU AGU-3' (let-7-G4U) (SEQ ID NO: 38), 5'-UGA GUU AGU-3' (let-7-G5U) (SEQ ID NO: 39), 5'-UGA GGU AUU-3' (let-7-G8U) (SEQ ID NO: 40), 5'-UUA UGU AGU-3' (let-7-G2,4U) (SEQ ID NO: 41), 5'-UUA GUU AGU-3' (let-7-G2,5U) (SEQ ID NO: 42), 5'-UUA GGU AUU-3' (let-7-G2,8U) (SEQ ID NO: 43), 5'-UGA UUU AGU-3' (let-7-G4,5U) (SEQ ID NO: 44), 5'-UGA UGU AUU-3' (let-7-G4,8U) (SEQ ID NO: 45), or 5'-UGA GUU AUU-3' (let-7-G5,8U) (SEQ ID NO: 46), and preferably, having a modified base sequence in which at least one guanine is substituted with uracil in the sequence in positions 2 to 7 based on the 5' end, such as 5'-UUA GGU AGU-3' (let-7-G2U) (SEQ ID NO: 37), 5'-UGA UGU AGU-3' (let-7-G4U) (SEQ ID NO: 38), 5'-UGA GUU AGU-3' (let-7-G5U) (SEQ ID NO: 39), 5'-UUA UGU AGU-3' (let-7-G2,4U) (SEQ ID NO: 41), 5'-UUA GUU AGU-3' (let-7-G2,5U) (SEQ ID NO: 42) or 5'-UGA UUU AGU-3' (let-7-G4,5U) (SEQ ID NO: 44) as an RNA interference-inducing nucleic acid suppressing a non-canonical target gene of let-7;
an RNA interference-inducing nucleic acid preferably having a modified base sequence in which at least one guanine is substituted with uracil in the sequence in positions 2 to 7 based on the 5' end, such as 5'-UAA UUG CUU-3' (miR-302a-G4U) (SEQ ID NO: 47), 5'-UAA GUU CUU-3' (miR-302a-G6U) (SEQ ID NO: 48), or 5'-UAA UUU CUU-3' (miR-302a-G4,6U) (SEQ ID NO: 49), as an RNA interference-inducing nucleic acid suppressing the non-canonical target gene of miR-302a; or
an RNA interference-inducing nucleic acid preferably having a modified base sequence in which at least one guanine is substituted with uracil in the sequence in positions 2 to 7 from the 5' end, such as 5'-AAA UUG CUG-3' (miR-372-G4U) (SEQ ID NO: 50), 5'-AAA GUU CUG-3' (miR-372-G6U) (SEQ ID NO: 51), 5'-AAA GUG CUU-3' (miR-372-G9U) (SEQ ID NO: 52), 5'-AAA UUU CUG-3' (miR-372-G4,6U) (SEQ ID NO: 53), 5'-AAA UUG CUU-3' (miR-372-G4,9U) (SEQ ID NO: 54), or 5'-AAA GUU CUU-3' (miR-372-G6,9U) (SEQ ID NO: 55), as an RNA interference-inducing nucleic acid suppressing the non-canonical target gene of miR-372.

Preferably, the RNA interference-inducing nucleic acid has one or more of the following base sequences:
as an RNA interference-inducing nucleic acid suppressing the non-canonical target gene of miR-124,
an RNA interference-inducing nucleic acid having a base sequence of 5'-UAA UGC ACG CGG UGA AUG CCAA-3' (miR-124-G4U) (SEQ ID NO: 56), 5'-UAA GUC ACG CGG UGAAUG CCAA-3'(miR-124-G5U) (SEQ ID NO: 57) or 5'-UAA UUC ACG CGG UGAAUG CCAA-3'(miR-124-G4,5U) (SEQ ID NO: 58);
as an RNA interference-inducing nucleic acid suppressing the non-canonical target gene of miR-1,
an RNA interference-inducing nucleic acid having a base sequence of 5'-UUG AAU GUAAAG AAG UAU GUA U-3' (miR-1-G2U) (SEQ ID NO: 59), 5'-UGU AAU GUA AAG AAG UAU GUA U-3' (miR-1-G3U) (SEQ ID NO: 60), 5'-UGG AAU UUAAAG AAG UAU GUA U-3' (miR-1-G7U) (SEQ ID NO: 61), 5'-UUU AAU GUA AAG AAG UAU GUA U-3' (miR-1-G2,3U) (SEQ ID NO: 62), 5'-UGU AAU UUA AAG AAG UAU GUA U-3' (miR-1-G3,7U) (SEQ ID NO: 63), 5'-UUG AAU UUA AAG AAG UAU GUA U-3' (miR-1-G2,7U) (SEQ ID NO: 64) or 5'-UUU AAU UUAAAG AAG UAU GUA U-3' (miR-1-G2,3,7U) (SEQ ID NO: 65);
as an RNA interference-inducing nucleic acid suppressing the non-canonical target gene of miR-122,
an RNA interference-inducing nucleic acid having a base sequence of 5'-UUG AGU GUG ACA AUG GUG UUU G-3' (miR-122-G2U) (SEQ ID NO: 66), 5'-UGU AGU GUG ACA AUG GUG UUU G-3 (miR-122-G3U) (SEQ ID NO: 67), 5'-UGG AUU GUG ACA AUG GUG UUU G-3' (miR-122-G5U) (SEQ ID NO: 68), 5'-UGG AGU UUG ACA AUG GUG UUU G-3' (miR-122-G7U) (SEQ ID NO: 69), 5'-UGG AGU GUU ACA AUG GUG UUU G-3' (miR-122-G9U) (SEQ ID NO: 70), 5'-UUU AGU GUG ACAAUG GUG UUU G-3' (miR-122-G2,3U) (SEQ ID NO: 71), 5'-UUG AUU GUG ACA AUG GUG UUU G-3' (miR-122-G2,5U) (SEQ ID NO: 72), 5'-UUG AGU UUG ACA AUG GUG UUU G-3' (miR-122-G2,7U) (SEQ ID NO: 73), 5'-UUG AGU GUU ACA AUG GUG UUU G-3' (miR-122-G2,9U) (SEQ ID NO: 74), 5'-UGU AUU GUG ACAAUG GUG UUU G-3 (miR-122-G3,5U) (SEQ ID NO: 75), 5'-UGU AGU UUG ACA AUG GUG UUU G-3 (miR-122-G3,7U) (SEQ ID NO: 76), 5'-UGU AGU GUU ACAAUG GUG UUU G-3 (miR-122-G3,9U) (SEQ ID NO: 77), 5'-UGG AUU UUG ACA AUG GUG UUU G-3' (miR-122-G5,7U) (SEQ ID NO: 78), 5'-UGG AUU GUU ACA AUG GUG UUU G-3' (miR-122-G5,9U) (SEQ ID NO: 79) or 5'-UGG AGU UUU ACAAUG GUG UUU G-3' (miR-122-G7,9U) (SEQ ID NO: 80);
as an RNA interference-inducing nucleic acid suppressing the non-canonical target gene of miR-133, an RNA interference-inducing nucleic acid having the base sequence of 5'-UUU UGU CCC CUU CAA CCA GCU G -3' (miR-133-G4U) (SEQ ID NO: 81), 5'-UUU GUU CCC CUU CAA CCA GCU G-3' (miR-133-G5U) (SEQ ID NO: 82) or 5'-UUU UUU CCC CUU CAA CCA GCU G-3'(miR-133-G4,5U) (SEQ ID NO: 83);
as an RNA interference-inducing nucleic acid suppressing the non-canonical target gene of let-7, an RNA interference-inducing nucleic acid having a base sequence of 5'-UUA GGU AGU AGG UUG UAU AGU U-3' (let-7-G2U) (SEQ ID NO: 84), 5'-UGA UGU AGU AGG UUG UAU AGU U-3' (let-7-G4U) (SEQ ID NO: 85), 5'-UGA GUU AGU AGG UUG UAU AGU U-3' (let-7-G5U) (SEQ ID NO: 86), 5'-UGA GGU AUU AGG UUG UAU AGU U-3' (let-7-G8U) (SEQ ID NO: 87), 5'-UUA UGU AGU AGG UUG UAU AGU U-3' (let-7-G2,4U) (SEQ ID NO: 88), 5'-UUA GUU AGU AGG UUG UAU AGU U-3' (let-7-G2,5U) (SEQ ID NO: 89), 5'-UUA GGU AUU AGG UUG UAU AGU U-3' (let-7-G2,8U) (SEQ ID NO: 90), 5'-UGA UUU AGU AGG UUG UAU AGU U-3' (let-7-G4,5U) (SEQ ID NO: 91), 5'-UGA UGU AUU AGG UUG UAU AGU U-3' (let-7-G4,8U) (SEQ ID NO: 92) or 5'-UGA GUU AUU AGG UUG UAU AGU U-3' (let-7-G5,8U) (SEQ ID NO: 93);
as an RNA interference-inducing nucleic acid suppressing the non-canonical target gene of miR-302a, an RNA interference-inducing nucleic acid having a base sequence of 5'-UAA UUG CUU CCA UGU UUU GGU GA-3' (miR-302a-G4U) (SEQ ID NO: 94), 5'-UAA GUU CUU CCA UGU UUU GGU GA-3' (miR-302a-G6U) (SEQ ID NO: 95), or 5'-UAA UUU CUU CCA UGU UUU GGU GA-3' (miR-302a-G4,6U) (SEQ ID NO: 96); or
as an RNA interference-inducing nucleic acid suppressing the non-canonical target gene of miR-372, an RNA interference-inducing nucleic acid having a base sequence of 5'-AAA UUG CUG CGA CAU UUG AGC GU -3' (miR-372-G4U) (SEQ ID NO: 97), 5'-AAA GUU CUG CGA CAU UUG AGC GU -3' (miR-372-G6U) (SEQ ID NO: 98), 5'-AAA GUG CUU CGA CAU UUG AGC GU -3' (miR-372-G9U) (SEQ ID NO: 99), 5'-AAA UUU CUG CGA CAU UUG AGC GU -3' (miR-372-G4,6U) (SEQ ID NO: 100), 5'-AAA UUG CUU CGA CAU UUG AGC GU -3' (miR-372-G4,9U) (SEQ ID NO: 101) or 5'-AAA GUU CUU CGA CAU UUG AGC GU -3' (miR-372-G6,9U) (SEQ ID NO: 102).

The present invention provides a composition for inhibiting the expression of a non-canonical target gene of miRNA, which includes an RNA interference-inducing nucleic acid.

The present invention provides a method of inhibiting the expression of a non-canonical target gene of miRNA, which includes administering the composition including an RNA interference-inducing nucleic acid into a subject.

The present invention provides the use of an RNA interference-inducing nucleic acid for inhibiting the expression of a non-canonical target gene of miRNA.

The present invention provides a composition for regulating cell cycling, differentiation, dedifferentiation, morphology, migration, division, proliferation or apoptosis, which includes an RNA interference-inducing nucleic acid.

The present invention provides a method of regulating cell cycling, differentiation, dedifferentiation, morphology, migration, division, proliferation or apoptosis, which includes administering the composition including an RNA interference-inducing nucleic acid into a subject.

The present invention provides the use of an RNA interference-inducing nucleic acid for regulating cell cycling, differentiation, dedifferentiation, morphology, migration, division, proliferation or apoptosis.

Preferably, the composition is any one or more of the compositions selected from:
a composition for inducing cancer cell death, which includes an RNA interference-inducing nucleic acid suppressing a non-canonical target gene of miR-124;
a composition for inducing neurite differentiation, which includes an RNA interference-inducing nucleic acid suppressing a non-canonical target gene of miR-124;
a composition for inducing cell cycle arrest in liver cancer cells, which includes an RNA interference-inducing nucleic acid suppressing a non-canonical target gene of miR-122;
a composition for promoting differentiation of muscle cells or muscle fibrosis, which includes an RNA interference-inducing nucleic acid suppressing a non-canonical target gene of miR-1;
a composition for inducing muscle cell death, which includes an RNA interference-inducing nucleic acid suppressing a non-canonical target gene of miR-155;
a composition for inducing cell death of neuroblastomas, which includes an RNA interference-inducing nucleic acid suppressing a non-canonical target gene of miR-124;
a composition for promoting cell division or proliferation of neuroblastomas, which includes an RNA interference-inducing nucleic acid suppressing a non-canonical target gene of miR-124;
a composition for inducing myocardial hypertrophy, which includes an RNA interference-inducing nucleic acid suppressing a non-canonical target gene of miR-1;
a composition for inducing myocardial hypertrophy, which includes an RNA interference-inducing nucleic acid suppressing a non-canonical target gene of miR-133;
a composition for inducing cell cycle arrest in cancer cells, which includes an RNA interference-inducing nucleic acid suppressing a non-canonical target gene of let-7;
a composition for inducing the cell cycle progressing activity of hepatocytes, which includes an RNA interference-inducing nucleic acid suppressing a non-canonical target gene of let-7;
a composition for promoting dedifferentiation, which includes an RNA interference-inducing nucleic acid suppressing a non-canonical target gene of miR-302a;
a composition for promoting dedifferentiation, which includes an RNA interference-inducing nucleic acid suppressing a non-canonical target gene of miR-372; or
a composition for inhibiting cell migration of liver cancer cells, which includes an RNA interference-inducing nucleic acid suppressing a non-canonical target gene of miR-122.

The present invention provides a method of preparing an RNA interference-inducing nucleic acid, which inhibits the expression of a non-canonical target gene of the miRNA by modifying a partial sequence of specific miRNA in one or more single strands of the double strands of the nucleic acid inducing RNA interference, the method including the following steps:
constructing an RNA interference-inducing nucleic acid having a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, to allow non-canonical target base pairs bound to a bulge generated in the target gene between positions 5 and 6 of the miRNA to be a consecutive base pair by the disappearance of the bulge, or
constructing an RNA interference-inducing nucleic acid having a modified base sequence in which at least one guanine is substituted with uracil in a base sequence between the first to ninth bases from the 5' end of specific miRNA, in which a G:A or G:U wobble pair at the corresponding site becomes a canonical base sequence of U:A or A:U.

In addition, the present invention provides a method of screening a test material for regulating cell cycling, differentiation, dedifferentiation, morphology, migration, division, proliferation or death, which includes the following steps:
transfecting an RNA interference-inducing nucleic acid into a target cell;
treating the target cell with a test material; and
confirming the expression level or expression of a non-canonical target gene of miRNA suppressing an RNA interference-inducing nucleic acid in target cells.

In addition, the present invention provides an RNA interference-inducing nucleic acid as follows:

### 1. 2 'OMe-modified RNA interference-inducing nucleic acid

The present invention provides an RNA interference-inducing nucleic acid suppressing a non-canonical target gene of miRNA by modifying a partial sequence of specific miRNA in one or more single strands of the double strands of the nucleic acid inducing RNA interference, wherein
the RNA interference-inducing nucleic acid has a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, to allow non-canonical target base pairs bound to a bulge generated in the target gene between positions 5 and 6 of the miRNA to be a consecutive base pair by the disappearance of the bulge, and
the specific miRNA is characterized by adding a methyl group (2'OMe) to the 2' position of the ribosyl ring of the 6^{th} nucleotide from the 5' end.

Preferably, the RNA interference-inducing nucleic acid inhibits only the expression of a canonical seed target gene of the corresponding RNA interference-inducing nucleic acid.

Preferably, the RNA interference-inducing nucleic acid is characterized by specifically suppressing a non-canonical nucleation bulge site of the specific miRNA, and removing a non-canonical nucleation bulge site which may be newly generated.

In addition, the present invention provides a composition for inhibiting the expression of a non-canonical target gene of miRNA, which includes an RNA interference-inducing nucleic acid.

In addition, the present invention provides a method of inhibiting the expression of a non-canonical target gene of miRNA, which includes an RNA interference-inducing nucleic acid.

In addition, the present invention provides the use of an RNA interference-inducing nucleic acid for inhibiting the expression of a non-canonical target gene of miRNA.

In addition, the present invention provides a method of preparing an RNA interference-inducing nucleic acid, which inhibits the expression of a non-canonical target gene of the miRNA by modifying a partial sequence of specific miRNA in one or more single strands of the double strands of the nucleic acid inducing RNA interference, the method including the following steps:
constructing an RNA interference-inducing nucleic acid having a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, to allow non-canonical target base pairs bound to a bulge generated in the target gene between positions 5 and 6 of the miRNA to be a consecutive base pair by the disappearance of the bulge; and
adding a methyl group (2'OMe) to the 2' position of the ribosyl ring of the 6^{th} nucleotide from the 5' end of the specific miRNA.

### 2. RNA interference-inducing nucleic acids suppressing non-canonical nucleation bulge target site of miRNA (SEQ ID NOs: 103 to 528)

The present invention provides an RNA interference-inducing nucleic acid, which suppresses a non-canonical target gene of miRNA by modifying a partial sequence of specific miRNA in one or more single strands of the double strands of the nucleic acid inducing RNA interference, wherein
the RNA interference-inducing nucleic acid has a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, to allow non-canonical target base pairs bound to a bulge generated in the target gene between positions 5 and 6 of the miRNA to be a consecutive base pair by the disappearance of the bulge.

Preferably, the RNA interference-inducing nucleic acid is characterized by selectively suppressing only a non-canonical nucleation bulge site and not suppressing a canonical target gene of miRNA.

Preferably, the specific miRNA consists of 6 to 24 bases, which includes the sequence of four bases in positions 2 to 5 from the 5' end of the same seed sequence of one selected from the group consisting of let-7/98/4458/4500, miR-125a-5p/125b-5p/351/670/4319, miR-124/124ab/506, miR-9/9ab, miR-29abcd, miR-103a/107/107ab, miR-221/222/222ab/1928, miR-26ab/1297/4465, miR-15abc/16/16abc/195/322/424/497/1907, miR-126-3p, miR-30abcdef/30abe-5p/384-5p, miR-33ab/33-5p, miR-34ac/34bc-5p/449abc/449c-5p, miR-19ab, miR-99ab/100, miR-17/17-5p/20ab/20b-5p/93/106ab/427/518a-3p/519d, miR-27abc/27a-3p, miR-218/218a, miR-22/22-3p, miR-185/882/3473/4306/4644, miR-181abcd/4262, miR-338/338-3p, miR-127/127-3p, miR-101/101ab, miR-149, miR-324-5p, miR-24/24ab/24-3p, miR-33a-3p/365/365-3p, miR-139-5p, miR-138/138ab, miR-143/1721/4770, miR-25/32/92abc/363/363-3p/367, miR-574-5p, miR-7/7ab, miR-145, miR-135ab/135a-5p, miR-148ab-3p/152, miR-28-5p/708/1407/1653/3139, miR-130ac/301ab/301b/301b-3p/454/721/4295/3666, miR-3132, miR-155, miR-485-3p, miR-132/212/212-3p, hsa-miR-9-3p, miR-374ab, miR-129-3p/129ab-3p/129-1-3p/129-2-3p, hsa-miR-126-5p, miR-425/425-5p/489, miR-423-3p, miR-21/590-5p, miR-31, hsa-miR-20b-3p, hsa-let-7d-3p, miR-191, miR-18ab/4735-3p, miR-369-3p, hsa-miR-5187-5p, miR-382, miR-485-5p/1698/1703/1962, hsa-miR-136-3p, miR-576-3p, miR-204/204b/211, miR-769-5p, miR-342-5p/4664-5p, miR-361-5p, miR-199ab-3p/3129-5p, miR-142-3p, miR-299-5p/3563-5p, miR-193/193b/193a-3p, hsa-miR-1277-5p, miR-140/140-5p/876-3p/1244, hsa-miR-30a/d/e-3p, hsa-let-7i-3p, miR-409-5p/409a, miR-379/1193-5p/3529, miR-136, miR-154/872, miR-4684-3p, miR-361-3p, miR-335/335-5p, miR-423a/423-5p/3184/3573-5p, miR-371/373/371b-5p, miR-1185/3679-5p, miR-3613-3p, miR-93/93a/105/106a/291a-3p/294/295/302abcde/372/373/428/519a/520be/520acd-3p/1378/1420ac, miR-876-5p/3167, miR-329/329ab/362-3p, miR-582-5p, miR-146ac/146b-5p, miR-380/380-3p, miR-499-3p/499a-3p, miR-551a, miR-142-5p, hsa-miR-17-3p, miR-199ab-5p, miR-542-3p, miR-1277, hsa-miR-29c-5p, miR-3145-3p, hsa-miR-106b-3p, hsa-miR-22-5p, miR-744/1716, hsa-miR-132-5p, miR-488, miR-501-3p/502-3p/500/502a, miR-486-5p/3107, miR-450a/451a, hsa-miR-30c-3p, miR-499-5p, miR-421, miR-197, miR-296-5p, miR-326/330/330-5p, miR-214/761/3619-5p, miR-612/1285/3187-5p, miR-409-3p, miR-378/422a/378bcdefhi, miR-342-3p, miR-338-5p, miR-625, miR-200bc/429/548a, hsa-miR-376a-5p, miR-584, miR-411, miR-573/3533/3616-5p/3647-5p, miR-885-5p, hsa-miR-99-3p, miR-876-3p, miR-654-3p, hsa-miR-340-3p, miR-3614-5p, hsa-miR-124-5p, miR-491-5p, miR-96/507/1271, miR-548a-3p/548ef/2285a, hsa-miR-32-3p, miR-3942-5p/4703-5p, miR-34b/449c/1360/2682, hsa-miR-23a/b-5p, miR-362-5p/500b, miR-677/4420, miR-577, miR-3613-5p, miR-369-5p, miR-150/5127, miR-544/544ab/544-3p, hsa-miR-29a-5p, miR-873, miR-3614-3p, miR-186, miR-483-3p, hsa-miR-374a-3p, miR-196abc, hsa-miR-145-3p, hsa-miR-29b-2-5p, hsa-miR-221-5p, miR-323b-3p, miR-616, miR-330-3p, hsa-miR-7-3p, miR-187, hsa-miR-26a-3p, miR-452/4676-3p, miR-129-5p/129ab-5p, miR-223, miR-4755-3p, miR-1247, miR-3129-3p, hsa-miR-335-3p, miR-542-5p, hsa-miR-181a-3p, hsa-miR-186-3p, hsa-miR-27b-5p, miR-491-3p, miR-4687-3p, hsa-miR-101-5p, miR-4772-5p, miR-337-3p, hsa-miR-223-5p, hsa-miR-16/195-3p, miR-3677-3p, hsa-miR-766-5p, miR-299/299-3p/3563-3p, miR-3140-3p, miR-532-5p/511, hsa-miR-24-5p, miR-4778-5p, miR-642b, miR-483-5p, miR-767-5p, hsa-miR-31-3p, miR-574-3p, miR-3173-3p, miR-2127/4728-5p, hsa-miR-103a-2-5p, miR-3591-3p, hsa-miR-625-3p, hsa-miR-15b-3p, miR-522/518e/1422p, miR-548d-3p/548acbz, hsa-miR-452-3p, miR-192/215, miR-1551/4524, hsa-miR-425-3p, miR-3126-3p, hsa-miR-125b-2-3p, miR-324-3p/1913, hsa-miR-141-5p, hsa-miR-365a/b-5p, hsa-miR-29b-1-5p, miR-563/380-5p, miR-1304, miR-216c/1461/4684-5p, hsa-miR-2681-5p, miR-194, miR-296-3p, hsa-miR-205-3p, miR-888, miR-4802-3p, hsa-let-7a/g-3p, miR-762/4492/4498, hsa-miR-744-3p, hsa-miR-148b-5p, miR-514/514b-3p, miR-28-3p, miR-550a, hsa-miR-125b-1-3p, hsa-miR-506-5p, hsa-miR-1306-5p, miR-3189-3p, miR-675-5p/4466, hsa-miR-34a-3p, hsa-miR-454-5p, miR-509-5p/509-3-5p/4418, hsa-miR-19a/b-5p, miR-4755-5p, hsa-miR-93-3p, miR-3130-5p/4482, hsa-miR-488-5p, hsa-miR-378a-5p, miR-575/4676-5p, miR-1307, miR-3942-3p, miR-4677-5p, miR-339-3p, miR-548b-3p, hsa-miR-642b-5p, miR-188-5p, hsa-miR-652-5p, miR-2114, miR-3688-5p, hsa-miR-15a-3p, hsa-miR-181c-3p, miR-122/122a/1352, miR-556-3p, hsa-miR-218-2-3p, miR-643, miR-140-3p, miR-1245, hsa-miR-2115-3p, miR-518bcf/518a-3p/518d-3p, miR-3200-3p, miR-545/3065/3065-5p, miR-1903/4778-3p, hsa-miR-302a-5p, hsa-miR-183-3p, miR-3144-5p, miR-582-3p, miR-4662a-3p, miR-3140-5p, hsa-miR-106a-3p, hsa-miR-135a-3p, miR-345/345-5p, miR-125a-3p/1554, miR-3145-5p, miR-676, miR-3173-5p, hsa-miR-5586-3p, miR-615-3p, miR-3688-3p, miR-4662a-5p, miR-4659ab-5p, hsa-miR-5586-5p, hsa-miR-514a-5p, miR-10abc/10a-5p, hsa-miR-888-3p, miR-3127-5p, miR-508-3p, hsa-miR-185-3p, hsa-miR-200c-5p,hsa-miR-550a-3p, miR-513c/514b-5p, miR-490-3p, hsa-miR-5187-3p, miR-3664-3p, miR-3189-5p, miR-4670-3p, miR-105/105ab, hsa-miR-135b-3p, hsa-miR-5010-3p, miR-493/493b, miR-3605-3p, miR-188-3p, hsa-miR-449c-3p, miR-4761-5p, miR-224, miR-4796-5p, hsa-miR-551b-5p, miR-556-5p, hsa-miR-122-3p, miR-4677-3p, miR-877, miR-576-5p, miR-490-5p, hsa-miR-589-3p, miR-4786-3p, hsa-miR-374b-3p, hsa-miR-26b-3p, miR-3158-3p, miR-4423-3p, miR-518d-5p/519bc-5p520c-5p/523b/526a, miR-4707-3p, hsa-miR-10a-3p, miR-526b, hsa-miR-676-5p, hsa-miR-660-3p, hsa-miR-5004-3p, miR-193a-5p, hsa-miR-222-5p, miR-4661-3p, hsa-miR-25-5p, miR-4670-5p, miR-659, miR-1745/3194-3p, hsa-miR-182-3p, miR-298/2347/2467-3p, hsa-miR-130b-5p, miR-4746-3p, miR-1893/2277-5p, miR-3619-3p, hsa-miR-138-1-3p, miR-4728-3p, miR-3127-3p, miR-671-3p, hsa-miR-211-3p, hsa-miR-2114-3p, hsa-miR-877-3p, miR-3157-5p, miR-502-5p, miR-500a, miR-548g, miR-523, hsa-miR-584-3p, miR-205/205ab, miR-4793-5p, hsa-miR-363-5p, hsa-miR-214-5p, miR-3180-5p, miR-1404/2110, miR-3157-3p, hsa-miR-191-3p, miR-1346/3940-5p/4507, miR-4746-5p, miR-3939, hsa-miR-181a-2-3p, hsa-miR-500a-3p, hsa-miR-196b-3p, hsa-miR-675-3p, hsa-miR-548aj/g/x-5p, miR-4659ab-3p, hsa-miR-5001-3p, hsa-miR-1247-3p, miR-2890/4707-5p, hsa-miR-150-3p, hsa-miR-629-3p, miR-2277-3p, miR-3547/3663-3p, miR-34bc-3p, miR-518ef, miR-3187-3p, miR-1306/1306-3p, miR-3177-3p, miR-1ab/206/613, miR-128/128ab, miR-1296, miR-598/598-3p, miR-887, miR-1-5p, miR-376c/741-5p, miR-374c/655, miR-494, miR-651, miR-1301/5047, miR-381-5p, miR-216a, miR-300/381/539-3p, miR-1249, miR-579, miR-656, miR-433, miR-1180, miR-597/1970, miR-190a-3p, miR-1537, miR-874-5p, miR-410/344de/344b-1-3p, miR-370, miR-219-2-3p/219-3p, miR-3620, miR-504/4725-5p, miR-2964/2964a-5p, miR-450a-2-3p, miR-511, miR-6505-3p, miR-433-5p, miR-6741-3p, miR-370-5p, miR-579-5p, miR-376c-5p,miR-376b-5p, miR-552/3097-5p, miR-1910, miR-758, miR-6735-3p, miR-376a-2-5p, miR-585, miR-451 and miR-137/137ab, and bases in position 6 and 7 are the same and complementary to a base capable of pairing with the 6^{th} base of the specific miRNA.

Preferably, the RNA interference-inducing nucleic acid includes a base sequence represented by any one or more of SEQ ID NOs: 103 to 528 (see Table 3).

In addition, the present invention provides a composition for inhibiting the expression of a non-canonical target gene of miRNA, which includes an RNA interference-inducing nucleic acid.

In addition, the present invention provides a method of inhibiting the expression of a non-canonical target gene of miRNA, which includes administering a composition including an RNA interference-inducing nucleic acid, into a subject.

In addition, the present invention provides the use of an RNA interference-inducing nucleic acid for suppressing a non-canonical target gene of miRNA.

In addition, the present invention provides a method of preparing an RNA interference-inducing nucleic acid, which inhibits the expression of a non-canonical target gene of miRNA in which a partial sequence of specific miRNA is modified in one or more single strands of the double strands of the nucleic acid inducing RNA interference, the method including the following steps:
constructing an RNA interference-inducing nucleic acid having a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, to allow non-canonical target base pairs bound to a bulge generated in the target gene between positions 5 and 6 of the miRNA to be a consecutive base pair by the disappearance of the bulge.

### 3. RNA interference-inducing nucleic acids suppressing non-canonical G:A wobble target site of miRNA (SEQ ID NOs: 529 to 863)

The present invention provides an RNA interference-inducing nucleic acid suppressing a non-canonical target gene of miRNA by modifying a partial sequence of specific miRNA in one or more single strands of the double strands of the nucleic acid inducing RNA interference, wherein
the RNA interference-inducing nucleic acid has a modified base sequence in which at least one guanine is substituted with uracil in a base sequence between the second to ninth bases from the 5' end of specific miRNA, and the G:A wobble at the corresponding site becomes the canonical base pair of U:A.

Preferably, the RNA interference-inducing nucleic acid includes a sequence of 6 to 8 consecutive bases, starting from the second base from the 5' end of specific miRNA, in which
at least one guanine base is substituted with an uracil base.

Preferably, the RNA interference-inducing nucleic acid selectively suppresses only a non-canonical target gene of miRNA binding to a G:A wobble pair, and does not suppress a canonical target gene of miRNA.

Preferably, the specific miRNA consists of 6 to 24 bases, which includes one selected from the group consisting of hsa-miR-1-3p, hsa-miR-194-5p, hsa-miR-193a-5p, hsa-miR-15b-3p, hsa-miR-200c-5p, hsa-miR-214-5p, hsa-miR-134-5p, hsa-miR-145-3p, hsa-miR-22-5p, hsa-miR-423-3p, hsa-miR-873-3p, hsa-miR-122-5p, hsa-miR-143-3p, hsa-miR-485-5p, hsa-miR-409-5p, hsa-miR-24-3p, hsa-miR-223-3p, hsa-miR-144-5p, hsa-miR-379-5p, hsa-miR-146b-5p/hsa-miR-146a-5p, hsa-miR-539-5p, hsa-miR-296-5p, hsa-miR-767-5p, hsa-miR-34a-5p/hsa-miR-34c-5p, hsa-let-7f-5p/hsa-let-7d-5p/hsa-let-7b-5p/hsa-let-7a-5p/hsa-let-7e-5p/hsa-miR-202-3p/hsa-let-7i-5p/hsa-miR-98-5p/hsa-let-7c-5p/hsa-let-7g-5p, hsa-miR-1271-3p, hsa-miR-138-5p, hsa-miR-19b-3p/hsa-miR-19a-3p, hsa-miR-27a-5p, hsa-miR-146b-3p, hsa-miR-7-5p, hsa-miR-423-5p, hsa-miR-324-5p, hsa-miR-629-5p, hsa-miR-139-3p, hsa-miR-30d-5p/hsa-miR-30e-5p/hsa-miR-30a-5p/hsa-miR-30c-5p/hsa-miR-30b-5p, hsa-miR-221-3p/hsa-miR-222-3p, hsa-miR-509-3p, hsa-miR-769-5p, hsa-miR-142-3p, hsa-miR-185-5p, hsa-miR-508-3p/hsa-miR-219a-5p, hsa-miR-31-5p, hsa-miR-103a-3p/hsa-miR-107, hsa-miR-542-3p, hsa-miR-219a-2-3p, hsa-miR-29c-3p/hsa-miR-29a-3p/hsa-miR-29b-3p, hsa-miR-125b-1-3p, hsa-miR-411-5p, hsa-miR-196a-5p/hsa-miR-196b-5p, hsa-miR-3622a-5p, hsa-miR-127-5p, hsa-miR-22-3p, hsa-miR-153-3p, hsa-miR-15b-5p/hsa-miR-16-5p/hsa-miR-424-5p, hsa-let-7g-3p/hsa-miR-493-5p/hsa-let-7c-3p, hsa-let-7i-3p, hsa-miR-218-5p, hsa-miR-1307-5p, hsa-miR-127-3p, hsa-miR-210-3p, hsa-miR-187-3p, hsa-miR-192-3p, hsa-miR-192-5p, hsa-miR-21-5p, hsa-miR-500a-3p, hsa-miR-203a-3p, hsa-miR-30c-2-3p, hsa-miR-488-3p, hsa-miR-301a-3p/hsa-miR-301b-3p, hsa-miR-126-3p, hsa-miR-26b-3p, hsa-miR-324-3p, hsa-miR-3065-3p, hsa-miR-124-5p, hsa-miR-345-5p, hsa-miR-615-3p, hsa-miR-889-5p/hsa-miR-135a-5p/hsa-miR-135b-5p, hsa-miR-18a-5p, hsa-miR-708-5p/hsa-miR-28-5p, hsa-miR-224-5p, hsa-miR-100-3p, hsa-miR-873-5p, hsa-miR-4662a-5p, hsa-miR-99b-3p/hsa-miR-99a-3p, hsa-miR-433-5p, hsa-miR-3605-5p, hsa-miR-744-5p, hsa-miR-1296-5p, hsa-miR-133a-3p, hsa-miR-382-5p, hsa-miR-425-5p, hsa-miR-377-5p, hsa-miR-3180-3p, hsa-miR-758-3p, hsa-miR-93-3p, hsa-miR-154-5p, hsa-miR-124-3p, hsa-miR-194-3p, hsa-miR-375, hsa-miR-148a-5p, hsa-miR-2277-5p, hsa-miR-17-3p, hsa-miR-4772-3p, hsa-miR-329-5p, hsa-miR-182-5p/hsa-miR-96-5p, hsa-miR-2467-5p/hsa-miR-485-5p, hsa-miR-149-5p, hsa-miR-29b-2-5p, hsa-miR-122-3p, hsa-miR-302a-3p/hsa-miR-520a-3p/hsa-miR-519b-3p/hsa-miR-520b/hsa-miR-519c-3p/hsa-miR-520c-3p/hsa-miR-519a-3p, hsa-miR-532-5p, hsa-miR-132-5p, hsa-miR-541-5p, hsa-miR-671-3p, hsa-miR-518e-3p, hsa-miR-487a-5p, hsa-miR-589-5p/hsa-miR- 146b-5p/hsa-miR- 146a-5p, hsa-miR-196b-5p/hsa-miR-196a-5p, hsa-miR-486-3p, hsa-miR-378a-3p, hsa-miR-27b-5p, hsa-miR-6720-3p, hsa-miR-574-3p, hsa-miR-29a-5p, hsa-miR-30c-2-3p/hsa-miR-30c-1-3p, hsa-miR-199b-3p, hsa-miR-574-5p, hsa-miR-4677-3p, hsa-miR-654-3p, hsa-miR-652-3p, hsa-miR-19a-3p/hsa-miR-19b-3p, hsa-let-7c-5p/hsa-miR-98-5p/hsa-let-7g-5p/hsa-let-7f-5p/hsa-miR-202-3p/hsa-let-7b-5p/hsa-let-7e-5p/hsa-let-7a-5p/hsa-let-7d-5p/hsa-let-7i-5p, hsa-miR-3663-3p, hsa-miR-152-3p/hsa-miR-148b-3p/hsa-miR-148a-3p, hsa-miR-193b-5p, hsa-miR-502-3p/hsa-miR-501-3p, hsa-miR-299-3p, hsa-miR-140-5p, hsa-miR-96-5p/hsa-miR-182-5p, hsa-miR-193b-3p, hsa-miR-365a-3p, hsa-miR-486-5p, hsa-miR-493-3p, hsa-miR-548am-5p, hsa-miR-20b-5p/hsa-miR-20a-5p/hsa-miR-93 -5p/hsa-miR-17-5p/hsa-miR-106b-5p, hsa-miR-541-3p, hsa-miR-452-5p, hsa-miR-221-5p, hsa-miR-518f-3p, hsa-miR-370-3p, hsa-miR-107/hsa-miR-103a-3p, hsa-miR-338-3p, hsa-miR-409-3p, hsa-let-7d-5p/hsa-let-7g-5p/hsa-let-7i-5p/hsa-let-7f-5p/hsa-let-7e-5p/hsa-let-7a-5p/hsa-let-7b-5p/hsa-let-7c-5p, hsa-miR-130b-3p/hsa-miR-301a-3p/hsa-miR-130a-3p/hsa-miR-301b-3p, hsa-miR-512-3p, hsa-miR-191-5p, hsa-miR-509-3-5p, hsa-miR-92a-3p/hsa-miR-92b-3p/hsa-miR-363-3p/hsa-miR-25 -3p/hsa-miR-32-5p, hsa-miR-183-5p, hsa-miR-1307-3p, hsa-miR-499a-5p/hsa-miR-208a-3p, hsa-miR-186-5p, hsa-miR-450b-5p, hsa-miR-450a-5p, hsa-miR-101-3p/hsa-miR-144-3p, hsa-miR-320a, hsa-miR-199b-5p/hsa-miR-199a-5p, hsa-miR-135a-5p, hsa-miR-145-5p, hsa-miR-26a-5p, hsa-miR-34c-5p, hsa-miR-125b-5p, hsa-miR-526b-5p, hsa-miR-16-5p/hsa-miR-15b-5p/hsa-miR-424-5p/hsa-miR-15a-5p, hsa-miR-9-3p, hsa-miR-363-5p, hsa-miR-1298-3p, hsa-miR-148a-3p, hsa-miR-302a-3p, hsa-miR-9-5p, hsa-miR-28-3p, hsa-miR-508-3p, hsa-miR-137, hsa-miR-5010-5p, hsa-miR-523-5p, hsa-miR-128-3p, hsa-miR-199a-5p/hsa-miR-199b-5p, hsa-miR-181a-2-3p, hsa-miR-27a-3p/hsa-miR-27b-3p, hsa-let-7d-3p, hsa-miR-129-5p, hsa-miR-424-3p, hsa-miR-181a-3p, hsa-miR-10a-5p, hsa-miR-196b-5p, hsa-miR-92a-1-5p, hsa-miR-483-5p, hsa-miR-1537-3p, hsa-miR-106b-5p/hsa-miR-20a-5p/hsa-miR-17-5p/hsa-miR-93-5p, hsa-miR-30a-3p/hsa-miR-30e-3p, hsa-miR-374a-3p, hsa-miR-675-5p, hsa-miR-503-5p, hsa-miR-340-5p, hsa-miR-208a-3p, hsa-miR-200b-3p/hsa-miR-200c-3p, hsa-miR-518f-5p/hsa-miR-523-5p, hsa-miR-625-3p, hsa-miR-194-5p, hsa-let-7g-3p, hsa-miR-514a-5p, hsa-miR-381-3p, hsa-miR-513c-5p/hsa-miR-514b-5p, hsa-miR-520a-5p, hsa-miR-125b-5p/hsa-miR-125a-5p, hsa-miR-141-3p, hsa-miR-874-3p, hsa-miR-202-5p, hsa-miR-140-3p, hsa-miR-361-3p, hsa-miR-513b-5p, hsa-miR-33a-5p, hsa-let-7a-5p/hsa-let-7c-5p/hsa-let-7b-5p/hsa-let-7d-5p/hsa-let-7f-5p/hsa-let-7e-5p/hsa-let-7i-5p/hsa-let-7g-5p, hsa-miR-136-3p, hsa-miR-508-5p, hsa-miR-204-5p/hsa-miR-211-5p, hsa-miR-146a-5p/hsa-miR-146b-5p, hsa-miR-23a-3p, hsa-miR-21-3p, hsa-miR-877-5p, hsa-miR-302a-5p, hsa-miR-139-5p, hsa-miR-99a-5p/hsa-miR-100-5p/hsa-miR-99b-5p, hsa-miR-216a-5p and hsa-miR-3157-3p, and preferably, a modified base sequence in which at least one guanine base is substituted with uracil in the sequence in positions 2 to 7 or 2 to 9 based on the 5' end, to allow the G:A wobble pair at the corresponding site to be a canonical base pair of U:A.

Preferably, the RNA interference-inducing nucleic acid has a sequence of 6 to 8 consecutive bases containing 2 to 7 or 2 to 9 bases from the 5' end, represented by any one or more of SEQ ID NOs: 529 to 863 (see Table 4).

In addition, the present invention provides a composition for inhibiting the expression of a non-canonical target gene of miRNA, which includes the RNA interference-inducing nucleic acid.

In addition, the present invention provides a method of inhibiting the expression of a non-canonical target gene of miRNA, which includes administering a composition including an RNA interference-inducing nucleic acid into a subject.

In addition, the present invention provides the use of an RNA interference-inducing nucleic acid for inhibiting the expression of a non-canonical target gene of miRNA.

In addition, the present invention provides a method of preparing an RNA interference-inducing nucleic acid, which inhibits the expression of a non-canonical target gene of miRNA by modifying a partial sequence of specific miRNA in one or more single strands of the double strands of the nucleic acid inducing RNA interference, the method including the following step:
constituting an RNA interference-inducing nucleic acid to have a modified base sequence in which at least one guanine base is substituted with a uracil base in the sequence of 6 to 8 consecutive bases, starting from the second base from the 5' end of specific miRNA.

Hereinafter, the present invention will be described in detail.

When miRNA binds with target mRNA, even if not exactly complementary to a miRNA seed region, it is recognized as a miRNA target and binds to mRNA, and this is called non-canonical target recognition. The inventors focused on the non-canonical target recognition of miRNA to develop an RNA interference-inducing nucleic acid selectively suppressing the non-canonical target gene of miRNA by modifying a partial sequence of miRNA and elucidated its efficiency, and thus the present invention was completed.

The present invention provides an RNA interference-inducing nucleic acid, which suppresses a non-canonical target gene of miRNA by modifying a partial sequence of specific miRNA in one or more single strands of the double strands of the nucleic acid inducing RNA interference, wherein
the RNA interference-inducing nucleic acid has a sequence of four bases in positions 2 to 5 from the 5' end of the specific miRNA, and bases in positions 6 and 7, which are the same and complementary to a base capable of pairing with the 6^{th} base of specific miRNA, including all complementary bases including a G:A or G:U wobble pair, to allow non-canonical target base pairs bound to a bulge generated in the target gene between positions 5 and 6 of the miRNA to be a consecutive base pair by the disappearance of the bulge, or
the RNA interference-inducing nucleic acid has a modified base sequence in which at least one guanine base is substituted with uracil or adenine in the base sequence between the first to 9^{th} bases from the 5' end of specific miRNA, and particularly, in the sequence from the 2^{nd} to 7^{th} bases based on the 5' end to allow a G:A or G:U wobble pair at the corresponding site to be a canonical base pair of U:A or A:U.

The RNA interference-inducing nucleic acid according to the present invention may be one or more single strands of the double strands of the nucleic acid inducing RNA interference, preferably including miRNA, small hairpin RNA (shRNA) and small interfering RNA (siRNA), DsiRNA, lsiRNA, ss-siRNA, asiRNA, piRNA or endo-siRNA.

The RNA interference-inducing nucleic acid according to the present invention is based on two types of non-canonical target recognition of miRNA.

More specifically, the miRNA recognizes a normal target gene as well as a non-canonical target gene. The non-canonical target gene recognized by the miRNA does not have a complementary relationship exactly corresponding to the miRNA.

Aspects of a non-canonical target gene recognized by the miRNA and an RNA interference-inducing nucleic acid suppressing the non-canonical target gene are as follows.

One type of recognition of miRNA-binding non-canonical target genes has a consecutive complementary relationship with all of the bases in positions 1 to 5 from the 5' end of miRNA. However, in a gene recognized by the base in position 6 from the 5' end of miRNA (paired with the base in position 6 from the 5' end of miRNA), the base recognized by the base in position 6 from the 5' end of miRNA is a base that has a complementary relationship with miRNA after pushing an immediately consecutive base in a bulge form, rather than a base that is immediately contiguous to a base having a complementary relationship with the base in position 5 from the 5' end of the miRNA.

The RNA interference-inducing nucleic acid suppressing the non-canonical target gene recognized by miRNA of one aspect has a four-base sequence in positions 2 to 5 from the 5' end of specific miRNA. In addition, two consecutive bases in the four-base sequence are the same and have a base sequence complementary to a base that can be paired with the 6^{th} base of the specific miRNA, and the base that can be paired with the 6^{th} base of the specific miRNA includes all complementary bases including G:A and G:U wobble pairs.

The RNA interference-inducing nucleic acid suppressing a non-canonical target gene recognized by the specific miRNA commonly includes a sequence of at least four bases in positions 2 to 5 from the 5' end of the specific miRNA, compared with the base sequence of the miRNA.

In addition, the 6^{th} and 7^{th} bases from the 5' end of the RNA interference-inducing nucleic acid may be the same, and these two bases may be complementary to a base that can be paired with the 6^{th} base of specific miRNA, which includes all complementary bases including G:A and G:U wobble pairs. For example, an arrangement of the 6^{th} base of the specific miRNA: a base that is able to be paired with the 6^{th} base: a base complementary to the base that is able to be paired with the 6^{th} base may be (A: U, G: A,C), (G: A,U,C: U,A,G), (U: G,A: C,U) or (C: G: C). For example, when the 6^{th} base of the specific miRNA is A, a sequence of the 6^{th} and 7^{th} bases from the 5' end of the RNA interference-inducing nucleic acid may be AA or CA; when the 6^{th} base of the specific miRNA is G, a sequence of the 6^{th} and 7^{th} bases from the 5' end of the RNA interference-inducing nucleic acid may be UG, AG or GG; when the 6^{th} base of the specific miRNA is U, a sequence of the 6^{th} and 7^{th} bases from the 5' end of the RNA interference-inducing nucleic acid may be CU or UU; or when the 6^{th} base of the specific miRNA is C, a sequence of the 6^{th} and 7^{th} bases from the 5' end of the RNA interference-inducing nucleic acid may be CC.

Preferably, the 6^{th} base from the 5' end of the RNA interference-inducing nucleic acid and the 6^{th} base from the 5' end of the specific miRNA are the same, and the 7^{th} base from the 5' end of the RNA interference-inducing nucleic acid and the 6^{th} base from the 5' end of the specific miRNA are the same.

In addition, the RNA interference-inducing nucleic acid preferably includes the 7^{th} base from the 5' end of the specific miRNA as the 8^{th} base from the 5' end thereof.

In still another aspect, aspects of a non-canonical target gene recognized by miRNA and an RNA interference-inducing nucleic acid suppressing the non-canonical target gene are as follows.

In the case of the non-canonical target gene recognized by miRNA, bases of the miRNA and the target gene recognized by the miRNA have a G:A or G:U wobble pair relationship, in addition to complementary pairing of A:U, G:C, C:G or U:A.

When the miRNA and the target gene recognized by the miRNA are in a G:A wobble relationship, an arrangement of the base of the specific miRNA: the base of the specific gene recognized by the specific miRNA: the base of the RNA interference-inducing nucleic acid suppressing the target gene recognized by the specific miRNA is G:A:U. In addition, when the miRNA and the target gene recognized by the miRNA have a G:U wobble relationship, an arrangement of the base of the specific miRNA: the base of the specific gene recognized by the specific miRNA: the base of the RNA interference-inducing nucleic acid suppressing the target gene recognized by the specific miRNA is G:U:A.

The RNA interference-inducing nucleic acid according to the present invention has a modified base sequence in which one or more guanine (G) bases of the base sequence from the 1^{st} to 9^{th} bases from the 5' end of the specific miRNA are substituted with uracil (U) bases. In addition, the RNA interference-inducing nucleic acid according to the present invention has a modified base sequence in which one or more guanine (G) bases are substituted with adenine (A) bases in the sequence from the 1^{st} to 9^{th} bases, and preferably, the 2^{nd} to 7^{th} bases from the 5' end of the specific miRNA. When one or more guanine (G) bases are included in the base sequence from the 1^{st} to 9^{th} bases from the 5' end of the specific miRNA, all of the included guanine (G) bases may be substituted with uracil (U) or adenine (A), and at least one guanine base is substituted with uracil (U) or adenine (A). When at least one guanine (G) is included in the base sequence from the 1^{st} to 9^{th} bases, and preferably, the 2^{nd} to 7^{th} bases from the 5' end of the specific miRNA, the other bases except the base substituted with uracil or adenine may be the same as the bases of the specific miRNA.

The RNA interference-inducing nucleic acid according to the present invention selectively suppresses a non-canonical target gene of the miRNA, and does not suppress a canonical target gene of the miRNA.

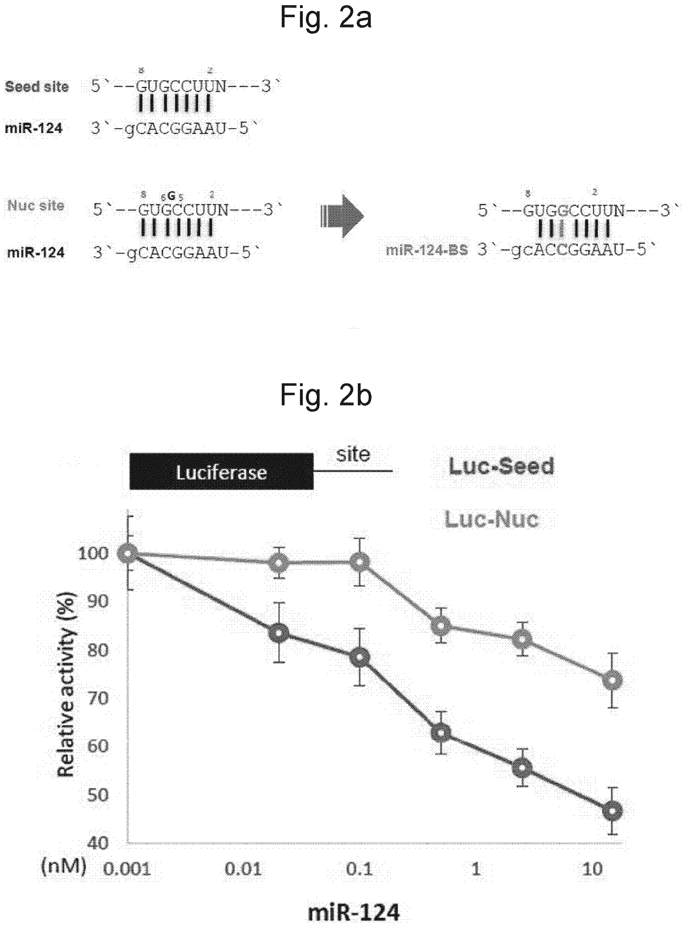

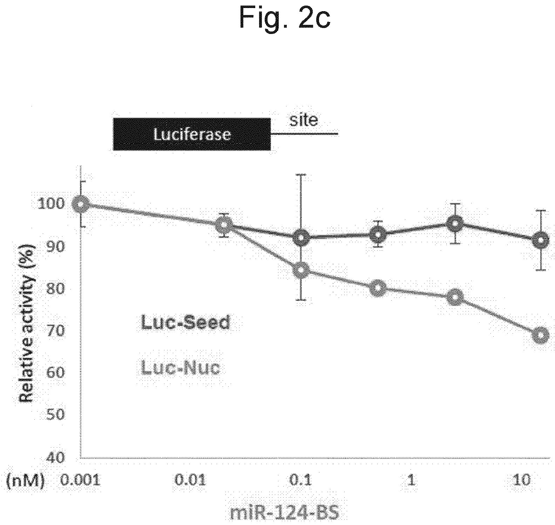

According to an exemplary embodiment of the present invention (see FIG. 2), the function of regulating the miRNA expression of a target gene of the RNA interference-inducing nucleic acid according to the present invention is specific for a non-canonical target gene, and an inhibitory effect is not shown for a canonical target gene.

Therefore, when the RNA interference-inducing nucleic acid according to the present invention is used, only the expression of a non-canonical target gene may be selectively inhibited, thereby selectively inducing only an effect expected by the expression inhibition of the RNA interference-inducing nucleic acid.

In the present invention, the specific miRNA may be one or more selected from the group consisting of miR-124, miR-155, miR-122, miR-1, miR-133, let-7, mR-302a and miR-372.

In the present invention, the base sequence of each of human miR-124, miR-155, miR-122, miR-1, miR-133, let-7, miR-302a and miR-372 is described in MIMAT0000422, MIMAT0000646, MIMAT0000421, MIMAT0000416, MIMAT0000427, MIMAT0000062, MIMAT0000684 or MIMAT0000724, respectively, in the miRNA sequence database, miRBase (http://www.mirbase.org/).

However, the specific miRNA according to the present invention is not limited to human miRNA, and includes miRNAs derived from animals including mammals.

As the RNA interference-inducing nucleic acid suppressing a non-canonical target gene recognized by the specific miRNA according to the present invention, the RNA interference-inducing nucleic acid which has a sequence of four bases in positions 2 to 5 from the 5' end of the specific miRNA, and bases in positions 6 and 7, which are e the same and complementary to a base capable of being paired with the 6^{th} base of the specific miRNA, including all complementary bases including G:A and G:U wobble pairs, has a length of 6 or more bases, but the present invention is not limited thereto. The RNA interference-inducing nucleic acid normally has a length of approximately 21 to 24 bases.

The RNA interference-inducing nucleic acid according to the present invention is an RNA interference-inducing nucleic acid suppressing a non-canonical target gene of miR-124 when the specific miRNA is miR-124. The RNA interference-inducing nucleic acid has a sequence of four bases in positions 2 to 5 from the 5' end of the miR-124, and bases in positions 6 and 7, which are the same and complementary to a base capable of being paired with the 6^{th} base of miR-124, including all complementary bases including G:A and G:U wobble pairs.

For example, the RNA interference-inducing nucleic acid may be an RNA interference-inducing nucleic acid (miR-124BS) in which the sequence of the 2^{nd} to 7^{th} bases from the 5' end is 5'-AA GGC C-3'. More preferably, the RNA interference-inducing nucleic acid is an RNA interference-inducing nucleic acid (miR-124BS) having the base sequence of 5'-UAA GGC CAC GCG GUG AAU GCC-3'.

The RNA interference-inducing nucleic acid according to the present invention is an RNA interference-inducing nucleic acid suppressing a non-canonical target gene of miR-122 when the specific miRNA is miR-122, which has a sequence of four bases in positions 2 to 5 from the 5' end of miR-122, and bases in positions 6 and 7, which are the same and complementary to a base capable of being paired with the 6^{th} base of miR-122, including all complementary bases having a G:A or G:U wobble pair.

For example, the RNA interference-inducing nucleic acid may be an RNA interference-inducing nucleic acid (miR-122BS) in which the sequence of the 2^{nd} to 7^{th} bases from the 5' end is 5'-GG AGU U-3. More preferably, the RNA interference-inducing nucleic acid is an RNA interference-inducing nucleic acid (miR-122BS) having a base sequence of 5'-UGG AGU UGU GAC AAU GGU GUU-3'.

The RNA interference-inducing nucleic acid according to the present invention may be an RNA interference-inducing nucleic acid suppressing a non-canonical target gene of miR-155 when the specific miRNA is miR-155, which has a sequence of four bases in positions 2 to 5 from the 5' end of miR-155, and bases in positions 6 and 7, which are the same and complementary to a base capable of being paired with the 6^{th} base of miR-155, including all complementary bases including G:A and G:U wobble pairs.

For example, the RNA interference-inducing nucleic acid may be an RNA interference-inducing nucleic acid (miR-155BS) in which the base sequence in positions 2 to 7 from the 5' end is 5'-UA AUG G-3'. More preferably, the RNA interference-inducing nucleic acid is an RNA interference-inducing nucleic acid (miR-155BS) having a base sequence of 5'-UUA AUG GC UAA U CGU GAU AGG-3'.

The RNA interference-inducing nucleic acid according to the present invention may be an RNA interference-inducing nucleic acid suppressing a non-canonical target gene of miR-1 when the specific miRNA is miR-1, which has a sequence of four bases in positions 2 to 5 from the 5' end of the miR-1, and bases in positions 6 and 7, which are the same and complementary to a base capable of being paired with the 6^{th} base of the miR-1, including all complementary bases including G:A and G:U wobble pairs.

For example, the RNA interference-inducing nucleic acid may be an RNA interference-inducing nucleic acid (miR-1BS) in which the base sequence in positions 2 to 7 from the 5' end is 5'-GG AAU U-3'. More preferably, the RNA interference-inducing nucleic acid is an RNA interference-inducing nucleic acid (miR-1BS) having a base sequence of 5'-UGG AAU UGU AAA GAA GUA UGU-3'.

As the RNA interference-inducing nucleic acid suppressing a non-canonical target gene recognized by specific miRNA according to the present invention, the RNA interference-inducing nucleic acid having a modified base sequence in which at least one guanine (G) base is substituted with uracil (U) or adenine (A) in the base sequence in positions 1 to 9 from the 5' end of the specific miRNA, preferably, the sequence from the 2^{nd} to 7^{th} bases from the 5' end, has a length of 6 or more bases, but the present invention is not limited thereto. The RNA interference-inducing nucleic acid may normally have approximately 21 to 24 bases in length.

The RNA interference-inducing nucleic acid according to the present invention is an RNA interference-inducing nucleic acid suppressing a non-canonical target gene of miR-124 when the specific miRNA is miR-124, which has a modified base sequence in which at least one guanine (G) base is substituted with uracil (U) or adenine (A) in the base sequence in positions 1 to 9 from the 5' end of the miR-124, preferably, the sequence from the 2^{nd} to 7^{th} bases from the 5' end.

For example, the RNA interference-inducing nucleic acid may be an RNA interference-inducing nucleic acid in which the base sequence in positions 1 to 9 from the 5' end is 5'-UAA UGC AC-3' (miR-124-G4U), 5'-UAA GUC AC-3' (miR-124-G5U) or 5'-UAA UUC AC-3' (miR-124-G4,5U). More preferably, the RNA interference-inducing nucleic acid is an RNA interference-inducing nucleic acid having a base sequence of 5'-UAA UGC ACG CGG UGA AUG CCAA-3' (miR-124-G4U), 5'-UAAGUC ACG CGG UGAAUG CCAA-3' (miR-124-G5U) or 5'-UAA UUC ACG CGG UGAAUG CCAA-3' (miR-124-G4,5U).

The RNA interference-inducing nucleic acid according to the present invention is an RNA interference-inducing nucleic acid suppressing a non-canonical target gene of miR-1 when the specific miRNA is miR-1, which has a modified base sequence in which at least one guanine (G) base is substituted with uracil (U) or adenine (A) in the base sequence in positions 1 to 9 from the 5' end of the miR-1.

For example, the RNA interference-inducing nucleic acid may be an RNA interference-inducing nucleic acid in which the base sequence in positions 1 to 9 from the 5' end is 5'-UUG AAU GUA-3' (miR-1-G2U), 5'-UGU AAU GUA-3' (miR-1-G3U), 5'-UGG AAU UUA-3' (miR-1-G7U), 5'-UUU AAU GUA-3' (miR-1-G2,3U), 5'-UGU AAU UUA-3' (miR-1-G3,7U), 5'-UUG AAU UUA-3' (miR-1-G2,7U) or 5'-UUU AAU UUA-3' (miR-1-G2,3,7U). More preferably, the RNA interference-inducing nucleic acid is an RNA interference-inducing nucleic acid having a base sequence of 5'-UUG AAU GUA AAG AAG UAU GUA U-3' (miR-1-G2U), 5'-UGU AAU GUA AAG AAG UAU GUA U-3' (miR-1-G3U), 5'-UGG AAU UUA AAG AAG UAU GUA U-3' (miR-1-G7U), 5'-UUU AAU GUA AAG AAG UAU GUA U-3' (miR-1-G2,3U), 5'-UGU AAU UUA AAG AAG UAU GUA U-3' (miR-1-G3,7U), 5'-UUG AAU UUA AAG AAG UAU GUA U-3' (miR-1-G2,7U) or 5'-UUU AAU UUA AAG AAG UAU GUA U-3' (miR-1-G2,3,7U).

The RNA interference-inducing nucleic acid according to the present invention is an RNA interference-inducing nucleic acid suppressing a non-canonical target gene of miR-122 when the specific miRNA is miR-122, which has a modified base sequence in which at least one guanine (G) base is substituted with uracil (U) or adenine (A) in the base sequence in positions 1 to 9 from the 5' end of the miR-122, preferably, the sequence from the 2^{nd} to 7^{th} bases from the 5' end.

For example, the RNA interference-inducing nucleic acid may be an RNA interference-inducing nucleic acid in which the base sequence in positions 1 to 9 from the 5' end is 5'-UUG AGU GUG-3' (miR-122-G2U), 5'-UGU AGU GUG-3' (miR-122-G3U), 5'-UGG AUU GUG-3' (miR-122-G5U), 5'-UGG AGU UUG-3' (miR-122-G7U), 5'-UGG AGU GUU-3' (miR-122-G9U), 5'-UUU AGU GUG-3' (miR-122-G2,3U), 5'-UUG AUU GUG-3' (miR-122-G2,5U), 5'-UUG AGU UUG-3' (miR-122-G2,7U), 5'-UUG AGU GUU-3' (miR-122-G2,9U), 5'-UGU AUU GUG (miR-122-G3,5U), 5'-UGU AGU UUG-3' (miR-122-G3,7U), 5'-UGU AGU GUU-3' (miR-122-G3,9U), 5'-UGG AUU UUG-3' (miR-122-G5,7U), 5'-UGG AUU GUU-3' (miR-122-G5,9U) or 5'-UGG AGU UUU-3 (miR-122-G7,9U). More preferably, the RNA interference-inducing nucleic acid is an RNA interference-inducing nucleic acid having a base sequence of 5'-UUG AGU GUG ACA AUG GUG UUU G-3' (miR-122-G2U), 5'-UGU AGU GUG ACA AUG GUG UUU G-3 (miR-122-G3U), 5'-UGG AUU GUG ACA AUG GUG UUU G-3' (miR-122-G5U), 5'-UGG AGU UUG ACA AUG GUG UUU G-3' (miR-122-G7U), 5'-UGG AGU GUU ACA AUG GUG UUU G-3' (miR-122-G9U), 5'-UUU AGU GUG ACAAUG GUG UUU G-3' (miR-122-G2,3U), 5'-UUG AUU GUG ACAAUG GUG UUU G-3' (miR-122-G2,5U), 5'-UUG AGU UUG ACA AUG GUG UUU G-3' (miR-122-G2,7U), 5'-UUG AGU GUU ACA AUG GUG UUU G-3' (miR-122-G2,9U), 5'-UGU AUU GUG ACA AUG GUG UUU G-3 (miR-122-G3,5U), 5'-UGU AGU UUG ACA AUG GUG UUU G-3 (miR-122-G3,7U), 5'-UGU AGU GUU ACA AUG GUG UUU G-3 (miR-122-G3,9U), 5'-UGG AUU UUG ACA AUG GUG UUU G-3' (miR-122-G5,7U), 5'-UGG AUU GUU ACAAUG GUG UUU G-3' (miR-122-G5,9U) or 5'-UGG AGU UUU ACAAUG GUG UUU G-3' (miR-122-G7,9U).

The RNA interference-inducing nucleic acid according to the present invention is an RNA interference-inducing nucleic acid suppressing a non-canonical target gene of miR-133 when the specific miRNA is miR-133, which has a modified base sequence in which at least one guanine (G) base is substituted with uracil (U) or adenine (A) in the base sequence in positions 1 to 9 from the 5' end of the miR-133, preferably, the sequence from the 2^{nd} to 7^{th} bases from the 5' end.

For example, the RNA interference-inducing nucleic acid may be an RNA interference-inducing nucleic acid in which the base sequence in positions 1 to 9 from the 5' end is 5'-UUU UGU CCC-3' (miR-133-G4U), 5'-UUU GUU CCC-3' (miR-133-G5U) or 5'-UUU UUU CCC-3'(miR-133-G4,5U). More preferably, the RNA interference-inducing nucleic acid may be an RNA interference-inducing nucleic acid having a base sequence of 5'-UUU UGU CCC CUU CAA CCA GCU G-3' (miR-133-G4U), 5'-UUU GUU CCC CUU CAA CCA GCU G-3' (miR-133-G5U) or 5'-UUU UUU CCC CUU CAA CCA GCU G-3'(miR-133-G4,5U).

The RNA interference-inducing nucleic acid according to the present invention is an RNA interference-inducing nucleic acid suppressing a non-canonical target gene of let-7 when the specific miRNA is let-7, which has a modified base sequence in which at least one guanine (G) base is substituted with uracil (U) or adenine (A) in the base sequence in positions 1 to 9 from the 5' end of the let-7, preferably, the sequence from the 2^{nd} to 7^{th} bases from the 5' end.

For example, the RNA interference-inducing nucleic acid may be an RNA interference-inducing nucleic acid in which the base sequence in positions 1 to 9 from the 5' end is 5'-UUA GGU AGU-3' (let-7-G2U), 5'-UGA UGU AGU-3' (let-7-G4U), 5'-UGA GUU AGU-3' (let-7-G5U), 5'-UGA GGU AUU-3' (let-7-G8U), 5'-UUA UGU AGU-3' (let-7-G2,4U), 5'-UUA GUU AGU-3' (let-7-G2,5U), 5'-UUA GGU AUU-3' (let-7-G2,8U), 5'-UGA UUU AGU-3' (let-7-G4,5U), 5'-UGA UGU AUU-3' (let-7-G4,8U) or 5'-UGA GUU AUU-3' (let-7-G5,8U). More preferably, the RNA interference-inducing nucleic acid is an RNA interference-inducing nucleic acid having a base sequence of 5'-UUA GGU AGU AGG UUG UAU AGU U-3' (let-7-G2U), 5'-UGA UGU AGU AGG UUG UAU AGU U-3' (let-7-G4U), 5'-UGA GUU AGU AGG UUG UAU AGU U-3' (let-7-G5U), 5'-UGA GGU AUU AGG UUG UAU AGU U-3' (let-7-G8U), 5'-UUA UGU AGU AGG UUG UAU AGU U-3' (let-7-G2,4U), 5'-UUA GUU AGU AGG UUG UAU AGU U-3' (let-7-G2,5U), 5'-UUA GGU AUU AGG UUG UAU AGU U-3' (let-7-G2,8U), 5'-UGA UUU AGU AGG UUG UAU AGU U-3' (let-7-G4,5U), 5'-UGA UGU AUU AGG UUG UAU AGU U-3' (let-7-G4,8U) or 5'-UGA GUU AUU AGG UUG UAU AGU U-3' (let-7-G5,8U).

The RNA interference-inducing nucleic acid according to the present invention is an RNA interference-inducing nucleic acid suppressing a non-canonical target gene of miR-302a when the specific miRNA is miR-302a, which has a modified base sequence in which at least one guanine (G) base is substituted with uracil (U) or adenine (A) in the base sequence in positions 1 to 9 from the 5' end of the miR-302a, preferably, the sequence from the 2^{nd} to 7^{th} bases from the 5' end.

For example, the RNA interference-inducing nucleic acid may be an RNA interference-inducing nucleic acid in which the base sequence in positions 1 to 9 from the 5' end is 5'-UAA UUG CUU-3' (miR-302a-G4U), 5'-UAA GUU CUU-3' (miR-302a-G6U) or 5'-UAA UUU CUU-3' (miR-302a-G4,6U). More preferably, the RNA interference-inducing nucleic acid is an RNA interference-inducing nucleic acid having a base sequence of 5'-UAA UUG CUU CCA UGU UUU GGU GA-3' (miR-302a-G4U), 5'-UAA GUU CUU CCA UGU UUU GGU GA-3' (miR-302a-G6U) or 5'-UAA UUU CUU CCA UGU UUU GGU GA-3' (miR-302a-G4,6U).

The RNA interference-inducing nucleic acid according to the present invention is an RNA interference-inducing nucleic acid suppressing a non-canonical target gene of miR-372 when the specific miRNA is miR-372, which has a modified base sequence in which at least one guanine (G) base is substituted with uracil (U) or adenine (A) in the base sequence in positions 1 to 9 from the 5' end of the miR-372, preferably, the sequence from the 2^{nd} to 7^{th} bases from the 5' end.

For example, the RNA interference-inducing nucleic acid may be an RNA interference-inducing nucleic acid in which the base sequence in positions 1 to 9 from the 5' end is 5'-AAA UUG CUG-3' (miR-372-G4U), 5'-AAA GUU CUG-3' (miR-372-G6U), 5'-AAA GUG CUU-3' (miR-372-G9U), 5'-AAA UUU CUG-3' (miR-372-G4,6U), 5'-AAA UUG CUU-3' (miR-372-G4,9U), or 5'-AAA GUU CUU-3' (miR-372-G6,9U). More preferably, the RNA interference-inducing nucleic acid is an RNA interference-inducing nucleic acid suppressing a non-canonical target gene of miR-372, having a base sequence of 5'-AAA UUG CUG CGA CAU UUG AGC GU -3' (miR-372-G4U), 5'-AAA GUU CUG CGA CAU UUG AGC GU -3' (miR-372-G6U), 5 '-AAA GUG CUU CGA CAU UUG AGC GU -3' (miR-372-G9U), 5'-AAA UUU CUG CGA CAU UUG AGC GU -3' (miR-372-G4,6U), 5'-AAA UUG CUU CGA CAU UUG AGC GU -3' (miR-372-G4,9U) or 5'-AAA GUU CUU CGA CAU UUG AGC GU -3' (miR-372-G6,9U).

In addition, the present invention provides an RNA interference-inducing nucleic acid, which suppresses a non-canonical target gene of miRNA by modifying a partial sequence of specific miRNA in one or more single strands of the double strands of the nucleic acid inducing RNA interference,
the RNA interference-inducing nucleic acid has a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, to allow non-canonical target base pairs bound to a bulge generated in the target gene between positions 5 and 6 of the miRNA to be a consecutive base pair by the disappearance of the bulge, and
the specific miRNA is characterized by adding a methyl group (2'OMe) to the 2' position of the ribosyl ring of the 6^{th} nucleotide from the 5' end.

The RNA interference-inducing nucleic acid according to the present invention has a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, and when a methyl group (2'OMe) is added to the 2' position of the ribosyl ring of the 6^{th} nucleotide from the 5' end of the specific miRNA, there is no limitation on the rest of the base sequence except this, and any base sequence may be included. Here, the RNA interference-inducing nucleic acid may include a sequence of 6 to 24 bases, preferably, 6 to 15 bases, and more preferably, 6 to 8 bases, but there is no particular limitation on the length of the nucleic acid.

The RNA interference-inducing nucleic acid according to the present invention includes a base sequence chemically modified by adding a methyl group (2'OMe) to the 2' position of the ribosyl ring of the 6^{th} nucleotide from the 5' end of the specific miRNA, the RNA interference-inducing nucleic acid in which the 2'OMe modification occurs may selectively inhibit only the expression of a canonical seed target gene thereof, but not inhibit the expression of a non-canonical seed target gene. Therefore, the RNA interference-inducing nucleic acid in which the 2'OMe modification occurs may maintain the purpose of the present invention for specifically suppressing only a non-canonical nucleation bulge site of the specific miRNA, and may have an effect of completely removing a newly generated non-canonical nucleation bulge site.

In the RNA interference-inducing nucleic acid according to the present invention, there is no limitation on the type of miRNA to which the methyl group (2'OMe) can be added as long as the 2'OMe is capable of being added to the 2' position of the ribosyl ring of the 6^{th} nucleotide from the 5' end. However, according to an exemplary embodiment of the present invention, the miRNA to which the 2'OMe may be added may be miR-124 or miR-1.

In addition, the present invention provides an RNA interference-inducing nucleic acid which suppress a non-canonical target gene of miRNA by modifying a partial sequence of specific miRNA in one or more single strands of the double strands of a nucleic acid inducing RNA interference, wherein
the RNA interference-inducing nucleic acid has a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, to allow non-canonical target base pairs bound to a bulge generated in the target gene between positions 5 and 6 of the miRNA to be a consecutive base pair by the disappearance of the bulge.

As long as the RNA interference-inducing nucleic acid according to the present invention has a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, it may have any base sequence without limitation on the rest of the base sequence except the above-described characteristics. Here, the RNA interference-inducing nucleic acid may include a sequence of 6 to 24 bases, preferably, 6 to 15 bases, and more preferably, 6 to 8 bases, but there is not specific limitation on the length of the nucleic acid.

The RNA interference-inducing nucleic acid according to the present invention has a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, to allow non-canonical target base pairs bound to a bulge generated in the target gene between positions 5 and 6 of the miRNA to be a consecutive base pair by the disappearance of the bulge, thereby selectively suppressing only a non-canonical nucleation bulge site and not suppressing a canonical target gene of miRNA.

The RNA interference-inducing nucleic acid according to the present invention may have a base sequence represented by any one or more of SEQ ID NOs: 103 to 528 (see Table 3).

In addition, the present invention provides an RNA interference-inducing nucleic acid which suppresses only the expression of a non-canonical target gene of miRNA by modifying a partial sequence of specific miRNA in one or more single strands of the double strands of the nucleic acid inducing RNA interference according to the present invention, wherein
the RNA interference-inducing nucleic acid has a modified base sequence in which at least one guanine (G) base is substituted with an uracil base in the sequence between the 2^{nd} to 9^{th} bases from the 5' end of the specific miRNA or the sequence in positions 2 to 7 from the 5' end to allow a G:A wobble pair at the corresponding site to be a canonical base pair of U:A.

The RNA interference-inducing nucleic acid according to the present invention may have at least one guanine (G) base substituted with an uracil base in the sequence between the 2^{nd} to 9^{th} bases from the 5' end of the specific miRNA or the sequence between the 2^{nd} to 7^{th} bases from the 5' end, and as long as it has a modified base sequence in which at least one guanine base between the 2^{nd} to 9^{th} bases from the 5' end of the specific miRNA is substituted with an uracil base, the RNA interference-inducing nucleic acid may have any base sequence without limitation on the rest of the sequence except the above-described characteristics. Here, in the RNA interference-inducing nucleic acid according to the present invention, all bases except the base substituted with an uracil (U) base in the sequence between the 2^{nd} to 9^{th} bases from the 5' end of the specific miRNA or between the 2^{nd} to 7^{th} bases from the 5' end may be the same as the bases of the specific miRNA.

As the RNA interference-inducing nucleic acid suppressing a non-canonical target gene recognized by specific miRNA according to the present invention, an RNA interference-inducing nucleic acid having a modified base sequence in which at least one guanine base is substituted with an uracil base in the base sequence between the 2^{nd} to 9^{th} bases from the 5' end of the specific miRNA or the sequence in positions 2 to 7 from the 5' end, may have a sequence of 6 to 24 bases, preferably, 6 to 15 bases, and more preferably, 6 to 8 bases, but there is no specific limitation on the length of the nucleic acid as long as it is an RNA interference-inducing nucleic acid having a modified base sequence in which at least one guanine base is substituted with an uracil base in the sequence of 6 to 8 consecutive bases, starting with the 2^{nd} base from the 5' end of the specific miRNA. Here, as long as the RNA interference-inducing nucleic acid has a modified base sequence in which at least one guanine base is substituted with an uracil base in the sequence of 6 to 8 consecutive bases, starting with the 2^{nd} base from the 5' end of the specific miRNA, there is no limitation on the rest of the base sequence except the above-mentioned characteristics, and any base sequence may be included.

The RNA interference-inducing nucleic acid according to the present invention has a modified base sequence in which at least one guanine (G) base is substituted with an uracil (U) base in the sequence of 6 to 8 consecutive bases, starting with the 2^{nd} base from the 5' end to allow a G:A wobble pair at the corresponding site to be a canonical base pair of U:A, thereby selectively suppressing only a non-canonical target gene of miRNA binding as a G:A wobble pair and not suppressing a canonical target gene of miRNA.

The RNA interference-inducing nucleic acid according to the present invention may include a base sequence represented by any one or more of SEQ ID NOs: 529 to 863 (see Table 4).

When the above-described RNA interference-inducing nucleic acid according to the present invention is used, a non-canonical target gene of miRNA may be specifically suppressed.

Therefore, the present invention provides a composition for inhibiting the expression of a non-canonical target gene of miRNA, which includes an RNA interference-inducing nucleic acid, or a kit for inhibiting the expression of a non-canonical target gene of miRNA, which includes an RNA interference-inducing nucleic acid.

In addition, when the above-described RNA interference-inducing nucleic acid according to the present invention is used, an action caused by the inhibition of the expression of a non-canonical target gene of miRNA by specifically suppressing the non-canonical target gene of miRNA may be selectively regulated.

More specifically, when the RNA interference-inducing nucleic acid according to the present invention is used, the non-canonical target gene of miRNA is specifically suppressed, and thus the action (e.g., cell cycle, differentiation, dedifferentiation, morphology, migration, division, proliferation or death) caused by the expression of a non-canonical target gene of miRNA may be specifically regulated by using the above-described RNA interference-inducing nucleic acid according to the present invention.

Accordingly, the present invention provides a composition or kit for regulating cell cycling, differentiation, dedifferentiation, morphology, migration, division, proliferation or death, which includes an RNA interference-inducing nucleic acid.

The "cell cycle" used herein is a continuous process of growing, dividing and growing cells again, and refers that cells repeatedly go through four phases of an M phase (mitosis), a G1 phase (first division preparation phase), an S phase (DNA synthesis phase) and a G2 phase (second division preparation phase). The G1 phase is for preparing for DNA synthesis from immediately after cell division to the initiation of DNA synthesis, and the G2 phase is for preparing for cell division from the termination of DNA synthesis to the initiation of the cell division.

The "regulation of a cell cycle" used herein includes induction of cell transition between the four phases, or promotion or arrest of the transition, for example, the induction of cells in the G2/M phase to transition to the G1/G2 phase.

The "cell differentiation" used herein is a process by which cells acquire morphological and functional specificities, in which the cells are changed in size or shape, membrane potential, metabolic activity, and response to a signal through differentiation.

The "regulation of cell differentiation" used herein refers to the proliferation or delay of a rate of cell differentiation, induction to initiate differentiation, or inhibition not to initiate differentiation, and for example, includes induction of nerve cell differentiation to acquire morphological specificity (e.g., neurite differentiation). In addition, for example, the induction of myocyte differentiation to acquire morphological specificity (e.g., the fibrosis of myocytes) may be included as an example of the regulation of cell differentiation.

The "dedifferentiation" used herein refers to a phenomenon in which differentiation reverses or completely-differentiated cells acquire pluripotency in the undifferentiated period before differentiation and then are changed like cells in mesophase.

The "regulation of dedifferentiation" used herein includes induction or promotion of cell dedifferentiation, or inhibition or delay of the progression of dedifferentiation, and the result of regulating dedifferentiation may be determined by confirming, for example, the form of cell growth (e.g., formation of a population, etc.), the activity of dedifferentiation factors (Oct3/4, Sox2, c-Myc, Klf4), etc.

The "cell morphology" used herein refers to phenotypic features including a cell shape, structure, size and the like, and varies depending on the type of organism, and the type of tissue or organ even in the same organism.

The "regulation of cell morphology" used herein refers to a change in phenotypic features including the shape, structure and size of cells, and includes the promotion, delay, induction or inhibition of the natural change in cell morphology and the promotion or induction of a non-natural change in cell morphology. For example, the induction of a myocardial hypertrophy of myocardial cells may be included as an example of the regulation of cell morphology.

The "cell migration" used herein refers to the mobility of cells, which is an important process for the growth and physiological activity of an animal, and cells usually respond rapidly to a given stimulus and normally migrate in a non-collective shape, whereas other types of cells may migrate only during a specific growth period or in special circumstances. Cell migration mainly occurs during the development of neural and vascular tubes or the wound healing of epithelial tissue, and the migration of cell populations contributes to the metastasis of various types of tumors.

The "regulation of cell migration" used herein refers to the delay, promotion, induction or inhibition (suppression) of cell migration.

The "cell division and proliferation" used herein is a process by which a parent cell is divided into two daughter cells to increase a cell number.

The "regulation of cell division and proliferation" used herein includes the delay, promotion, inhibition or induction of cell division and proliferation. When cells are induced to the M phase (mitosis) in the cell cycle, it may include cell division and proliferation. For example, an increase in the number of cells distributed in the G0/G1 phase and a decrease in the number of cells distributed in the G2/M phase may be an example of the regulation of cell division and proliferation.

The "apoptosis" used herein refers to a stage leading to death due to genetic properties while cells maintain their functional role, and includes early apoptosis and late apoptosis. According to apoptosis, a new protein is synthesized as the entire cell atrophies, leading to death by a suicide gene of the cells.

The "regulation of apoptosis" used herein includes the induction, delay, promotion or inhibition of apoptosis.

More specifically, the present invention provides a composition for inducing cancer cell death, which includes an RNA interference-inducing nucleic acid (preferably, miR-124BS) suppressing a non-canonical target gene of miR-124;
a composition for inducing neurite differentiation, which includes an RNA interference-inducing nucleic acid (preferably, miR-124BS) suppressing a non-canonical target gene of miR-124;
a composition for inducing cell cycle arrest in liver cancer cells, which includes an RNA interference-inducing nucleic acid (preferably, miR-122BS) suppressing a non-canonical target gene of miR-122;
a composition for promoting differentiation of muscle cells or muscle fibrosis, which includes an RNA interference-inducing nucleic acid (preferably, miR-1BS) suppressing a non-canonical target gene of miR-1;
a composition for inducing muscle cell death, which includes an RNA interference-inducing nucleic acid (preferably, miR-155BS) suppressing a non-canonical target gene of miR-155;
a composition for inducing cell death of neuroblastomas, which includes an RNA interference-inducing nucleic acid (preferably, miR-124-G5U) suppressing a non-canonical target gene of miR-124;
a composition for promoting cell division or proliferation of neuroblastomas, which includes an RNA interference-inducing nucleic acid (preferably, miR-124-G4U) suppressing a non-canonical target gene of miR-124;
a composition for inducing myocardial hypertrophy, which includes an RNA interference-inducing nucleic acid (preferably, miR-1-G2U, miR-1-G3U or miR-1-G7U) suppressing a non-canonical target gene of miR-1;
a composition for inducing myocardial hypertrophy, which includes an RNA interference-inducing nucleic acid (preferably, miR-133-G4U) suppressing a non-canonical target gene of miR-133;
a composition for inducing cell cycle arrest in cancer cells, which includes an RNA interference-inducing nucleic acid (preferably, let-7-G2U, let-7-G2,4U) suppressing a non-canonical target gene of let-7;
a composition for inducing the cell cycle progressing activity of hepatocytes, which includes an RNA interference-inducing nucleic acid (preferably, let-7-G4U) suppressing a non-canonical target gene of let-7;
a composition for promoting dedifferentiation, which includes an RNA interference-inducing nucleic acid (preferably, miR-302a-G4U) suppressing a non-canonical target gene of miR-302a;
a composition for promoting dedifferentiation, which includes an RNA interference-inducing nucleic acid (preferably, miR-372-G4U) suppressing a non-canonical target gene of miR-372; or
a composition for inhibiting cell migration of liver cancer cells, which includes an RNA interference-inducing nucleic acid (preferably, miR-122-G2U or miR-122-G2,3U) suppressing a non-canonical target gene of miR-122.

In addition, when the RNA interference-inducing nucleic acid according to the present invention is used, the action of the non-canonical target gene of miRNA may be selectively regulated by specifically suppressing the non-canonical target gene of miRNA, and therefore may be applied in various fields (e.g., pharmaceuticals, cosmetics, cytology, etc.).

In addition, the RNA interference-inducing nucleic acid according to the present invention may be applied in a method of screening a test material for regulating cell cycling, differentiation, dedifferentiation, morphology, migration, division, proliferation or apoptosis.

More specifically, the present invention provides a method of screening a test material for regulating cell cycling, differentiation, dedifferentiation, morphology, migration, division, proliferation or apoptosis, which includes:
transfecting the RNA interference-inducing nucleic acid according to the present invention into target cells;
treating the target cells with a test material; and
checking an expression level or expression of the non-canonical target gene of miRNA, which is suppressed by the RNA interference-inducing nucleic acid, in the target cells.

In the present invention, the target cells are derived from mammals including a human without limitation, and tissue from which the target cells are extracted or the type of cells is not limited. The target cells may include, for example, neural cells, cardiomyocytes, cancer cells and muscle cells.

In the present invention, the introduction of the RNA interference-inducing nucleic acid into target cells may be conducted properly according to various methods known in the art, and for example, the induction may be performed by methods well known to those of ordinary skill in the art to which the present invention belongs, for example, transfection using calcium phosphate (Graham, FL et al., Virology, 52:456 (1973)), transfection by DEAE dextrin, transfection by microinjection (Capecchi, MR, Cell, 22:479 (1980)), transfection by a cationic lipid (Wong, TK et al., Gene, 10:87 (1980)), electroporation (Neumann E et al., EMBO J, 1:841 (1982)), transduction or transfection, as described in the documents (Basic methods in molecular biology, Davis et al., 1986 and Molecular cloning: A laboratory manual, Davis et al., 1986). The introduction is preferably performed by transfection. Transfection efficiency may vary greatly depending on the type of cells, as well as cell culture conditions or a transfection reagent.

In the present invention, the test material refers to an unknown material used in screening to examine whether cell cycling, differentiation, dedifferentiation, morphology, division, proliferation or apoptosis is affected by regulating the expression of the non-canonical target gene of miRNA, and includes a compound, an extract, a protein or a nucleic acid, but the present invention is not limited thereto.

In the present invention, the expression level or expression of the non-canonical target gene of miRNA may be confirmed by various expression analysis methods, for example, RT-PCR, ELISA (see Sambrook, J et al, Molecular Cloning A Laboratory Manual, 3rd ed Cold Spring Harbor Press (2001)), western blotting or FACS analysis, but the present invention is not limited thereto.

The expression level or expression of the non-canonical target gene of miRNA in the present invention is confirmed, and as a result of comparison with the case of only treatment with the RNA interference-inducing nucleic acid without a test material, when there is a difference, it can be determined that the test material affects the expression of the non-canonical target gene of miRNA. Further, it can be determined that the test material has a function of regulating cell cycling, differentiation, dedifferentiation, morphology, migration, division, proliferation or apoptosis.

In addition, the present invention provides a method of preparing the above-described RNA interference-inducing nucleic acid.

More specifically, the present invention provides a method of preparing an RNA interference-inducing nucleic acid which suppresses a non-canonical target gene of miRNA by modifying a partial sequence of specific miRNA in one or more single strands of the double strands of a nucleic acid inducing RNA interference, which includes the following steps:
constructing an RNA interference-inducing nucleic acid to have a sequence of four bases in positions 2 to 5 from the 5' end of the specific miRNA, and bases in positions 6 and 7, which are the same and complementary with a base capable of being paired with the 6^{th} base of specific miRNA, including all complementary bases including G:A and G:U wobble pairs, or
constructing an RNA interference-inducing nucleic acid to have a modified base sequence in which at least one guanine base is substituted with uracil or adenine in the base sequence between the 1^{st} to 9^{th} bases from the 5' end of specific miRNA.

In addition, the present invention provides a method of preparing an RNA interference-inducing nucleic acid, which inhibits the expression of a non-canonical target gene of miRNA by modifying a partial sequence of specific miRNA in one or more single strands of the double strands of a nucleic acid inducing RNA interference, the method including the following steps:
constructing an RNA interference-inducing nucleic acid having a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, to allow non-canonical target base pairs bound to a bulge generated in the target gene between positions 5 and 6 of the miRNA to be a consecutive base pair by the disappearance of the bulge; and
adding a methyl group (2'OMe) to the 2' position of the ribosyl ring of the 6^{th} nucleotide from the 5' end of the specific miRNA.

In addition, the present invention provides a method of preparing an RNA interference-inducing nucleic acid, which inhibits the expression of a non-canonical target gene of miRNA by modifying a partial sequence of specific miRNA in one or more single strands of the double strands of a nucleic acid inducing RNA interference, the method including the following steps:
constructing an RNA interference-inducing nucleic acid having a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, to allow non-canonical target base pairs bound to a bulge generated in the target gene between positions 5 and 6 of the miRNA to be a consecutive base pair by the disappearance of the bulge.

In addition, the present invention provides a method of preparing an RNA interference-inducing nucleic acid, which inhibits the expression of a non-canonical target gene of miRNA by modifying a partial sequence of specific miRNA in one or more single strands of the double strands of a nucleic acid inducing RNA interference, the method including the following steps:
constituting an RNA interference-inducing nucleic acid to have a modified base sequence in which at least one guanine base is substituted with an uracil base in the sequence of 6 to 8 consecutive bases, starting from the second base from the 5' end of specific miRNA.

The RNA interference-inducing nucleic acid has already been described, and thus to avoid duplication, the description will be omitted.

The construction of the RNA interference-inducing nucleic acid is performed according to various methods known in the art, and the methods are not limited to a specific method.

In addition, the present invention provides an interface-induced nucleic acid modified by adding a methyl group (2'OME) to a 2' site of the ribosyl group of the 6^{th} nucleotide from the 5' end of specific miRNA.

The modified interface-induced nucleic acid maintains the purpose of the present invention for specifically suppressing only a non-canonical nucleation bulge site of the corresponding miRNA, and has an effect of completely removing non-canonical nucleation bulge binding which may newly occur.

### [Advantageous Effects]

When an interference-inducing nucleic acid according to the present invention is used, a biological function exhibited by suppressing a non-canonical target gene of conventional miRNA can be effectively improved, or a part of the functions of the conventional miRNA, that is, only a biological function exhibited by suppressing a non-canonical target gene, can be selectively exhibited. In addition, a cell cycle, differentiation, dedifferentiation, morphology, migration, proliferation or apoptosis can be regulated by the interference-inducing nucleic acid, and thus it is expected to be used in various fields such as pharmaceuticals, cosmetics, etc.

### [Description of Drawings]

FIG. 1 illustrates the action mechanism of an interference-inducing nucleic acid suppressing a non-canonical target of miRNA.
FIGS. 2A to 2C show the non-canonical target-specific expression inhibitory effect of miR-124-BS by measuring the suppression of target genes of a canonical target (seed) of miR-124 and a non-canonical nucleation bulge target using a luciferase reporter enzyme.
FIGS. 3A and 3B show the result of fluorescence-activated cell sorting (FACS) performed on miR-124-BS to confirm the apoptosis induced by the canonical target (seed) and non-canonical nucleation bulge target of miR-124 in cervical cancer cells (HeLa).
FIGS. 4A and 4B show the result of observing cell morphology and a marker expression pattern through expression of miR-124-BS in human neural tumor blastoma (Sh-Sy-5y), confirming neurite differentiation induced by a non-canonical nucleation bulge target of miR-124.
FIGS. 5A and 5B show the result of observing cell morphological and molecular biological characteristics, confirming neurite branch differentiation induced by miR-124-BS(4753), which is a natural form of miR-124-BS regulating a nucleation bulge of miR-124 with a canonical target (seed) and miR-124-(3714) having the same seed sequence as miR-124.
FIGS. 6A to 6C show the result of observing cell morphological and molecular biological characteristics, confirming that neurite branch differentiation induced by a non-canonical nucleation bulge target of miR-124 in a mouse neuroblastoma (N2a) is a mechanism caused by the regulation of the expression of an MAPRE1 gene.
FIGS. 7A to 7C show the RNA-Seq analysis result of confirming the inhibition of the expression of a non-canonical nucleation bulge target gene induced by miR-124-BS in mouse neuroblastoma (N2a) at the transcriptome level.
FIGS. 8A to 8C show the result of observing cell cycle arrest induced by a non-canonical nucleation bulge target of miR-122 in a human liver cancer cell line (HepG2) using miR-122-BS and flow cytometry.
FIGS. 9A to 9C show the cell morphological and molecular biological features and flow cytometry of miR-1-BS and miR-155-BS, confirming the increase in thickness of myocytes induced by a non-canonical nucleation bulge target of miR-1 and the inhibition of myocyte differentiation and induction of apoptosis, which are induced by a non-canonical nucleation bulge target of miR-155, in a muscle cell line.
FIGS. 10A to 10D show the bioinformatics analysis results of Ago HITS-CLIP, which are the RNA data of Argonaute protein-bound miRNA and a target in human brain tissue, confirming that there are a canonical seed target as well as a non-canonical G:A wobble seed target, which naturally occur.
FIGS. 11A to 11D show the cell morphology and flow cytometry result for observing apoptosis of miR-124-G4U and miR-124-G5U, confirming that the non-canonical 4G:A wobble seed target and the non-canonical 5G:A wobble seed target of miR-124 in mouse neuroblastomas (N2a) show totally different functions from miR-124 in brain cell differentiation.
FIGS. 12A and 12B show the flow cytometry (fluorescence-activated cell sorting: FACS) result for analyzing cell proliferation and cell cycling with miR-124-G4U and miR-124-G5U to confirm the biological functions of suppressing the non-canonical 4G:A wobble seed target and the non-canonical 5G:A wobble seed target of miR-124 in human neuroblastomas (SH-sy-5y).
FIGS. 13A to 13D show the flow cytometry (fluorescence-activated cell sorting; FACS) result for a cardiomyocyte cell line (h9c2) with a fluorescent protein reporter, confirming that a non-canonical 7G:A wobble seed target of miR-1 has a gene-suppressing function.
FIGS. 14A to 14C show the result of observing cell morphological and molecular biological features of a myocardial hypertrophy-inducing effect after miR-1-G2U, miR-1-G3U and miR-1-G7U are expressed in rat neonatal cardiomyocytes, confirming the functions of the non-canonical 2G:A, 3G:A and 7G:A wobble seed targets of miR-1.
FIGS. 15A and 15B show the result of observing cell morphological features for a myocardial hypertrophy-inducing effect after miR-133-G4U is expressed in myocardial cells (h9c2) to confirm the function of a non-canonical 4G:A wobble seed target of miR-133.
FIGS. 16A to 16B show the results of measuring the enzyme activity of a luciferase reporter in a human liver cancer cell line (HepG2), confirming that gene expression is inhibited through non-canonical 2G:A and 3G:A wobble seed targets of miR-122.
FIGS. 17A to 17D show the result of observing cell morphological features of the expression of miR-122-G2U and miR-122-G2,3,U to confirm a function in which the inhibition of the expression of a non-canonical 2G:A wobble seed target and a non-canonical 2,3G:A wobble seed target suppresses the migration in a human liver cancer cell line (HepG2) by miR-122.
FIGS. 18A and 18B show the results of flow cytometry for the expression of miR-122-G2,3U to confirm that the inhibition of the expression of a non-canonical 2,3G:A wobble seed target by miR-122 induces cell cycle arrest in a human liver cancer cell line (HepG2).
FIGS. 19A and 19B show the result of flow cytometry for the expression of miR-122-G2,3U and miR-122-G2,7U to confirm that the inhibition of the expression of a non-canonical 2,3G:A wobble seed target and a non-canonical 2,7G:A wobble seed target by miR-122 induces cell cycle arrest of a human liver cancer cell line (HepG2).
FIGS. 20A and 20B show the result of flow cytometry for the expression of let-7a-G2U, let-7a-G2,4U and let-7-G4U to confirm that the inhibition of the expression of non-canonical 2G:A and 2,4G:A wobble seed targets by let-7 induces cell cycle arrest of a human liver cancer cell line (HepG2) and the inhibition of the expression of a non-canonical 4G:A wobble seed target promotes a cell cycle.
FIGS. 21A to 21C show that miR-372-G4U or miR-302a-G4U inducing the inhibition of the expression of a non-canonical 4G:A wobble seed target by miR-372 or miR-302a promotes dedifferentiation with miR-372 or miR-302a, confirmed with an Oct4 gene expression reporter.
FIGS. 22A and 22B show the results of confirming the phenomenon in which a 2'OMe modification in position 6 from the 5' end does not suppress a non-canonical nucleation bulge target of a corresponding RNA interference derivative.
FIGS. 23A and 23B show the results of confirming that a 2'OMe modification in position 6 from the 5' end does not suppress total non-canonical nucleation bulge target mRNAs in a transcriptome.
FIGS. 24A and 24B show the results of analyzing miRNAs binding to non-canonical nucleation bulge sites through Ago HITS CLIP experiments.
FIGS. 25A to 25F show the results of identifying a G-to-U modification recognizing non-canonical GA wobble seed target as a canonical target in tumor miRNA by analyzing sequence variations in a miRNA sequencing database (TCGA) of cancer patients.

### [Modes of the Invention]

Hereinafter, the present invention will be described in further detail with reference to the following examples. The following examples are merely provided to exemplify the present invention, and the present invention are not limited to the following examples.

### [Example 1] Action mechanism of interference-inducing nucleic acid suppressing non-canonical target of miRNA

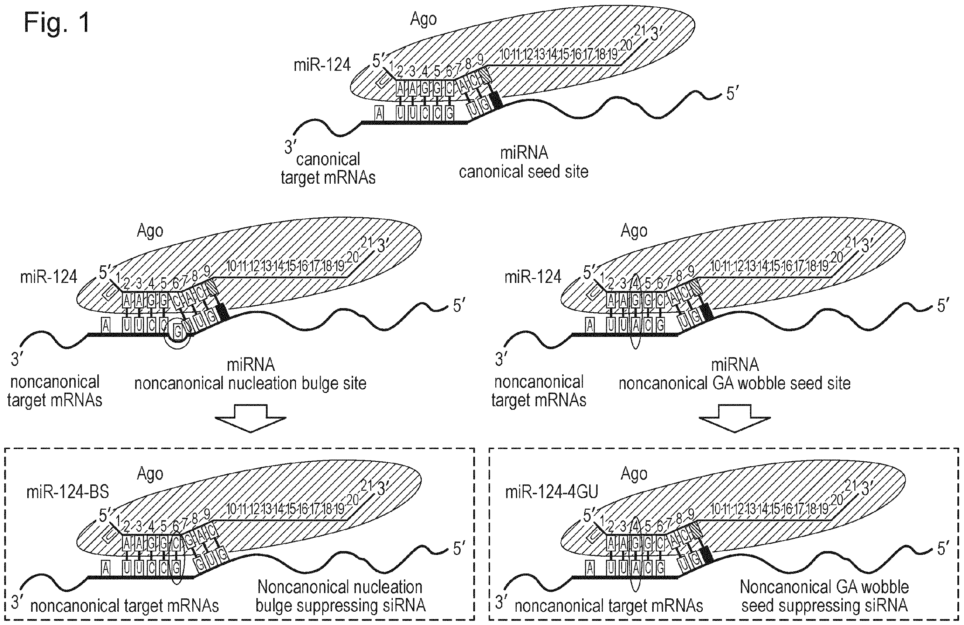

Through Ago HITS CLIP assay, the inventors confirmed from an Argonaute protein-binding RNA complex sequence that, when miRNA is base-paired with a target site in Argonaute protein, the miRNA binds with target messenger RNA (mRNA) without making a complete complementary pairing with the miRNA seed, in addition to a canonical seed target having a complete complementary pairing with the miRNA seed. Moreover, as a result of sequencing of RNA having the above-described characteristics, it was confirmed that there are a target base-paired by the induction of a nucleation bulge to the target by the 6^{th} base of the miRNA (non-canonical nucleation bulge target site of miRNA) and a target site allowing G:A wobble pairing in a miRNA seed region (non-canonical G:A wobble seed target site of miRNA), which naturally occur (FIG. 1).

As illustrated in FIG. 1, miR-124 has at least 6 consecutive complementary base pairings to the base sequence of a target site, which is 5'-GUGCCUUA-3' with a seed base sequence (5'-UAAGGCAC-3') by means of the Argonaute protein, and the target site having such base pairings has been reported as a canonical seed target site (canonical seed site) of miRNA (Nature, 2009, 460 (7254): 479-86).

In addition, in the Argonaute-bound RNA complex, a target in which the miR-124 seed base sequence (5'-UAAGGCAC-3') makes complementary base pairing with a non-canonical nucleation bulge target site (5'-GUGGCCUU-3') of miRNA to allow G of target mRNA in positions 5 and 6 from the 5' end of miR-124 to be arranged in a bulge was identified, and based on this, the seed base sequence of miR-124 enabling complementary base pairing with the non-canonical nucleation bulge target site of miRNA has a base sequence (5-UAAGGCCA-3) in which the 6^{th} base is repeated once more between positions 6 and 7 of the miR-124 seed. The consecutive and perfectly complementary sequence with respect to the above-described sequence was used as miR-124BS (BS: bulge site) siRNA suppressing a non-canonical nucleation bulge target of miR-124.

When miRNA-bound target mRNA sequence in the Argonaute-bound RNA complex was analyzed, other than a conventional complementary base sequence, it was confirmed that G:A wobble base pairing occurs in the binding of the target mRNA with the seed sequence of miRNA. Therefore, in the case of miR-124, the 4^{th} G base from the 5' end of the seed base sequence (5'-UAAGGCAC-3') makes a G:A wobble pair, thereby recognizing a non-canonical G:A wobble seed target site of miRNA (5'-UGGCAUU-3'). Based on this, siRNA was designed with a consecutively and perfectly complementary sequence with the non-canonical G:A wobble seed target site of miR-124 to invent miR-124-GU, and siRNA was used as siRNA inhibiting the expression of the non-canonical G:A wobble seed target of miR-124.

### [Example 2] Confirmation of inhibitory ability of miRNA-BS in which non-canonical nucleation bulge target site and consecutively and perfectly complementary base sequence are included in seed region

In a canonical seed target site of natural miR-124, the sequence complementary to the 2^{nd} to 7^{th} bases from the 5' end of miR-124 is 5'-GUGCCUU-3', and the canonical seed target site makes perfectly complementary base pairing with at least 6 consecutive bases with the seed of miR-124 (5'-UAAGGCAC-3') (FIG. 2A).

A non-canonical nucleation bulge target site (Nuc site; nucleation bulge site) of miR-124 found by Ago HITS CLIP assay is 5'-GUGGCCUU-3', the 6^{th} base (C) of miR-124 is base-paired with the target by forming a nucleation bulge, and therefore, G of target RNA paired between positions 5 and 6 from the 5' end of miR-124 is formed in a bulge. Based on this, siRNA was designed using the base sequence having consecutively and perfectly complementary binding with the non-canonical nucleation bulge target site (nucleation bulge site) (5'-UAAGGCCA-3') as a miRNA seed and named miR-124-BS siRNA (FIG. 2A). It is considered that miR-124-BS recognizes a non-canonical target of miR-124, which is a nucleation bulge target site (Nuc site), as a canonical seed target, and specifically and more strongly inhibits gene expression of the non-canonical target, which was confirmed through luciferase reporter assay (FIGS. 2B and C).

First, to confirm how much miR-124 can suppress a non-canonical target, which is a nucleation bulge target site, compared with a conventional canonical target, which is a seed target, a corresponding site for miR-124 was inserted into a luciferase reporter, and various concentrations (0, 0.01, 0.1, 0.5, 2.5 and 15 nM) of miR-124 were also transfected into cells, followed by measuring inhibitory concentration 50 (IC₅₀) with luciferase activity. As a result, it can be confirmed that the IC₅₀ of the canonical seed site was approximately 0.5 nM, which was similar to the IC₅₀ of the non-canonical nucleation bulge site of approximately 0.2 nM (FIG. 2B). However, the highest inhibitory ratio for the canonical seed site was approximately 50%, and the highest inhibitory ratio for the non-canonical nucleation bulge site was approximately 80%, indicating that the inhibition through the non-canonical nucleation bulge site is slightly weak. Particularly, through the measurement of luciferase activity, it was confirmed that gene suppression is initiated at a concentration of approximately 0.01 nM or more for the canonical seed target and 0.1 nM or more for the non-canonical nucleation bulge target (FIG. 2B). Accordingly, it was able to be seen that miR-124 regulates the expression of the canonical target and the non-canonical nucleation bulge target, and has high canonical target expression regulatory efficiency.

The luciferase reporter assay performed herein was conducted by cloning the sequence of the corresponding miR-124 target site into a *Renilla* luciferase gene (3' untranslated region (3'UTR)) site of a psi-check2 vector (Promega) twice in a row. The sequence of the non-canonical bulge site uses a naturally-occurring non-canonical nucleation bulge target site in the 3' UTR site of MINK1. The miR-124, as a human-derived sequence, was prepared as a duplex of a guide strand and a passenger strand, which are chemically synthesized by Bioneer according to a miRBase database, separated by HPLC, according to by the method provided by the manufacturer. The miR-124 and the luciferase reporter vector were co-transfected into approximately 10,000 cervical cancer cells (HeLa: ATCC CCL-2) using a Lipofectamine 2000 reagent (Invitrogen) according to the manufacturer's protocol. The HeLa cells were incubated in Dulbecco's modified Eagle's medium (Invitrogen) supplemented with 10% fetal bovine serum (FBS), 100 U/ml penicillin and 100 µg/ml streptomycin, and during transfection, the cells were incubated in an antibiotic-free complete medium. Twenty-four hours after transfection, luciferase activity was measured using a dual-luciferase reporter assay system (Promega) according to the manufacturer's protocol, and here, the measurement of *Renilla* luciferase activity was repeated at least three times using a Glomax Luminometer (Promega), and calculated by normalization by firefly luciferase activity.

To confirm the functions of miR-124-BS invented to specifically suppress only the non-canonical nucleation bulge target of the miR-124, an experiment was conducted with a luciferase reporter in the same manner as described above. As a result, the miR-124-BS did not inhibit luciferase reporter enzyme activity with respect to the canonical seed target of miR-124 at any concentration (0 to 10 nM), but an effect of inhibiting luciferase reporter enzyme activity with respect to the non-canonical nucleation bulge target reporter of miR-124 was possibly confirmed at a concentration of 0.1 nM or more (FIG. 2C). Here, the gene inhibitory efficiency was measured by IC50, showing that the inhibition of the non-canonical nucleation bulge target is approximately 0.5 nM. In terms of this, the function of regulating the expression of the miR-124 non-canonical nucleation bulge target of miR-124-BS may be interpreted as being specific for the non-canonical nucleation bulge target site (FIG. 2C). The luciferase reporter assay used herein was performed by synthesizing miR-124-BS by Bioneer in the same manner as described above, co-transfecting HeLa cells (ATCC CCL-2) with a corresponding luciferase reporter vector, and measuring luciferase enzyme activity for 24 hours.

In this example, the inventors invented miRNA-BS having a consecutive and perfectly complementary seed site sequence with respect to a corresponding site sequence in order to suppress only a non-canonical organic target of miRNA, and when a luciferase reporter assay was performed using miR-124 to verify the effect of miRNA-BS, it was able to be demonstrated that the miR-124-BS does not inhibit the expression of a canonical seed target, and is able to effectively inhibit the expression of a non-canonical bulge target.

### [Example 3] Observation of apoptosis of cervical cancer cells induced by inhibition of expression of non-canonical nucleation bulge target of miR-124

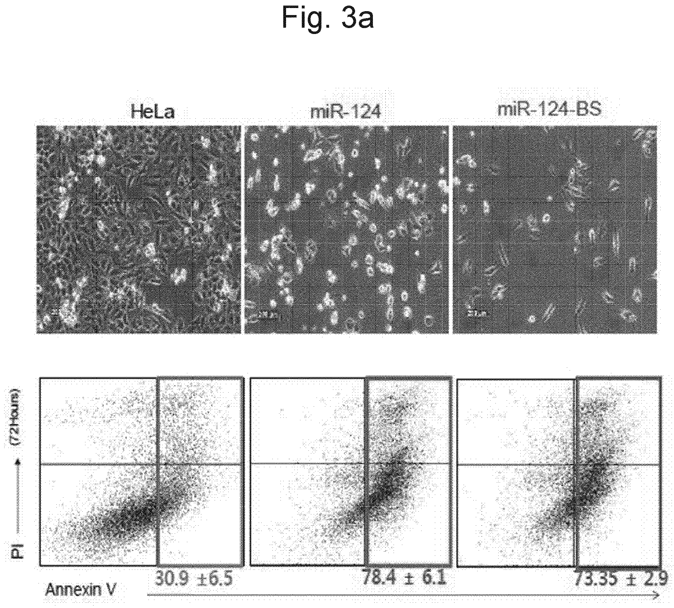

After confirming that the miR-124-BS suppresses only a non-canonical bulge target among several targets suppressed by miR-124 in the above-described example, through this, it was intended to confirm whether only a specific function of the miR-124 can be exhibited. Since specifically expressed only in nerve cells, the miR-124 is known to mainly play a role related to nerve cells. However, miR-124 expression decreases in gliomas generated by tumors, with regard to this, when the miR-124 expression is artificially induced in tumor cells, it was observed that the apoptosis of tumor cells may occur. Therefore, to confirm whether various and different functions of miR-124 vary according to the type of target, the miR-124-BS was transfected into cervical cancer cells (HeLa), and the apoptosis of the cells was observed through flow cytometry (FIG. 3A).

The experiment was performed by transfecting 75 nM of the miR-124 or miR-124-BS duplex into cervical cancer cells (HeLa: ATCC CCL-2) using an RNAiMAX reagent (Invitrogen) according to the manufacturer's protocol, treating the cells with trypsin 72 hours after transfection, detaching the cells from the culture dish, treating the cells using an Annexin V: FITC Apoptosis Detection Kit II (BD Pharmingen) according to the method provided by the manufacturer to detect annexin V for apoptosis and propidium iodide (PI) for necrosis by BD Aria I (BD Biosciences). Here, as a control, a sequence was synthesized to be the same as the sequence of cel-miR-67, which is miRNA of *C*. *elegans.*

As a result of comparing these experimental results for the control, miR-124 expression and miR-124-BS expression, when miR-124 was expressed, it was observed that, in cervical cancer (HeLa) cells, compared with the control, a ratio of progressing cell death by apoptosis (the number of cells stained with annexin V) increases from approximately 30% to approximately 78%, and it was confirmed that the miR-124-BS expression also increased to approximately 73%, which is 2-fold higher than the control (FIG. 3A). According to the result of Example 2 in that the miR-124-BS does not suppress a canonical seed site of the miR-124 and suppresses only a non-canonical bulge site, it can be seen that the apoptosis of cervical cancer cells occurs by the inhibition of the expression of the non-canonical nucleation bulge target.

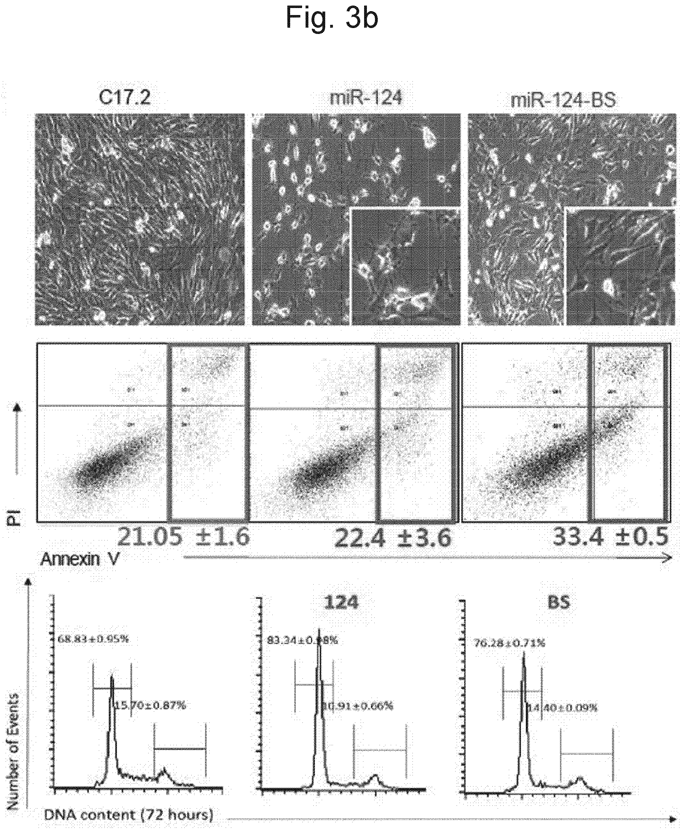

It has been reported that the miR-124 is a brain tissue-specific miRNA, and does not have a brain tissue-specific function in other cells not showing tissue cell-specific transcriptome expression (Farh K et al, 2005, Science, 310 (5755) 1817-21). Therefore, to observe a function of the miR-124 that is caused by suppressing a non-canonical bulge target in nerve cells, the miR-124-BS was transfected into a cerebellar neural stem cell line, C17.2, and then cell differentiation was observed in cell morphology (FIG. 3B, upper image). As a result, when the miR-124 was transfected, the neurite differentiation of C17.2 cells was extended and promoted, but when the miR-124-BS was transfected, it was observed that there was differentiation, and branches were short but different. Therefore, it can be seen that general neurite differentiation induced by the miR-124 occurs by suppressing a canonical seed site target. Here, the miRNA transfection was conducted with an RNAiMax reagent by synthesizing 50 nM miRNA in the same manner as in Example 2.

In addition, since the miR-124-BS previously induced apoptosis in HeLa cells, whether such a function is also exhibited in nerve cells was observed through flow cytometry for C17.2 cells in the same manner as in Example 2. As a result, in the case of the miR-124 expression, an apoptosis ratio was 22.4%, almost similar to the control which is 21%, and in the case of the miR-124-BS expression, an apoptosis ratio was 33.4%, which is slightly increased (FIG. 3B, middle image). This shows the same tendency as in cervical cancer cells in that apoptosis occurs through the suppression of a non-canonical bulge target of the miR-124, but the increase rate is much smaller than that in the cervical cancer cells. The miR-124 mainly plays a differentiation-involved role in neural stem cells, and here, the regulation of a cell cycle may play a critical role. Therefore, after miR-124 and miR-124-BS were transfected into C17.4 cells, a cell cycle was observed by propidium iodide (PI) staining through flow cytometry (FIG. 3B, lower image). Here, it was seen that the miR-124 induces cell cycle arrest in the G0/G1 phase to 83%, which is slightly higher than the control, which is 69%. In comparison, it was possible to observe that miR-124-BS expression does not significantly induce cell cycle arrest. This experiment was performed by transfecting corresponding miRNA in the same manner as in the above-described example, incubating the transfected cells for 48 hours, detaching the cells, fixing them with ethanol, reacting the cells with 1 mg/ml of PI (Sigma-Aldrich) and 0.2 mg/ml of RnaseA at 37 °C for 30 minutes, and analyzing them through flow cytometry using BD FACSCalibur (BD Biosciences).

Summarizing the results in the above examples, only by inhibition of the expression of a non-canonical nucleation bulge target of miR-124 by the miR-124-BS, the death of cervical cancer cells (Hela) was induced like the miR-124, and therefore, it can be seen that the inhibitory function of the non-canonical bulge target of the miR-124 is the death of cancer cells. In addition, in neural stem cells, as neurite differentiation occurs due to miR-124 expression, little apoptosis and cell cycle arrest occur, and in the miR-124-BS suppressing only a non-canonical bulge target of miR-124, such a function is exhibited in a slightly different form. Accordingly, it can be seen that the nerve cell differentiation function of miR-124 is usually caused by the suppression of a canonical seed target gene which is conventionally known, or in a different form, it was able to be considered that the nerve cell differentiation function of miR-124 may occur by a non-canonical bulge target.

### [Example 4] Confirmation of neuronal differentiation in different form with many branches and short neurites (branched neurite outgrowth) by inhibiting expression of miR-124 non-canonical nucleation bulge target by miR-124-BS

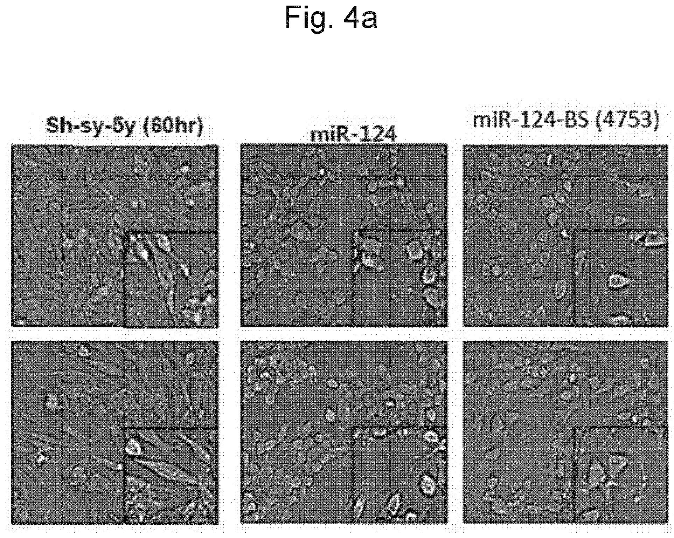

After confirming the fact that the miR-124-BS induces a different type of neurite differentiation from miR-124 in the previous example, to further examine this, a human neuroblastomas (sh-sy-5y) cell line (CRL-2266) was used for the experiment. Here, in addition to the miR-124-BS, miR-124-BS(4753) designed to have the same miRNA sequence having the same miR-124 seed sequence which is able to recognize and suppress a non-canonical nucleation bulge site of the miR-124, but the other part of which is different was synthesized by the method described in the previous example and used for the experiment. The sequence of the miR-124-BS(4753) used herein is 5'-CAAGGCCAAAGGAAGAGAACAG-3', and a control was used by being synthesized to be the same as the miRNA of C. elegans, cel-miR-67 (NT; non-targeting). The corresponding miRNA was transfected by the same method as described in the previous example, the cells were cultured for 60 hours, and then the morphological change of the cells was observed using an optical microscope (FIG. 4A).

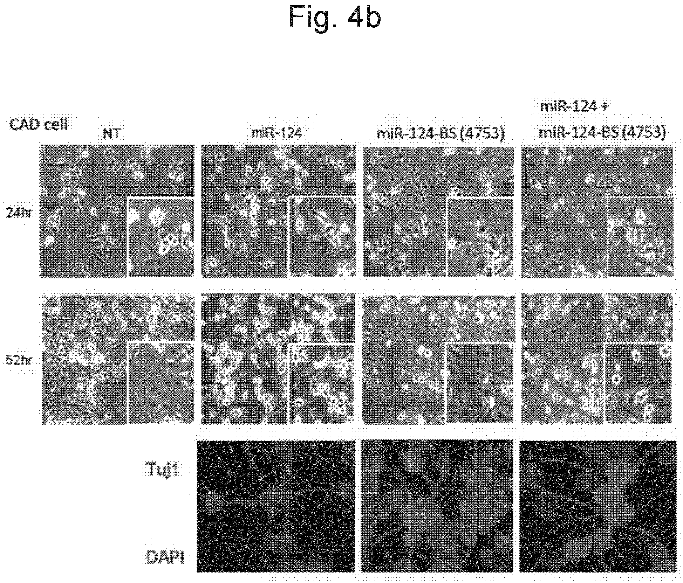

As a result, the miR-124 expression was possible to observe by the morphological change which makes a neurite of the human neuroblastoma (Sh-sy-5y) cell differentiate longer, whereas in the miR-124-BS(4753) having the same eed sequence that can suppress only a non-canonical bulge target of miR-124 as the miR-124-BS, a highly branched neurite outgrowth was observed in one cell (FIG. 4A). Based on this, the same experiment was additionally performed on catecholamine-containing nerve cells (CAD, CRL-11179). Here, corresponding miRNA was transfected, the cells were incubated for 52 hours, and then the cells were observed (FIG. 4B). As a result, like the result shown from the human neuroblastoma (Sh-sy-5y, CRL-2266) cells, the miR-124 is small in number but forms a longer neurite, whereas it was possible to observe that the miR-124-BS(4753) shows a differentiation phenomenon in which many short neurite branches overgrow (FIG. 4B). Additionally, miR-124 and miR-124-BS(4753) are transferred into cells at the same ratio, cells with long neurites and cells with many neurite branches were observed simultaneously. This shows the possibility that, when both of the invented miR-124-BS and the conventional miR-124 were expressed, it can artificially promote various types of neuronal differentiation similar to brain cells shown in a more complicated neural network.

Additionally, the neuronal differentiation of the CAD cell line promoted by the miRNA was confirmed by immunostaining for a nerve cell-specific marker, Tuj1 (FIG. 4B, lower image). The experiment performed herein was performed by transfecting corresponding miRNA, fixing the CAD cells with 4% paraformaldehyde 52 hours after transfection, immunostaining the cells with a primary antibody, Tuj 1 (MRB-435P, Covance Antibody Products) at 1:1000 and an Alexa 594 fluorescent material-conjugated secondary antibody (Abcam: ab150076) at 1:1000, and staining the nucleus with DAPI.

From the result of the above-described example, it can be seen that miR-124 has a function of forming many short neurite branches, which is different from the function of the conventional miR-124 by suppressing only a non-canonical nucleation bulge target. Particularly, there are a different sequence from the miR-124-BS and the same seed sequence, which was observed using the miR-124-BS(4753) capable of suppressing only the non-canonical nucleation bulge target, elucidating the fact that an RNA interference derivative including the seed sequence capable of recognizing the non-canonical nucleation bulge target exhibits the same effect as the miR-124-BS. In addition, it can be shown that complex neurite branches generated by the miR-124-BS can be applied as a method of promoting various types of neuronal differentiation similar to human brain cells showing a complicated neural network.

### [Example 5] Confirmation of morphological and molecular biological features of branched neurite outgrowth induced thereby after miR-124-BS is expressed using siRNA and shRNA vector

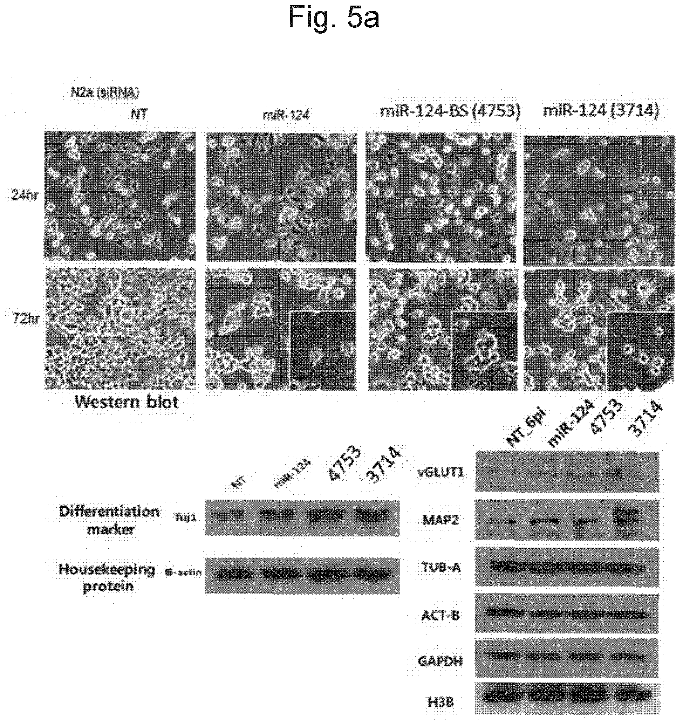

To additionally confirm the specific branched neurite outgrowth phenomenon induced by the miR-124-BS expression in mouse neuroblastomas N2a (CCL-131) cells, as in Example 4, the miR-124 and the miR-124-BS(4753) were transfected into the cells, and then the morphological change of the cells was observed while the cells were incubated for 72 hours (FIG. 5A). In addition, simultaneously, the miR-124(3714) which has the same miR-124 seed sequence, but is different in the other part of the sequence was synthesized as the sequence of 5'-GAAGGCAGCAGUGCUCCCCUGU-3' to form an siRNA-type duplex and then transfected into cells for an experiment. Here, a control was used by being synthesized to be the same as the sequence of cel-miR-67 (NT; non-targeting), which is miRNA of C. *elegans.*

As a result, in the experiment group into which the miR-124 is delivered, nerve cells having a long neurite similar to that in catecholamine-containing nerve cells, CAD, were able to be observed, and in cells to which miR-124-BS(4753) was transfected, the morphological feature of a cell having a short neurite branched in various directions was possible to confirm (FIG. 5A, upper). In addition, in an experimental group into which miR-124(3714) was transfected, like the miR-124, it was possible to observe that there are many cells having a relatively longer neurite (FIG. 5A, upper). Such morphological changes in cells were able to be confirmed by detecting an increase in expression levels of an antibody against a corresponding protein expressed in differentiated nerve cells, Tuj1 (Covance Antibody Products, MRB-435P), vGLUT1 (Synaptic Systems, 135-303) and MAP2 (Millipore, MAB3418) through immunoblotting, compared with the expression of four types of control proteins (ACT-B, TUB-A, GAPDH and H3B) (FIG. 5A, lower). Accordingly, it was possible to confirm that the neuronal differentiation caused by miR-124-BS(4753) that is similar to but different from that by miR-124 is slightly different in a morphological aspect, but induced in the same manner in terms of a gene expression marker, compared with the case caused by miR-124, and it was able to seen that the miR-124(3714) shown to mainly suppress a canonical seed target in the same manner as miR-124 because it has the same seed sequence exhibits a characteristic of differentiating the branch of a nerve cell longer than expected. In addition, when both of the miR-124-BS(4753) and miR-124(3714) are expressed, the expression levels of proteins shown in the differentiated nerve cells increased, confirming that these have molecular characteristics of differentiated forms of nerve cells.

In all of the examples described above, the miRNA expression was performed by transfecting a synthesized duplex into cells, or also by transfecting a vector for expressing the form of shRNA, which is a hairpin-shaped RNA capable of generating the miRNA. Therefore, a vector for expressing miR-124, miR-124-BS(4753) or miR-124(3714) in the form of shRNA was formed using a pCAG-miR30 plasmid (Addgene #14758), which is a vector for expressing pre-miRNA. The pCAG-miR30 vector used herein used a CAG promoter to strongly express miRNA, was designed to express a backbone of miRNA-30, thereby having an advantage of maximizing miRNA expression (Paddison P et al, Nature methods, 2004, 1(2): 163-7).

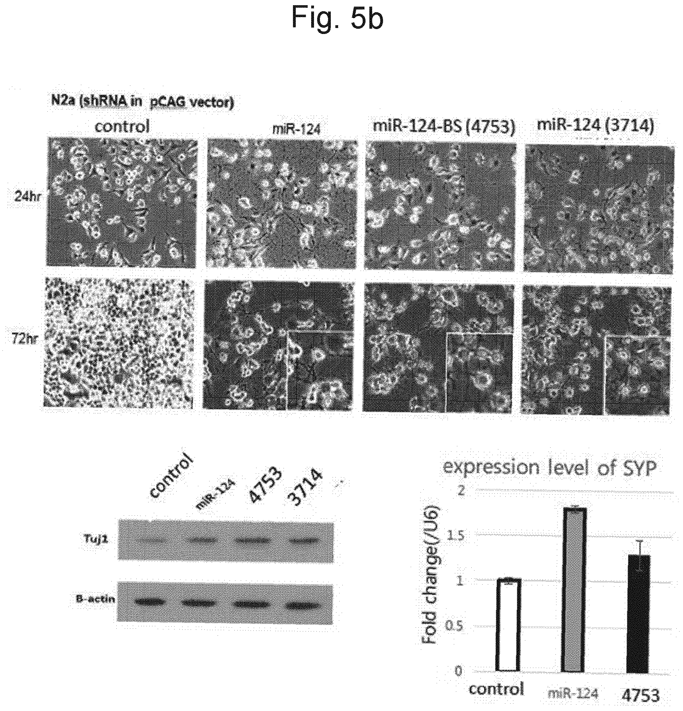

The pCAG vector formed to express each of the miR-124, miR-124-BS(4753) and miR-124(3714) was transfected into N2a cells, using a pCAG vector not expressing anything, and then cell morphology was observed (FIG. 5B). In an experiment group in which a hair-pin structure was transfected into neuroblastoma (N2a) cells, similar changes to the changes in cell morphology shown by the miR-124, miR-124-BS(4753) and miR-124(3714) duplexes were shown. When incubated for 72 hours after transfection, in the experimental groups in which the miR-124 and the miR-124(3714) are expressed, respectively, neuroblastoma cells with a long neurite were able to be observed. In contrast, in the experimental group in which the miR-124-BS(4753) is expressed, neuroblastoma cells with a short neurite branching in various directions were able to be observed (FIG. 5B, upper). Such changes in cell morphology were confirmed from the increase in expression of Tuj1 (O. von Bohlen et al. 2007 Cell and Tissue Research, 329, 3, 409-20), which is a protein expressed in differentiated nerve cells, or expression of synaptophysin (SYP) (FIG. 5B, lower). Therefore, it was possible to confirm that the cells into which the miR-124, miR-124-BS(4753), and miR-124(3714) hair-pin vectors are introduced have the characteristics of differentiated nerve cells. The increase in TUJ1 was observed by immunoblotting in the same manner as in the above-described example, and synaptophysin was detected using the primer sequences disclosed in the previous document (S. E. Kwon et al. 2011 Neuron 70, 847-54) through quantitative polymerase chain reaction (qPCR). This assay was conducted by transfecting a corresponding vector into N2a cells using a Lipofectamine 3000 reagent (Invitrogen) according to the manufacturer's protocol, 24 hours after transfection, extracting total RNA using an RNeasy kit (Qiagen), performing reverse transcription with Superscript III RT (Invitrogen) according to the corresponding protocol, performing qPCR with an SYBR Green PCR Master Mix (Applied Biosystems) to measure an mRNA level of SYP as a corresponding primer, and then normalizing it to a GAPDH mRNA level.

From the results in the example, it can be seen that, even when a vector expressing the miRNA transfection in the form of shRNA, if only having the same seed sequence of the miR-124 (miR-124(3714)), like the miR-124, generally long neurite differentiation occurs by the inhibition of the expression of a canonical target gene. In addition, when the non-canonical bulge target of miR-124 is also expressed in the same manner as the miR-124-BS through the seed region in the form of shRNA (miR-124-BS(4753)), branched neurite outgrowth may occur. It was possible to confirm that both of the canonical target suppression and non-canonical bulge target suppression by the miR-124 show molecular gene marker expression in the form of neurite differentiation of nerve cells, and both show differentiation into nerve cells.

### [Example 6] Confirmation of induction of neurite differentiation of mouse neuroblastoma (N2a) cells by miR-124-BS through regulation of MAPRE1 expression

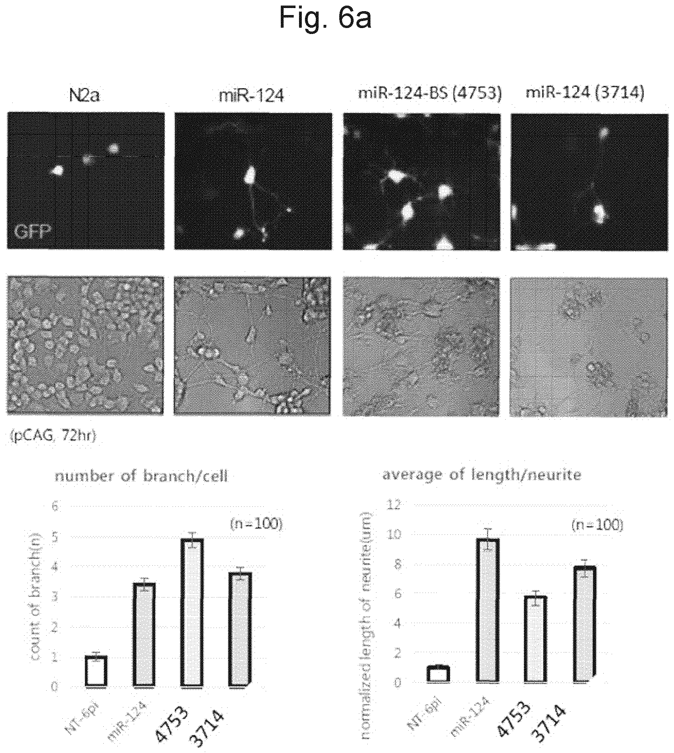

Additionally, the miR-124, miR-124-BS(4753) and miR-124(3714)-induced neurite differentiation in neuroblastoma (N2a) cells was observed in terms of cell morphology, which was quantitatively analyzed (FIG. 6A). During quantitative analysis, to distinguish a neurite branch derived from each nerve cell, a pUltra (Addgene #24129) plasmid expressing a green fluorescent protein (GFP) and a corresponding miRNA expression vector were co-transfected in the same manner as in the above-described example, cell culture was conducted for 72 hours, neurites were observed using a fluorescent microscope (Leica) to count the number of neurite branches, and the length of the neurite generated per cell was analyzed using the ImageJ program.

As a result, with respect to 100 cells, the number of neurite branches expressed per cell was increased in the miR-124, miR-124-BS(4753) and miR-124(3714) experimental groups approximately 3- to 5-fold compared to the control (FIG. 6A, lower left). However, when the miR-124-BS(4753) is expressed, compared with the miR-124 or miR-124(3714) is expressed, significantly more branches were observed. In addition, when it was confirmed that, in the miR-124-BS(4753) and miR-124(3714) experimental groups, the length of a neurite generated per cell was increased 4- to 6-fold compared to the control, and in further detail, it was observed that, when the miR-124 or miR-124(3714) was expressed, compared with when the miR-124-BS(4753) was expressed, a longer neurite branch is produced (FIG. 6A, lower right). This is the same result as observed in cell morphology in the previous examples (FIGS. 4, 5 and 6).

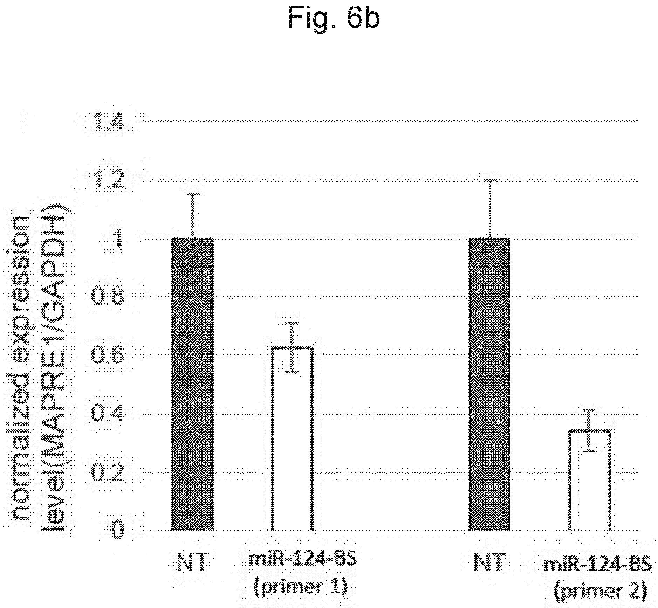

To investigate whether the specific neuronal differentiation phenomenon caused by the miR-124-BS is induced by a non-canonical bulge site of any gene, the non-canonical bulge target sequence of miR-124 was searched for in the sequence of the 3' UTR region of the gene related to neuronal differentiation. As a result, a microtubule-associated protein RP/EB family member 1 (MAPRE1: Elena Tortosa et al. 2013 EMBO32,1293-1306) gene reported to regulate the differentiation of nerve cells was found. MAPRE1 may be a protein attached to the end of a microtubule constituting a neurite in the generation of the neurite, and may determine the shape of a neurite through the regulation of the formation of the corresponding microtubule. Accordingly, to confirm that the MAPRE1 gene is recognized as the non-canonical bulge target of miR-124 and inhibits the expression thereof, after miR-124-BS was transfected into N2a cells, the mRNA level of MAPRE1 was measured by qPCR assay according to the method performed in Example 5 (FIG. 6B). When qPCR was conducted with two different primers, it was possible to confirm that both of the two results were able to show that MAPRE1 expression decreased to approximately 40 to 70% in the miR-124-BS-transfected experimental group, compared with the control. Through this, it was possible to confirm that the expression of MAPRE1 is inhibited by the non-canonical bulge site of the miR-124.

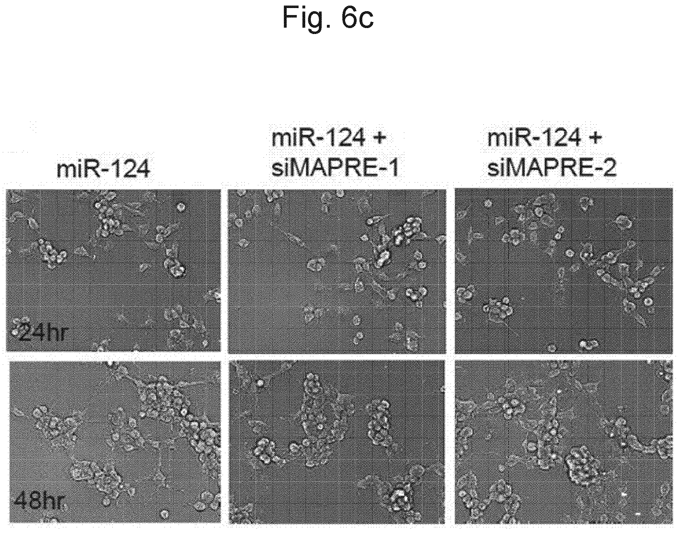

MAPRE1 is the non-canonical bulge target of the miR-124, and if it is a very important target, the type of neuronal differentiation caused by the suppression of the non-canonical bulge target of the miR-124-BS is further promoted by the reduction in MAPRE-1 expression. Accordingly, to confirm this, two types of siRNA capable of suppressing MAPRE1 were prepared and transfected into N2a cells with miR-124, followed by performing an experiment of confirming a change in cell morphology (FIG. 6C). As a result, compared with the control expressing only miR-124, in the experimental group expressing miR-124 with the siRNA for MAPRE1, it was possible to observe that the number of neurite branches is highly increased, which was observed to be the same as in the experiment using two types of MAPRE1 siRNA having a different sequence (FIG. 6C). In this experiment, when RNA is transfected into cells in the same manner as in the method used in the previous example, the morphology was observed while the cells were incubated for 48 hours thereafter.

Therefore, it was confirmed that the short, branched neurite outgrowth induced by the miR-124-BS is a biological function that can be shown by at least partially inhibiting the expression of a MAPRE1 gene by the non-canonical bulge target site of miR-124.

### [Example 7] Analysis of tendency of suppressing non-canonical bulge target gene of miR-124 when miR-124-BS is transfected into cells at transcriptome level

It was confirmed that a gene suppressed by miR-124-BS in mouse neuroblastoma (N2a) cells eventually induces the nerve cell differentiation which produces many neurite branches of neuroblastoma (FIG. 5). This phenomenon is considered to occur by a mechanism of recognizing and suppressing the non-canonical bulge target of miR-124, and such possibility was confirmed with MAPRE1 found as a target gene (FIG. 6). However, it may be actually a phenomenon caused by suppression of hundreds of non-canonical bulge target genes of miR-124.

After miR-124 and miR-124-BS were transfected into N2a cells, RNA-Seq assay was performed on the miR-124-BS invented by the inventors to confirm only the non-canonical bulge target gene of miR-124 at the transcriptome level expressing all genes. Here, as a control, a duplex having the same base sequence as miR-124 and an abasic substitution (pi) in position 6 from the 5' end (miR-124-6pi) was used, and it was reported that such substitution does not make pairing with target mRNA at all since there is no base in position 6 from the 5' end of miRNA (Lee HS et. Al. 2015, Nat Commun. 6:10154).

The RNA-Seq assay was performed by delivering each of the miR-124, the miR-124-BS and the miR-124-6pi duplex into N2a cells at 75 nM using a modification of the sequence of cel-miR-67, which is miRNA of C. elegans as an experimental control, in which the position 6 from the 5' end is substituted with an abasic spacer (NT-6pi), extracting total RNA with an RNAeasy (Qiagen) 24 hours after incubation, constructing a library at Otogenetics, and subjecting it to next-generation sequencing. Afterward, a FASTAQ file, which is the sequence data obtained from the assay, was mapped to a mouse genomic sequence (mm9) with the TopHat2 program, expression values (FPKM) were obtained with Cufflink and Cuffdiff programs and normalized to a result in mouse neuroblastomas (N2a) cells into which a control NT-6pi was transfected to perform analysis with a fold change, (log2 ratio).

To analyze whether the mRNA level of a gene having a target site of miRNA is reduced by the expression of the corresponding miRNA from the RNA-Seq result, genes with a phastCons score of 0.9 or more were screened to select those in which a canonical seed site (7mer paired with bases between positions 2 to 8 from the 5' end) of the miR-124 has 3' UTR and the sequence of the corresponding site is conserved in various species, and these profiling results were compared and analyzed with a cumulative fraction in the order of inhibition depending on the expression of the corresponding miRNA. In addition, the non-canonical bulge site (nuc, 7mer) of miR-124 was also identified in the same manner as described above, and the suppressive fraction of the corresponding gene was analyzed with a cumulative fraction in the same manner as described above. Here, since a gene having the non-canonical bulge site of the miR-124 also has a canonical seed site, it is difficult to determine the suppressive effect thereof, in contrast to when there is no canonical seed sequence of miR-124 in total mRNA (nuc only), the case where there is only a canonical seed site and no non-canonical bulge site (seed only) was also analyzed.

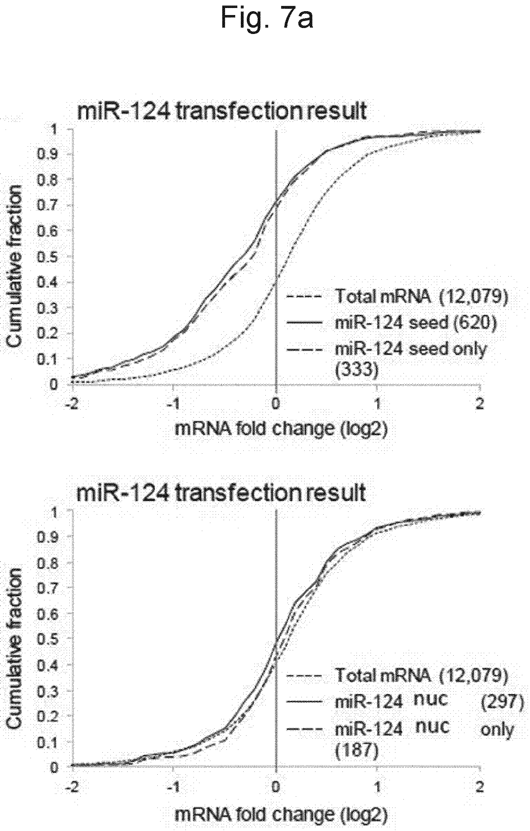

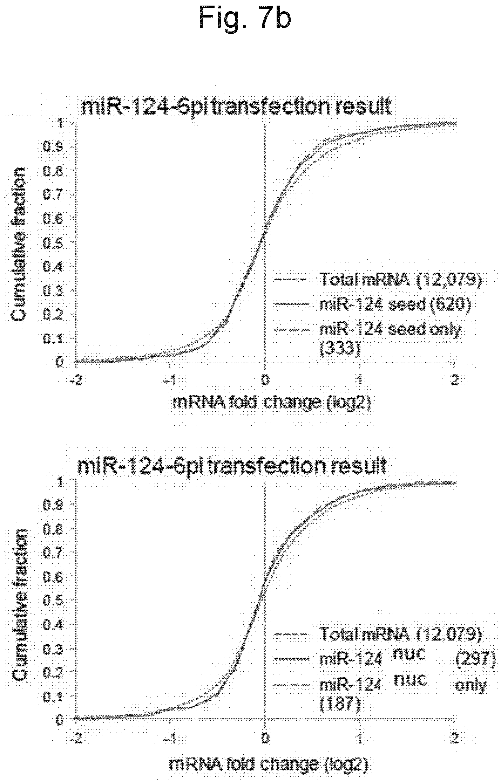

In the RNA-Seq sequencing for the miR-124-expressed experimental group, when a fold change according to the miR-124 expression was analyzed according to a cumulative fraction, a phenomenon in which a gene (miR-124 seed) having the canonical seed site conserved in the miR-124 in the 3' UTR is highly suppressed compared with the distribution of total mRNA was confirmed (FIG. 7A, upper), and compared with this, it was possible to observe that a gene having the non-canonical bulge site of the miR-124 is significantly suppressed and then very weakly suppressed (FIG. 7A, lower). However, such a suppressive phenomenon was not observed in miR-124-6pi-transfected cells as a control (FIG. 7B).

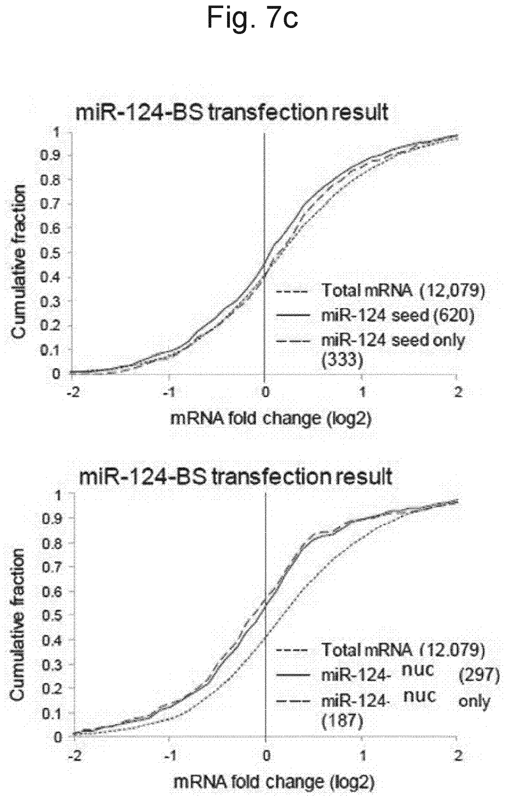

When the miR-124-BS developed by the inventors was expressed, in RNA-Seq sequencing, when a fold change according to the miR-124-BS expression was analyzed according to a cumulative fraction, it was confirmed that a weak inhibitory effect was shown in a gene (miR-124 seed) having the canonical seed site conserved in the miR-124 in the 3' UTR, but there is no change in a gene (miR-124 seed only) having the corresponding canonical seed site without a non-canonical bulge site of the miR-124 (FIG. 7C, upper). However, it was able to seen that the suppression of both of the gene having the non-canonical bulge target of the miR-124 (miR-124 nuc) and the gene having a corresponding target (miR-124 nuc only) occurs strongly and effectively (FIG. 7C, lower). In FIG. 7C, when the miR-124-BS was expressed, the gene having only the canonical seed site of miR-124 (miR-124 seed only) was not suppressed, demonstrating that the canonical seed site of miR-124 was not suppressed at all, but it is predicted that the weak suppression of the seed site target of miR-124 by the miR-124-BS is probably caused by the co-existence of the canonical site and the non-canonical bulge site of the miR-124.

From the result of the example, it was able to be seen that miRNA very strongly and effectively suppresses a conventional canonical seed target gene, but very weakly suppresses the non-canonical bulge target at the transcriptome level, and it was possible to confirm that miRNA-BS inhibits the expression of the non-canonical bulge target of miRNA, but does not suppress the conventional canonical seed sequence at the transcriptome level.

### [Example 8] Confirmation of cell cycle arrest induced by miR-122-BS in human liver cancer cell line (HepG2) through flow cytometry

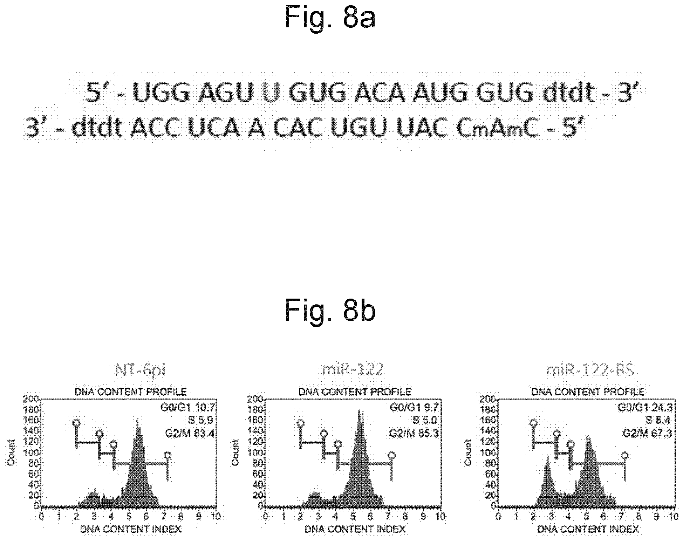

Based on the result of observing the induction of nerve cell differentiation of miR-124 by the non-canonical nucleation bulge target of miR-124, an experiment for the biological function of the non-canonical nucleation bulge target of another miRNA was performed. MiR-122 has 5'-UGG AGU GU-3' as a seed base sequence, and miR-122-BS, which is the base sequence of siRNA base-paired with the non-canonical nucleation bulge target site thereof, may have one more U in position 6, and in this case, thus have 19 bases represented by 5'-UGG AGU U GUG ACA AUG GUG-3' at the 5' end thereof and deoxy thymine nucleotides (dts) as bases in positions 20 and 21. Particularly, in a duplex structure, the corresponding dt parts may consist of a guide strand and a passenger strand to form a two-nucleotide overhang at the 3' end. Here, the passenger strand made perfectly complementary base pairing with the guide strand, and included two 2'OMes at both of the positions 1 and 2 from the 5' end and two dts at the end of the base sequence (FIG. 8A). The guide strand and the passenger strand of the miR-122-BS were chemically synthesized by Bionia, separated by HPLC, and were prepared in a duplex according to the method provided by the manufacturer.

The miR-122-BS prepared as described above (FIG. 8A) was transfected into a human liver cancer cell line (HepG2), and the change in cell cycle was then observed through flow cytometry. In this experiment, each of NT-6pi as a control and the miR-122 and miR-122-BS duplexes was transfected into HepG2 cells at 50 nM using an RNAiMAX (Invitrogen) reagent, and the cells were incubated for 24 hours, treated with 100 ng/ml of nocodazole for 16 hours and synchronized to the G2/M phase (division preparation phase/division phase), followed by analyzing a cell cycle with a MuseTM Cell Cycle Kit (Catalog No. MCH100106, Millipore) and a Muse Cell Analyzer (Millipore) according to the experimental method provided by the manufacturer.

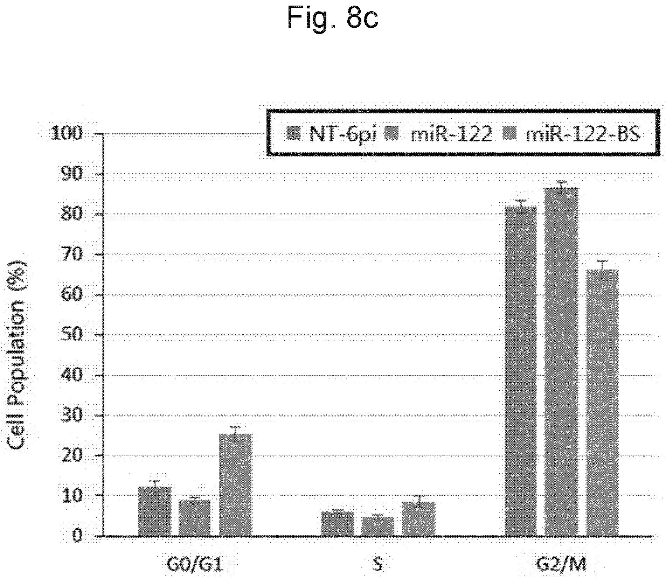

As a result, compared with the cell cycle analysis for the NT-6pi-transfected control, in the miR-122-BS experimental group, the G0/G1-phase cells increased approximately 2-fold from 10.7% to 24.3%, confirming that the cell cycle arrest in the liver cancer (HepG2) cells increased (FIGS. 8B and 8C). An increase in the GO/G1-phase cells was not observed in the miR-122-transfected cells. Subsequently, the G2/M-phase cell distribution of the miR-122-BS-transfected human liver cancer cell line (HepG2) was reduced from 83.4% to 67.3% compared with the control, and this result was not observed in the miR-122-transfected cells (FIGS. 8B and 8C).

Accordingly, it was possible to observe that the miR-122-BS shows a biological function of inducing cell cycle arrest in a human liver cancer cell line through regulation of the expression of the non-canonical nucleation bulge target of the miR-122, which is a completely different function from the biological function of the miR-122.

### [Example 9] Confirmation of muscle fibrosis function in skeletal muscle cells (C2C12), induced by miR-1-BS, and apoptosis mediated by miR-155-BS

Since a novel biological function different from the function shown by the miRNA-BS-mediated inhibition of the expression of the canonical seed target of miRNA has been observed, to observe how such a function is exhibited in muscle cells, miRNA-BS was applied to miR-1 and miR-155, which are expressed in muscle cells and play a critical role.

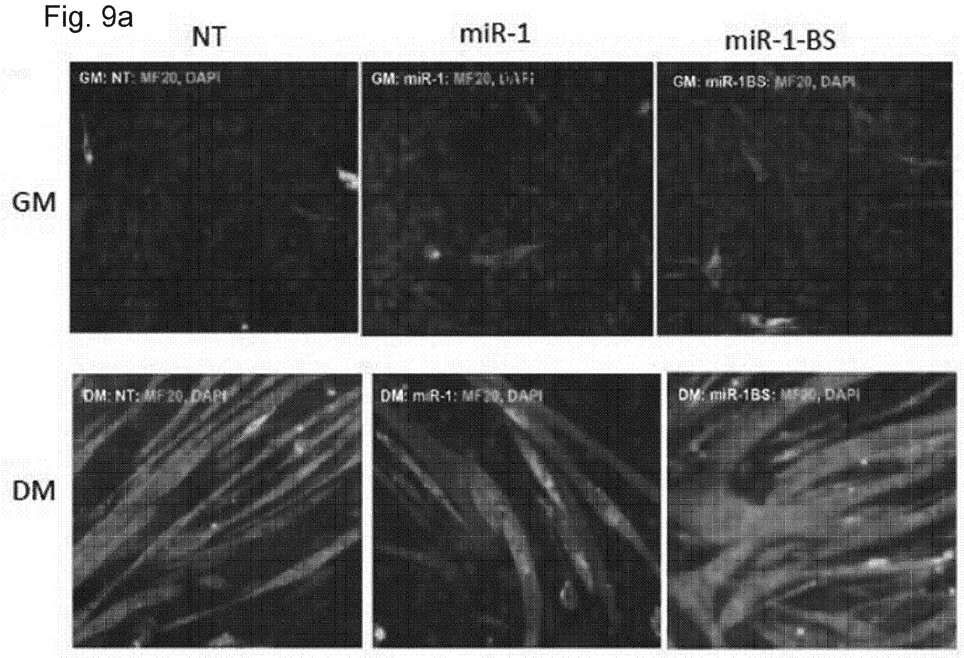

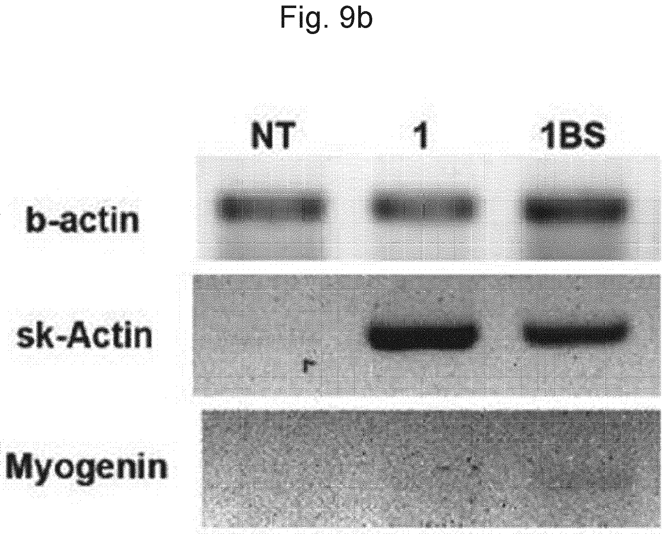

First, since the miR-1 is a muscle tissue-specific miRNA, and has been reported to function in muscle cell differentiation (Chen J et.al, Nature genetics, 2006, 38(2): 228-233), miR-1-BS was expressed in the skeletal muscle cell line, C2C12, and then a cell morphological change (FIG. 9A) and the expression of a gene marker expressed in differentiation (FIG. 9B) were investigated. Here, miR-1-BS was synthesized in the same manner as described in the previous example and transfected into the cells, and the cells were incubated in Dulbecco's modified Eagle's medium (Invitrogen) supplemented with 10% fetal bovine serum (FBS), 100 U/ml penicillin and 100 (µg/ml of streptomycin for 48 hours and then observed after being divided under a growth media (GM) condition (FIG. 9A, upper) and further incubated for 48 hours under a FBS-removed deprivation media condition (DM) (FIG. 9A, lower). Afterward, the cells were treated with 4% para-formaldehyde (PFA) to fix the cells, a myosin 2 heavy chain protein expressed in differentiated muscle cells was reacted with a primary antibody (MF20: Developmental Studies Hybridoma Bank) and then stained with an Alexa 488 fluorescent material-attached mouse secondary antibody (ab150105) to observe cell morphology and differentiation (FIG. 9A).

As a result, when miR-1-BS was expressed under the growth media (GM) condition in the mouse skeletal muscle cells (C2C12), compared with the NT (control) or miR-1 duplex-transfected cells, a larger number of differentiated muscle cells was possible to confirm by MF20 staining. Under the deprivation media (DM) condition that reduces serum during incubation, the progression of the differentiation into a muscle fiber was observed, and it was possible to observe a fiber in a thicker form compared with the skeletal muscle cells expressing NT as a control (FIG. 9A). This is caused by faster induction of muscle cell differentiation by miR-1-BS under the GM condition. The differentiation of muscle cells can be confirmed by the expression level of a gene whose expression is increased during differentiation as a marker at the molecular level, and therefore, sk-actin and myogenin expression levels were detected through reverse transcription (RT)-PCR using a primer specific for the corresponding mRNA, compared with the mRNA level of b-actin, which has no difference in expression level in differentiation (FIG. 9B). As a result, when both of the miR-1(1) and miR-1-BS(1BS) are expressed, it was possible to confirm that the transcription levels of the sk-actin and the myogenin increase, and the transcription level of the myogenin is increased more by the miR-1-BS than the miR-1.

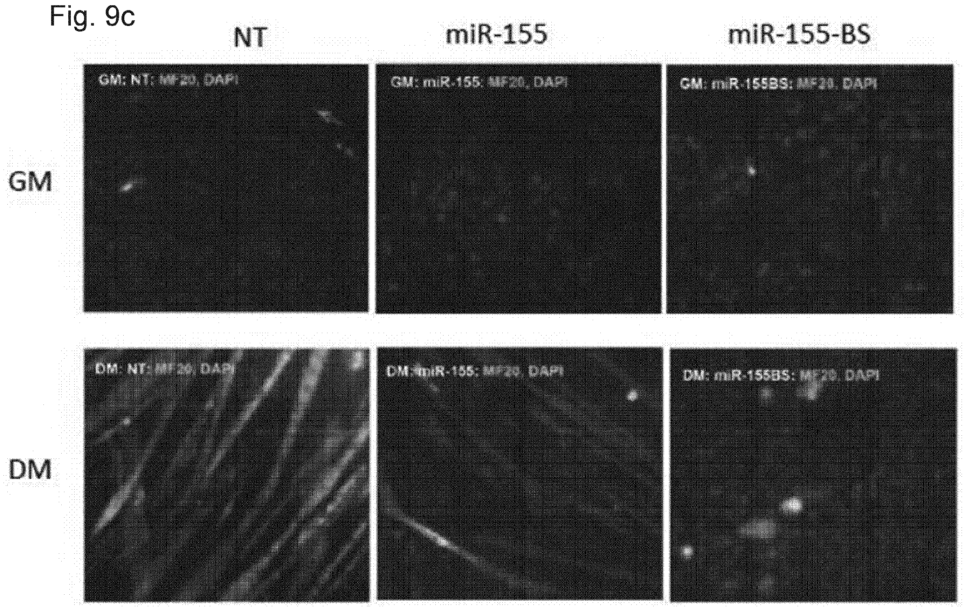

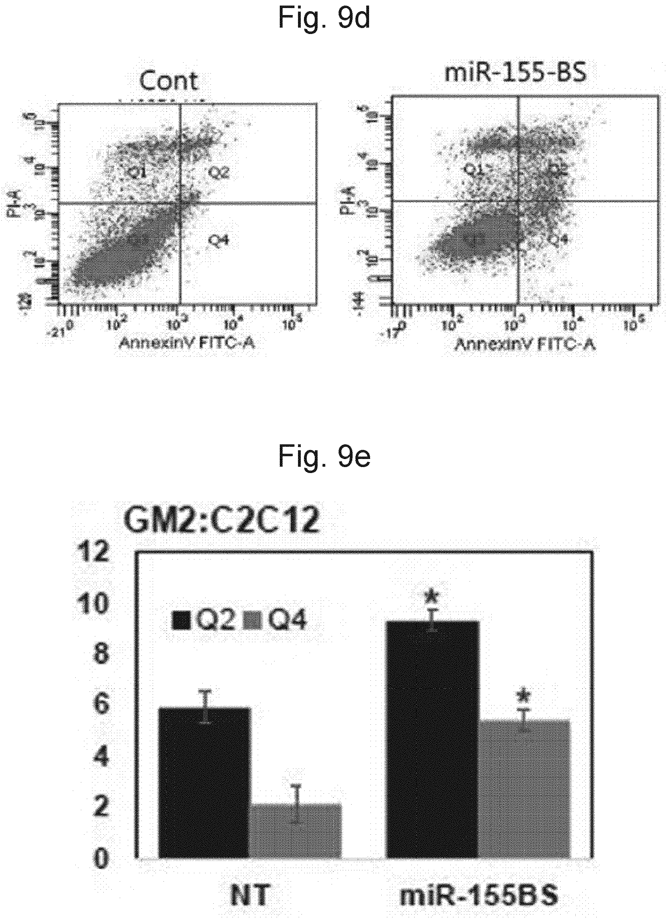

The miR-155 is miRNA inhibiting muscle cell differentiation (Seok H et. Al, JBC, 286(41):35339-46, 2011), the effect of miR-155-BS was observed through the differentiation of skeletal muscle cells (C2C12) in the same manner as performed for the miR-1 above (FIG. 9C). As a result, after NT as a control, miR-155 and miR-155-BS were expressed, it was observed that no significant morphological differences were observed under the GM condition, whereas under the DM condition inducing muscle cell differentiation, the control was differentiated, when the miR-155 was expressed, the differentiation was inhibited, and when the miR-155-BS was expressed, it was confirmed that differentiation disappeared through immunostaining with a myosin 2 heavy chain protein (FIG. 9C). Particularly, when closely examining the C2C12 cells expressing miR-155-BS, it was observed that the miR-155-BS induces apoptosis. In contrast, in the miR-155-transfected muscle cells, apoptosis was not observed at all (FIG. 9C, lower). For further investigation, the C2C12 cells expressing the corresponding miRNA were grown under the GM condition for 48 hours, and then subjected to flow cytometry in the same manner as in Example 3 to examine apoptosis (FIG. 9D). As a result, it was confirmed that, in the miR-155-BS-transfected experimental group, apoptosis increased approximately 1.5 to 2.5-fold compared with the control (FIGS. 9D and 9E).

In this example, it was possible to confirm that the miR-1-BS promotes the differentiation of skeletal muscle cells and induces the differentiation of a thick muscle fiber, the miR-155-BS induces the death of skeletal muscle cells, and these functions are different from the function of the conventional miR-1 for contracting muscle cells and the function of the miR-155 for inhibiting muscle cell differentiation.

### [Example 10] Discovery of non-canonical target site allowing wobble base pairing at seed position of miRNA through Ago HITS CLIP assay

As a method of analyzing a miRNA target at the transcriptome level, an experimental method called Ago HITS CLIP has been developed and is widely used (Nature, 2009, 460 (7254): 479-86). The Ago HITS CLIP assay is a method of inducing covalent bonding between RNA and Argonaute protein in cells by UV irradiation of cells or a tissue sample, isolating the generated RNA-Argonaute complex through immunoprecipitation using an antibody specifically recognizing Argonaute, and analyzing the isolated RNA through next-generation sequencing. The base sequence data obtained thereby may not only identify target mRNA of miRNA through bioinformatics analysis, but also precisely analyze its binding site and sequence (Nature, 2009, 460 (7254): 479-86). Ago HITS-CLIP was first applied to mouse cerebral cortex tissue to perform mapping for the miRNA target in the brain tissue. Since then, this method has been applied to various tissues. Through numerous Ago HITS-CLIP assays, miRNA binding sites in various tissues were identified, and it was found that there are many non-canonical binding sites which make similar miRNA seed binding particularly in the above-mentioned target sequence, but different binding in the sequence of target mRNA (Nat Struct Mol Biol. 2012 Feb 12;19(3):321-7).

It was observed that some of the known non-canonical binding rules are present as wobbles through G:U base pairing, other than the conventional known base pairings. The wobble base pairing is found in specific RNA, different from the conventional base pairing, and it was shown that the well-known G:U wobble pair may also be paired to a non-canonical target of miRNA. However, compared with this, a G:A wobble pair which has weak binding and is not often found may form base pairing in specific RNA, but other than this, has not been investigated at all. However, in the case of the seed sequence of miRNA, since the free energy of base pairing is structurally stabilized by the Argonaute protein, generally weak G:A base pairing may be relatively significant, and based on fact described above, the inventors conducted analysis as follows.

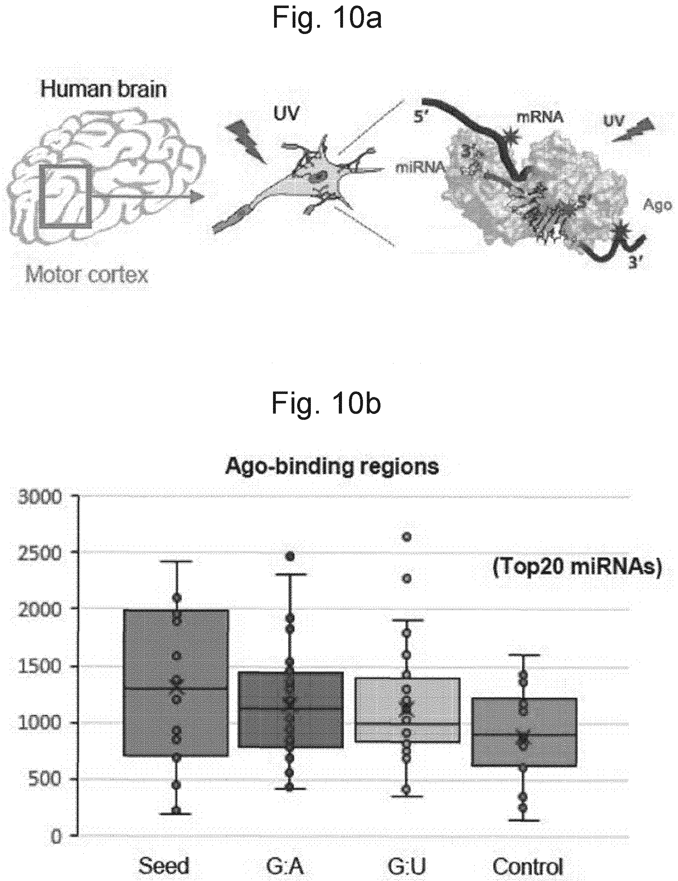

First, to confirm whether wobble base pairing including a G:A pair is present in the miRNA target of human brain tissue, Ago HITS-CLIP data from the gray matter of the brain tissue after death was analyzed (Boudreau RL et al, Neuron, Vol.81 (2), 2014, 294-305) (FIG. 10A). This analysis was performed by pairing an RNA sequence that had been bound with Argonaute-miRNA according to the method used in Ago HITS-CLIP development (Nature, 2009, 460 (7254): 479-86) to a human genomic sequence by Bowtie2 using a FASTQ file. In addition, the corresponding sequencing result paired to a human miRNA sequence at the same time so as to also analyze 20 types of miRNAs (top 20 miRNAs; FIG. 10B) frequently binding to the Argonaute protein in the corresponding brain tissue was analyzed (FIG. 10B). Here, it was observed that a considerably large number of canonical seed target sites are found at miRNA binding sites (FIG. 3B, median: 1292.5 sites, error range: +/- 706.62). Afterward, from the Ago HITS-CLIP data, whether non-canonical target sites that are bound with G:U or G:A wobble pairing are present in such a miRNA seed base sequence was examined. Here, for analysis, a target sequence which cannot achieve complementary pairing of bases due to a base sequence identical to the miRNA seed base sequence, was used as a control.

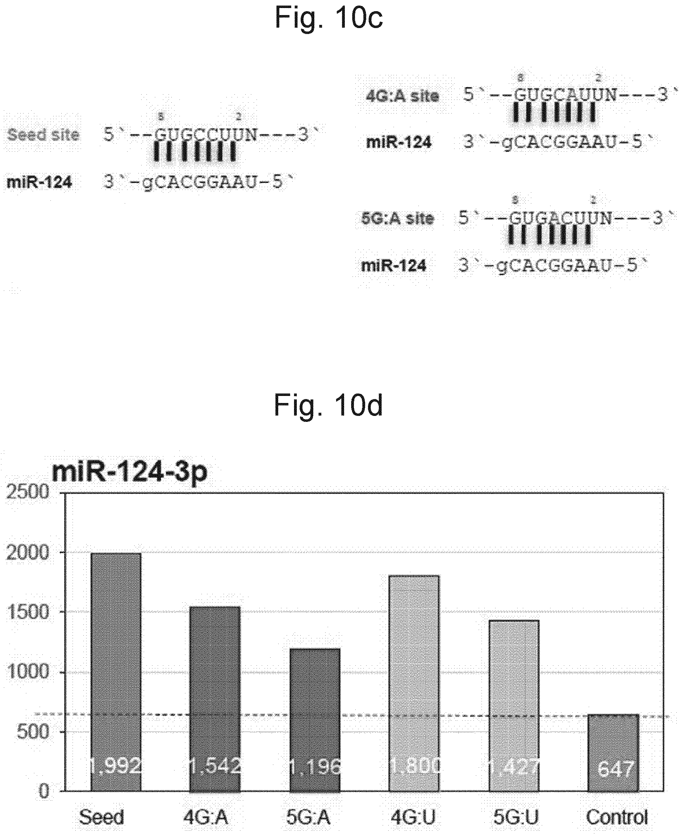

As a result of the analysis, all of G:A wobble pairs pair-allowed target sites (median: 1119.5 sites, error range: +/- 526.48) and G:U wobble pair-allowed target sites (median: 995 sites, error range: +/- 523.22) showed higher distribution than the control (median 891 sites, error range: +/- 410.71), indicating that, particularly, there are approximately 1.1 times more miRNA target sites having a G:A wobble pair than those having a G:U wobble pair (FIG. 10B). MiR-124 specifically expressed in brain tissue has two G bases in positions 4 and 5 from the 5' end in the seed region, and thus can achieve G:U and G:A wobble base pairing (FIG. 10C). Accordingly, as a result of distinguishing and analyzing the G:A and G:U wobble base pairing by the G bases at the specific sites (FIG. 10D), the largest number (1,992) of canonical seed target sites (seed sites) of miR-124 were found. However, as compared with the above, 1,542 sites with a G:U wobble pair at the base in position 4 were found, and then 1,542 sites with a G:A wobble pair were observed. In addition, 1,800 target sites with a G:U wobble pair and 1,196 target sites with a G:A wobble pair were observed, and it was possible to confirm that all of the investigated sites are present in a number greater than the number of the control (647 sites) (FIG. 10D). Accordingly, when target mRNA is recognized with the base sequence of the seed region, a non-canonical G:U or G:A wobble base pair as well as a canonical base sequence with respect to the seed region is allowed, and it was able to be seen that through these types of pairing, miRNA can bind with target mRNA through such G:A wobble base pairing. Such miRNA target recognition through G:A wobble base pairing is a novel result that has not been reported yet, and as observed above, it was possible to confirm that non-canonical targets are recognized in many miRNAs.

In this example, as it was found that the recognition of the non-canonical target of miRNA allows G:A wobble pairing to the seed sequence, it is considered that many miRNAs may bind to the mRNA of a target gene, thereby inhibiting the expression of the gene.

### [Example 11] Confirmation of development of mIRNA-GU recognizing G:A seed site, which is non-canonical target of miRNA, and effect of increasing nerve cell death of miR-124-G5U when miR-124 is applied

In Example 10, it was found that the non-canonical target recognition of miRNA may allow a G:A wobble pair in the seeding sequence, which is referred to as a non-canonical G:A seed site, and in contrast, miRNA-GU, which is the sequence of a new RNA interference-inducing material capable of complementarily pairing was invented. When applying such sequencing technology to miR-124, the miR-124 is the base sequence in which a seed sequence is 5'-AAGGCAC-3' and a non-canonical G:A wobble seed target is capable of having G:A wobble base pairing at the 4^{th} and 5^{th} G bases, and therefore, when such a G:A wobble pair is changed into the miRNA-GU sequence, which is a format for changing the wobble pair into a conventional complementary base pair, the sequence may be changed into miR-124-G4U (5'-AAUGCAC-3') or miR-124-G5U (5'-AAGUCAC-3'). Since the miR-124-G4U and miR-124-G5U changed as described above may be complementarily paired with a non-canonical target site weakly paired in a G:A wobble pair and recognized by the conventional miR-124, the function of the non-canonical G:A seed target in the miR-124 may be exhibited.

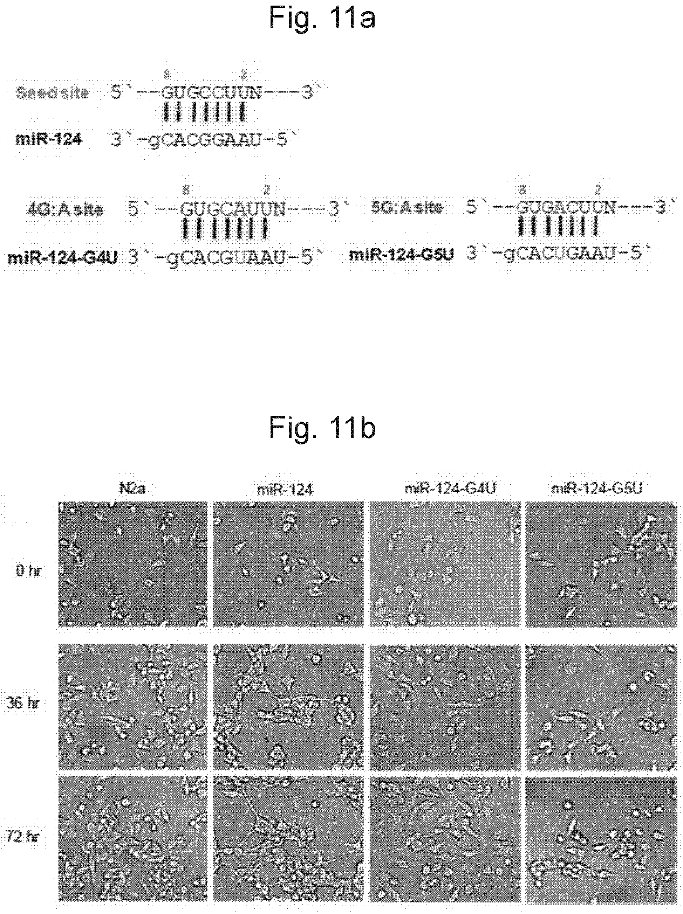

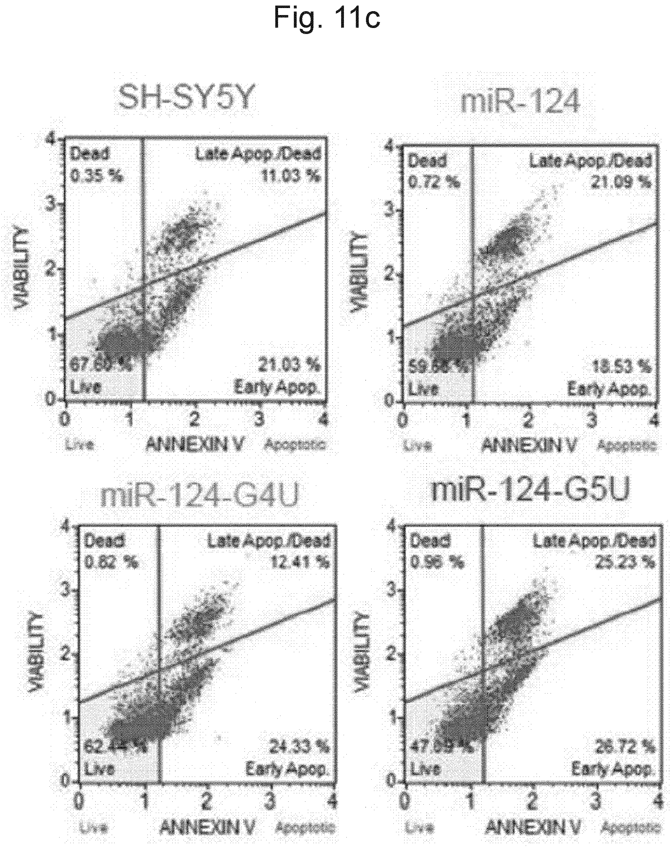

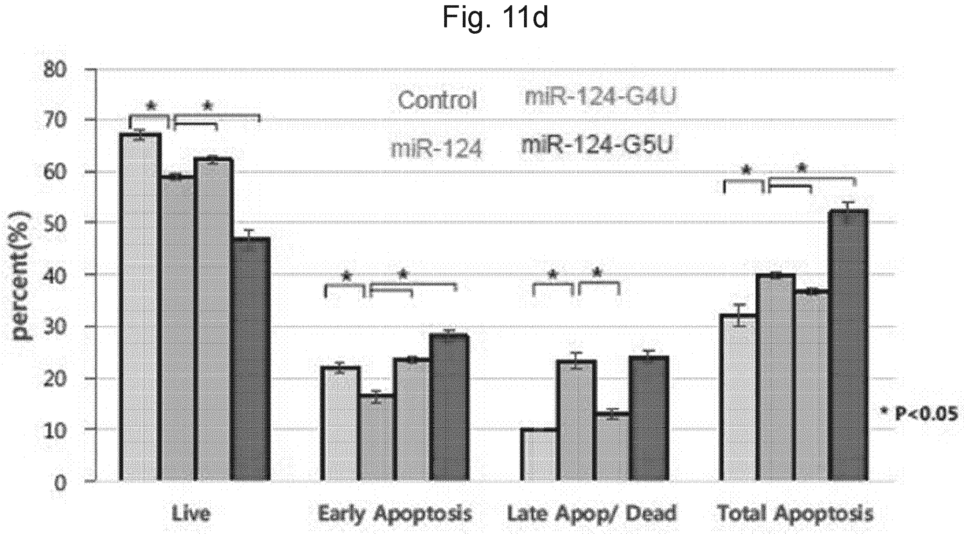

Accordingly, an experiment was performed by applying a non-canonical G:A wobble seed target to the miR-124 to examine the biological function mediated by the non-canonical G:A wobble seed target of the miRNA. To this end, NT (referred to as N2a of FIG. 11B) as a control, the miR-124, the miR-124-G4U or the miR-124-G5U were transfected into neuroblastomas N2a cells according to the same manner as described in the previous example, and incubated for 72 hours to observe cell morphology (FIG. 11B). As a result, when the miR-124 is expressed, the differentiation phenomenon in which neurites are formed occurs, but in the case of the miR-124-G4U or miR-124-G5U-transfected experimental group, a phenomenon in which differentiation does not occur is observed (FIG. 11B). Since neuronal differentiation ability, which is the conventional miR-124 function, is changed by the miRNA-GU, a change in apoptosis was observed in a human neuroblastomas (Sh-sy-5y) cell line. Here, the flow cytometry was performed with a Muse Annexin V and Dead Cell Assay Kit (Millipore), similar to the method used in Example 9, using a Muse™ Cell Analyzer (Millipore). As a result, it was confirmed that the miR-124-G-5U expression increases 2- or more fold compared with the control (FIG. 11C), and when such increase was analyzed by dividing into early apoptosis and late apoptosis, it was analyzed that, compared with the miR-124-transfected experimental group, in the miR-124-G-5U-transfected experimental group, both of early apoptosis and late apoptosis significantly increase (FIG. 11D). In the miR-124-G-4U-transfected experimental group, compared with the miR-124-transfected experimental group, it was possible to observe that late apoptosis was significantly inhibited (FIGS. 11C and 11D), and the number of living cells is also high (FIG. 11B).

Based on this, it was possible to confirm that the miR-124-GU-mediated suppression of the non-canonical G:A wobble seed target eliminates the neuronal differentiation ability induced by miR-124, and exhibits an apoptosis regulating function, which is another biological function, instead of the neuronal differentiation ability.

### [Example 12] Promotion of cell division of neuroblasts induced by miR-124-G4U

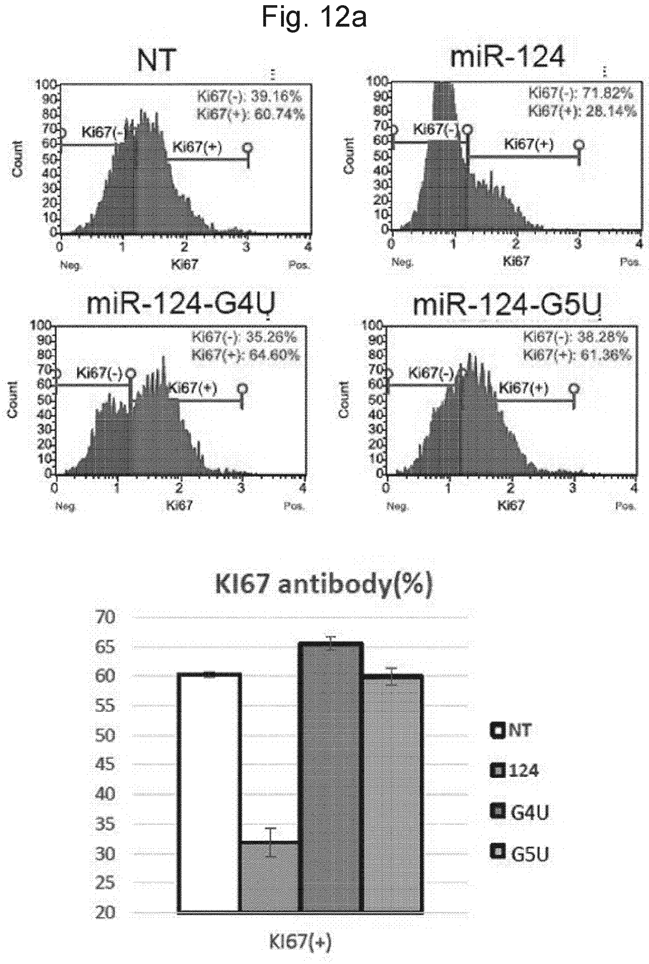

In the previous example, since the miR-124-G4U lost neural differentiation ability and did not have a specific function in apoptosis, cell division was examined. That is, each of the control NT, the miR-124, the miR-124-G4U and the miR-124-G5U was transfected into neuroblastoma (N2a) cells in the same manner as described in the previous example, and flow cytometry for the cell division and proliferation phenomenon was performed (FIG. 12A). The experiment used herein was performed by treating cells using a Muse Ki67 Proliferation Kit (Millipore) according to the experimental method provided by the manufacturer, and analyzing cell division using a muse cell analyzer (Millipore). This method is for quantitatively analyzing the number of cells increased by cell division and proliferation by measuring the number of Ki67-stained cells. When miR-124 is expressed in N2a cells, compared with the control NT, as the cells are differentiated, the number of cells with reduced Ki67 staining intensity increases. However, in the miR-124-G4U-transfected experimental group, compared with the control (NT), the number of proliferated cells, Ki67-stained cells, slightly increased from 61% to 65%. In contrast, it was possible to observe that the miR-14-G5U has no difference from the control.

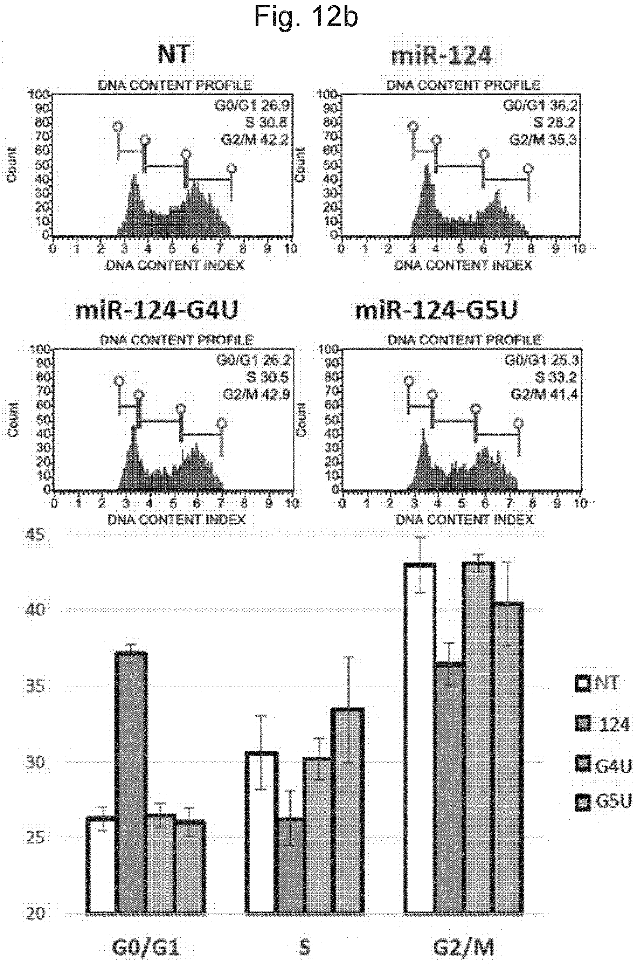

Additionally, to investigate an effect of miR-124-G4U on a cell cycle, flow cytometry was performed using a human neuroblastomas cell line (sh-sy-5y) (FIG. 12B). The analysis was performed using a Muse™ Cell Cycle Kit (Catalog No. MCH100106, Millipore) and a Muse Cell Analyzer (Millipore) to examine the functions of the miR-124-G4U and the miR-124-G5U by cell cycle. As a result, in the case of miR-124, cell cycle arrest was observed by an increase in the number of cells at the G0/G1 phase increases, but in the cases of the miR-124-G4U and the miR-124-G5U, the cell cycle arrest mediated by miR-124 disappears and the cell cycle goes back to the same as the control.

According to the above-described example, it was able to be seen that the miR-124-G4U of the RNA interference-inducing modified sequences suppressing the non-canonical G:A wobble seed target of miR-124 has a function of increasing cell division and proliferation of neuroblasts.

### [Example 13] Confirmation that miR-1 can inhibit the expression of a non-canonical G:A wobble seed target gene in a cardiomyocyte cell line using a fluorescent protein reporter

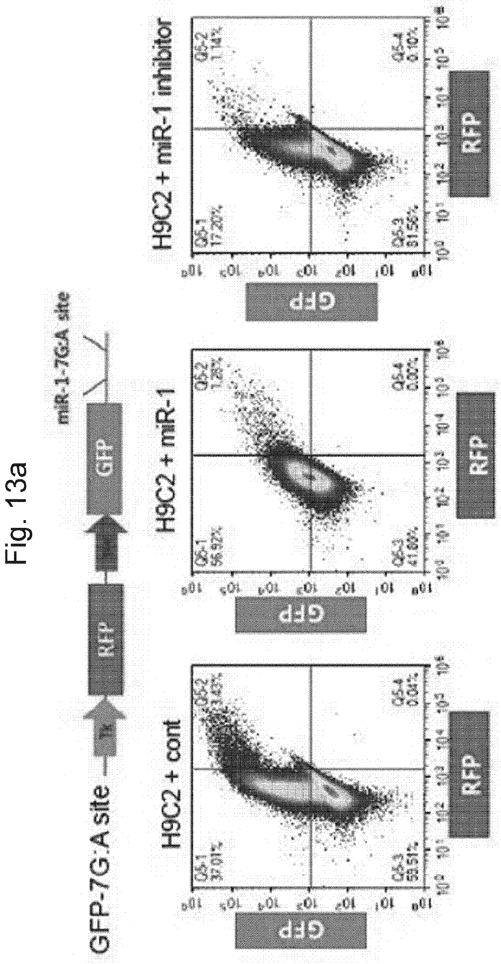

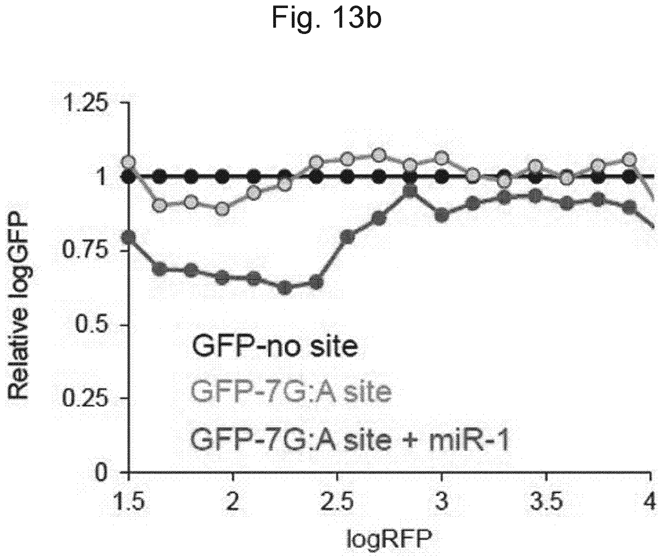

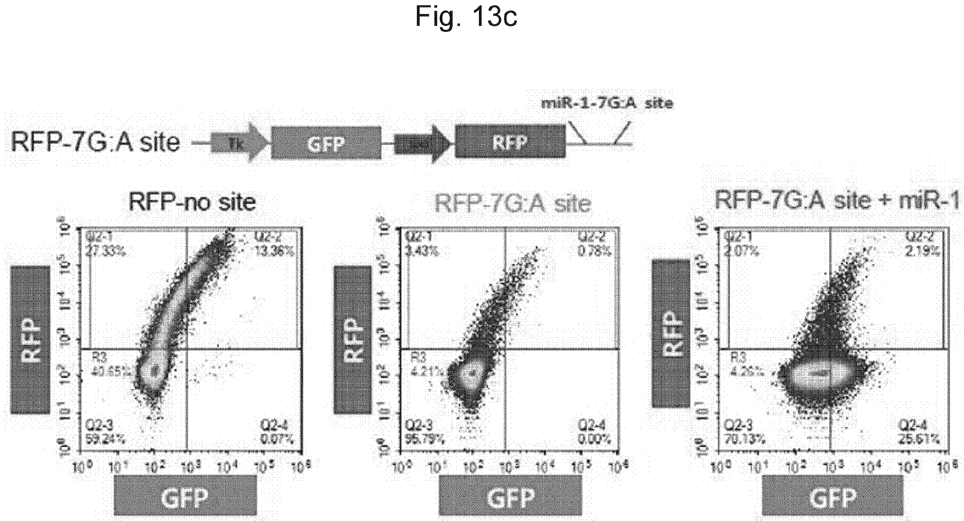

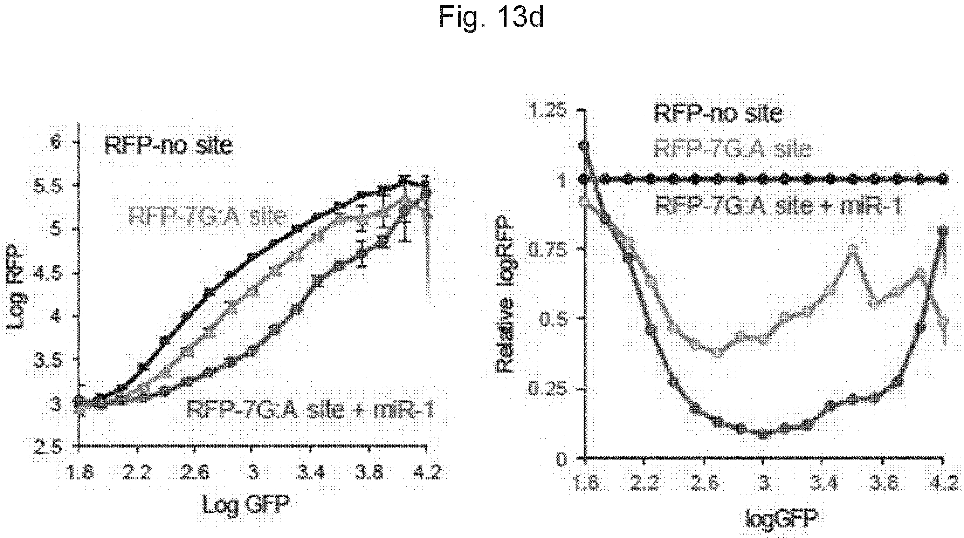

In the above-described example, the miRNA was able to non-canonically bind with target mRNA by allowing a G:A wobble pair in a seed sequence, and it was found that the resulting complex was able to have a novel function, and then a reporter assay for confirming whether such binding can actually induce the suppression of a target gene was conducted at the cellular level. Here, this assay was conducted on miR-1 known to be highly expressed in muscle-like cells such as cardiomyocytes h9c2, and particularly, on a G:A wobble base pair, focusing on G present in position 7 from the 5' end of miR-1 (FIG. 13). To measure the gene suppression mediated by miRNA more precisely at the individual cell level, a fluorescent protein expression reporter system was constructed and used (FIG. 13). The fluorescent protein expression reporter system was formed to express fluorescent proteins with two colors in one vector. Here, a green fluorescent protein (GFP) was expressed in a SV40 promoter, several miRNA target sites were consecutively arranged at the 3'untranslated region (3' UTR), a miRNA-mediated gene inhibitory effect was measured by the intensity of the GFP, and the red fluorescent protein (RFP) was expressed in a TK promoter so that a GF signal was normalized to the RFP intensity and used (FIG. 13A, upper).

A reporter was formed by inserting a non-canonical target site (7G:A-site) which is complementary to the bases in positions 2 to 8 from the 5' end of miR-1 and has a G:A wobble at the 7^{th} base (G) into the fluorescent protein expression reporter vector constructed as described above, and then whether the reporter is suppressed by miR-1 was confirmed through an experiment. Here, in this experiment, 500 ng of the GFP-7G:A site reporter vector and 25 µM of miR-1 were co-transfected into a cardiomyocyte cell line (H9c2) using a Lipofectamine 2000 (Invitrogen) reagent according to the manufacturer's protocol, and 24 hours after transfection, each fluorescent signal was measured through flow cytometry using Attune NxT (Life Technology). As a result, it was confirmed that the 7G:A site of the miR-1 was very effectively suppressed by miR-1 expression (FIG. 13A, middle) as compared with cells into which the control nucleic acid (cont; NT) was transfected (FIG. 13A, left). That is, as a result of analyzing the degrees of the expression of two types of fluorescent proteins in the cardiomyocyte cell line (h9c2) in four parts (Q5-1, Q5-2, Q5-3 and Q5-4), in the miR-1-transfected cells, compared with the control NT-transfected cells, the distribution of cells showing the RFP at an intensity of 103 and the GFP at an intensity of 103 was reduced from 59.51% (control: Q5-3) to 41.80% (miR-1: Q5-3).

In the control, it was observed that the GFP-7G:A site reporter vector was inhibited to some extent even in the h9c2 cells without miR-1 transfection. It may be predicted that the inhibition is mediated by miR-1 previously expressed in the h9c2 cells through G:A wobble base pairing with the reporter target mRNA. To confirm this, a miRIDIAN microRNA hairpin inhibitor (miR-1 inhibitor, FIG. 13A, right) which can inhibit the miR-1 expression in the h9c2 cells was purchased from Dharmacon, and transfected into cells at 50 µM. As a result, compared with the control (FIG. 13A, left), when the miR-1 inhibitor was transfected (FIG. 13A, right), the distribution of cells showing the RFP at an intensity of 103 and the GFP at an intensity of 103 was increased to 81.56% (miR-1 inhibitor: Q5-3), confirming that the miR-1 present in the cells can inhibit gene expression by the 7G:A site of miR-1 (FIG. 13A).

This was confirmed by additionally transfecting each of a control (GFP-no site) in which a miRNA target site was not added to a fluorescent protein expression reporter and a miR-1 7G:A site-inserted reporter, that is, a GFP-7G:A site into the H9c2 cells and comparing their activities, and the result was quantitatively compared with the case of co-transfection with miR-1 as a positive control (FIG. 13B). For the comparative analysis, the RFP values measured by a flow cytometer were divided by section, and the GFP values were averaged and converted into a log ratio, and then the log ratio was relatively calculated as the GFP level of the control, that is, the GFP-no site, was set as 1 (relative log GFP). Here, when there is a site complementarily paired with the 7^{th} G base of the miR-1 through G:A wobble pairing (GFP-7G:A site), particularly, in the section showing RFP expression (log RFP) from 1.5 to 3, it was confirmed that GFP expression (relative log GFP) caused by a G:A wobble is reduced by approximately 10% from 1. Such inhibition was observed in the same manner as the pattern in which GFP expression (relative log GFP) was inhibited by approximately 40% to 10% in the positive control, particularly, in the section of RFP expression (log RFP) from 1.5 to 4.

To confirm that the result observed in the above example was not influenced by the intensities of different promoters used in the corresponding fluorescent protein reporter and the difference in fluorescent activity between two different fluorescent proteins, a fluorescent protein reporter, that is, p.UTA.3.0 (Lemus-Diaz N et al, Scientific Reports,(7), 2017), in which two fluorescent proteins were interchanged was purchased from Addgene (plasmid # 82447). Here, a reporter assay was conducted after constructing a reporter vector (RFP-7G:A site) by repeatedly inserting the miR-1-7G:A sequence five times into the 3' UTR of the RFP regulated by a SV40 promoter in a p.UTA.3.0 vector, and the RFP expression level was used by modifying a GFP value normalized to a degree of transfection into cells (FIG. 13C, upper). As a result, like the previous example, the suppression of miR-1 7G;A site by miR-1 expressed in h9c2 cells was confirmed by comparing the results with the control of a reporter having no miRNA target site (RFP-no site) and a miR-1 7G:A site-inserted reporter (RFP-7G:A site), and the same phenomenon as in the previous example was also observed by the co-transfection of the positive control and the miR-1 (FIGS. 13C and 13D)..

From the above, it was confirmed that the non-canonical target of miRNA found by Ago HITS-CLIP assay cannot only actually bind to the seed sequence of miRNA through G:A wobble pairing, but also inhibits the expression of the corresponding target gene. According to this, it is considered that the non-canonical target of miRNA is able to easily bind to the target site of miRNA through G:A wobble pairing, and if this can be similarly induced by miRNA-GU, only the biological function of miRNA mediated by the non-canonical target suppression will be used.

### [Example 14] Confirmation of function of inducing the hypertrophy of cardiomyocytes through regulation of non-canonical G:A wobble seed target gene at G2, G3 and G7 of miR-1

To confirm the possibility of regulating a biological function through the above-described non-canonical gene suppression by the G:A wobble base pair acting as a non-canonical target of miRNA by using miRNA-GU, an experiment was conducted with the miR-1 of a cardiomyocyte, which has been described in the previous example.

To investigate whether a non-canonical G:A wobble target has a specific biological function, miR-1 should recognize only a non-canonical G:A wobble pair target site, rather than a canonical target, and thus miRNA-GU prepared by substituting the corresponding G with U in the seed region of the miR-1 to be paired with A, not C, was used for the experiment. In this experiment, for physiological conditions more similar to the heart, primary rat neonatal cardiomyocytes derived from rat cardiac tissue were used. Here, for cardiomyocyte culture, cardiomyocytes were isolated from cardiac tissue of a neonatal rat (1 day after birth) through an enzyme reaction (Neomyt kit, Cellutron), and the cardiomyocytes were cultured in a 4:1 mixture of Dulbecco's modified Eagle's medium (Invitrogen) supplemented with 10% FBS (fetal bovine serum), 5% HS (horse serum), 100 U/ml penicillin and 100 (µg/ml streptomycin and M199 (WellGene). By adding brdU to the prepared media, the cells were cultured differently from other cells with cell cycles in cardiac tissue.

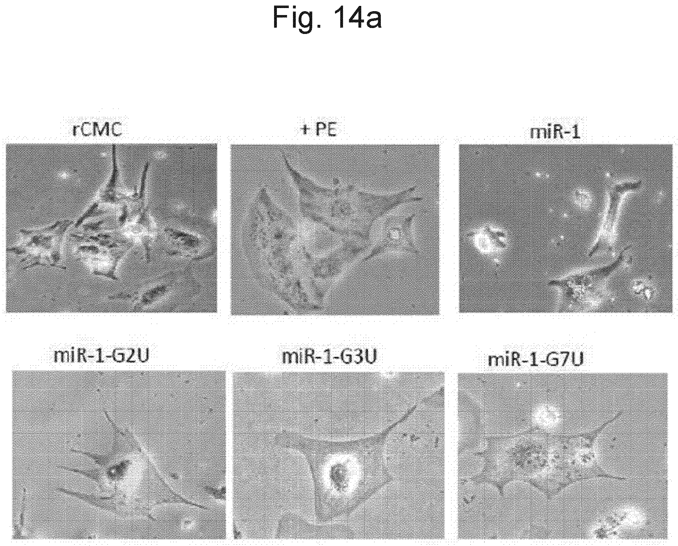

It was first confirmed whether myocardial hypertrophy is induced in the primarily cultured cardiomyocyte obtained as described above (FIG. 14A). Cardiomyocytes have a characteristic of being able to increase their size after cell cycle arrest, which is called myocardial hypertrophy. Myocardial hypertrophy is a heart disease model system whose cell morphology and gene expression profile change are well known, which plays a role in compensating for the deterioration in function of cardiomyocytes and is known as the intermediate physiological stage of a heart disease. In the myocardial hypertrophy cell model, when the cardiomyocytes were treated with an adrenergic receptor agonist such as phenylephrine (PE), myocardial hypertrophy may be induced (Molkentin, JD et al, 1998, Cell 93(2), 215-228). Accordingly, myocardial hypertrophy was induced by treating the cultured cardiomyocytes with 100 µM phenylephrine, and then compared with a control which is not treated at all (FIG. 14A, rCMC), thus the increase in size of the cardiomyocyte was morphologically confirmed (FIG. 14A, +PE). In addition, when miR-1 was transfected into the primary cardiomyocyte, a decrease in size of the cardiomyocyte was observed (FIG. 14A, upper right). This is concurrent with the well-known myocardial hypertrophy and the miR-1 function in cardiomyocytes (Molkentin, JD et al, 1998, Cell 93(2), 215-228, Ikeda S et al, Mol cell boil, 2009, vol29, 2193-2204).

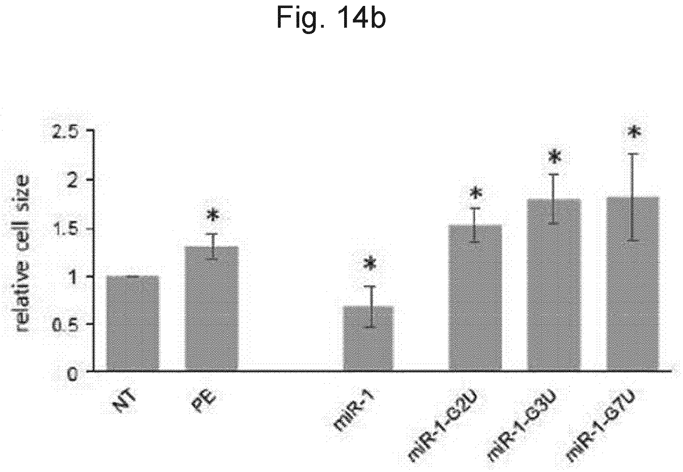

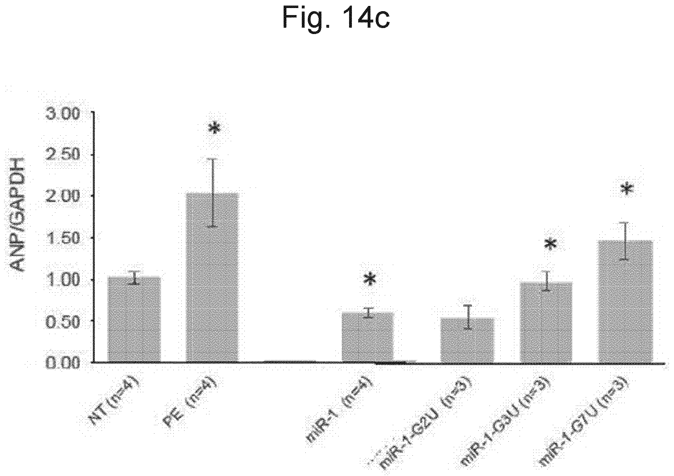

MiR-1 contains three Gs in the seed region, and therefore, to recognize only a non-canonical G:A wobble pair target site, not a canonical target, miR-1 in which G is substituted with U was designed and applied to the 2^{nd} position (miR-1-G2U), the 3^{rd} position (miR-1-G3U) and the 7^{th} position (miR-1-G7U) from the 5' end depending on where G is located. Here, the substituted base sequences of the miR-1 used in FIG. 14 are as follows (miR-1-G2U: 5'p-UUGAAUGUAAAGAAGUAUGUAU-3'; miR-1-G3U: 5'p-UGUAAUGUAAAGAAGUAUGUAU-3'; and miR-1-G7U: 5'p-UGGAAUUUAAAGAAGUAUGUAU-3'). These sequences were synthesized as a guide strand, and a passenger strand was prepared by being designed with reference to that of the conventional miR-1, chemically synthesized by Bioneer and separated by HPLC. The sequences were prepared in a duplex of a guide strand and a passenger strand according to the method provided by the manufacturer. The transfection of the corresponding miRNA into the primary cardiomyocyte cell line was performed using an RNAiMAX reagent (Invitrogen) at 50 (µM.

As a result, it was possible to observe that when miR-1-G2U, miR-1-G3U or miR-1 - G7U was transfected into the primary cardiomyocyte culture, the size of the cardiomyocytes was increased similar to the myocardial hypertrophy-induced cells (FIG. 14A, +PE). Here, to quantitatively analyze each cell size, 100 or more cells were quantified using the ImageJ program (NIH), and it was analyzed that all of the miR-1 substitutes (miR-1-G2U, miR-1-G3U and miR-1-G7U) synthesized to recognize only a G:A wobble pair site of miR-1 are 1.5 to 1.8-fold bigger than the case in which the control (NT) was transfected. This is a similar level to the size of cardiomyocytes in the phenylephrine (PE)-induced myocardial hypertrophy model, and miR-1 itself shows an effect of reducing cell size in terms of the morphology of the cardiomyocyte, showing that the canonical target inhibitory effect exhibits a function in cell morphology different from G:A wobble-mediated target suppression (FIG. 14B). In addition, to further confirm the myocardial hypertrophy observed in this example at the molecular level, the expression of an atrial naturiuretic peptide (ANP) gene known to be increased in expression as a marker was measured as a relative value, through quantitative polymerase chain reaction (qPCR), as compared with GAPDH (FIG. 14C). As a result, when the miR-1 substitutes (miR-1-G2U, miR-1-G3U and miR-1-G7U) synthesized to recognize only a G:A wobble pair site of the miR-1 was transfected into cardiomyocytes, it was possible to confirm through four repeated experiments that all of them significantly increased an ANP expression level approximately 1.2- to 1.5-fold compared to the control (NT). The increase in ANP expression is less than the increase in size of the phenylephrine (PE)-treated cardiomyocyte experimental group, which is approximately 2-fold higher than the control, which is, however, the opposite of the phenomenon in which ANP expression was significantly reduced when original miR-1 was transfected. This shows that miR-1 exhibits a completely different biological function due to a G:A wobble pair site.

From the above, it was finally seen that the non-canonical target suppressed by the G:A wobble pair in the miRNA seed region can exhibit a biologically different function from the conventional canonical target recognition. This finding indicates that the function caused by a canonical target of miRNA is different from the function caused by a non-canonical target thereof, and according to this, if only the G:A wobble target of the miRNA can be suppressed, only a biological function occurring due to the G:A wobble target will be selectively controlled.

### [Example 15] Observation of induction of myocardial hypertrophy through miR-133-G4U expression in cardiomyocyte cell line (H9c2)

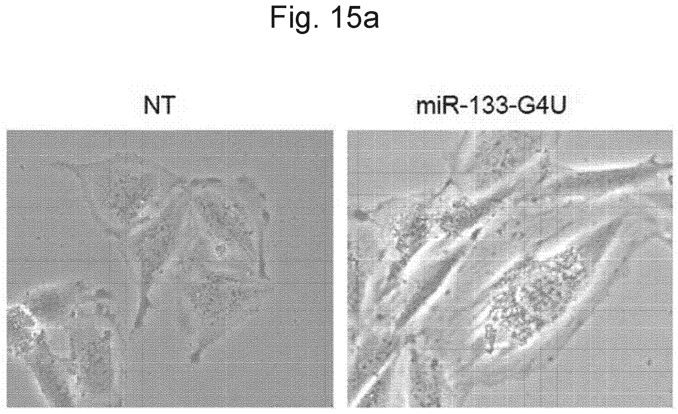

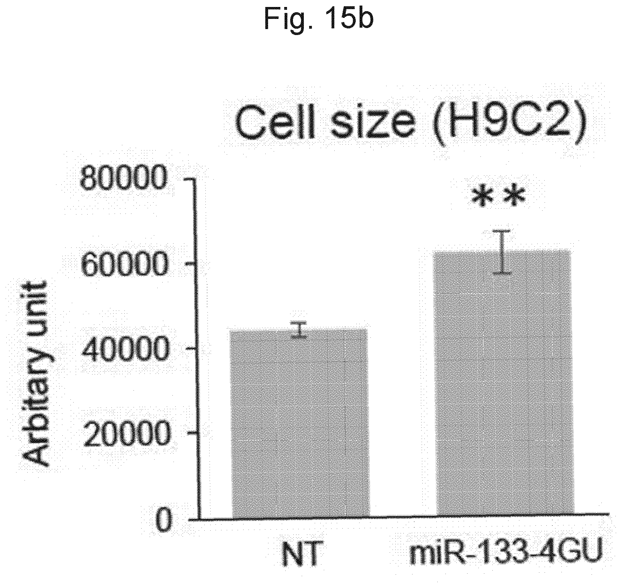

In terms of the non-canonical target phenomenon recognized by G:A wobble base pairing acting on the seed region of miRNA, to additionally confirm its biological function in cardiomyocytes, other than the miR-1 described in the previous example, miRNA-GU was applied to miR-133 known to function in cardiomyocytes. The miR-133 is known to have a function of regulating myocyte development and disease pathology and inhibiting myocardial hypertrophy (Nat Med. 2007, 13(5): 613-618; Ikeda S et al, Mol cell boil, 2009, vol29, 2193-2204). Therefore, the inventors transfected a 50 nM duplex prepared with miR-133-G4U (5'-UUUUGUCCCCUUCAACCAGCUG-3'), which is an interference-inducing nucleic acid specifically inhibiting target recognition through the G4 from the 5' end of a non-canonical GA wobble target site of the miR-133, synthesized by Bionia as described above into a cardiomyocyte cell line (h9c2), and observed a change in cell morphology (FIG. 15A). As a result, miR-133-G4U expression increased the size of the H9c2 cells compared with the control (NT) (FIG. 15A), and as a result of quantitatively analyzing the sizes of 100 or more cells using the ImageJ (NIH) program, it was possible to confirm that the cell size significantly approximately 2-fold increased (FIG. 15B).

Accordingly, it was possible to predict that the decrease in expression of the non-canonical G:A wobble seed target of miR-133, occurring by miR-133-G4U induces myocardial hypertrophy, which is a novel function different from the canonical seed target inhibitory function of miR-133 for inhibiting myocardial hypertrophy.

### [Example 16] Confirmation of effect of inhibiting expression of non-canonical GA wobble seed site gene by miR-122 in liver cancer cells using luciferase reporter

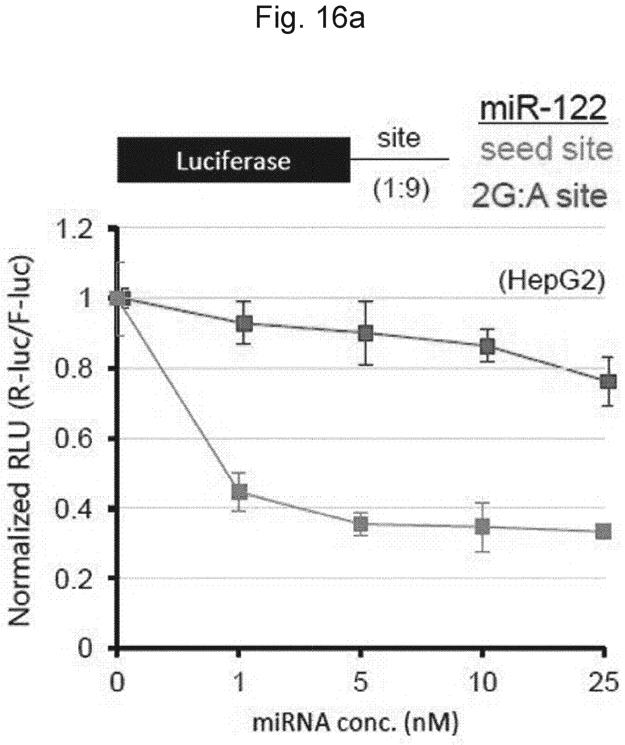

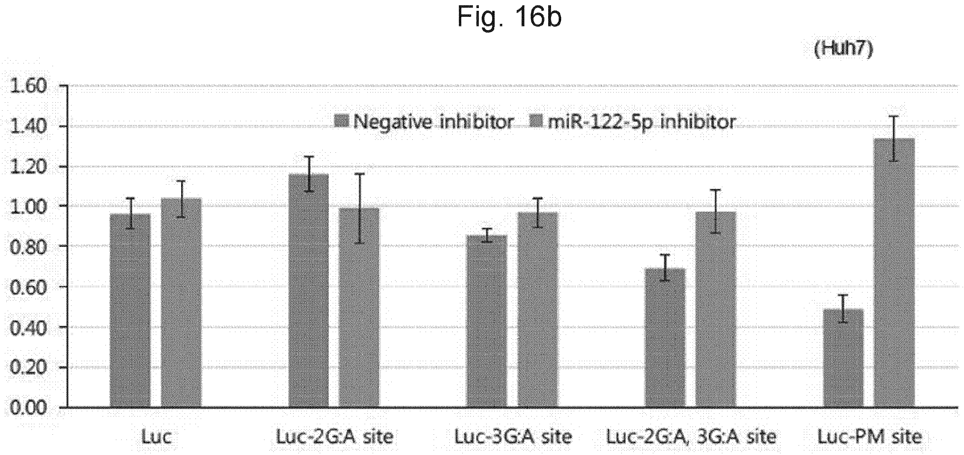

In Example 10 described above, from the finding that miRNA non-canonically binds with a G:A wobble pair seed site, miRNA-GU specifically recognizing only the non-canonical target binding was invented, and it was observed in Examples 11, 12, 13 or 14 that, when this was applied to miR-124, miR-1 or miR-133, an effect different from the conventional function was exhibited. In addition, to confirm whether the expression of a target gene having a non-canonical G:A wobble pair seed site in miRNA is able to be actually inhibited, the function in a myocardial tissue cell line was confirmed for miR-1 in Example 13. In addition, it was attempted to confirm whether the expression of a non-canonical G:A wobble seed target gene can be inhibited in miR-122 specifically expressed in liver tissue and liver cancer cells, using a luciferase reporter system (FIG. 16).

The miR-122 specifically expressed in liver cancer or liver tissue and thus functioning has Gs capable of making a G:A wobble pair in positions 2, 3, 5 and 7 from the 5' end of the seed sequence (the 1^{st} to 8^{th} bases from the 5'end: 5'-UGG AGU GU-3'). Therefore, first, in order to measure the inhibitory efficiency of the corresponding target site capable of being non-canonically recognized through G:A wobble pairing at G2, an inhibitory concentration 50 (IC50) was measured by the same method as described in Example 2 and compared with a canonical seed site (FIG. 16A).

To detect the inhibition of the expression of a non-canonical G:A wobble seed target enabling G:Abase pairing with G2 of miR-122, a luciferase reporter vector for an experiment was produced by introducing five sequentially arranged copies of a non-canonical G:A wobble seed site (2G:A) sequence (the 1^{st} to 9^{th} bases from the 5' end: 5'-CAC ACU CAA-3') for the G2 of miRNA-122 into the 3' untranslated region (3' UTR) of a *Renilla* luciferase gene in a psi-check2 (Promega) vector In addition, a luciferase reporter vector for a canonical seed site (the 1^{st} to 9^{th} bases from the 5' end: 5'-CAC ACU CCA-3') was simultaneously produced in the same manner as described above.

As a result of measuring an effect of inhibiting the expression of a target gene having a canonical seed site and a non-canonical G:A wobble seed site (2G:A site) at G2 from the 5' end at various concentrations of miR-122 by a change in activity of a corresponding luciferase reporter using the luciferase reporter vector produced as described above, and examining an IC50 value, the IC50 of the miR-122 was measured to be approximately 0.5 nM for a canonical seed target site, and to be approximately 7 nM for a non-canonical G:A wobble seed site (2G:A site), confirming that the miR-122 inhibits the expression of the gene having the non-canonical G:A wobble seed site (2G:A site), and showing that the efficiency is lower compared to the canonical seed site (FIG. 16A). In addition, when examining a concentration at which the inhibition of gene expression was initiated in a non-canonical G:A wobble seed target using a luciferase reporter, it was confirmed that luciferase enzyme activity is suppressed at 1.5 nM or more (FIG. 16A). This showed an approximately 2-fold difference in inhibitory efficiency from when the miR-122 inhibits the expression of a canonical seed target site.

This experiment was conducted by synthesizing a duplex of miR-122 in the same manner as used in this example, co-transfecting it with a corresponding psi-check2 vector (50 ng) at various concentrations (0, 1, 5, 10 and 25 nM) into approximately 10,000 liver cancer cells (Huh7, KCLB: 60104) according to the manufacturer's protocol using a Lipofectamine 2000 reagent (Invitrogen), and incubating the cells in a 96-well plate, and then luciferase activity was measured in the same manner as used in Example 2.

After confirming that miR-122 inhibits the expression of the non-canonical G:A wobble seed site gene, to confirm whether it is equally regulated by miR-122 present in cells, using a Huh7 cell line, which are liver cancer cells containing miR-122, a luciferase reporter assay was performed on a non-canonical G:A wobble seed site (2G:A) caused by G2 and the G3 from the 5' end (FIG. 16B). To perform this assay, additionally, a luciferase reporter vector (luc-3G:A site) for a non-canonical G:A wobble seed site (3G:A) caused by G3 from the 5' end, a luciferase reporter vector for a non-canonical G:A wobble seed site (luc-2G:A, 3G:A site) caused by G2 and G3 from the 5' end, and a luciferase reporter vector (luc-PM site) measuring a perfectly complementary sequence to the entire sequence of miR-122 were produced in the same manner as described above, and co-transfected with a luciferase reporter vector (luc-2G:A site) for the non-canonical G:A wobble seed site (2G:A) caused by G2 from the 5' end into a liver cancer cell line to measure luciferase activity (FIG. 16B).

As a result, when a luc-PM site vector was transfected into Huh7 cells known to have miR-122, it was observed that the activity was reduced by approximately 50% compared with the control into which only a luciferase vector was transfected, confirming that there is miR-122 in Huh7 cells, and simultaneously, the miR-122 suppresses the activity of a non-canonical G:A wobble seed target reporter (Luc-3G:A) at G3, and non-canonical G:A wobble seed target reporters (Luc-2G:A, 3G:A) at G2 and G3. In addition, to see whether the suppressive effect is induced by the miR-122, an miR-122 expression inhibitor (hsa-miR-122-5p inhibitor, IH-300591-06-0010) was purchased from Damacon and treated at 50 nM, confirming that all inhibitory phenomena disappeared.

According to the example, it was confirmed that the miR-122 is weaker than a canonical seed site, and can inhibit the expression of the non-canonical G:A wobble seed target caused by G2 from the 5' end, and miR-122 naturally present in liver cancer cells (Huh7) inhibits the expression of the non-canonical 3G:A wobble seed target gene and the non-canonical 2,3G:A wobble seed target gene based on the 5' end. Particularly, the inhibitory effect shown in the liver cancer cells (Huh7) disappears due to the treatment of the miR-122 expression inhibitor, which is regulated by miR-122 (FIG. 16B). Therefore, it can be seen that the miR-122 has a function of regulating the inhibition of the expression of a non-canonical G:A wobble seed target gene.

### [Example 17] Confirmation of phenomenon of inhibiting cell migration in liver cancer cell line through inhibition of expression of specific non-canonical G:A wobble seed target gene of miR-122

To examine the function of a non-canonical G:A wobble seed target of miR-122 in liver cancer cells, miRNA-GU complementarily recognizing and suppressing the corresponding non-canonical G:A wobble seed target site was applied to be transfected into liver cancer cells (hepG2), and then a wound healing assay was performed to examine how the cell migration ability among the properties of the liver cancer cells, which is important for cancer metastasis, changes.

The wound healing assay for the liver cell line (hepG2) was performed by first synthesizing miRNA-GU complementarily recognizing the non-canonical 2G:A wobble seed target, the non-canonical 3G:A wobble seed target, or the non-canonical 2,3G:A wobble seed target based on the 5' end of miR-122 with a corresponding sequence, transfecting the resulting sequence into the hepG2 cells in the same manner as described in the above-described example, making a scratch in a cell layer using a 1,000-µl tip 24 hours after culture, and incubating the cells until 48 hours and observing cell migration in an experimental group, and comparing with cells transfected with a control NT-6pi. The sequences of interference-inducing nucleic acids used in the assay are as follows: (miR-122: 5'-UG GAG UGU GAC AAU GGU GUU UG-3'; miR-122-G2U: 5'-UU GAG UGU GAC AAU GGU GUU UG-3'; miR-122-G3U: 5'-UG UAG UGU GAC AAU GGU GUU UG-3'; miR-122-G2,3U: 5'-UU UAG UGU GAC AAU GGU GUU UG-3').

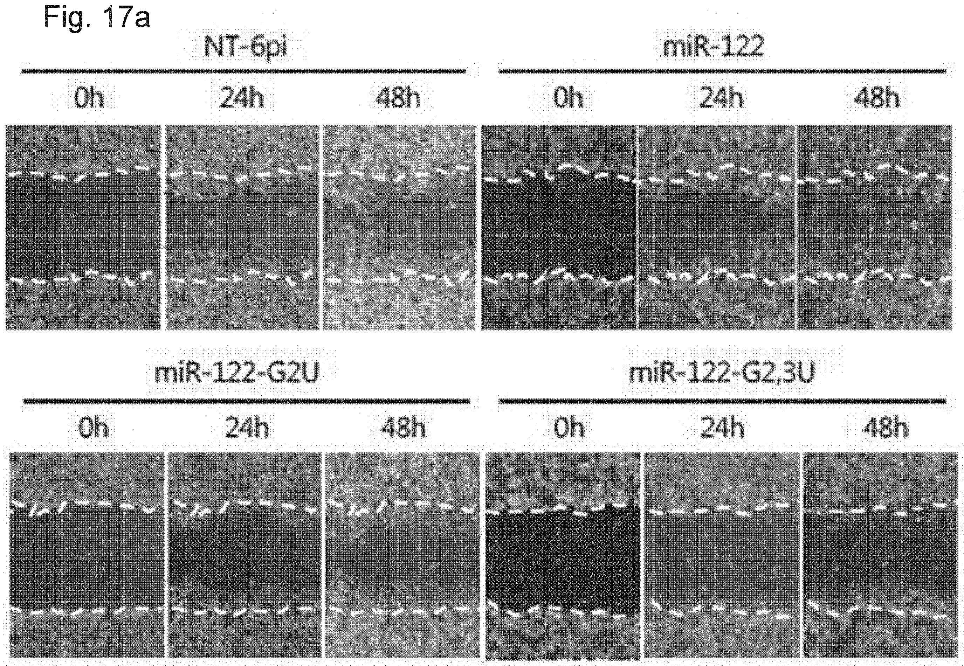

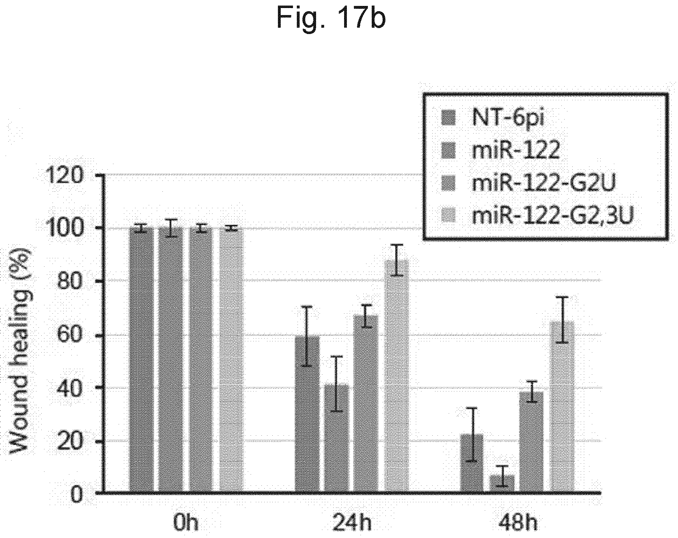

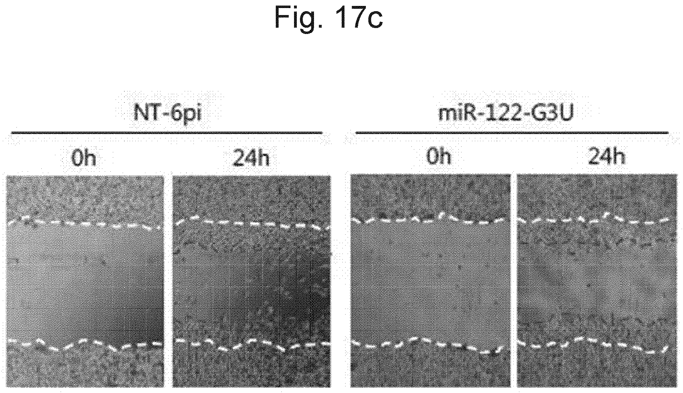

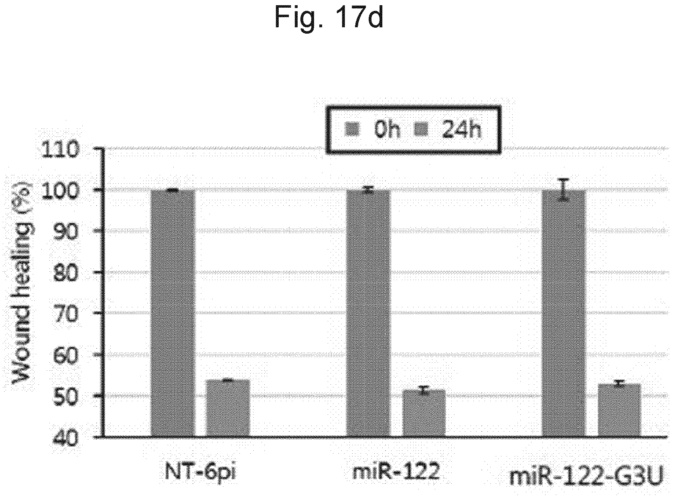

As a result, in the case of the control NT-6pi and the miR-122-transfected case, in 48-hour cell culture, it was possible to observe the migration of cells almost completely filling the scratch. However, in experimental groups transfected with miR-122-G2U and miR-122-G2,3U siRNA, it was seen that the cell migration is inhibited, and such an inhibitory phenomenon is most strongly shown in the miR-122-G2,3U siRNA-transfected experimental group (FIG. 17A). As quantitatively measured, in the cases of miR-122-G2U and miR-122-G2,3U siRNA, compared with the control, it was confirmed that the cell migration was inhibited 2- to 3-fold (FIG. 17B). The quantitative analysis was performed to observe the miR-122-mediated cell migration in terms of cell morphology, and the observation result was analyzed using the ImageJ program (NIH). Additionally, as a result of the experiment for the miR-122-G3U siRNA-induced cell migration, a cell migration inhibitory function by miR-122-G3U was not observed (FIG. 17C and 17D).

Based on this, it can be seen that the miR-122-mediated suppression of a non-canonical G:A wobble seed target inhibits the migration of liver cancer cells, indicating that G:A wobble at the 2^{nd} base preferentially acts for inhibition, and when the G:A wobble at the 3^{rd} base is added, the intensification of the cell migration inhibitory function is induced.

### [Example 18] Confirmation of cell cycle arrest by regulation of miR-122-G2,3U-mediated non-canonical G:A wobble seed target

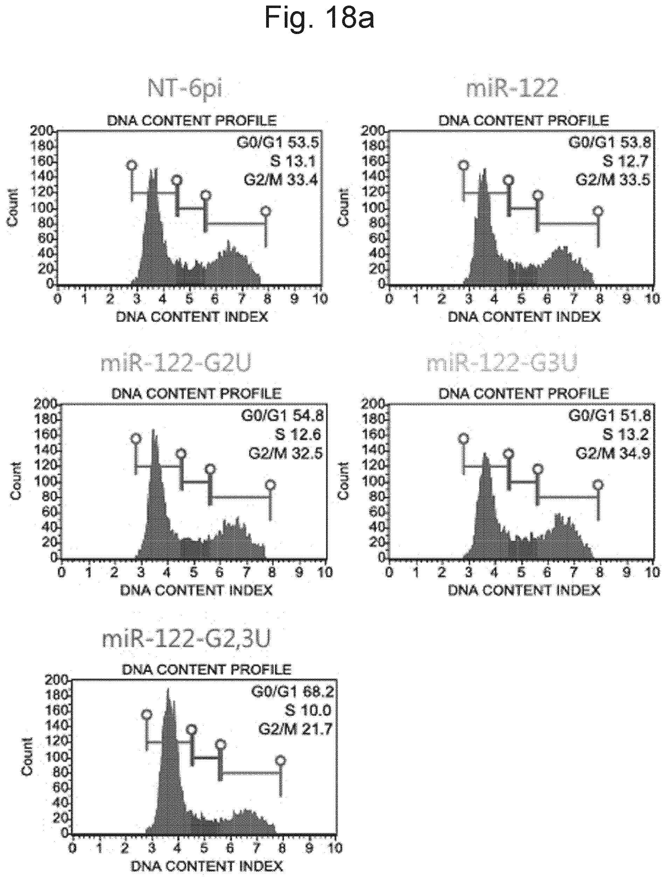

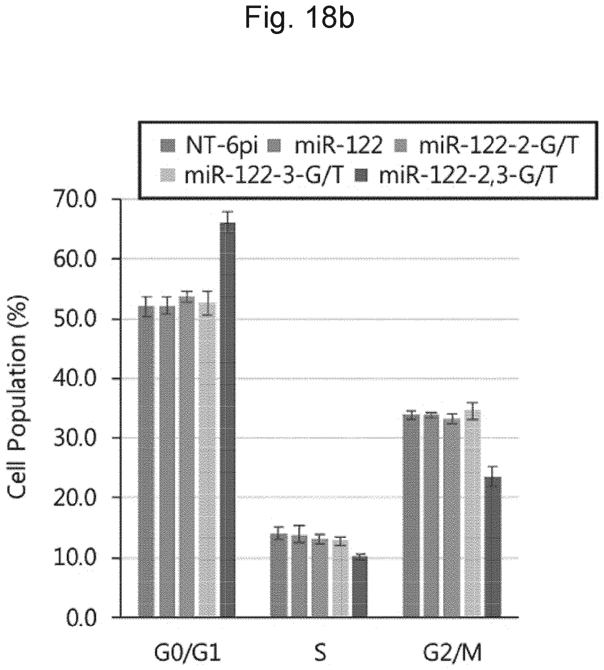

Cell migration is closely related to cell division, and widely observed, particularly, in cancer cells (Cancer research, 2004, 64(22):8420-8427). Accordingly, to confirm whether the miR-122-G2,3U-induced inhibition of liver cancer cell migration is related to the regulation of a cell cycle, flow cytometry was performed in the same manner as in Example 12. Here, as a result of measuring the cell cycle of each type of cells after NT-6pi, miR-122, miR-122-G2U, miR-122-3U and miR-122-G2,3U were delivered into the liver cancer cell line (HepG2), in the miR-122-G2,3U-transfected experimental group, it was confirmed that the ratio of the cells in the G0/G1 phase is increased by 52% to 68% on average compared with the control (NP-6pi), and the G2/M-phase cell distribution is significantly low (FIG. 18). However, in the cases of miR-122, miR-122-G2U and miR-122-G3U, compared with the control, significant differences were not observed. Here, the cell cycle analysis was performed by delivering the synthesized corresponding siRNA duplex into HepG at 50 nM and incubating for 24 hours. The analysis was conducted according to the manufacturer's protocol with a Muse Cell Cycle Kit (Catalog No. MCH100106, Millipore) and a Muse Cell Analyzer (Millipore).

Accordingly, it was confirmed that the cancer cell function regulated by miR-122-G2,3U reduces cell division (G2/M), and induces cell cycle arrest (G0/G1), and the result of the example may be interpreted as miR-122 recognizes non-canonical GA wobble seed sites through G:A wobble pairing at both of G2 and G3 based on the 5' end, and exhibits the function of preventing the progression of a liver cancer cell cycle.

### [Example 19] Observation of inducing cell cycle arrest by regulation of miR-122-G2,3U, miR-122-G2,7U-induced non-canonical G:A wobble seed targets

The miR-122 function at a non-canonical G:A wobble seed target identified that miR-122-G2,3U has a function of inhibiting liver cell migration in Example 17, and particularly, it was observed that the inhibition of cell migration induced by the expression of the miR-122-G2,3U is maximized when a regulatory effect of G3 is added to the biological function of the 2^{nd} base for regulating a non-canonical G:A wobble seed target (FIG. 17). Accordingly, to examine whether the biological function of suppressing a non-canonical G:A wobble seed target is changed with an additional combination of G2, G3, and G7 in miR-122, an experiment for additionally confirming a cell cycle regulatory function of miR-122-G7U, miR-122-G3,7U or miR-122-G2,7U siRNA.

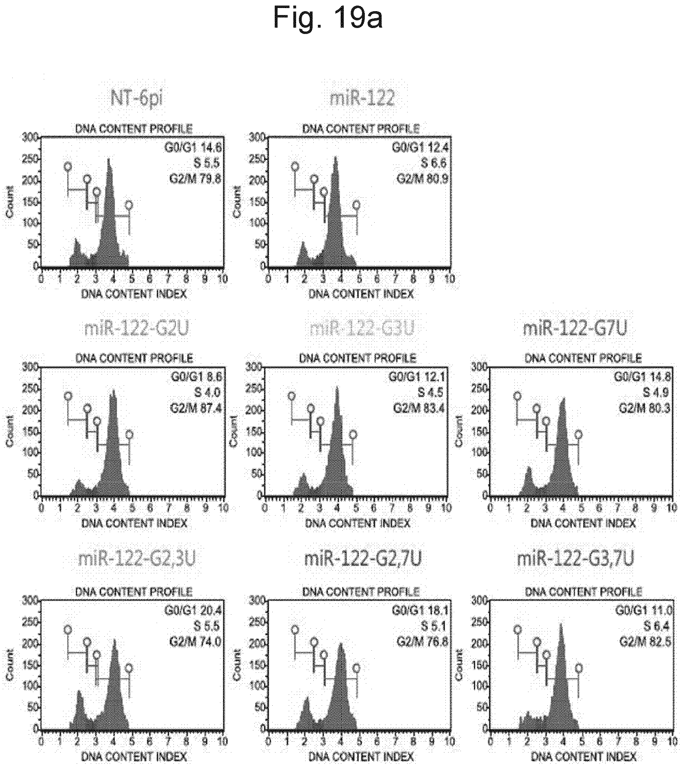

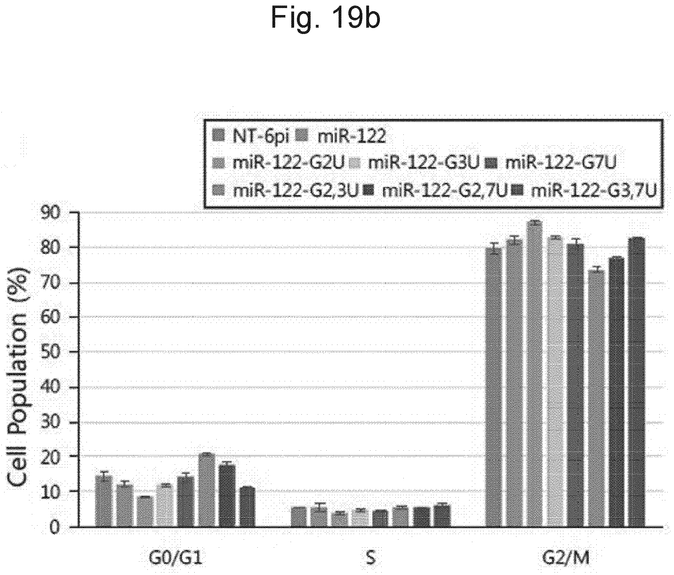

Here, for the experiment, a liver cancer cell line (HepG2) was used in the same manner as described above, and a corresponding miRNA-GU duplex was prepared in the same manner as in Example 18 above and transfected into the cells at 50 nM. However, in this cell cycle observation, after 24-hour culture, the cells were treated with 100 mg/mL of nocodazole for 16 hours, HepG2 cells at the G2/M phase (division preparation phase/division phase) was synchronized, and the amount of cells in a cell cycle arrest state at G0/G1 was measured using a Muse Cell Analyzer (Millipore) according to how many cells are arrested at G2/M (FIG. 19).

As a result, it was possible to confirm that, in the case of miR-122-G2U, compared with the control NT-6pi, there was no difference in number of cells at G0/G1, which is a cell cycle arrest state, but in the case of miR-122-G2,3U siRNA and miR-122-G2,7U siRNA, when a non-canonical G:A wobble seed target site was regulated in addition to G2, the proportion of the cells in the cell cycle arrest state (GO/G1) increased (FIG. 19A). However, miR-122-G3,7U did not exhibit a significant increase (FIG. 19A). According to quantitative analysis, in the miR-122-G2,3U siRNA-transfected experimental group, compared with the control, the proportion of the cells in a cell cycle arrest state (G0/G1) increased approximately 2-fold or more, and in the case of the miR-122-G2,7U experimental group, it was observed that the proportion of the cells in a cell cycle arrest state (G0/G1) increased approximately 1.5-fold (FIG. 19B).

Based on the above, it was confirmed that cell cycle arrest can be increased through the regulation of non-canonical G:A wobble seed targets mediated by miR-122-G2,3U siRNA and miR-122-G2,7U siRNA.

### [Example 20] Observation of induction of cell cycle arrest induced by regulation of non-canonical G:A wobble seed targets mediated by let-7a-G2U and let-7a-G2,4U

As a representative miRNA having a tumor suppressor function, there is the let-7 family. Since a function of regulating the development process of C. elegans has been reported (Nature, 2000, 403(6772): 901-906), and expression suppressed in various cancer cells (Cancer Res., 2004, 64(11): 3753-3756) and an anticancer function through the regulation of tumorigenesis (Cell, 2005, 120(5): 635-647; Genes Dev. 2007, 21(9): 1025-1030) have been reported, based on this, the let-7 family has been studied as significant genes having potential for cancer diagnosis and development of an anticancer agent. Accordingly, the present inventors conducted an experiment to examine the biological function induced by the suppression of the non-canonical G:A wobble seed target of let-7.

The seed sequence from the 1^{st} to 9^{th} bases from the 5' end of let-7a is 5'-UGA GGU AGU-3', Gs capable of making G:A wobble base pairing are present in positions 2,4, 5, and 8. The inventors focused on G2 and G4, and thus conducted an experiment in the same manner as in Example 19 above, modified them into miRNA-GU to synthesize a sequence, transfected the sequence into a liver cancer cell line, that is, HepG2 cells, and then examined how a cell cycle is affected. The base sequences for the let-7 synthesized and used in the experiment are as follows: (let-7a: 5'-U GAG GUA GUA GGU UGU AUA G UU-3'; let-7a-G2U: 5'-U UAG GUA GUA GGU UGU AUA G UU-3'; let-7a-G4U: 5'-U GAU GUA GUA GGU UGU AUAG UU-3'; let-7a-G2,4U: 5'-U UAU GUA GUA GGU UGU AUAGUU-3').

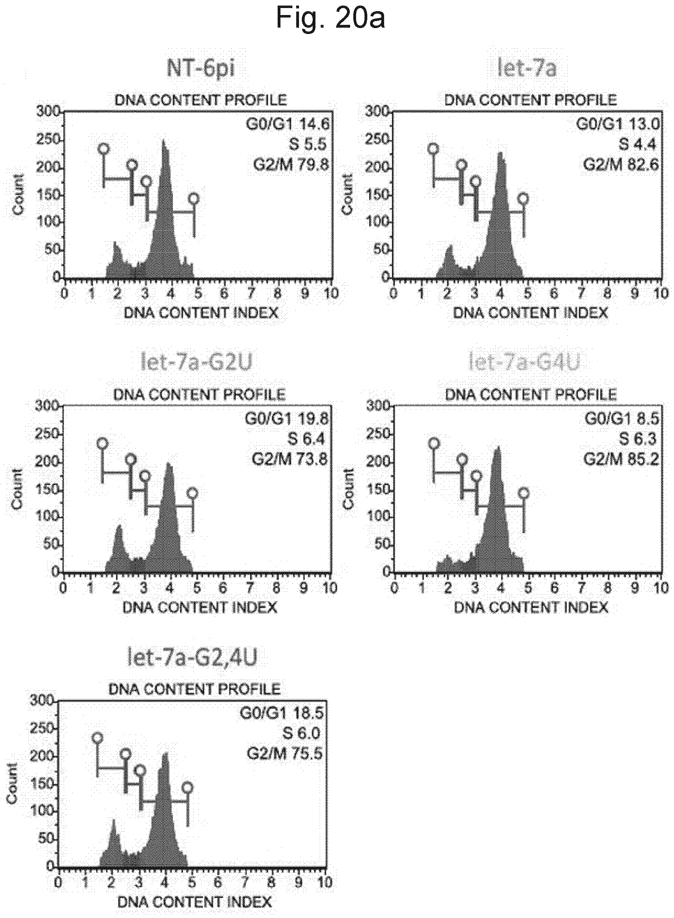

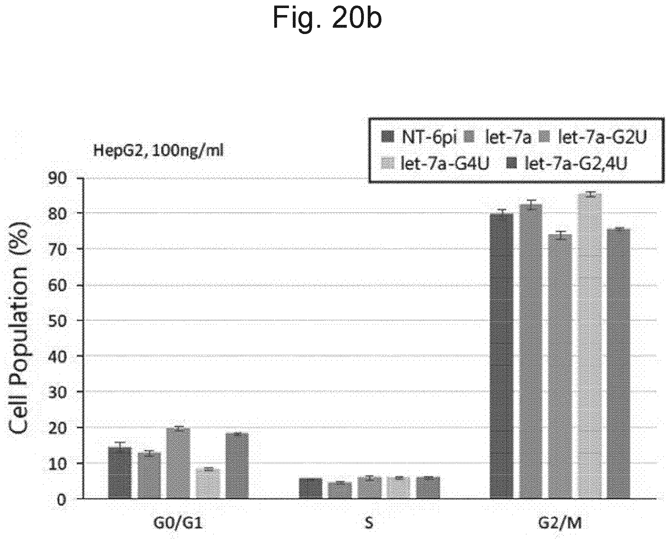

As a result, it was observed that, in the case of let-7a, compared with the control NT-6pi, there was no significant difference, but let-7a-G2U and let-7a-G2,4U increased cell cycle arrest at G0/G1, and let-7-G4U even increased the amount of cells at G2/M to make the cell cycle faster (FIG. 20A). Through quantification of the results of four-repeated experiments, it was possible to observe that, in the let-7a-G2U experimental group, compared with the control (Nt-6pi), the distribution of G1/G0-phase cells increased approximately 1.5-fold, and the distribution of G2/M-phase cells is reduced (FIG. 20B). Accordingly, it was possible to confirm that the inhibition of the expression of a non-canonical 2G:A wobble seed target of let-7a induces cell cycle arrest of the HepG2 cells. Subsequently, in the case of G4 present in the seed of let-7a, through a single GA wobble (let-7a-G4U), the induction of cell cycle arrest of HepG2 cells was reduced, but the cell cycle proceeded rapidly to the G2/M phase, resulting in a large number of cells remaining by the treatment with a nocodazole drug. In addition, when a G:A wobble was made at both G2 and G4 (let-7a-G2,4U), it was possible to observe the induction of the cell cycle arrest of HepG2 cells . Such cell cycle arrest was not observed in let-7a-transfected HepG2 cells (FIG. 20B). Therefore, the inhibition of the expression of non-canonical G:A wobble seed targets at the G2, and G2 and G4 of let-7a induced the cell cycle arrest of HepG2 cells, confirming that let-7a has a completely different function from the functions shown by the regulation of HepG2 cells performed by the inventors.

According to the example, let-7 serves to promote cancer cell cycle arrest by suppressing the gene of a non-canonical GA seed site at G2 based on the 5' end, and it can be seen that, more preferably, when both G2 and G4 are involved in G:A wobble pairing, its effect is the largest. In addition, as the non-canonical GA seed site through G4 based on the 5' end of let-7 promotes the progression of the cell cycle of cancer cells, it is considered that the proliferation of cancer cells will be induced.

### [Example 21] Observation that dedifferentiation induced by suppression of non-canonical G:A wobble seed target mediated is promoted by miR-302-4GU and miR-372-4GU, using OCT4 promoter reporter

Differentiated cells may acquire differentiation ability again by artificial expression of several transcription factors, and cells finally acquiring pluripotent differentiation ability again, such as embryonic cells, are referred to as induced pluripotent stem cells. It was reported that this technique can produce inducible pluripotent stem cells (iPSs) having differentiation diversity such as stem cells by transfecting four factors (Oct3/4, Sox2, c-Myc, Klf4) into mouse embryonic fibroblasts (MEM) (Cell, 2006,126 (4): 663-676). Similarly, it has been reported that iPSs can be made by delivering four factors (OCT4, SOX2, NANOG, and LIN28) into human somatic cells (Science,2007, 318(5858): 1917-1920). This is a field with very high applicability in that, through dedifferentiation, the possibility of regeneration that allows pluripotent differentiation can be created by any cells, including human cells, and after the first finding, there has been a consistent effort to increase iPS production efficiency. As part of this, as one of the methods for efficiently inducing pluripotency with MiRNA-induced pluripotent stem cells (mirPSs) reported, a function of dedifferentiating cells with miRNA such as miR-302a or miR-372 alone or in combination with four factors (Oct3/4, Sox2, c-Myc and Klf4) was reported (RNA, 2008 14(10): 2115-2124; Nat Biotechnol. 2011, 29 (5): 443-448). Therefore, the inventors conducted an experiment to examine whether the inhibition of the expression of non-canonical G:A wobble seed targets of miR-302a and miR-372 can promote a process of dedifferentiating cells.

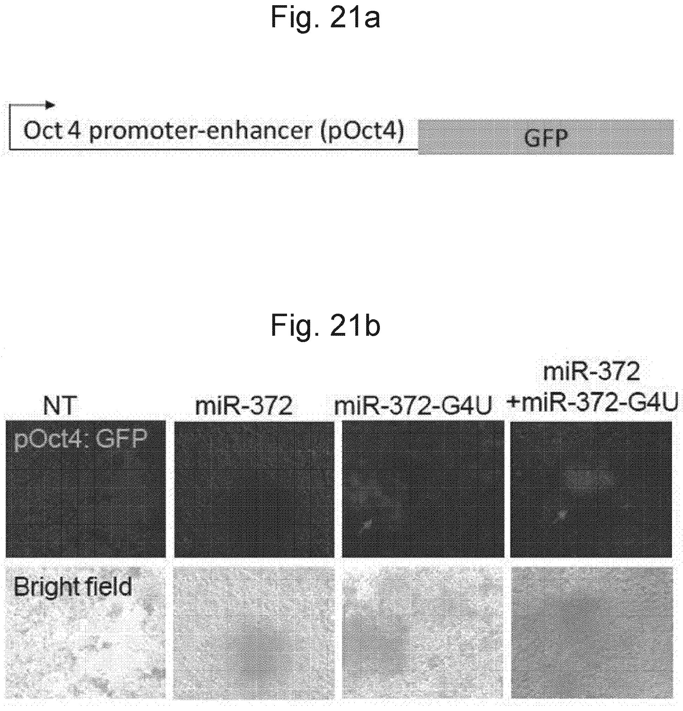

First, to monitor pluripotency induction, a Oct4 promoter reported to be activated in stem cells and a GFP-containing plasmid expressed depending on the promoter were purchased (Addgene #21319) (FIG. 21A). When such a Oct4 expression reporter vector (pOct4:GFP) is transfected into the differentiated cervical cancer cells (HeLa), GFP is not expressed, whereas when the corresponding cells are dedifferentiated to obtain differentiation ability, GFP is expressed in a Oct4 expression reporter. An experiment was performed on miRNAs such as miR-302a and miR-372, known to cause dedifferentiation, with the Oct4 expression reporter vector. In the seed sequences of miR-302a and miR-372, the sequence of the 2^{nd} to 8^{th} bases from the 5' end is "AA GUG CU", and thus the non-canonical G:A wobble seed can be made at G4 and G6 from the 5' end. However, the inventors conducted an experiment by synthesizing miR-302-G4U or miR-372-G4U, focusing on G4.

First, the experiment was conducted by delivering an Oct4 expression reporter vector (pOct4:GFP) into HeLa cells using a Lipofectamine 2000 reagent (Invitrogen) according to the manufacturer's protocol, synthesizing a guide strand and a passenger strand according to each of human miR-302 and miR-372 sequences in Bionia as described in the above example to prepare a duplex, and sequentially delivering the duplexes at 50 nM using an RNAiMAX reagent (Invitrogen). In addition, siRNAs specific for the non-canonical G:A wobble seed targets of miR-302 and miR-372 were prepared by substituting a G base with U in the seed in the same manner as in the above example, and then delivered into cells at 50 nM. The base sequences of the nucleic acids used herein are as follows: (miR-302: 5'-UAAGUGCUUCCAUGUUUUGGUGA-3'; miR-302-4GU: 5'-UAAUUGCUU CCAUGUUUUGGUGA-3'; miR-302 passenger strand: 5'-ACUUAAACGUGGAUGUACUUGCU-3'; miR-372: 5'-AAAGUGCUGCG ACAUUUGAGCGU-3'; miR-372-G4U: 5'- AAAUUGC UGCGACA UUUGAGCGU-3', and miR-372 passenger strand: 5'-CCUCAAAUGUGGAGCACUAUUCU-3'). HeLa cells into which a reporter vector and RNA were transfected were incubated in Dulbecco's modified Eagle's medium (Invitrogen) supplemented with 10% FBS, 100 U/ml penicillin, and 100 (µg/ml streptomycin for 14 days, and during transfection, incubated in an antibiotic-free complete medium.

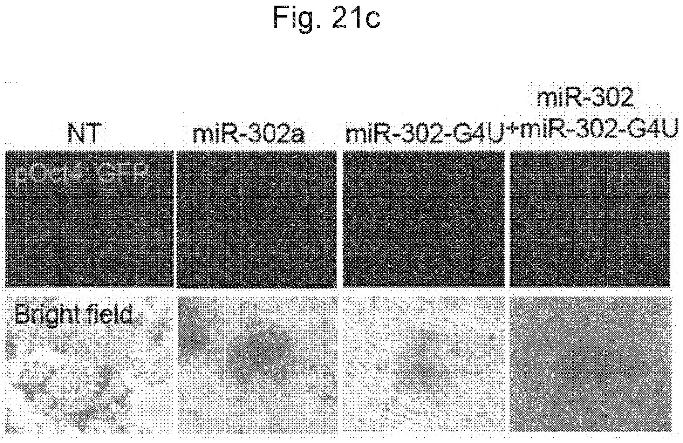

As a result, in the experimental group into which miR-302 or miR-372 was delivered independently, compared with the control, colony-forming cell growth was shown, but GFP expression showing the activity of the Oct4 promoter was not observed until the period of 14-day culture. However, in the experimental groups into which siRNAs in which the non-canonical G:A wobble seed targets of miR-302 and miR-372 are suppressed were transfected, GFP expression exhibiting colony-forming cell growth as well as the activity of the Oct4 promoter was observed (FIGS. 21B and 21C, red arrows), and when siRNAs suppressing miR-302 and the non-canonical G:A wobble seed target thereof and miR-372 and the non-canonical G:A wobble seed target thereof were simultaneously delivered into cells, more intensive and larger colonies of GFP-expressing cells were able to be observed (FIGS. 21B and 21C). Based on this, it was able to be seen that the suppression of the non-canonical G:A wobble seed targets of miR-302 and miR-372 may promote dedifferentiation of cells and acquisition of differentiation ability through Oct4 expression.

According to this example, it was possible to observe that miR-372-G4U and miR-302a-G4U may promote the dedifferentiation of cells more efficiently than when the induction of the dedifferentiation of cells is caused by expressing conventional miR-372 and miR-302 alone, , and therefore, it was able to be seen that the miR-372-G4U and the miR-302a-G4U may be used as materials for promoting the dedifferentiation of cells.

### [Example 22] Confirmation of phenomenon in which 2' OMe modification at 6^{th} base based on 5' end does not suppress non-canonical nucleation bulge target of corresponding RNA interference derivative

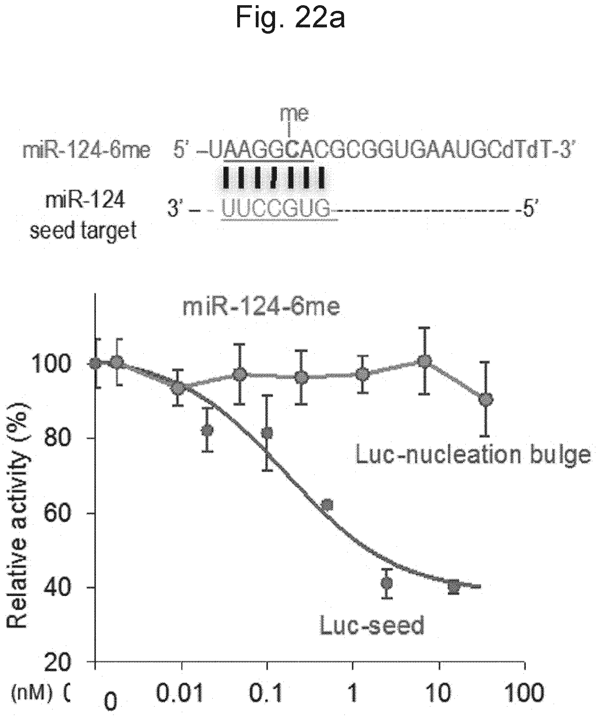

In the above-described example, a non-canonical nucleation bulge target had a novel biological function completely different from that of a canonical seed target, and thus miRNA-BS, which is an RNA interference-inducing nucleic acid, regulating only the inhibition of the expression of a non-canonical nucleation bulge target was invented, and its function was then confirmed. However, in the case of miRNA-BS, it was possible to confirm that, in order to complementarily arrange a non-canonical nucleation bulge site of the conventional miRNA, its seed sequence should be modified, but a novel nucleation bulge site may be generated again through the modified seed sequence. Accordingly, it is known that there is a need for technology in which an RNA interference-induced nucleic acid such as miRNA-BS recognizes only a canonical seed sequence, but does not recognize a non-canonical nucleation bulge site. Therefore, the inventors conducted an experiment to reduce the pairing strength at position 6, focusing on that the base in position 6 based on the 5' end is important in the seed sequence for non-canonical nucleation bulge pairing, and the degree of non-canonical nucleation bulge pairing may vary depending on the pairing strength of the base in position 6. That is, a methyl group (2' OMe) was added at the 2' position of the ribosyl ring of the 6^{th} nucleotide of miR-124 to modify the miR-124, and it was observed by a luciferase reporter assay whether the modified interference-inducing nucleic acid (miR-124-6me) has the function of suppressing a non-canonical nucleation bulge target of miR-124. The 2'OMe-modified miR-124 was prepared through synthesis (Bionia), and a luciferase reporter assay was conducted in the same manner as in Example 2 to measure gene inhibitory efficiency by IC50 (FIG. 22A). The luciferase reporter vector used herein was used for a canonical seed site (Luc-seed) and a non-canonical nucleation bulge site (Luc-nucleation bulge) of miR-124.

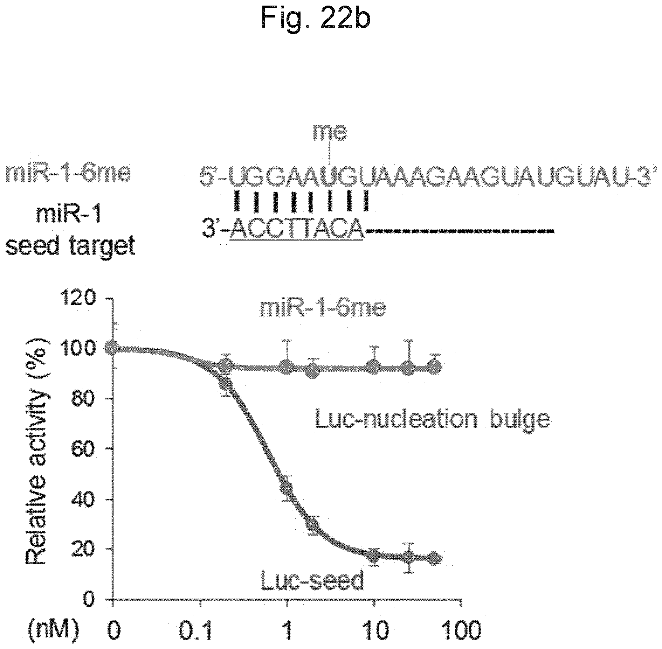

As a result, it was observed that an inhibitory effect on the non-canonical bulge target gene of miR-124 shown in Example 2 disappeared, whereas the inhibition of gene expression through the canonical seed site still exhibits an excellent inhibitory effect, exhibiting an IC50 of 0.3 nM, even when 2' OMe was added at the 6^{th} nucleotide (FIG. 22A). Additionally, when 2'OMe was added to the 6^{th} nucleotide of miR-1 (miR-1-6me), which is miRNA different from miR-124, in the same manner as described above, and a gene expression inhibitory effect on a target was examined using a luciferase vector including a canonical seed site (Luc-seed) and non-canonical nucleation bulge site (Luc-nucleation bulge) of miR-1 (FIG. 22B), like the result of the miR-124, the inhibitory effect on a miR-1-mediated non-canonical bulge target gene disappeared, whereas it was possible to confirm that the gene expression inhibition through a canonical seed site was excellent, an IC50 of 0.7 nM is shown. According to the example, when 2'OMe modification was applied to the 6^{th} nucleotide based on the 5' end of the RNA interference-inducing nucleic acid, it was possible to confirm that the inhibitory effect on the expression of a canonical seed target gene induced by the corresponding RNA interference-inducing nucleic acid is maintained, and the inhibition of the expression of a non-canonical nucleation bulge target gene disappears.

### [Example 23] Confirmation that 2' OMe modification at 6^{th} position based on 5' end does not suppress total non-canonical nucleation bulge target mRNA in transcriptome through RNA-Seq analysis

In Example 22, it was confirmed that when a 2'OM modification is applied to the 6^{th} nucleotide of miR-1, the inhibitory effect of the expression of a (miR-1-6me) canonical seed target gene is maintained, and the inhibition of the expression of a non-canonical nucleation bulge target gene is reduced. Accordingly, to confirm whether the miR-1-6me invented by the inventors, actually reduces the function of suppressing the non-canonical bulge target gene of miR-1 in a transcriptome of a cardiomyocyte cell line (h9c2) in which all genes actually related to the miR-1 function are expressed, the miR-1 and the miR-1-6me were transfected into h9c2 cells, and then subjected to RNA-Seq analysis. The RNA-Seq analysis was performed in the same manner as in Example 7 using the 6^{th} base based on the 5' end modified through abasic substitution (NT-6pi) in a cel-miR-67 sequence, which is miRNA of C. elegans as an experimental control. Here, an RNA-Seq library was prepared using a SENSE Total RNA-Seq Library Kit (Lexogen), and sequenced using MiniSeq (Illumina).

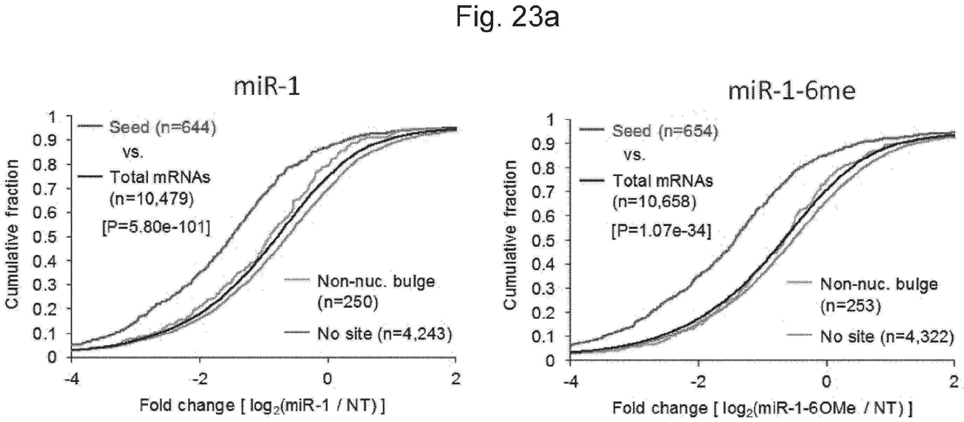

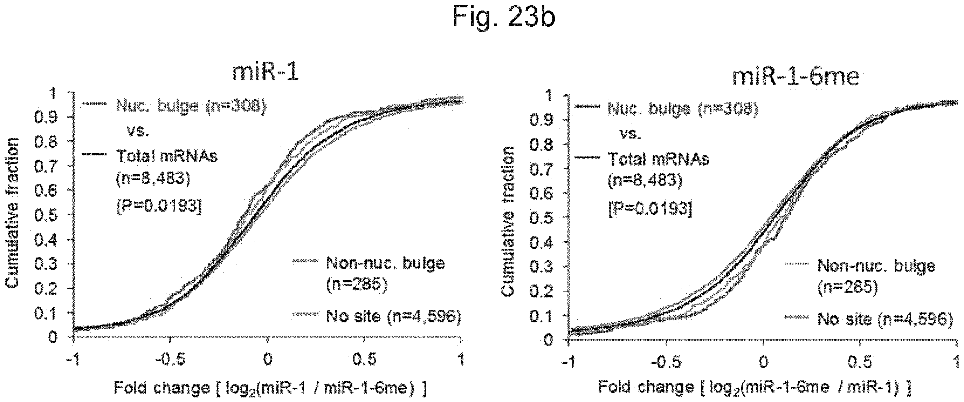

Afterward, the sequence data, FASTAQ file, obtained by the analysis was mapped to a mouse genomic sequence (m6) with the TopHat2 program, an expression value (FPKM) was calculated by Cufflink and Cuffdiff programs, normalized to the result in h9c2 cells into which the control NT-6pi was transfected and then expressed as a log2 ratio (fold change) for analysis. Here, to analyze the RNA-Seq result to confirm whether the amount of mRNA of a gene with a miRNA target site is inhibited by the expression of corresponding miRNA, a gene having the canonical seed site (7mer base-paired with a sequence from the 2^{nd} to 8^{th} bases from the 5' end) of miR-1 in the 3' UTR was selected, and the profile results were comparatively analyzed with a cumulative fraction in order of corresponding miRNA expression-dependent inhibition (FIG. 23A). As a result, it was confirmed that both miR-1 and miR-1-6me can well suppress a gene (seed) having the canonical seed site of miR-1 in the 3' UTR, compared with the total gene (mRNA) distribution. Afterward, as the gene having the non-canonical bulge site (nuc, 7mer) of miR-1 in the 3' UTR was also analyzed with a cumulative fraction in the same manner as described above, whether the inhibition of the expression of the non-canonical nucleation bulge target gene of miR-1 was reduced was analyzed by relatively comparing the amounts of corresponding target mRNA in miR-1-transfecetd cells and miR-1-6me-transfected cells (FIG.23B). As a result, in miR-1, when compared to the expression in miR-1-6me, it was observed that the non-canonical nucleation bulge target gene is still suppressed significantly when relatively compared with total mRNA, confirming that the inhibition of the expression of the corresponding non-canonical nucleation bulge target gene is reduced in miR-1-6me.

According to the result of the above-described example, it was able to be seen that conventional miRNA at the transcriptome level efficiently suppressed a non-canonical bulge target gene with the conventional canonical seed target gene, but miRNA in which a 2'OMe modification was applied to the 6^{th} nucleotide (miRNA-6me) still suppresses a canonical seed target gene, but not a non-canonical bulge target gene. Accordingly, while maintaining the original intention to specifically suppress only the non-canonical nucleation bulge site of corresponding miRNA by applying the 2'OMe modification applied to the 6^{th} base from the 5' end to miRNA-BS, its side effects can be minimized by completely eliminating non-canonical nucleation bulge pairing that may newly appear.

### [Example 24] Identification of miRNA binding to non-canonical nucleation bulge site through Ago HITS CLIP assay

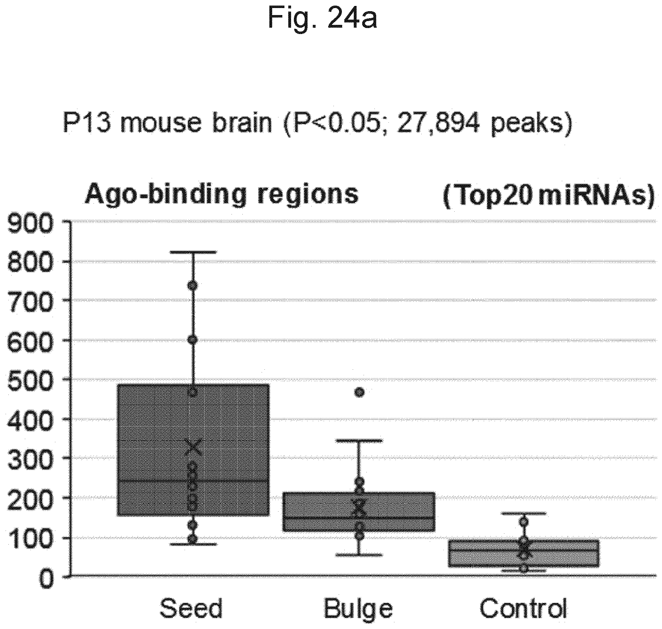

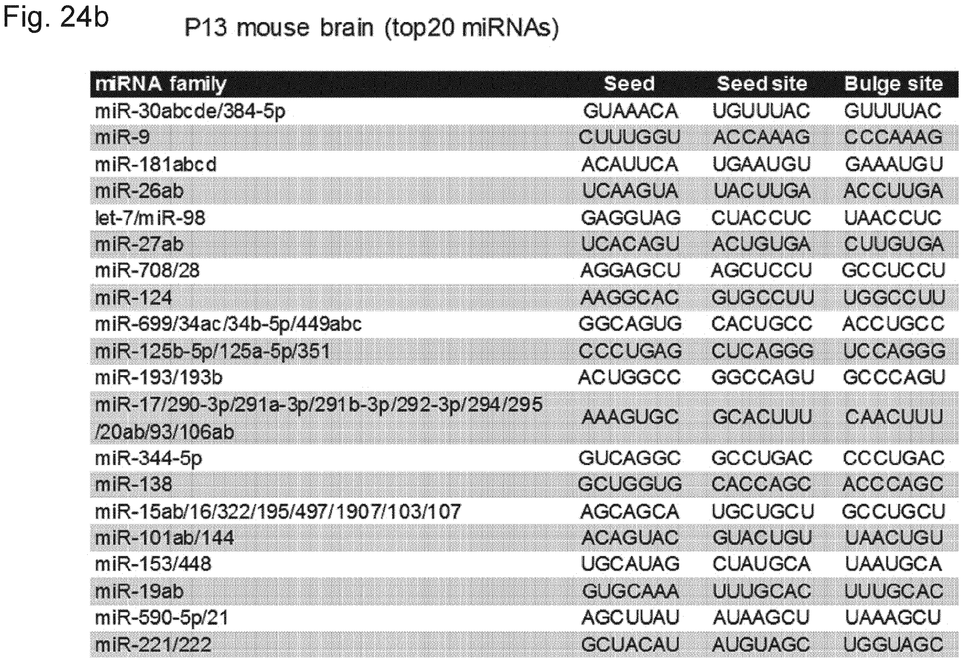

To identify miRNA to which the invention for specifically recognizing and suppressing a non-canonical nucleation bulge site by miRNA can be applied, by analyzing the result by a method of analyzing a miRNA target at the transcriptome level, that is, Ago HITS CLIP assay, miRNA binding to the non-canonical nucleation bulge site was identified. First, Ago HITS-CLIP data obtained from the cerebral cortex of a mouse 13 days after birth (p13) was sequenced in the same manner as in Example 10, confirming that the top 20 expressed miRNAs binding with Argonaute bind with the non-canonical nucleation bulge site of the target mRNA (FIG. 24A). Therefore, when the corresponding miRNA (FIG. 24B) recognizes target mRNA through a base sequence in the seed region, it was able to be seen that the miRNA can bind with target mRNA by canonical base pairing through the seed part as well as a non-canonical nucleation bulge site.

Expanding mouse Ago HITS-CLIP data analysis of the example, other Ago HITS-CLIP results obtained from human brain and cardiac tissues (Boudreau RL et al, 2014, Neuron, 81(2) 294-305, Spengler RM et al, 2016, Nucleic Acids Res, 44(15) 7120-7131) were analyzed in the same manner as in the above-described example, and the frequencies of the canonical seed site and non-canonical nucleation bulge site at which Ago and the corresponding miRNA interacted were calculated (human brain miRNA, Table 1; human heart miRNA, Table 2).

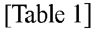

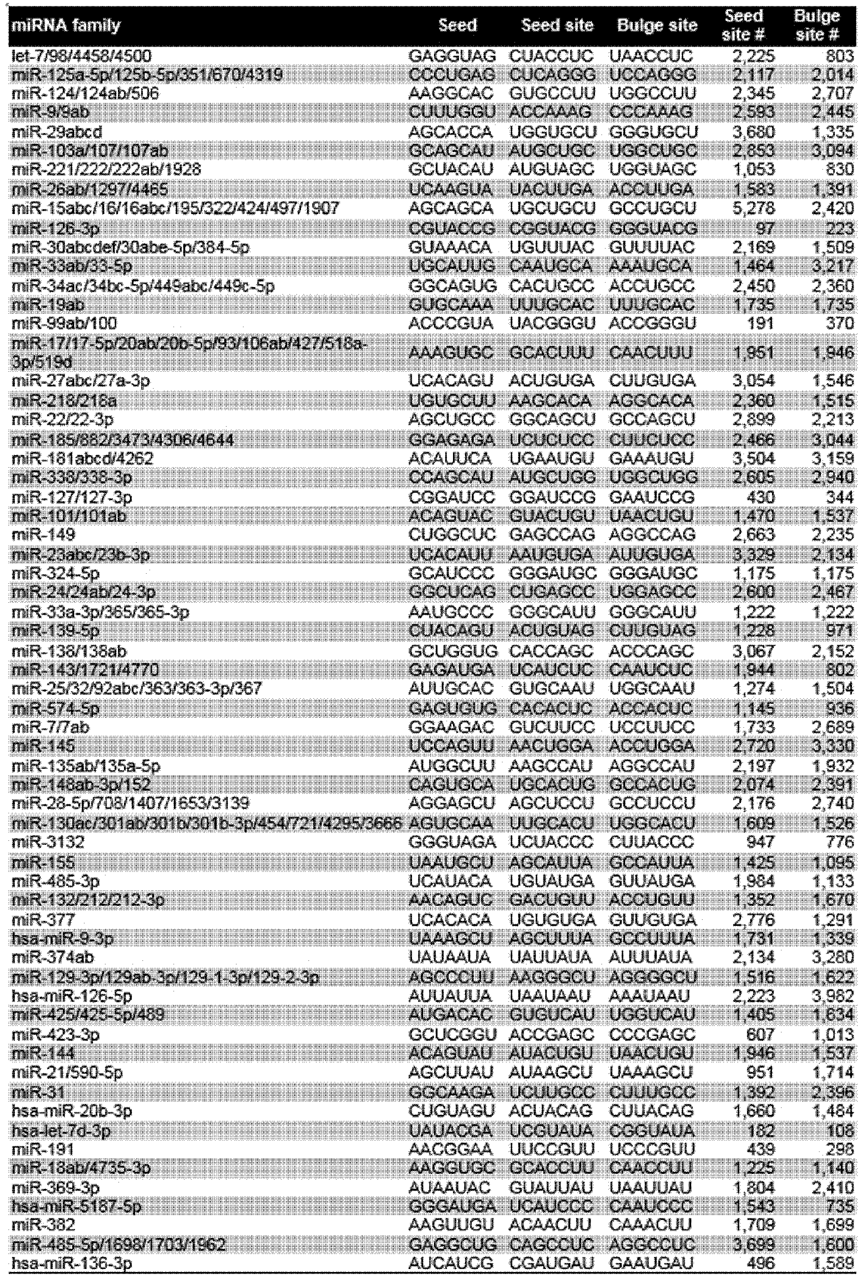

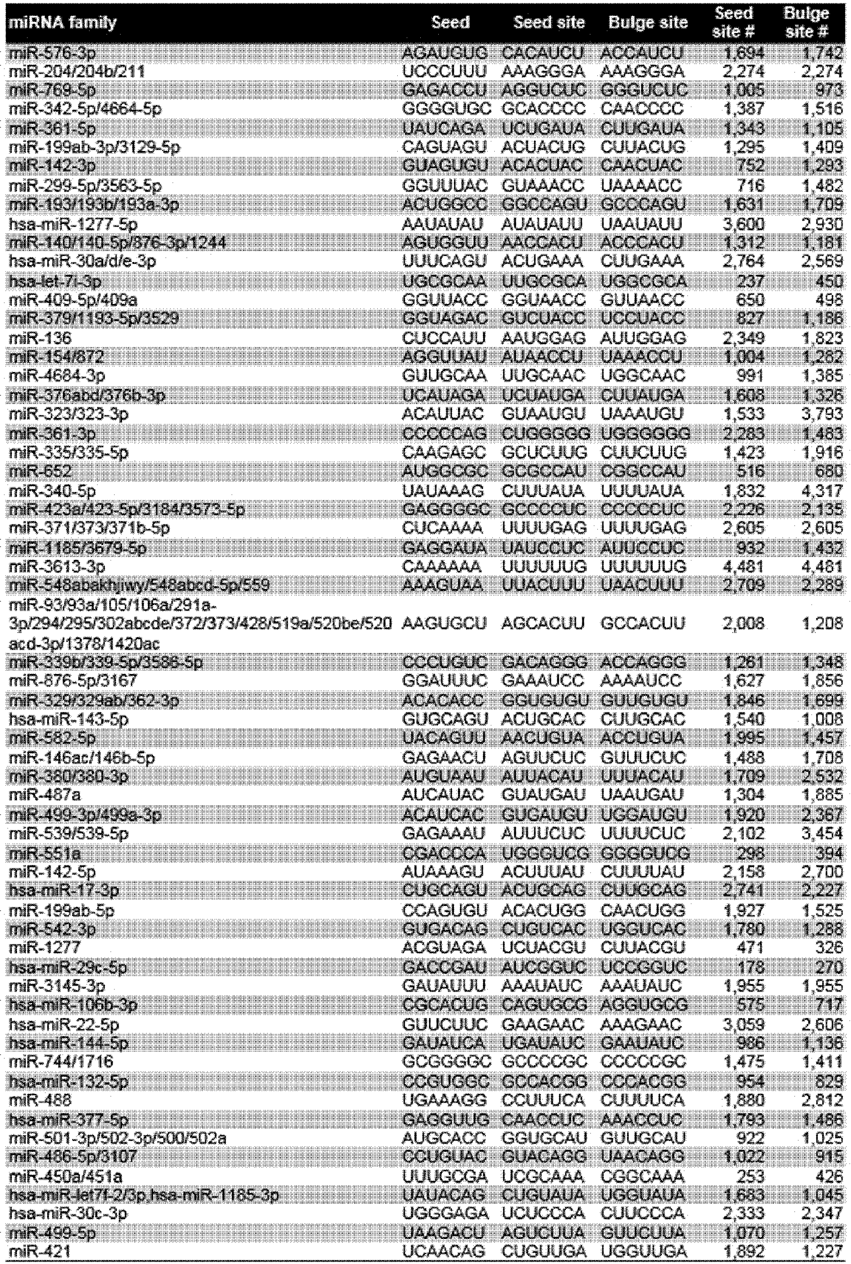

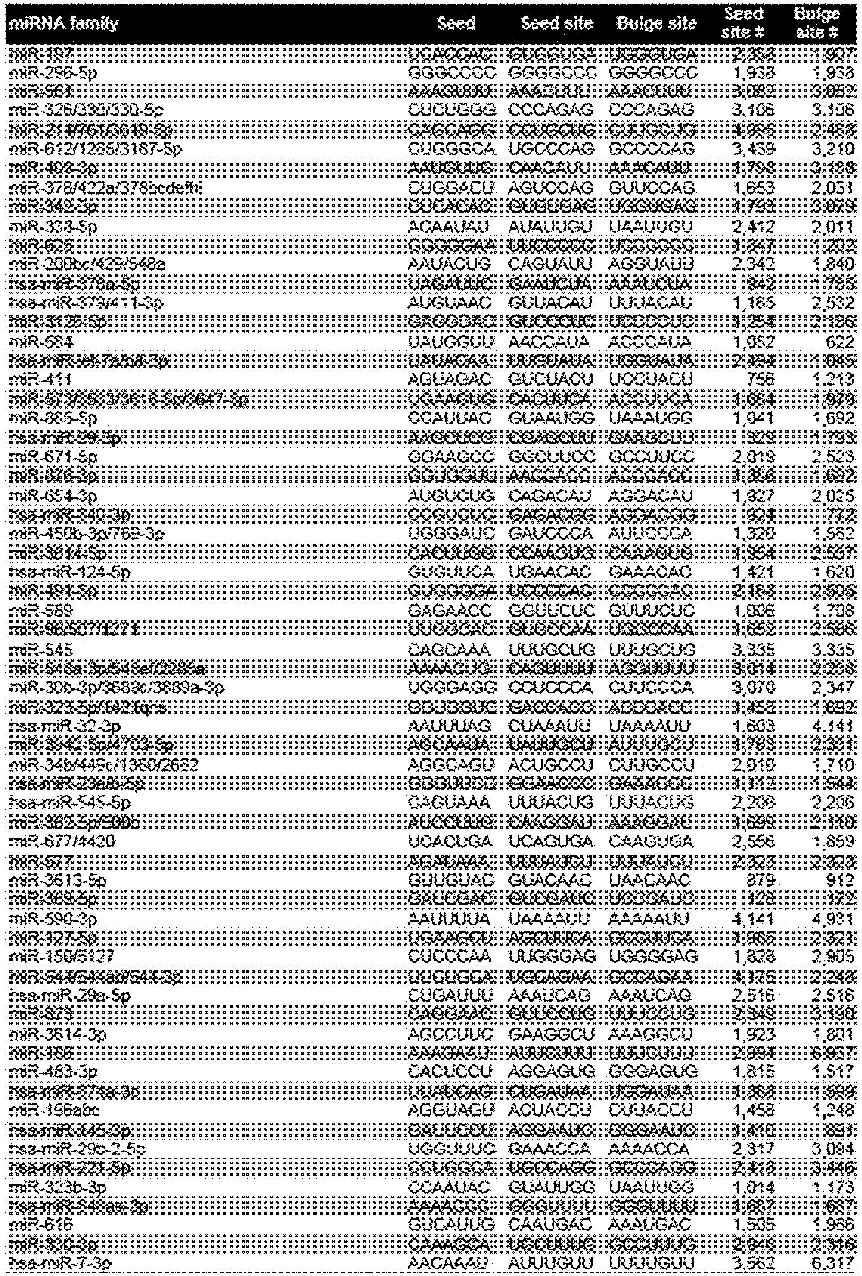

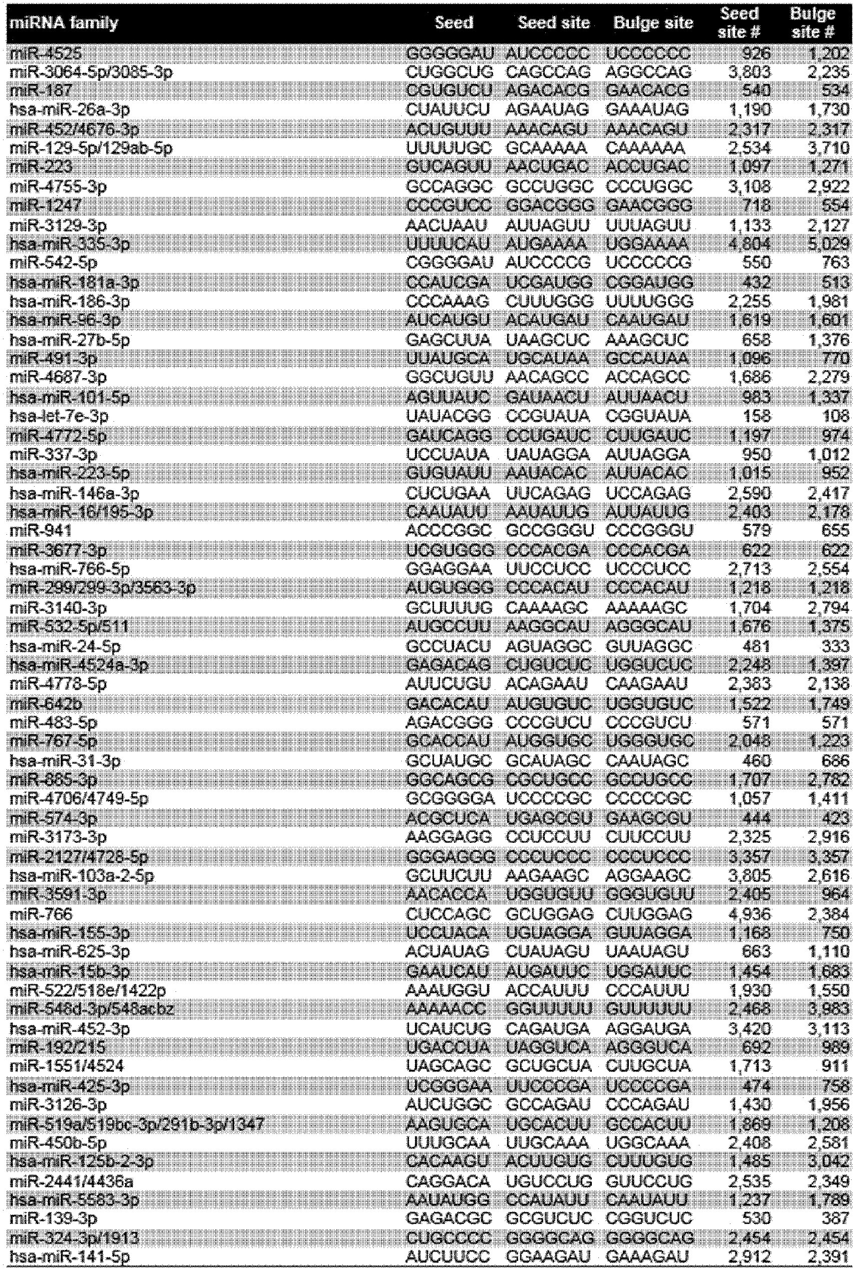

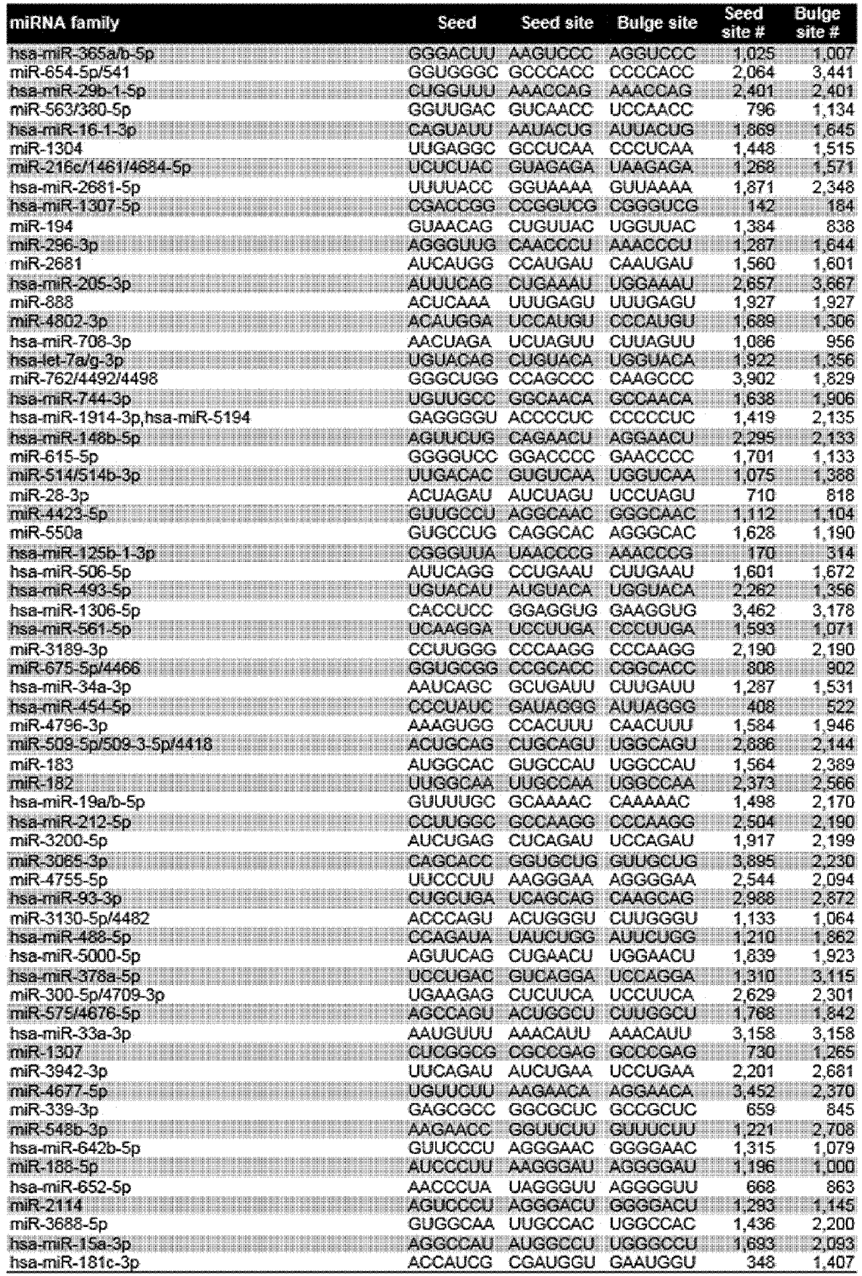

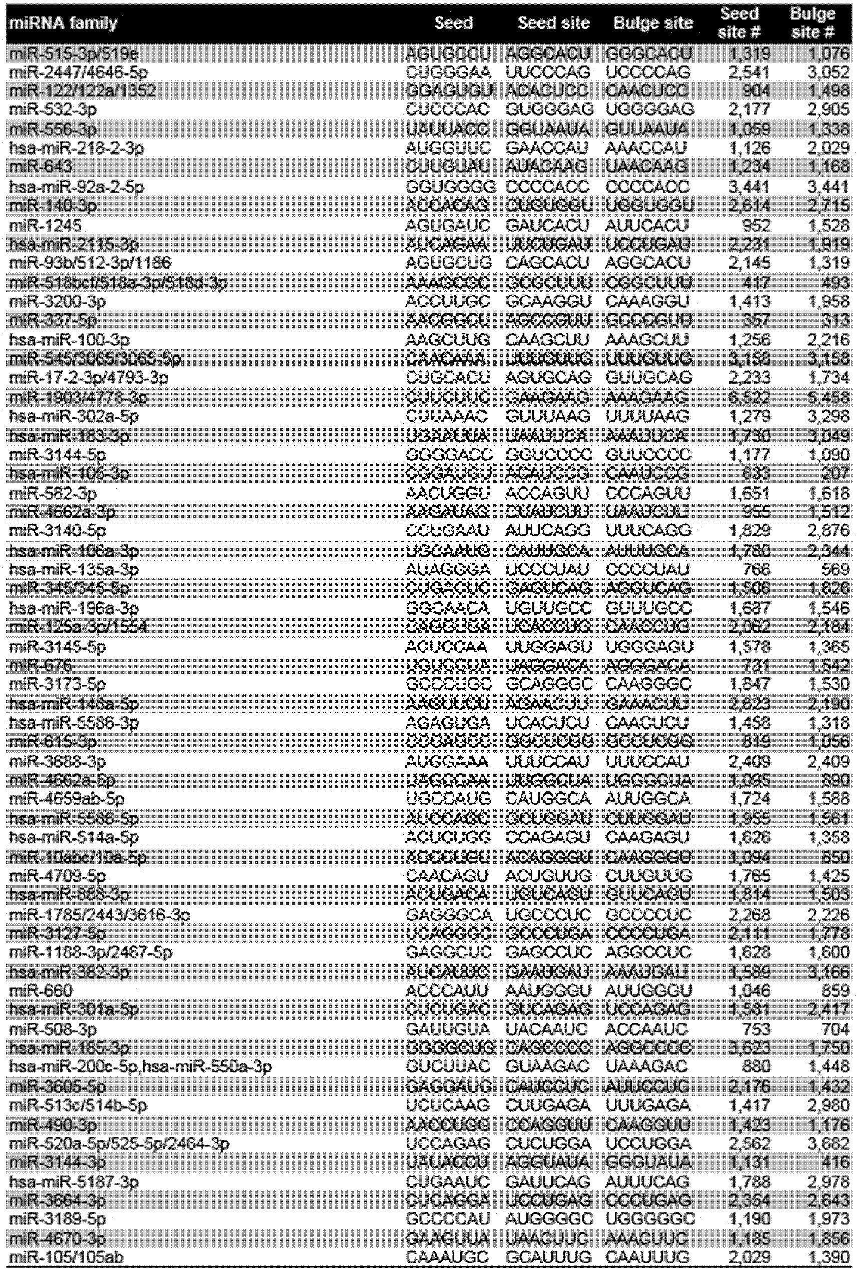

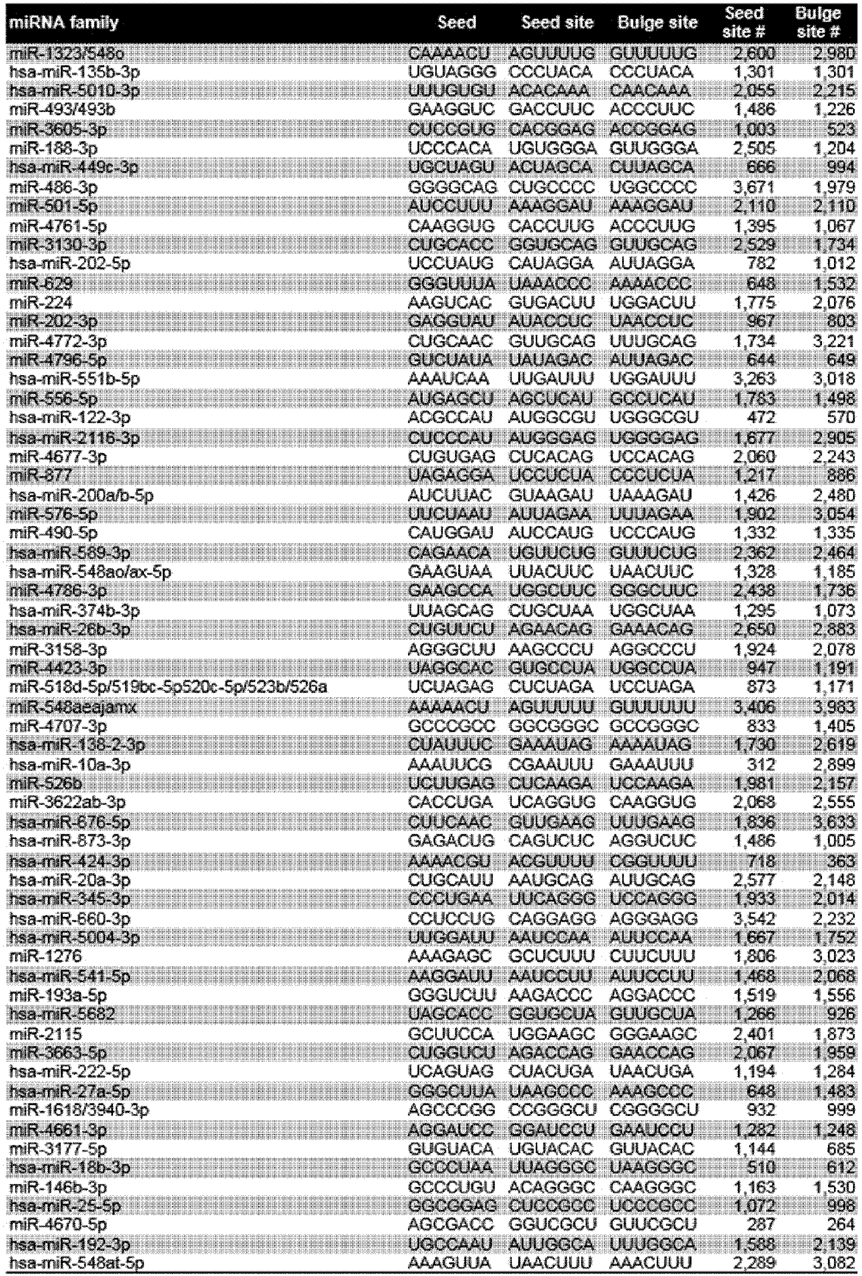

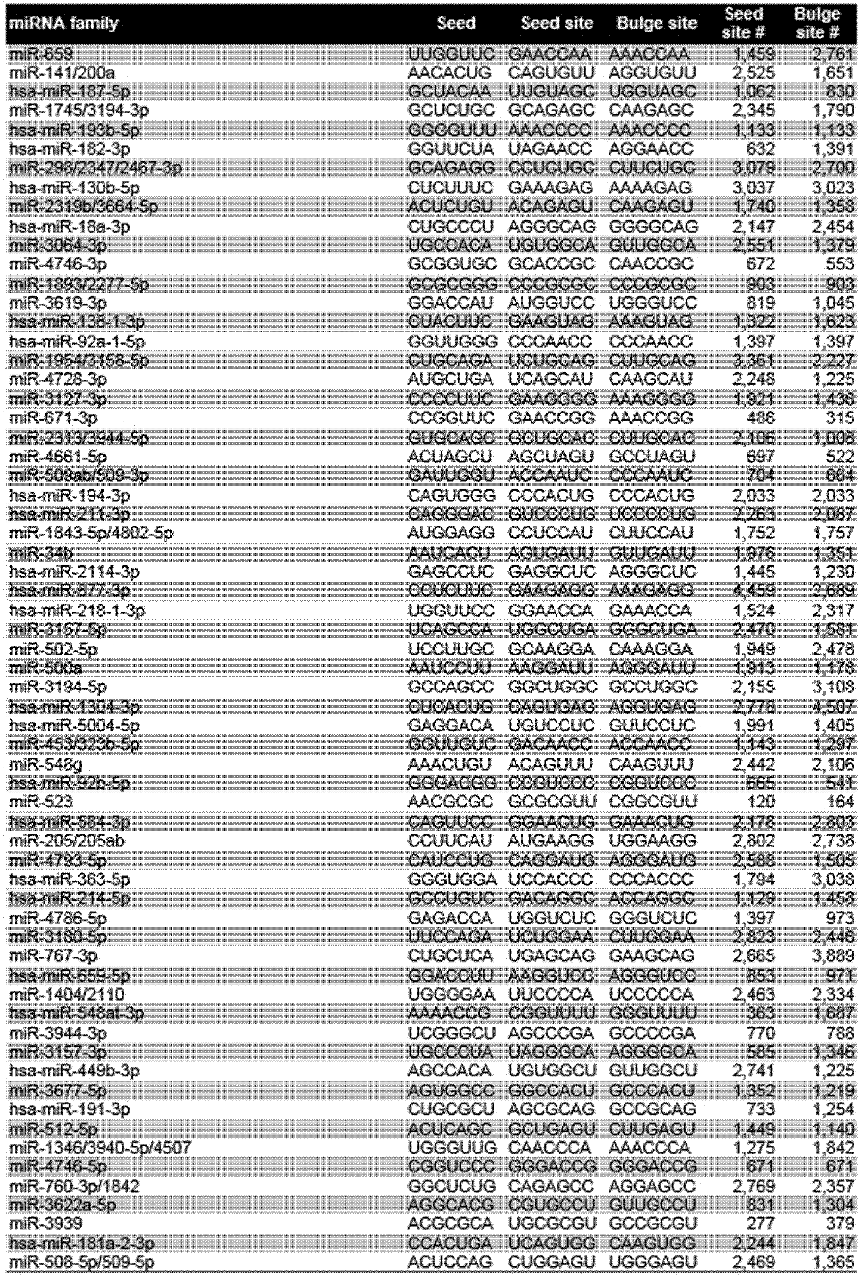

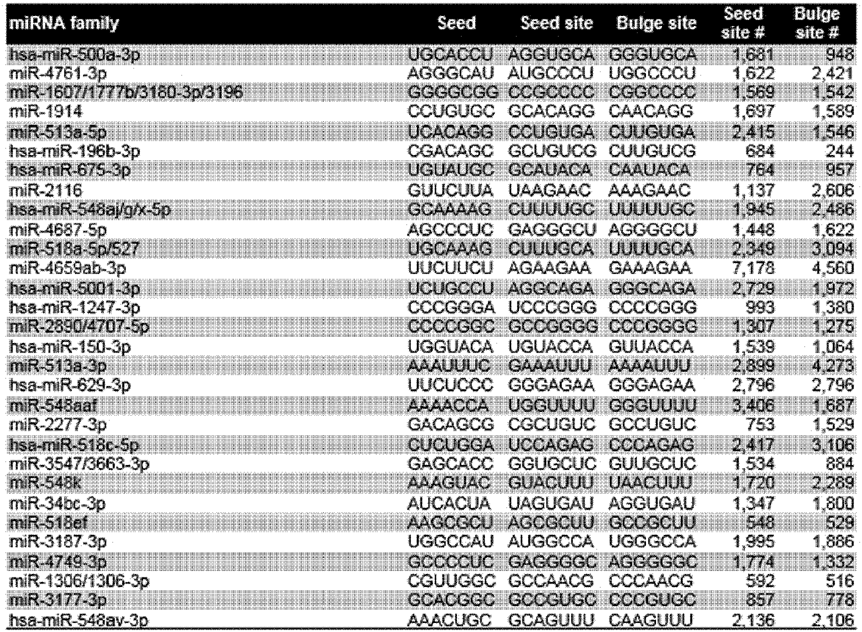

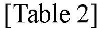

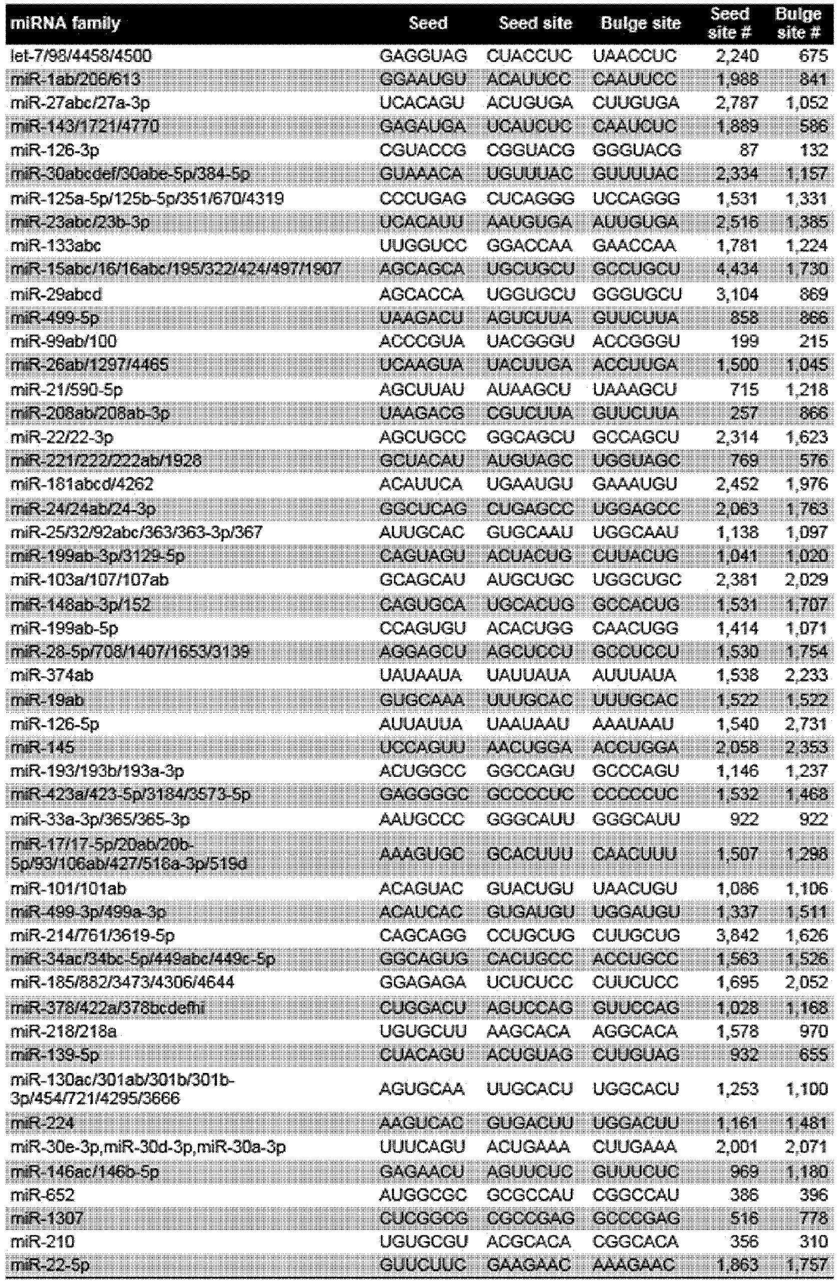

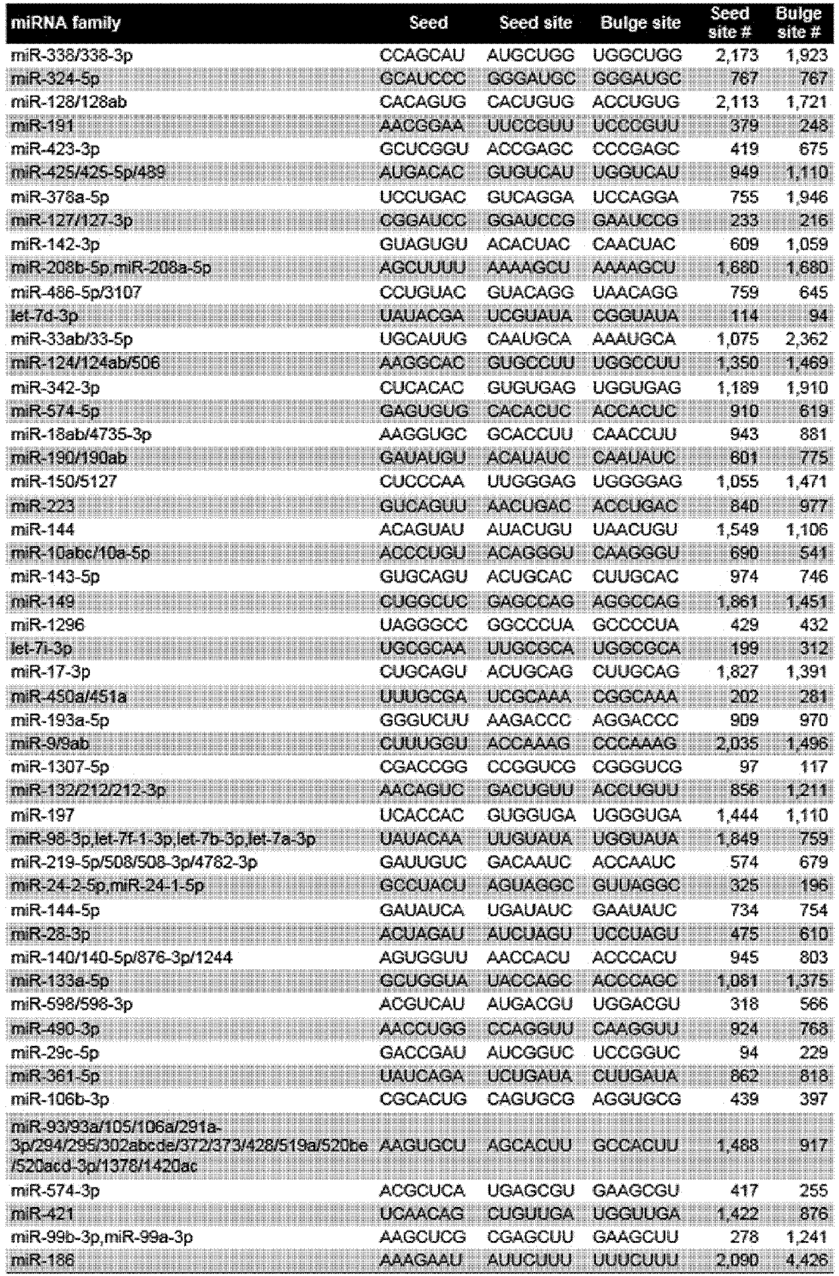

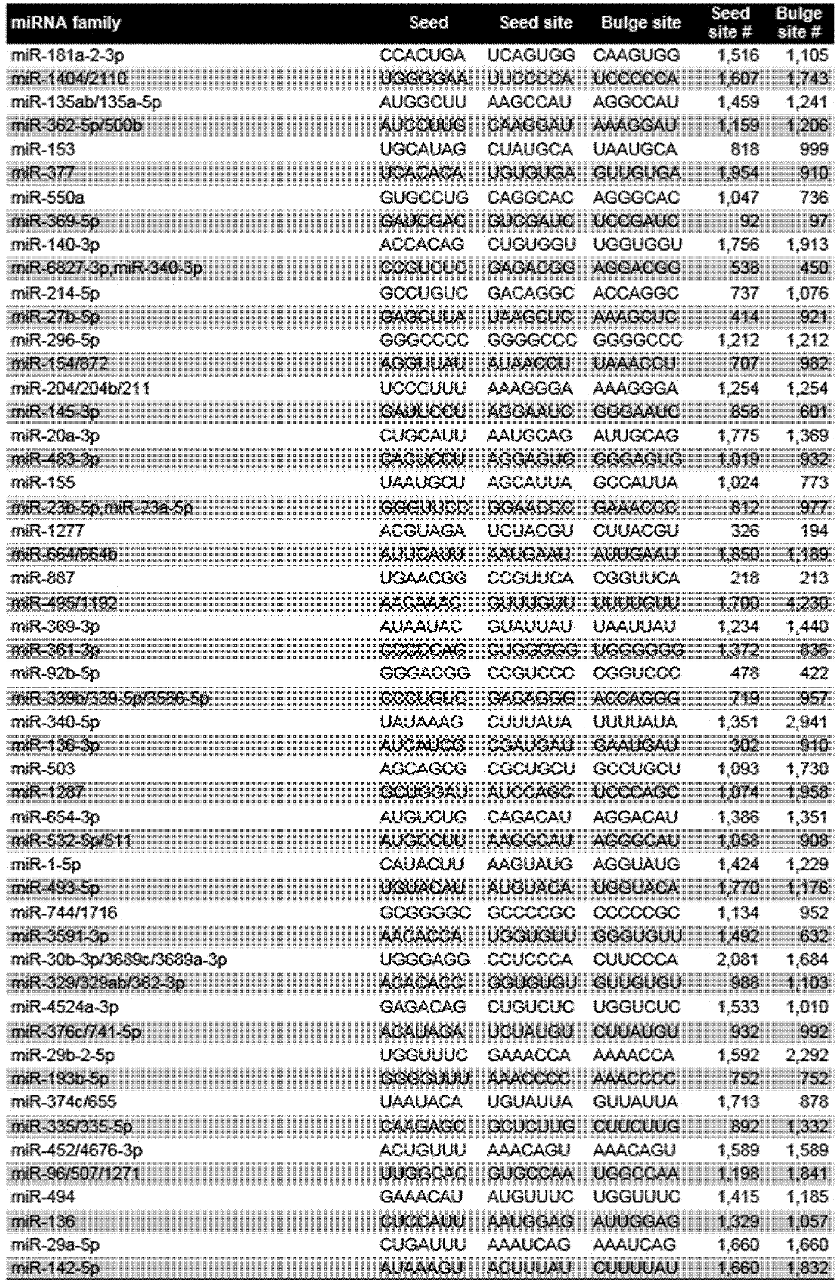

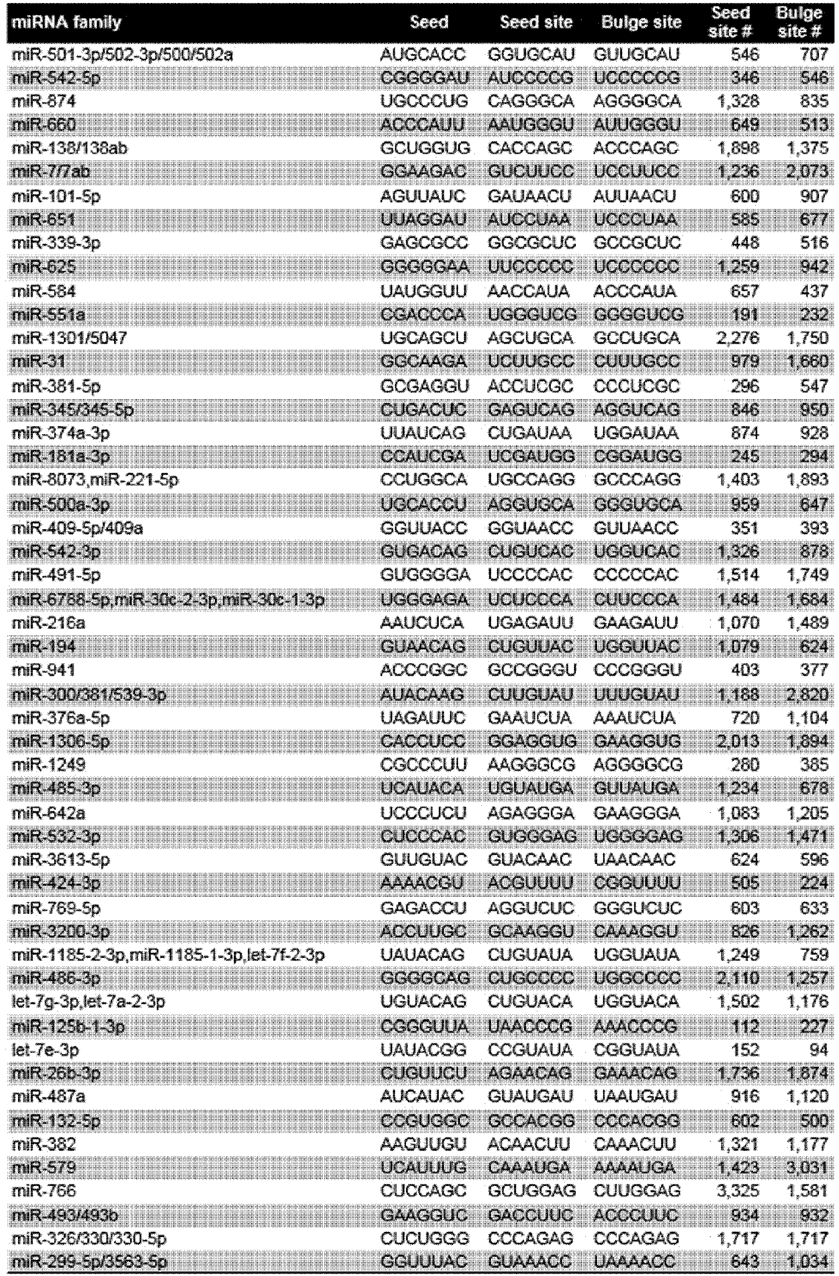

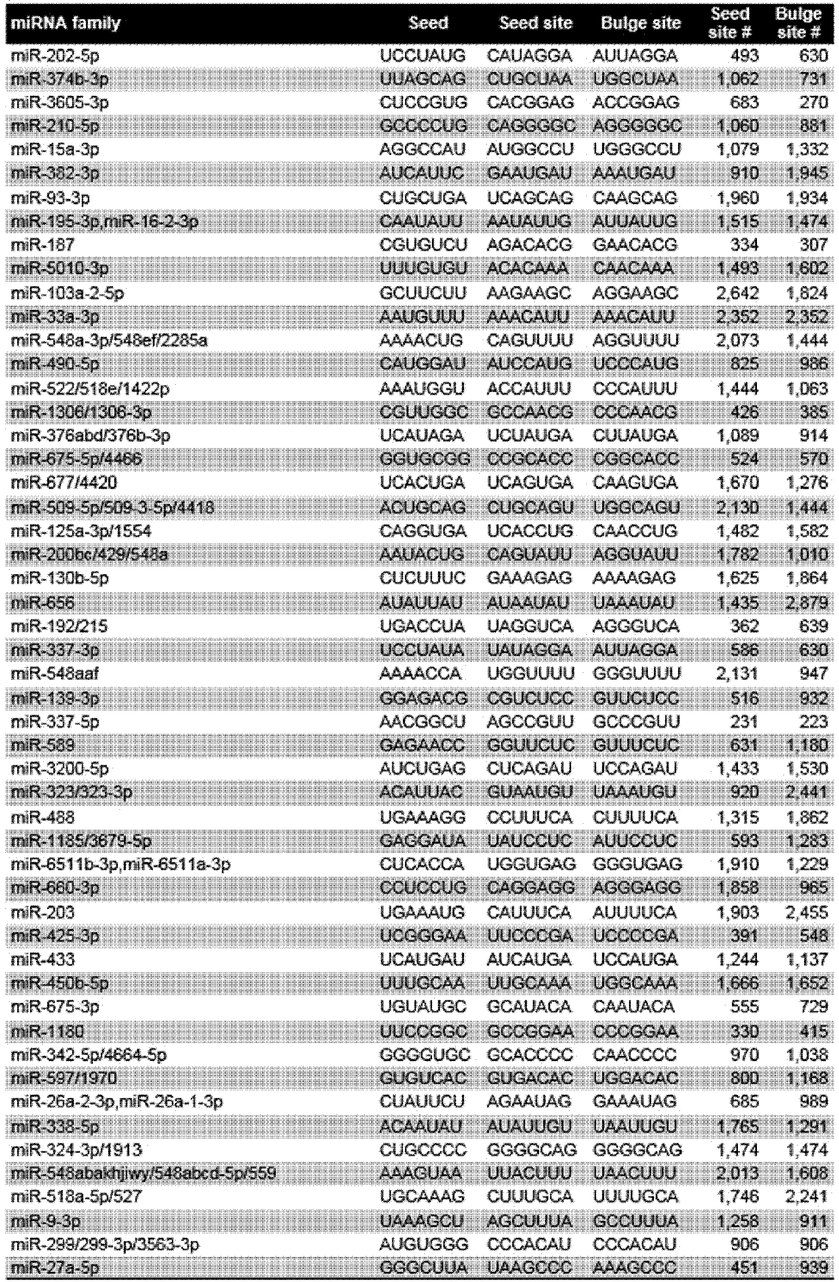

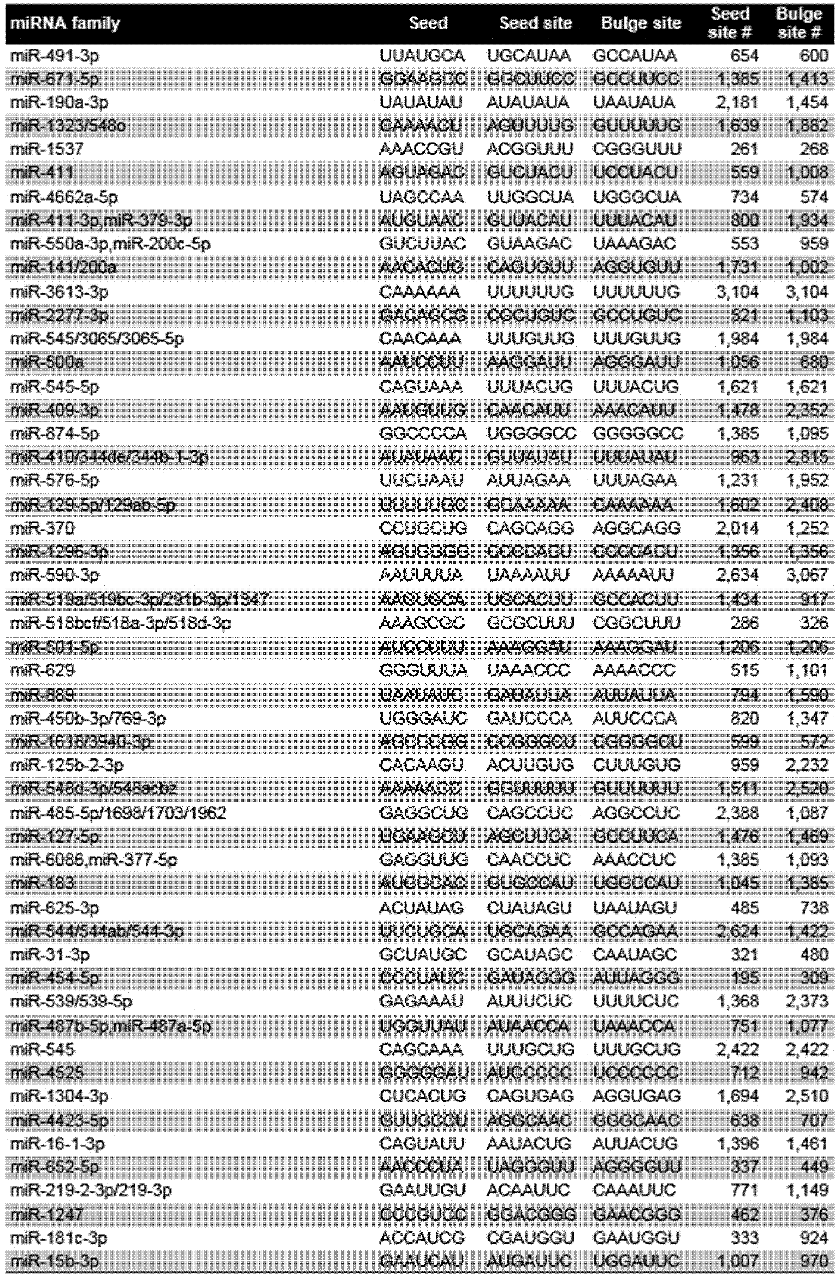

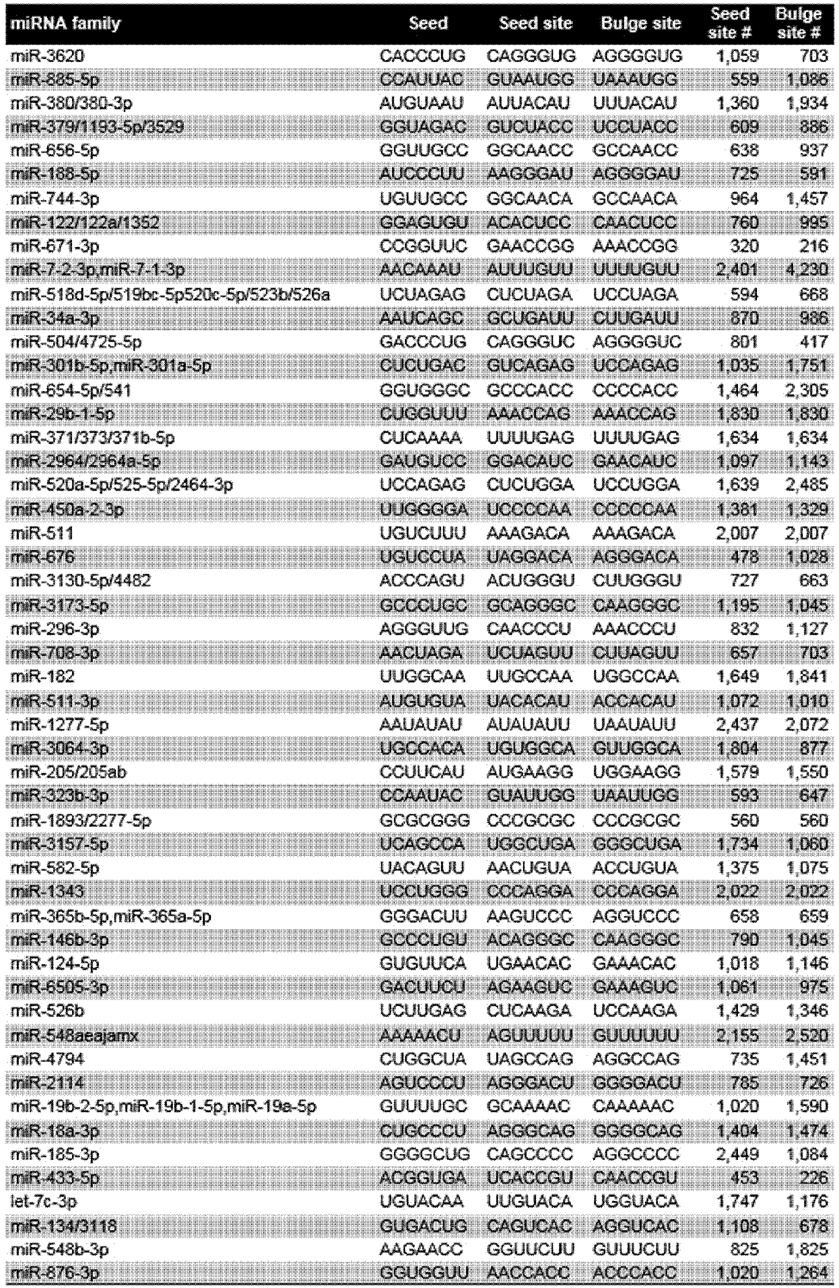

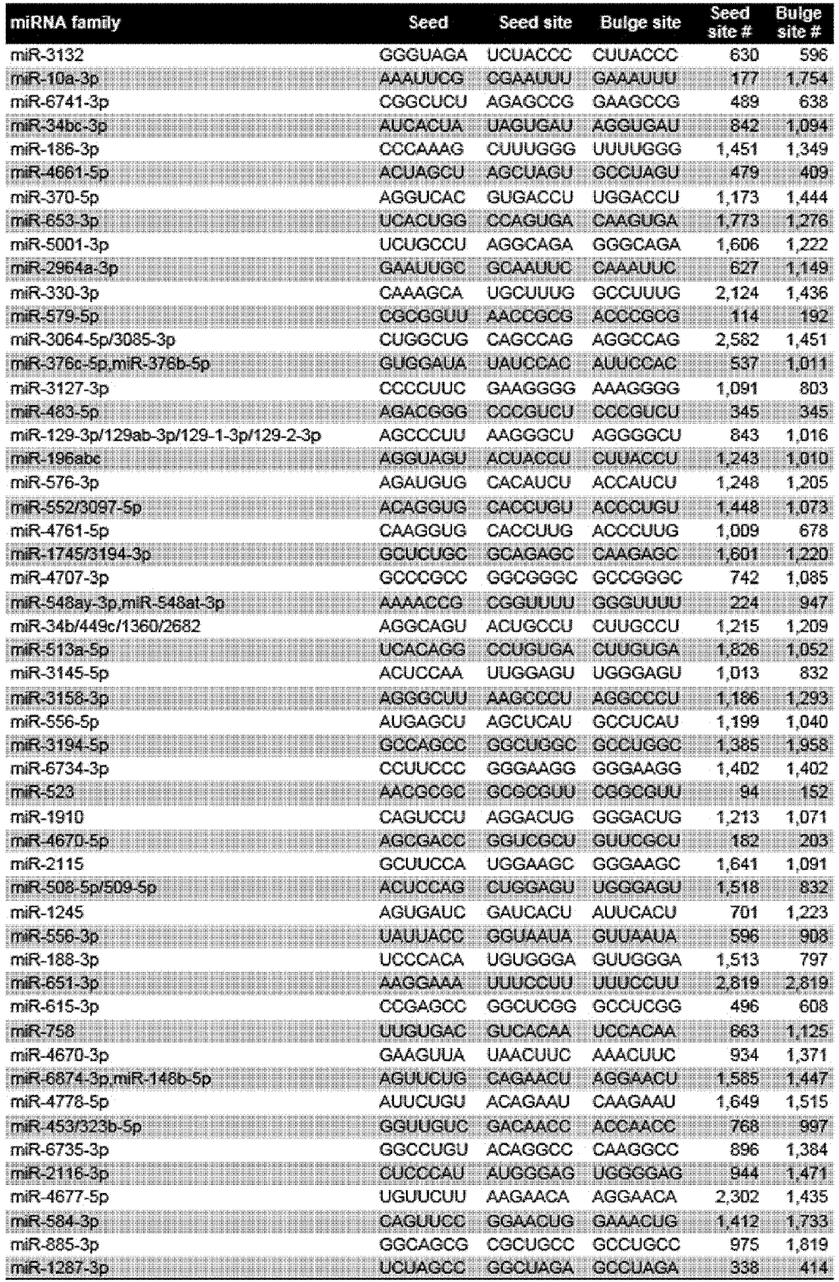

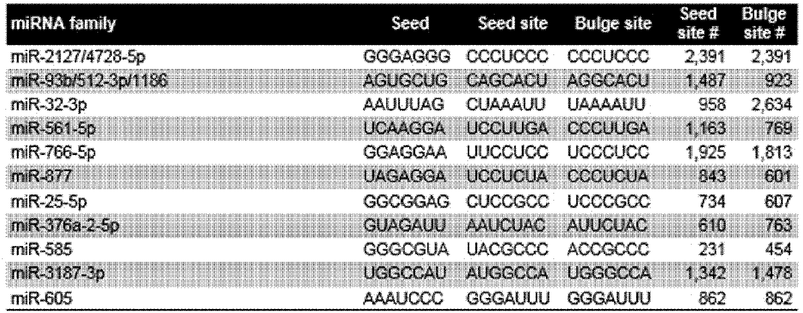

As a result, it was possible to confirm that miRNAs listed in the tables (Tables 1 and 2) bind with a non-canonical nucleation bulge site in a corresponding tissue. Therefore, when all of the data obtained through the Ago HITS-CLIP assay in the example was summarized, thereby identifying miRNA, and then the present invention for modifying a non-canonical nucleation bulge site to be recognized as a canonical seed site was applied to the miRNA, as shown in Table 3 below, each of a total of 426 sequences (BS sequences) consists of the 2^{nd} to 7^{th} nucleotides based on the 5' end of an RNA interference nucleic acid, and the modified RNA interference nucleic acid will specifically bind to the non-canonical bulge target of the corresponding miRNA and exhibit only the corresponding function.

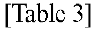

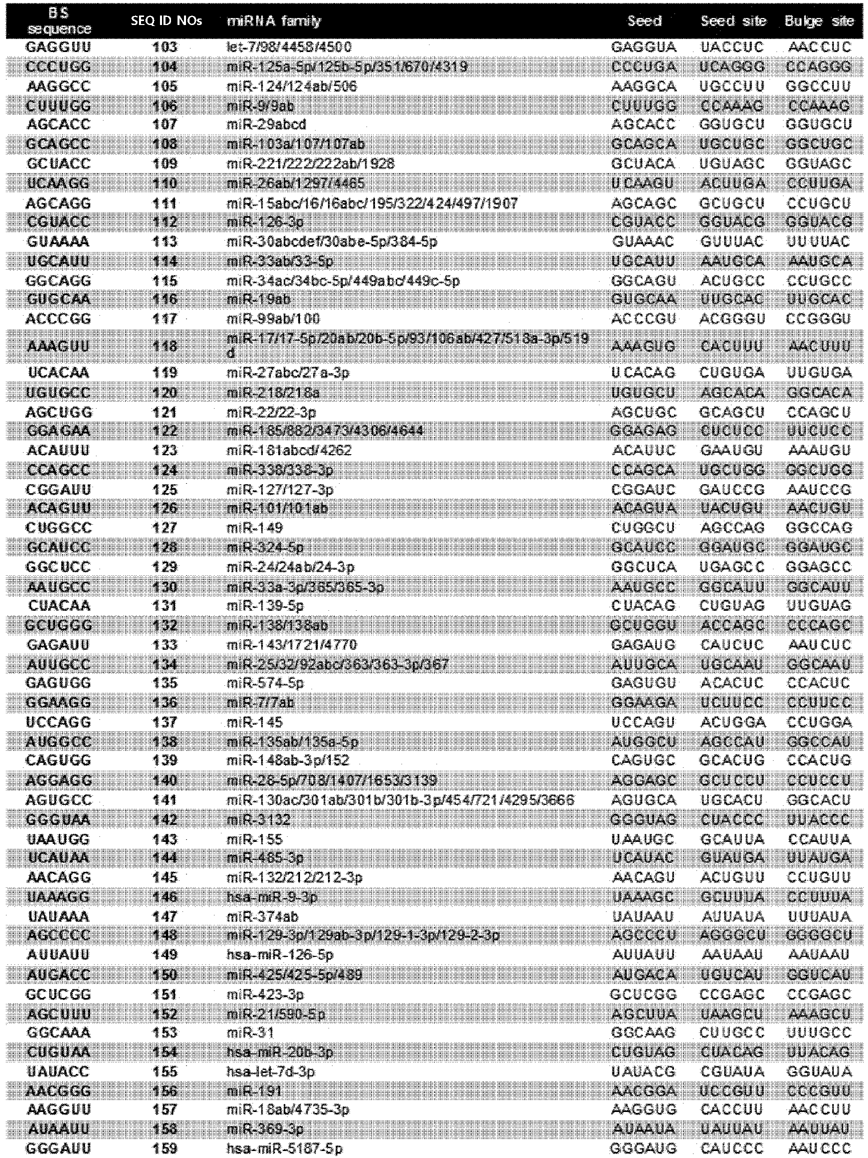

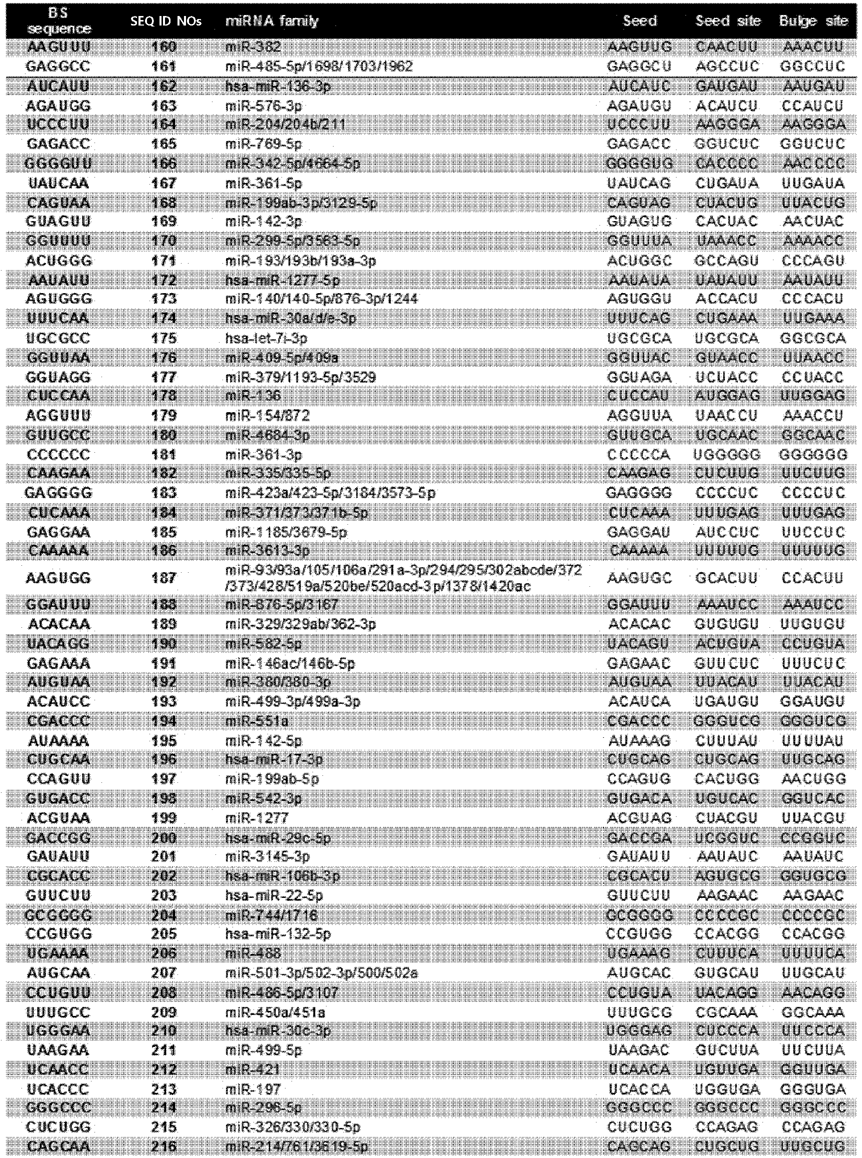

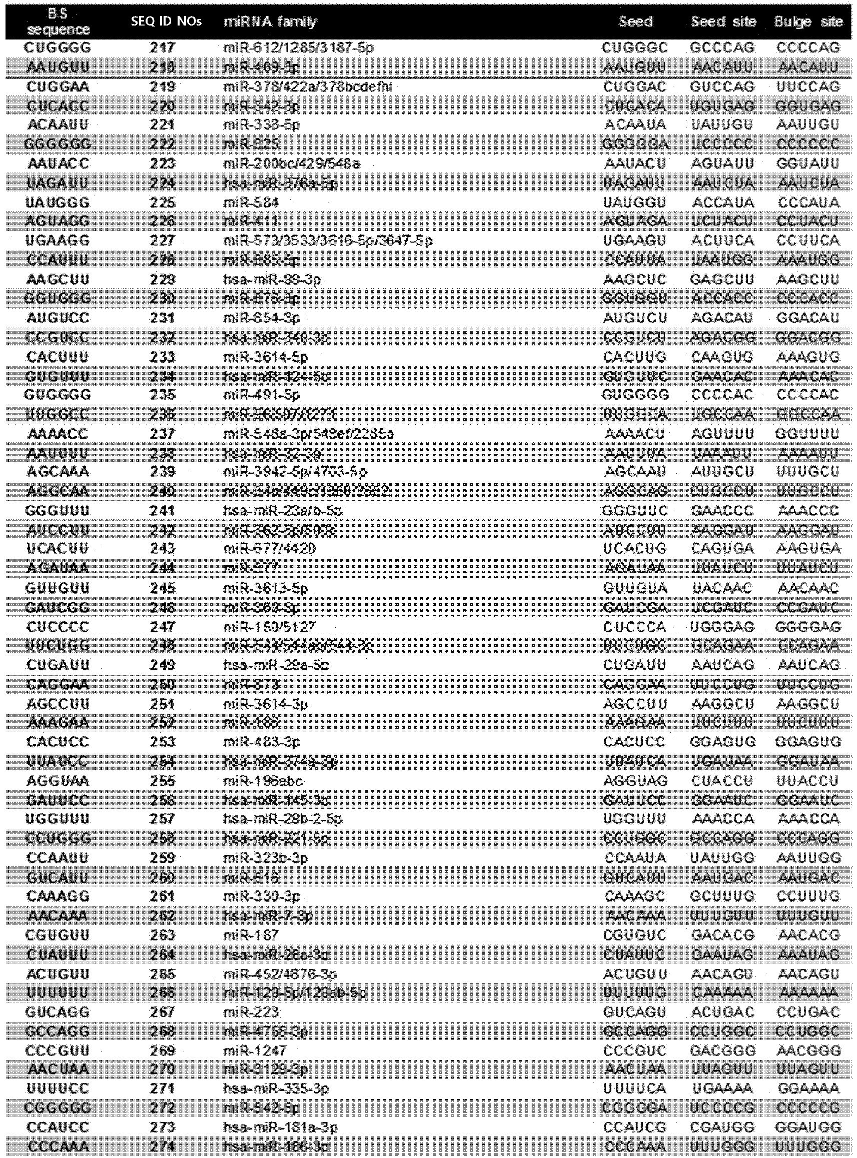

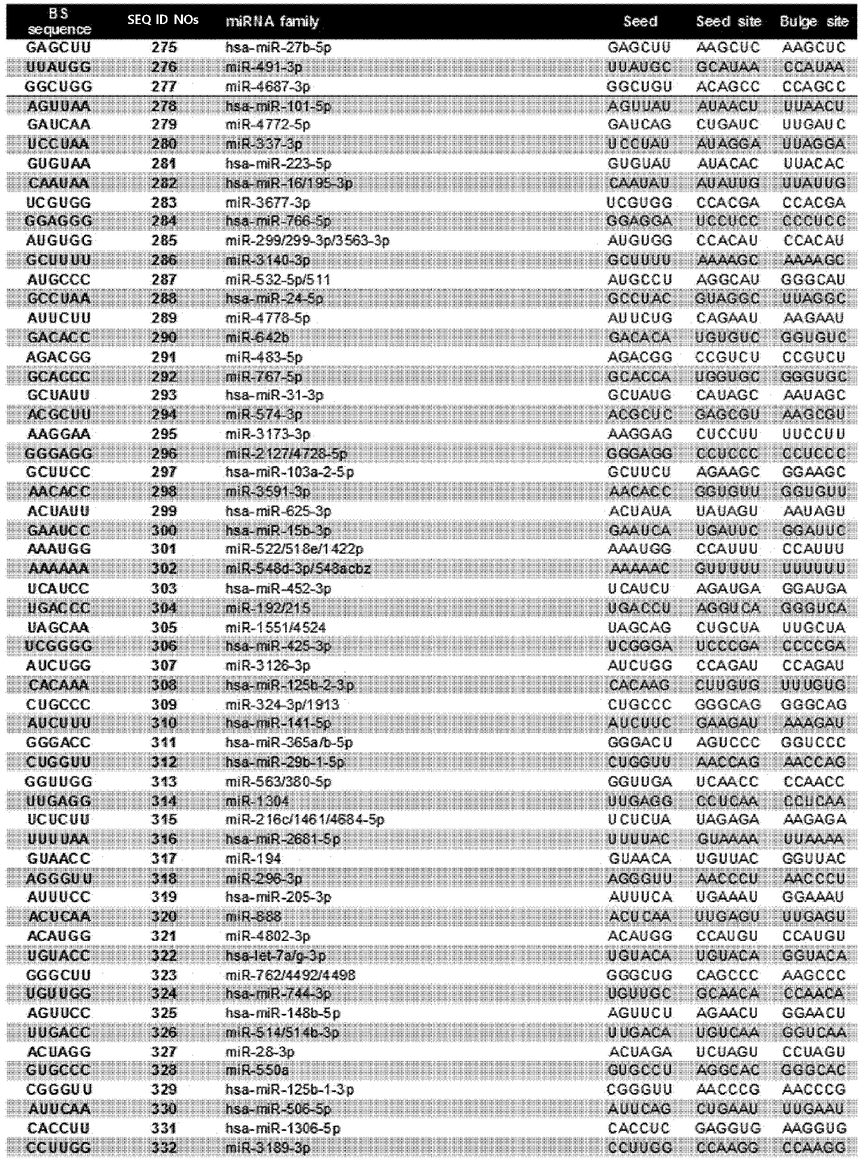

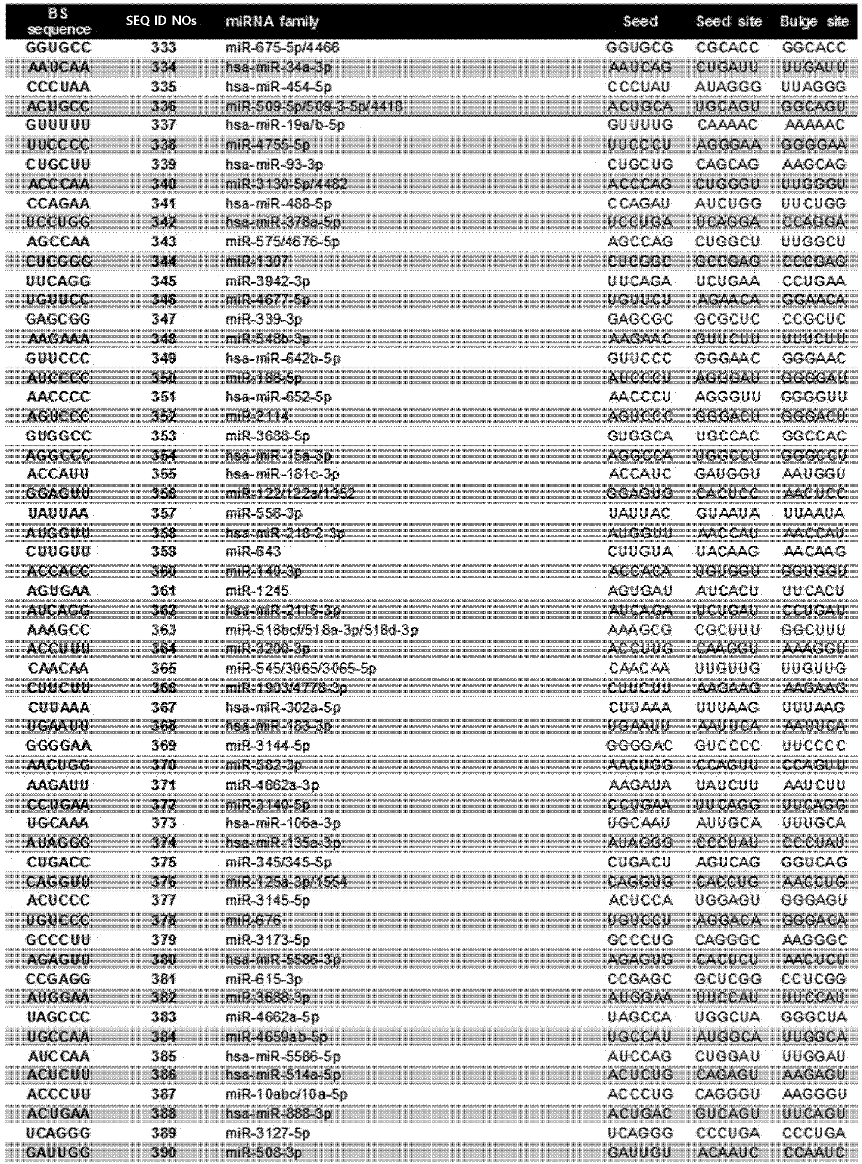

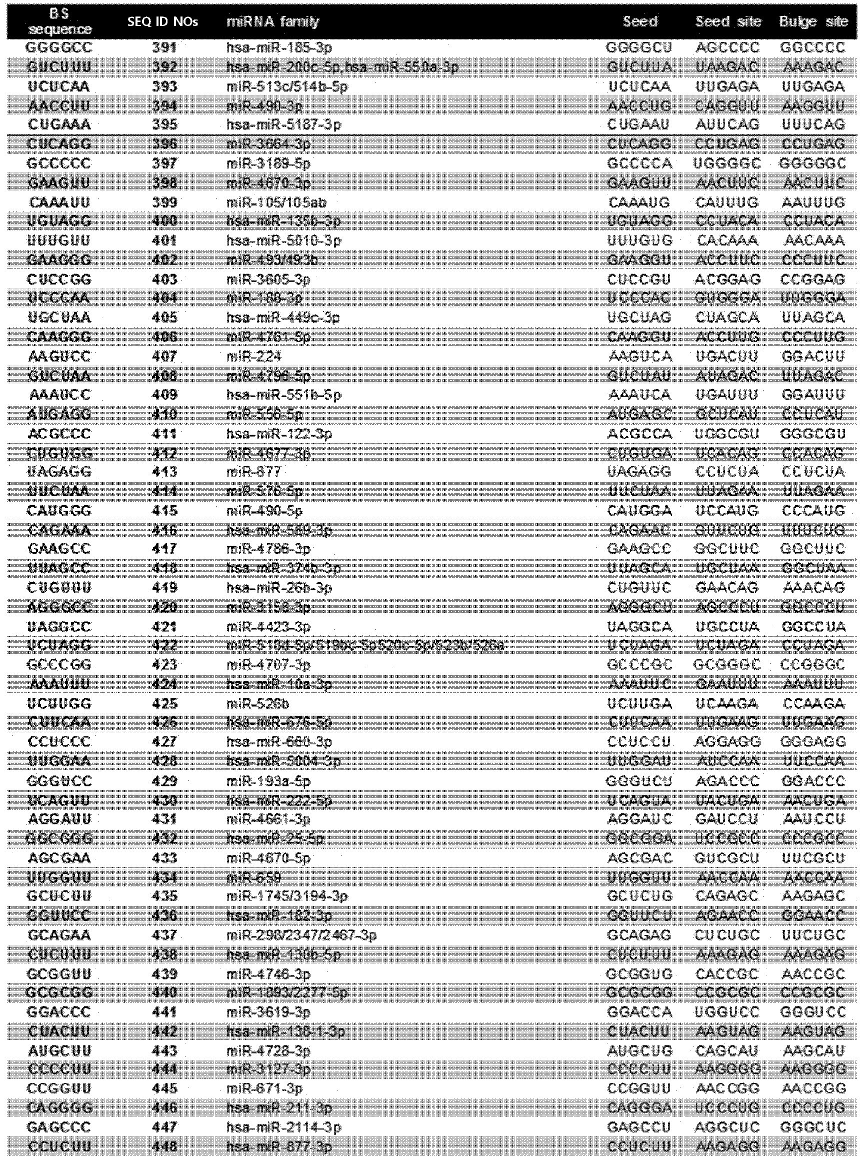

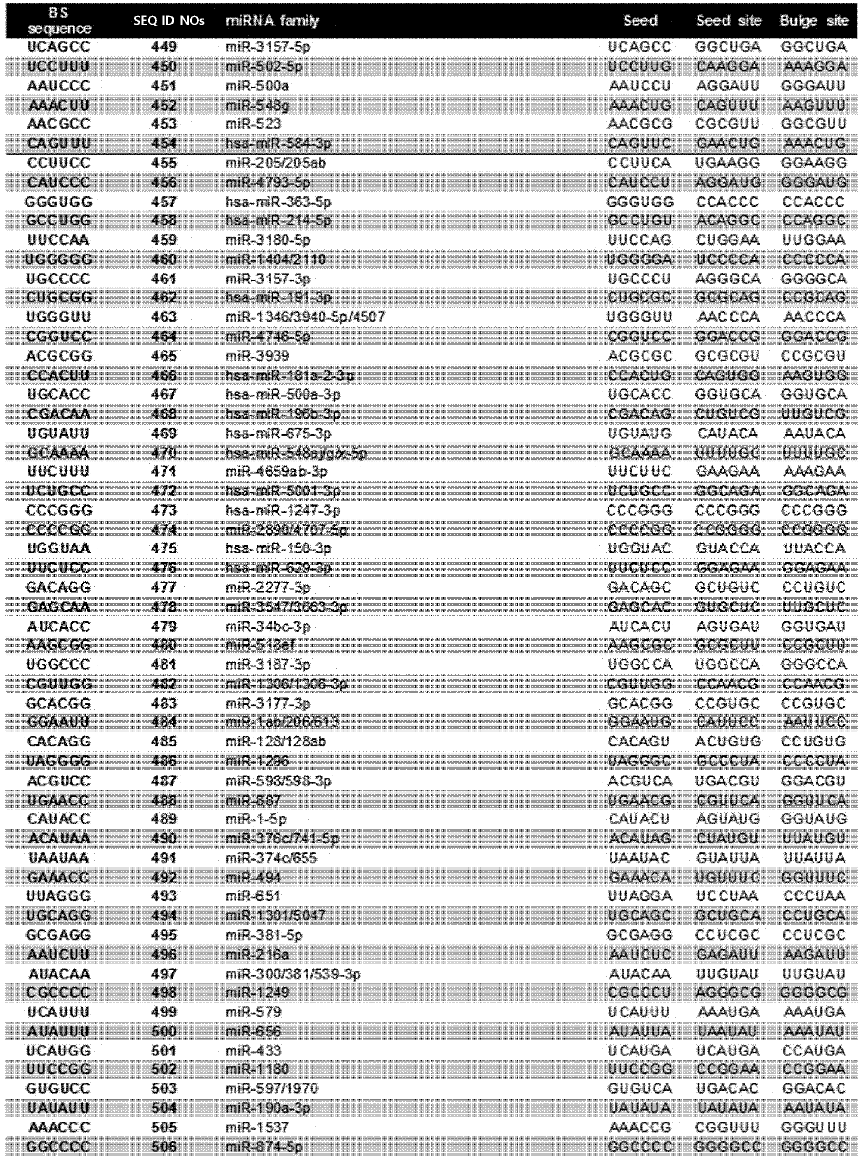

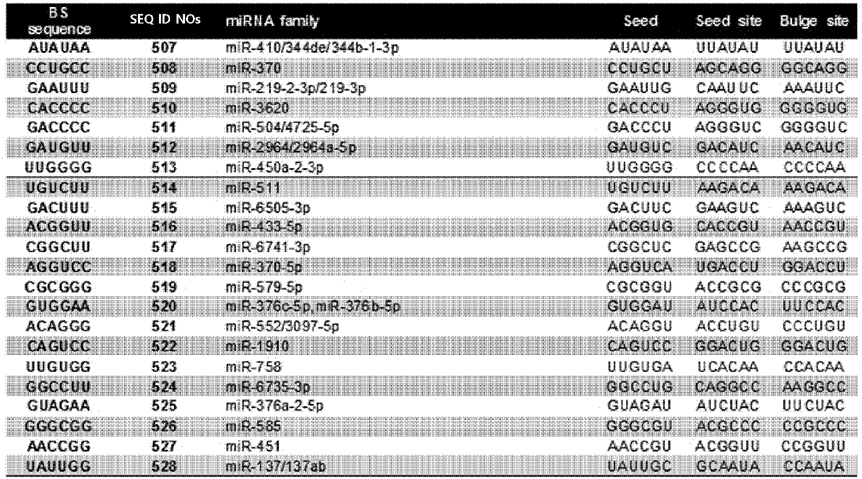

Through the Ago HITS-CLIP assay in the above example, miRNA binding to a non-canonical bulge target in several tissue cells was identified, and when the present invention for modifying miRNA to recognize a non-canonical nucleation bulge site as a canonical seed site is applied, a total of 426 sequences (BS sequences) were prepared, and therefore, technology for exhibiting only the function of suppressing the non-canonical target of miRNA was completed.

### [Example 25] Analysis of sequence variation in miRNA sequence database (TCGA) of cancer patients, and thereby identifying miRNA binding to non-canonical G:A wobble seed site

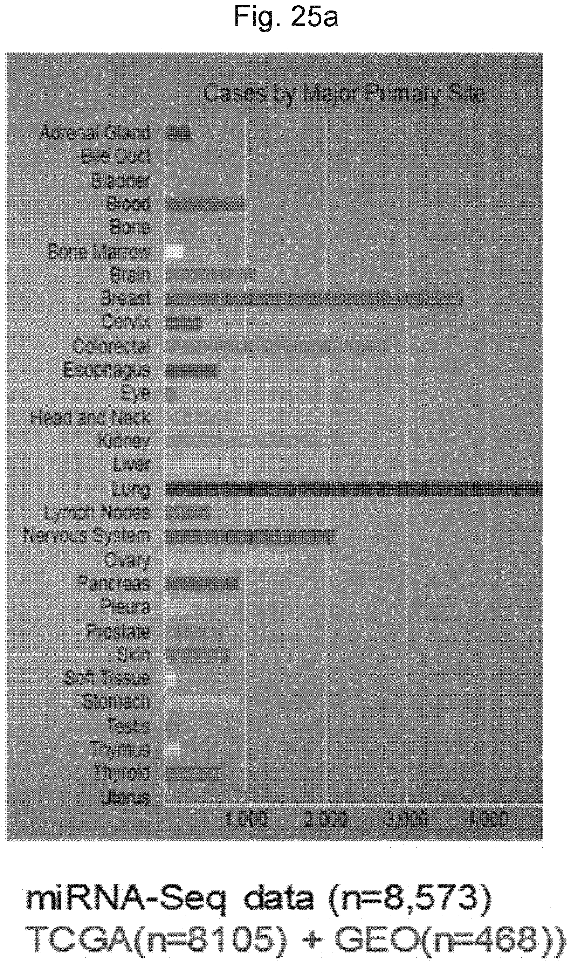

According to the above examples, it was confirmed that miRNA binds to a non-canonically G:A wobble site, thereby inhibiting the expression of a target gene, and therefore, in the present invention, a miRNA sequence modification method (miRNA-BS) of modifying a sequence to recognize a non-canonical G:A wobble site as a canonical seed site was developed. If a miRNA function is changed through wobble pairing between G in the conventional miRNA seed sequence and A of target mRNA, based on the idea that such a mechanism can be shown in a disease such as a tumor through sequence variation of miRNA, a result of sequencing miRNA from tissue of a cancer patient was analyzed (miRNA-Seq). Here, as 8,105 sequences were obtained from the miRNA sequencing-related TCGA database of cancer patients, and additionally, 468 sequences were obtained through cancer-related miRNA sequencing from the Gene Expression Omnibus (GEO) database, a total of 8,573 tumor miRNA sequencing results obtained thereby were mapped using the bowtie2 program in the same manner as in Example 10, and then sites with variations were analyzed. At this time, the miRNA sequencing data used in the analysis for each cancer type is as shown in FIG. 25A.

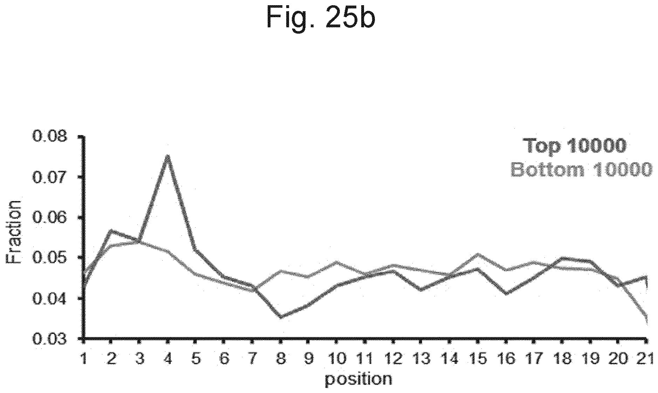

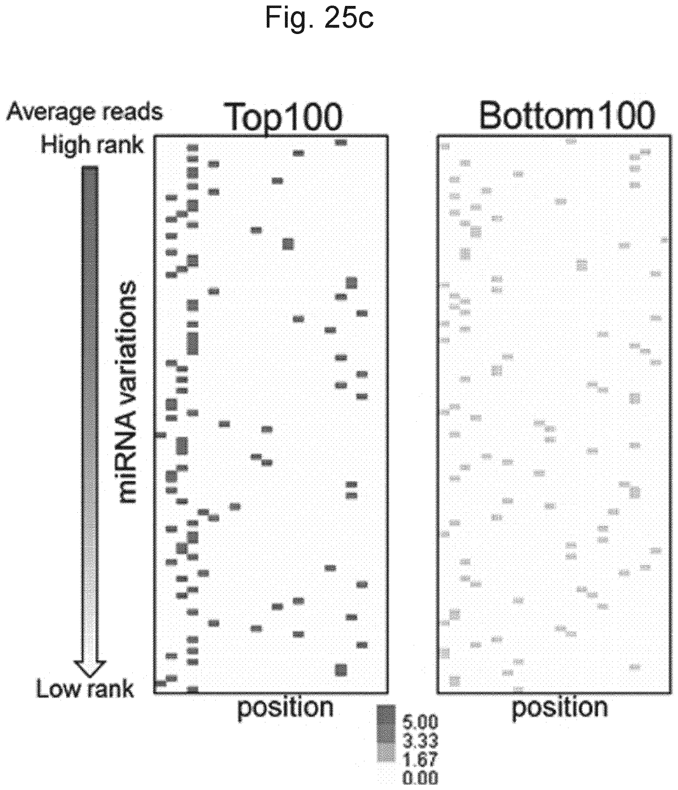

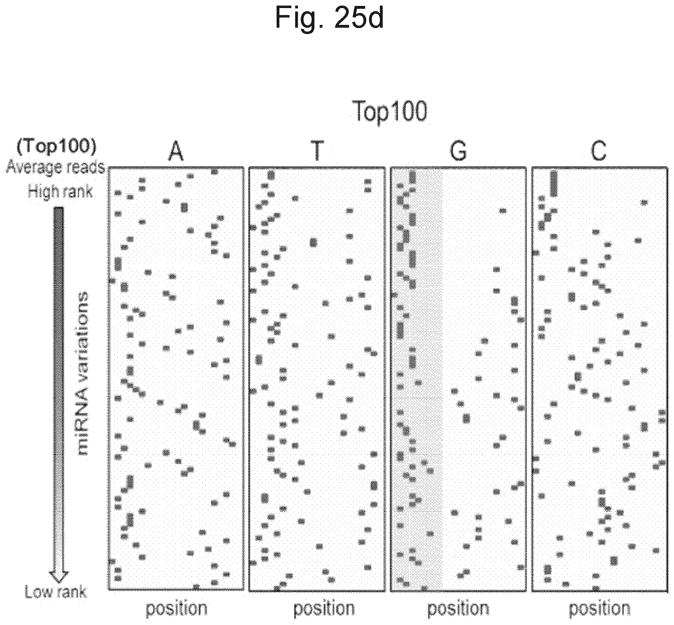

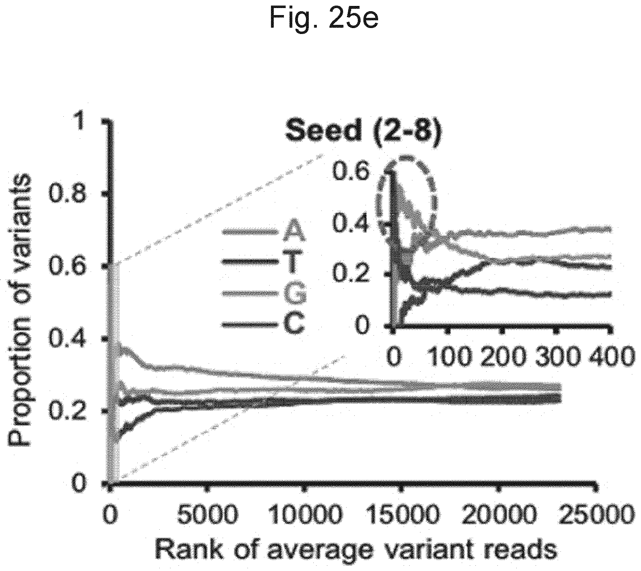

As a result of examining the distribution of the most frequent variations (top 10,000) and the least variations (bottom 10,000) by classifying variation fractions by position based on the 5' end of miRNA (FIG. 25B), it was possible to observe that the most frequent variations (top 10,000) mainly occur in the seed region (between the 2^{nd} to the 8^{th} bases) of miRNA. In addition, examining the most frequent variations (top 100) and the least variations (bottom 100) on a heatmap (FIG. 25C), it was able to be seen that the variations are concentrated in the seed region as in the examples above. Examining the sequencing result with DNA through reverse transcription of the miRNA by analyzing it with A, T, C and G, the sequence variation in the tumor miRNA which tends to be shown in the seed region was detected at only G in the seed sequence (FIG. 25D). Such tendency was also confirmed from that, in addition to top 100 variations, all variations mainly occur at G (FIG. 25E).

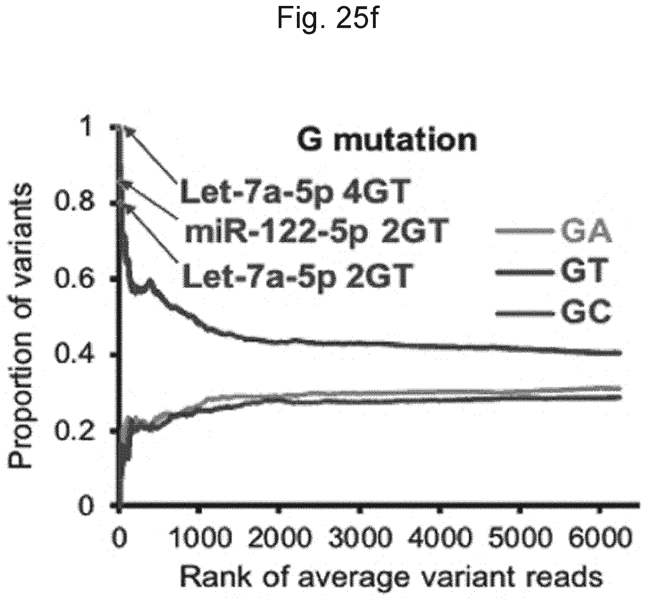

This may be a phenomenon in which G of miRNA recognizing the non-canonical G:A wobble noted by the inventors is changed to U to more tightly bind to A of the target mRNA on the opposite side, and as a result of analyzing which bases Gs, which show the higher variation rate, were changed to (FIG. 25F), in most of the corresponding results in which the miRNA is sequenced with DNA through reverse transcription, it was observed that G changes to T. This result indicates that miRNA pairs with A in a target by changing its G to U for G present in the seed region to well recognize a non-canonical G:A wobble seed target as a canonical seed in actual cancer tissue, and therefore, this type of variation indicates how to apply the sequencing method that allows recognition of a non-canonical G:A wobble seed sequence as a canonical seed sequence, developed in the present invention, to which G at which position of which miRNA. Accordingly, putting all of the results together, miRNA with a normalized variation frequency per cancer patient of 2 or more was selected (see Table 4). As shown in Table 4, a total of 335 sequences (sequence (G>U)) were designed to have variations from the 2^{nd} nucleotide based on the 5' end of an RNA interference nucleic acid, and the modified RNA interference nucleic acids will only interact with a non-canonical G:A bulge target for the corresponding miRNA and exhibit only a corresponding function.

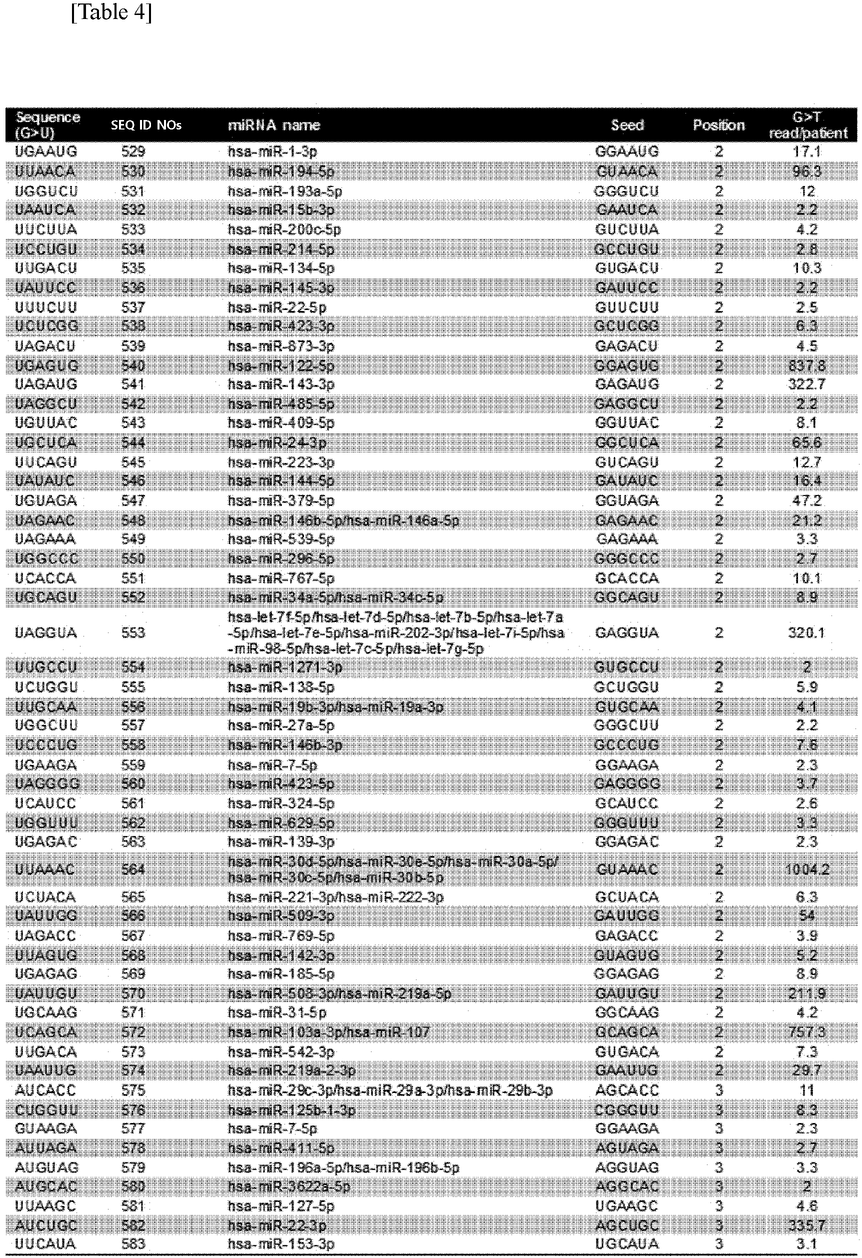

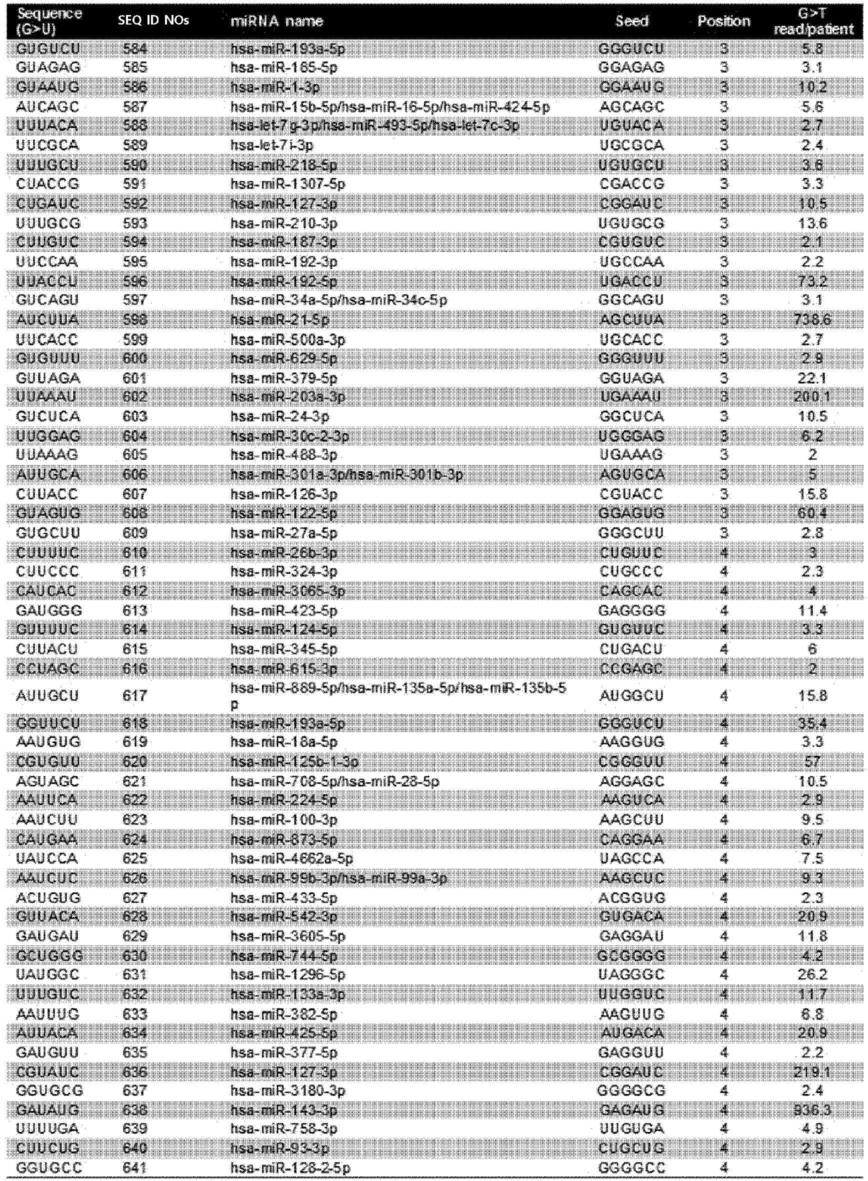

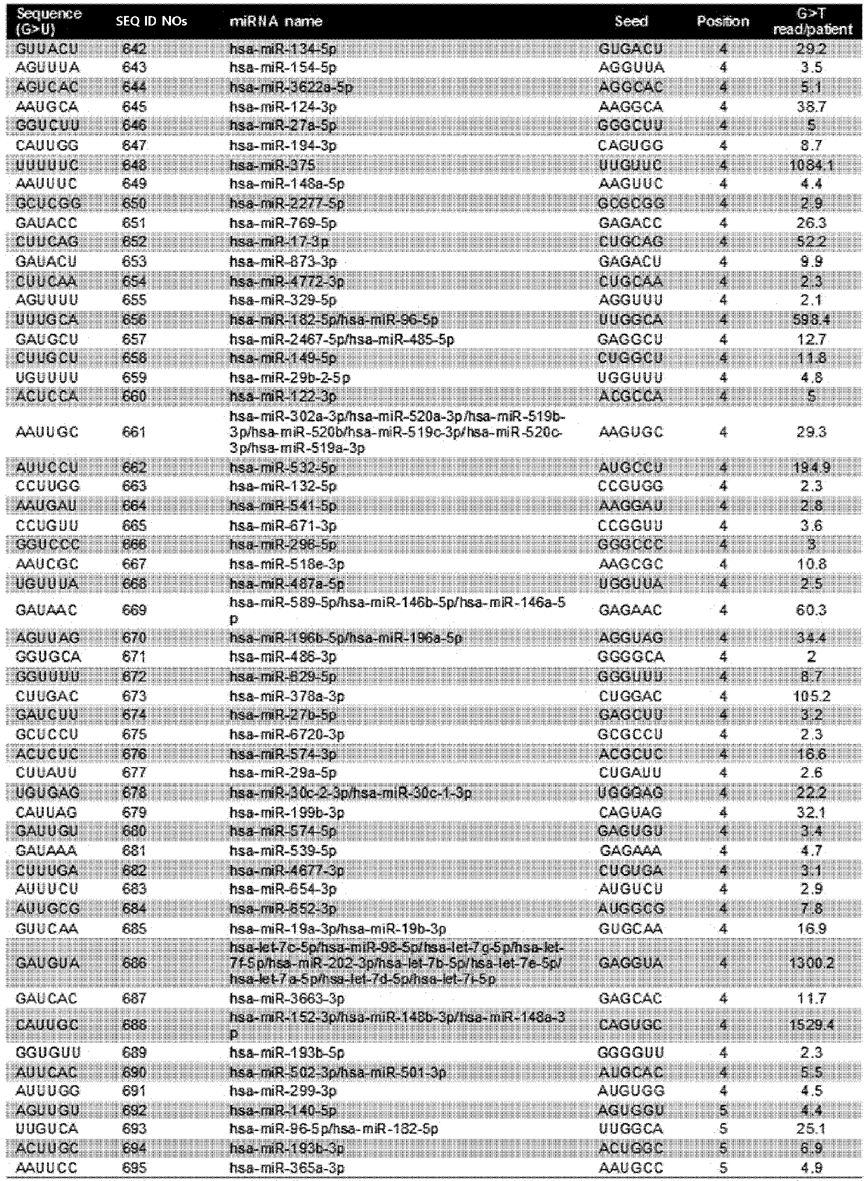

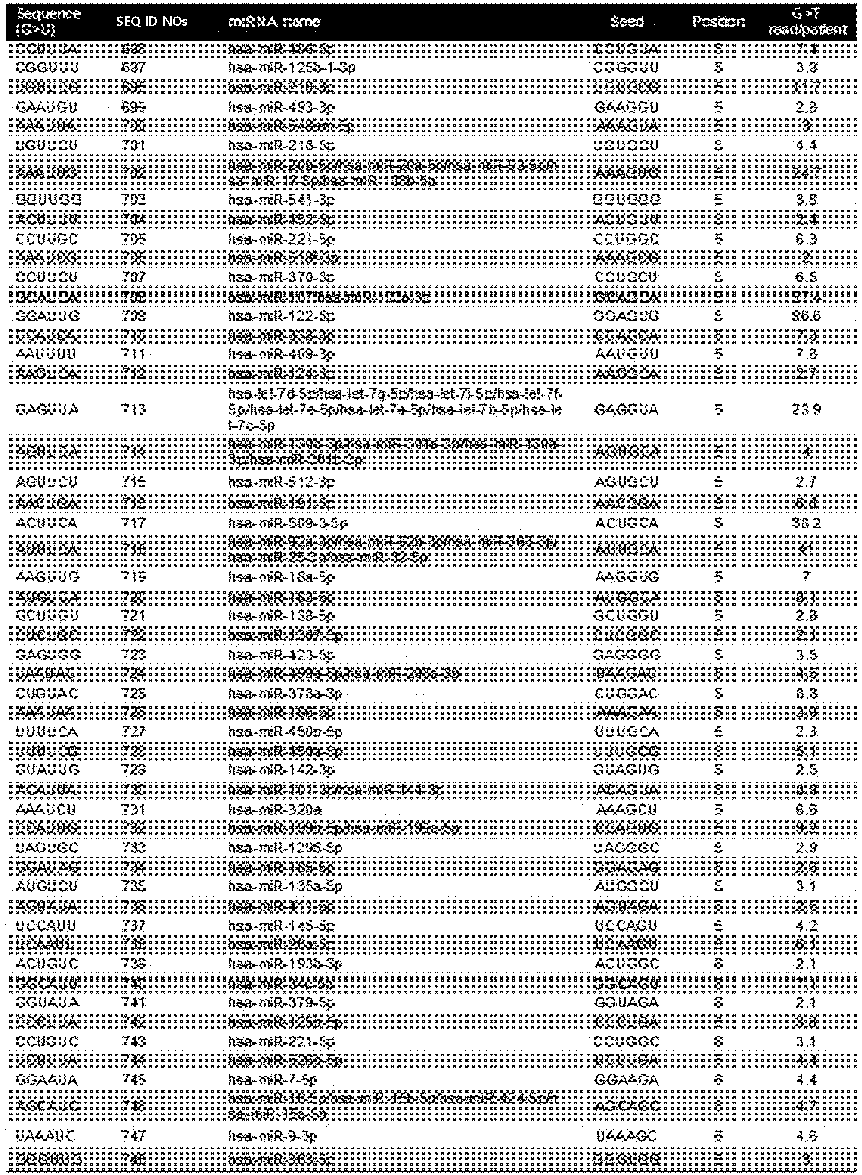

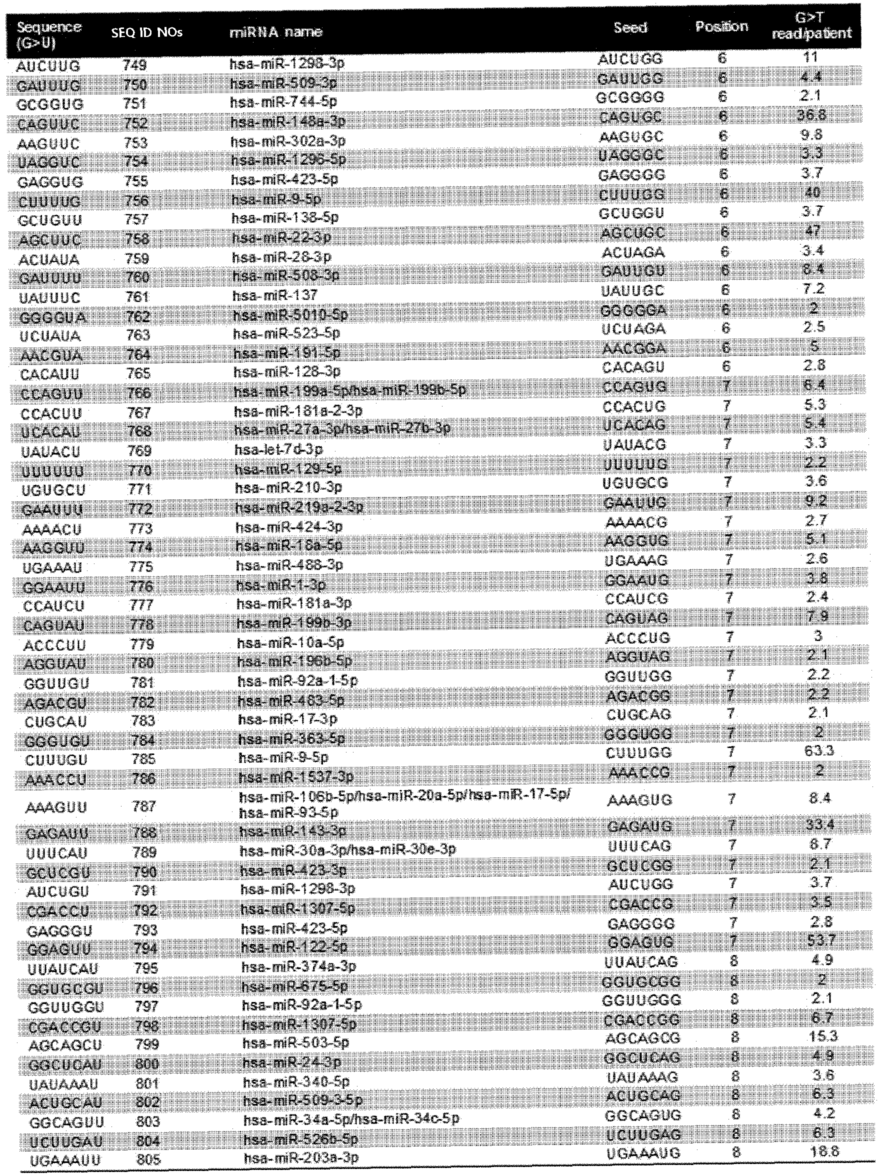

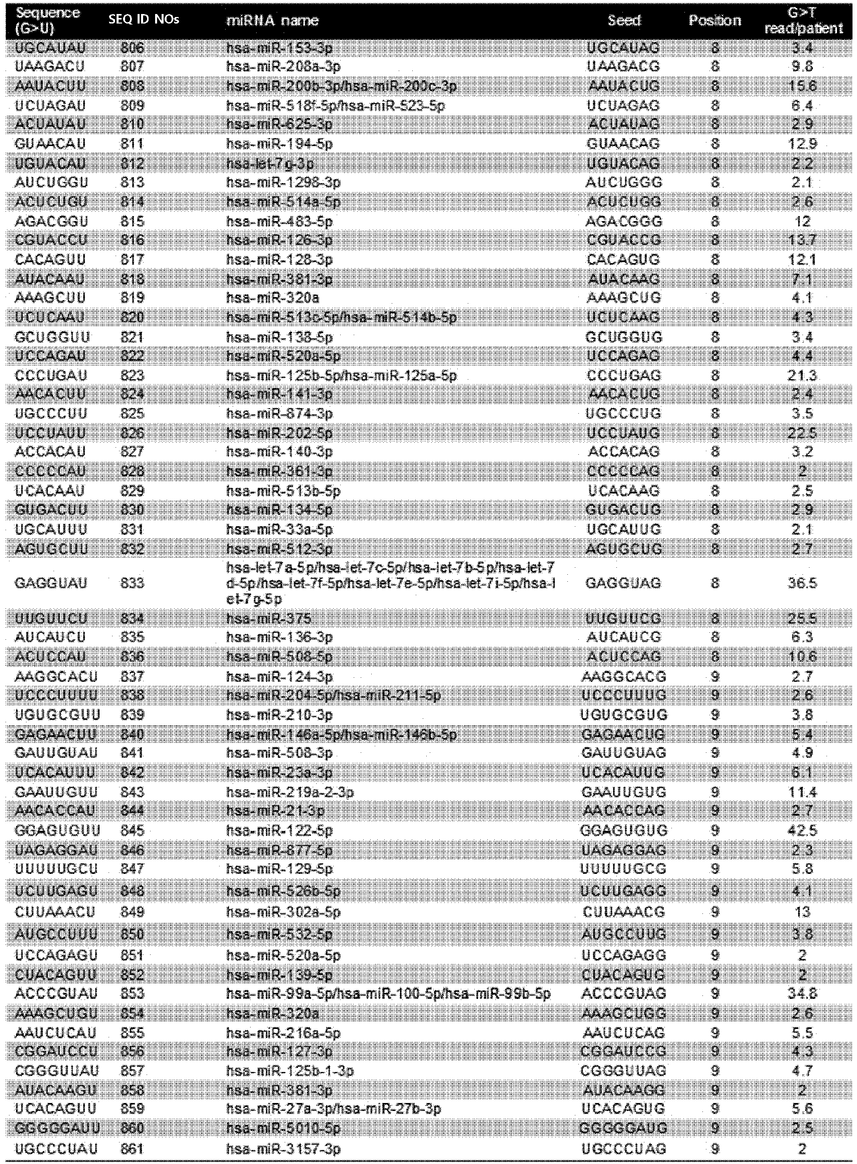

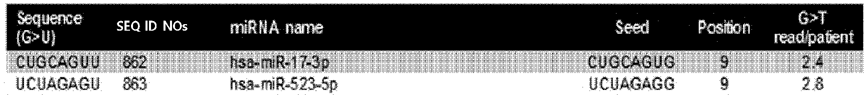

Through the miRNA sequencing variant analyses in the example, variant miRNAs recognizing a non-canonical G:A wobble seed target as a canonical target in various cancer patients were identified, and therefore, when the present invention (miRNA-GU) for modifying a miRNA sequence to recognize a non-canonical G:A wobble seed site as a canonical seed site is applied, a total of 335 sequences (sequence (G>U)) were made (Table 4), thereby completing the technology of exhibiting only the function of suppressing a non-canonical target of miRNA.

### [Industrial Applicability]

When an interference-inducing nucleic acid according to the present invention is used, a biological function exhibited by suppressing a non-canonical target gene of conventional miRNA may be effectively improved, or a part of the functions of the convention miRNA, that is, only a biological function exhibited by suppressing a non-canonical target gene may be selectively exhibited. In addition, cell cycling, differentiation, dedifferentiation, morphology, migration, proliferation or apoptosis may be regulated by the interference-inducing nucleic acid, and thus it is expected to be used in various fields such as pharmaceuticals, cosmetics, etc.

## Claims

1. An RNA interference-inducing nucleic acid, which suppresses a non-canonical target gene of microRNA (miRNA) by modifying a partial sequence of specific miRNA in one or more single strands of the double strands of the nucleic acid inducing RNA interference,
wherein the RNA interference-inducing nucleic acid has a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, to allow non-canonical target base pairs bound to a bulge generated in the target gene between positions 5 and 6 of the miRNA to be a consecutive base pair by the disappearance of the bulge, or
the RNA interference-inducing nucleic acid has a modified base sequence in which at least one guanine is substituted with uracil or adenine in a base sequence between the first to ninth bases from the 5' end of specific miRNA, in which a G:A or G:U wobble pair at the corresponding site becomes a canonical base sequence of U:A or A:U.

2. The RNA interference-inducing nucleic acid of claim 1, wherein the RNA interference-inducing nucleic acid selectively suppresses a non-canonical target gene of miRNA and does not suppress a canonical target gene of miRNA.

3. The RNA interference-inducing nucleic acid of claim 1, wherein the specific miRNA is one or more selected from the group consisting of miR-124, miR-155, miR-122, miR-1, let-7, miR-133, miR-302 and miR-372, which have the same seed sequence and consist of 18 to 24 bases.

4. The RNA interference-inducing nucleic acid of claim 1, wherein the RNA interference-inducing nucleic acid has the sequence from the 2^{nd} to 7^{th} bases from the 5' end, which is one or more selected from:
an RNA interference-inducing nucleic acid (miR-124-BS) having the base sequence of 5'-AA GGC C-3', which is an RNA interference-inducing nucleic acid suppressing a non-canonical target gene of miR-124;
an RNA interference-inducing nucleic acid (miR-122-BS) having the base sequence of 5'-GG AGU U-3', which is an RNA interference-inducing nucleic acid suppressing a non-canonical target gene of miR-122;
an RNA interference-inducing nucleic acid (miR-155-BS) having the base sequence of 5'-UA AUG G-3', which is an RNA interference-inducing nucleic acid suppressing a non-canonical target gene of miR-155; and
an RNA interference-inducing nucleic acid (miR-1-BS) having the base sequence of 5'-GG AAU U-3', which is an RNA interference-inducing nucleic acid suppressing a non-canonical target gene of miR-1.

5. The RNA interference-inducing nucleic acid of claim 4, wherein the RNA interference-inducing nucleic acid has any one or more base sequences as follows:
an RNA interference-inducing nucleic acid (miR-124-BS) having a base sequence of 5'-UAA GGC CAC GCG GUG AAU GCC-3' as an RNA interference-inducing nucleic acid suppressing a non-canonical target gene of miR-124;
an RNA interference-inducing nucleic acid (miR-122-BS) having a base sequence of 5'-UGG AGU UGU GAC AAU GGU GUU-3' as an RNA interference-inducing nucleic acid suppressing a non-canonical target gene of miR-122;
an RNA interference-inducing nucleic acid (miR-155-BS) having a base sequence of 5'-UUAAUG GC UAA U CGU GAU AGG-3' as an RNA interference-inducing nucleic acid suppressing a non-canonical target gene of miR-155; or
an RNA interference-inducing nucleic acid (miR-1-BS) having a base sequence of 5'-UGG AAU UGU AAA GAA GUA UGU-3' as an RNA interference-inducing nucleic acid suppressing a non-canonical target gene of miR-1.

6. The RNA interference-inducing nucleic acid of claim 1, wherein the RNA interference-inducing nucleic acid has any one or more base sequences between the 1^{st} to 9^{th} bases from the 5' end as follows:
as an RNA interference-inducing nucleic acid suppressing a non-canonical target gene of miR-124,
an RNA interference-inducing nucleic acid having a base sequence of 5'-UAA UGC ACG-3' (miR-124-G4U), 5'-UAA GUC ACG-3' (miR-124-G5U) or 5'-UAA UUC ACG-3' (miR-124-G4,5U);
as an RNA interference-inducing nucleic acid suppressing a non-canonical target gene of miR-1,
an RNA interference-inducing nucleic acid having a base sequence of 5'-UUG AAU GUA-3' (miR-1-G2U), 5'-UGU AAU GUA-3' (miR-1-G3U), 5'-UGG AAU UUA-3' (miR-1-G7U), 5'-UUU AAU GUA-3' (miR-1-G2,3U), 5'-UGU AAU UUA-3' (miR-1-G3,7U), 5'-UUG AAU UUA-3' (miR-1-G2,7U) or 5'-UUU AAU UUA-3' (miR-1-G2,3,7U);
as an RNA interference-inducing nucleic acid suppressing a non-canonical target gene of miR-122,
an RNA interference-inducing nucleic acid having a base sequence of 5'-UUG AGU GUG-3' (miR-122-G2U), 5'-UGU AGU GUG-3' (miR-122-G3U), 5'-UGG AUU GUG-3' (miR-122-G5U), 5'-UGG AGU UUG-3' (miR-122-G7U), 5'-UGG AGU GUU-3' (miR-122-G9U), 5'-UUU AGU GUG-3' (miR-122-G2,3U), 5'-UUG AUU GUG-3' (miR-122-G2,5U), 5'-UUGAGU UUG-3' (miR-122-G2,7U), 5'-UUGAGU GUU-3' (miR-122-G2,9U), 5'-UGU AUU GUG (miR-122-G3,5U), 5'-UGU AGU UUG-3' (miR-122-G3,7U), 5'-UGU AGU GUU-3' (miR-122-G3,9U), 5'-UGG AUU UUG-3' (miR-122-G5,7U), 5'-UGG AUU GUU-3' (miR-122-G5,9U) or 5'-UGG AGU UUU-3 (miR-122-G7,9U);
as an RNA interference-inducing nucleic acid suppressing a non-canonical target gene of miR-133,
an RNA interference-inducing nucleic acid having a base sequence of 5'-UUU UGU CCC-3' (miR-133-G4U), 5'-UUU GUU CCC-3' (miR-133-G5U) or 5'-UUU UUU CCC-3'(miR-133-G4,5U);
as an RNA interference-inducing nucleic acid suppressing a non-canonical target gene of let-7,
an RNA interference-inducing nucleic acid having a base sequence of 5'-UUA GGU AGU-3' (let-7-G2U), 5'-UGA UGU AGU-3' (let-7-G4U), 5'-UGAGUU AGU-3' (let-7-G5U), 5'-UGA GGU AUU-3' (let-7-G8U), 5'-UUA UGU AGU-3' (let-7-G2,4U), 5'-UUA GUU AGU-3' (let-7-G2,5U), 5'-UUA GGU AUU-3' (let-7-G2,8U), 5'-UGA UUU AGU-3' (let-7-G4,5U), 5'-UGA UGU AUU-3' (let-7-G4,8U) or 5'-UGAGUU AUU-3' (let-7-G5,8U);
as an RNA interference-inducing nucleic acid suppressing a non-canonical target gene of miR-302a,
an RNA interference-inducing nucleic acid having a base sequence of 5'-UAA UUG CUU-3' (miR-302a-G4U), 5'-UAAGUU CUU-3' (miR-302a-G6U), or 5'-UAA UUU CUU-3' (miR-302a-G4,6U); and
as an RNA interference-inducing nucleic acid suppressing a non-canonical target gene of miR-372,
an RNA interference-inducing nucleic acid having a base sequence of 5'-AAA UUG CUG-3' (miR-372-G4U), 5'-AAA GUU CUG-3' (miR-372-G6U), 5'-AAA GUG CUU-3' (miR-372-G9U), 5'-AAA UUU CUG-3' (miR-372-G4,6U), 5'-AAA UUG CUU-3' (miR-372-G4,9U) or 5'-AAAGUU CUU-3' (miR-372-G6,9U).

7. The RNA interference-inducing nucleic acid of claim 6, wherein the RNA interference-inducing nucleic acid has one or more base sequences as follows:
as an RNA interference-inducing nucleic acid suppressing a non-canonical target gene of miR-124,
an RNA interference-inducing nucleic acid having a base sequence of 5'-UAA UGC ACG CGG UGAAUG CCAA-3' (miR-124-G4U), 5'-UAAGUCACG CGG UGAAUG CCA A-3'(miR-124-G5U) or 5'-UAAUUC ACG CGG UGAAUG CCAA-3'(miR-124-G4,5U);
as an RNA interference-inducing nucleic acid suppressing a non-canonical target gene of miR-1,
an RNA interference-inducing nucleic acid having a base sequence of 5'-UUG AAU GUAAAG AAG UAU GUA U-3' (miR-1-G2U), 5'-UGU AAU GUA AAG AAG UAU GUA U-3' (miR-1-G3U), 5'-UGG AAU UUAAAGAAG UAU GUA U-3' (miR-1-G7U), 5'-UUU AAU GUA AAG AAG UAU GUA U-3' (miR-1-G2,3U), 5'-UGU AAU UUA AAG AAG UAU GUA U-3' (miR-1-G3,7U), 5'-UUG AAU UUA AAG AAG UAU GUA U-3' (miR-1-G2,7U) or 5'-UUU AAU UUA AAG AAG UAU GUA U-3' (miR-1-G2,3,7U);
as an RNA interference-inducing nucleic acid suppressing a non-canonical target gene of miR-122, an RNA interference-inducing nucleic acid having a base sequence of 5'-UUG AGU GUG ACA AUG GUG UUU G-3' (miR-122-G2U), 5'-UGU AGU GUG ACA AUG GUG UUU G-3 (miR-122-G3U), 5'-UGG AUU GUG ACAAUG GUG UUU G-3' (miR-122-G5U), 5'-UGG AGU UUG ACAAUG GUG UUU G-3' (miR-122-G7U), 5'-UGG AGU GUU ACAAUG GUG UUU G-3' (miR-122-G9U), 5'-UUU AGU GUG ACAAUG GUG UUU G-3' (miR-122-G2,3U), 5'-UUG AUU GUG ACAAUG GUG UUU G-3' (miR-122-G2,5U), 5'-UUG AGU UUG ACAAUG GUG UUU G-3' (miR-122-G2,7U), 5'-UUG AGU GUU ACA AUG GUG UUU G-3' (miR-122-G2,9U), 5'-UGU AUU GUG ACA AUG GUG UUU G-3 (miR-122-G3,5U), 5'-UGU AGU UUG ACA AUG GUG UUU G-3 (miR-122-G3,7U), 5'-UGU AGU GUU ACA AUG GUG UUU G-3 (miR-122-G3,9U), 5'-UGG AUU UUG ACA AUG GUG UUU G-3' (miR-122-G5,7U), 5'-UGG AUU GUU ACA AUG GUG UUU G-3' (miR-122-G5,9U) or 5'-UGG AGU UUU ACAAUG GUG UUU G-3' (miR-122-G7,9U);
as an RNA interference-inducing nucleic acid suppressing a non-canonical target gene of miR-133, an RNA interference-inducing nucleic acid having a base sequence of 5'-UUU UGU CCC CUU CAA CCA GCU G -3' (miR-133-G4U), 5'-UUU GUU CCC CUU CAA CCA GCU G-3' (miR-133-G5U) or 5'-UUU UUU CCC CUU CAA CCA GCU G-3'(miR-133-G4,5U);
as an RNA interference-inducing nucleic acid suppressing a non-canonical target gene of let-7, an RNA interference-inducing nucleic acid having a base sequence of 5'-UUA GGU AGU AGG UUG UAU AGU U-3' (let-7-G2U), 5'-UGA UGU AGU AGG UUG UAU AGU U-3' (let-7-G4U), 5'-UGA GUU AGU AGG UUG UAU AGU U-3' (let-7-G5U), 5'-UGA GGU AUU AGG UUG UAU AGU U-3' (let-7-G8U), 5'-UUA UGU AGU AGG UUG UAU AGU U-3' (let-7-G2,4U), 5'-UUA GUU AGU AGG UUG UAU AGU U-3' (let-7-G2,5U), 5'-UUA GGU AUU AGG UUG UAU AGU U-3' (let-7-G2,8U), 5'-UGA UUU AGU AGG UUG UAU AGU U-3' (let-7-G4,5U), 5'-UGA UGU AUU AGG UUG UAU AGU U-3' (let-7-G4,8U) or 5'-UGA GUU AUU AGG UUG UAU AGU U-3' (let-7-G5,8U);
as an RNA interference-inducing nucleic acid suppressing a non-canonical target gene of miR-302a, an RNA interference-inducing nucleic acid having a base sequence of 5'-UAA UUG CUU CCA UGU UUU GGU GA-3' (miR-302a-G4U), 5'-UAA GUU CUU CCA UGU UUU GGU GA-3' (miR-302a-G6U) or 5'-UAA UUU CUU CCA UGU UUU GGU GA-3' (miR-302a-G4,6U); and
as an RNA interference-inducing nucleic acid suppressing a non-canonical target gene of miR-372, an RNA interference-inducing nucleic acid having a base sequence of 5'-AAA UUG CUG CGA CAU UUG AGC GU -3' (miR-372-G4U), 5'-AAA GUU CUG CGA CAU UUG AGC GU -3' (miR-372-G6U), 5'-AAA GUG CUU CGA CAU UUG AGC GU -3' (miR-372-G9U), 5'-AAA UUU CUG CGA CAU UUG AGC GU -3' (miR-372-G4,6U), 5'-AAA UUG CUU CGA CAU UUG AGC GU -3' (miR-372-G4,9U) or 5'-AAA GUU CUU CGA CAU UUG AGC GU -3' (miR-372-G6,9U).

8. A composition for inhibiting the expression of a non-canonical target gene of microRNA (miRNA), comprising the RNA interference-inducing nucleic acid of any one of claims 1 to 7.

9. A composition for regulating cell cycling, differentiation, dedifferentiation, morphology, migration, division, proliferation or apoptosis, comprising the RNA interference-inducing nucleic acid of any one of claims 1 to 7.

10. The composition of claim 9, wherein the composition is any one or more selected from:
a composition for inducing cancer cell death, which comprises an RNA interference-inducing nucleic acid suppressing a non-canonical target gene of miR-124;
a composition for inducing neurite or dendrite differentiation, which comprises an RNA interference-inducing nucleic acid suppressing a non-canonical target gene of miR-124;
a composition for inducing cell cycle arrest in liver cancer cells, which comprises an RNA interference-inducing nucleic acid suppressing a non-canonical target gene of miR-122;
a composition for promoting differentiation of muscle cells or muscle fibrosis, which comprises an RNA interference-inducing nucleic acid suppressing a non-canonical target gene of miR-1;
a composition for inducing muscle cell death, which comprises an RNA interference-inducing nucleic acid suppressing a non-canonical target gene of miR-155;
a composition for inducing cell death of neuroblastomas, which comprises an RNA interference-inducing nucleic acid suppressing a non-canonical target gene of miR-124;
a composition for promoting cell division or proliferation of neuroblastomas, which comprises an RNA interference-inducing nucleic acid suppressing a non-canonical target gene of miR-124;
a composition for inducing myocardial hypertrophy, which comprises an RNA interference-inducing nucleic acid suppressing a non-canonical target gene of miR-1;
a composition for inducing myocardial hypertrophy, which includes an RNA interference-inducing nucleic acid suppressing a non-canonical target gene of miR-133;
a composition for inducing cell cycle arrest in cancer cells, which includes an RNA interference-inducing nucleic acid suppressing a non-canonical target gene of let-7;
a composition for inducing the cell cycle progressing activity of hepatocytes, which includes an RNA interference-inducing nucleic acid suppressing a non-canonical target gene of let-7;
a composition for promoting dedifferentiation, which includes an RNA interference-inducing nucleic acid suppressing a non-canonical target gene of miR-302a;
a composition for promoting dedifferentiation, which includes an RNA interference-inducing nucleic acid suppressing a non-canonical target gene of miR-372; and
a composition for inhibiting cell migration of liver cancer cells, which includes an RNA interference-inducing nucleic acid suppressing a non-canonical target gene of miR-122.

11. A method of preparing an RNA interference-inducing nucleic acid inhibiting the expression of a non-canonical target gene of microRNA (miRNA) by modifying a partial sequence of specific miRNA in one or more single strands of the double strands of the nucleic acid inducing RNA interference, the method comprising the following steps:
constructing an RNA interference-inducing nucleic acid having a sequence of four bases in positions 2 to 5 from the 5' end of specific miRNA and bases in positions 6 and 7, which are the same and complementary to a base capable of pairing with the 6^{th} base of the specific miRNA, including all complementary bases including G:Aand G:U wobble pairs, to allow non-canonical target base pairs bound to a bulge generated in the target gene between positions 5 and 6 of the miRNA to be a consecutive base pair by the disappearance of the bulge, or
constructing an RNA interference-inducing nucleic acid having a modified base sequence in which at least one guanine is substituted with uracil in a base sequence between the first to ninth bases from the 5' end of specific miRNA, in which a G:A or G:U wobble pair at the corresponding site becomes a canonical base sequence of U:A or A:U.

12. A method of screening a test material for regulating cell cycling, differentiation, dedifferentiation, morphology, migration, division, proliferation or apoptosis, comprising:
transfecting the RNA interference-inducing nucleic acid of any one of claims 1 to 7 into target cells;
treating the target cells with a test material; and
confirming an expression level or expression of a non-canonical target gene of microRNA (miRNA) inhibited by the RNA interference-inducing nucleic acid in the target cells.

13. An RNA interference-inducing nucleic acid suppressing a non-canonical target gene of microRNA (miRNA) by modifying a partial sequence of specific miRNA in one or more single strands of the double strands of a nucleic acid inducing RNA interference,
wherein the RNA interference-inducing nucleic acid has a sequence of four bases in positions 2 to 5 from the 5' end of the specific miRNA, and bases in positions 6 and 7, which are the same and complementary to a base capable of pairing with the 6^{th} base of specific miRNA, including all complementary bases including a G:A or G:U wobble pair, to allow non-canonical target base pairs bound to a bulge generated in the target gene between positions 5 and 6 of the miRNA to be a consecutive base pair by the disappearance of the bulge, and
the specific miRNA has a methyl group (2'OMe) added to the 2' position of the ribosyl ring of the 6^{th} nucleotide from the 5' end.

14. The RNA interference-inducing nucleic acid of claim 13, wherein the RNA interference-inducing nucleic acid inhibits only the expression of a canonical seed target gene of the corresponding RNA interference-inducing nucleic acid.

15. The RNA interference-inducing nucleic acid of claim 13, wherein the RNA interference-inducing nucleic acid specifically suppresses only a non-canonical nucleation bulge site of the specific miRNA, and removes the non-canonical nucleation bulge site which is able to be newly generated.

16. A composition for inhibiting the expression of a non-canonical target gene of microRNA (miRNA), which includes the RNA interference-inducing nucleic acid of any one of claims 13 to 15.

17. A method of preparing an RNA interference-inducing nucleic acid, which inhibits the expression of a non-canonical target gene of microRNA (miRNA) by modifying a partial sequence of specific miRNA in one or more single strands of the double strands of a nucleic acid inducing RNA interference, the method comprising the following steps:
constructing an RNA interference-inducing nucleic acid having a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:Aand G:U wobble pairs, to allow non-canonical target base pairs bound to a bulge generated in the target gene between positions 5 and 6 of the miRNA to be a consecutive base pair by the disappearance of the bulge; and
adding a methyl group (2'OMe) to the 2' position of the ribosyl ring of the 6^{th} nucleotide from the 5' end.

18. An RNA interference-inducing nucleic acid, which suppresses a non-canonical target gene of microRNA (miRNA) by modifying a partial sequence of specific miRNA in one or more single strands of the double strands of a nucleic acid inducing RNA interference,
wherein the RNA interference-inducing nucleic acid has a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:A and G:U wobble pairs, to allow non-canonical target base pairs bound to a bulge generated in the target gene between positions 5 and 6 of the miRNA to be a consecutive base pair by the disappearance of the bulge.

19. The RNA interference-inducing nucleic acid of claim 18, wherein the RNA interference-inducing nucleic acid selectively suppresses a non-canonical nucleation bulge target site and does not suppress a canonical target gene of miRNA.

20. The RNA interference-inducing nucleic acid of claim 18, wherein the RNA interference-inducing nucleic acid has a base sequence represented by any one of SEQ ID NOs: 103 to 528, shown in the following table:

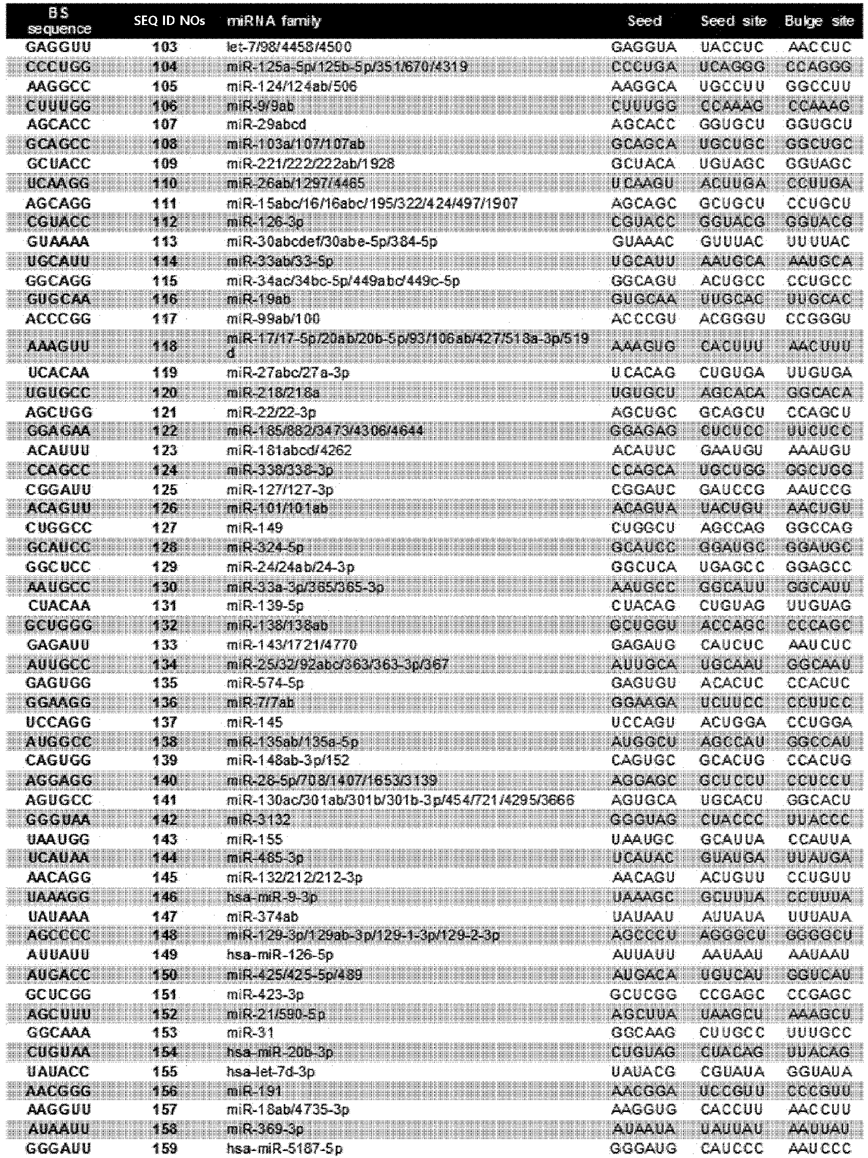

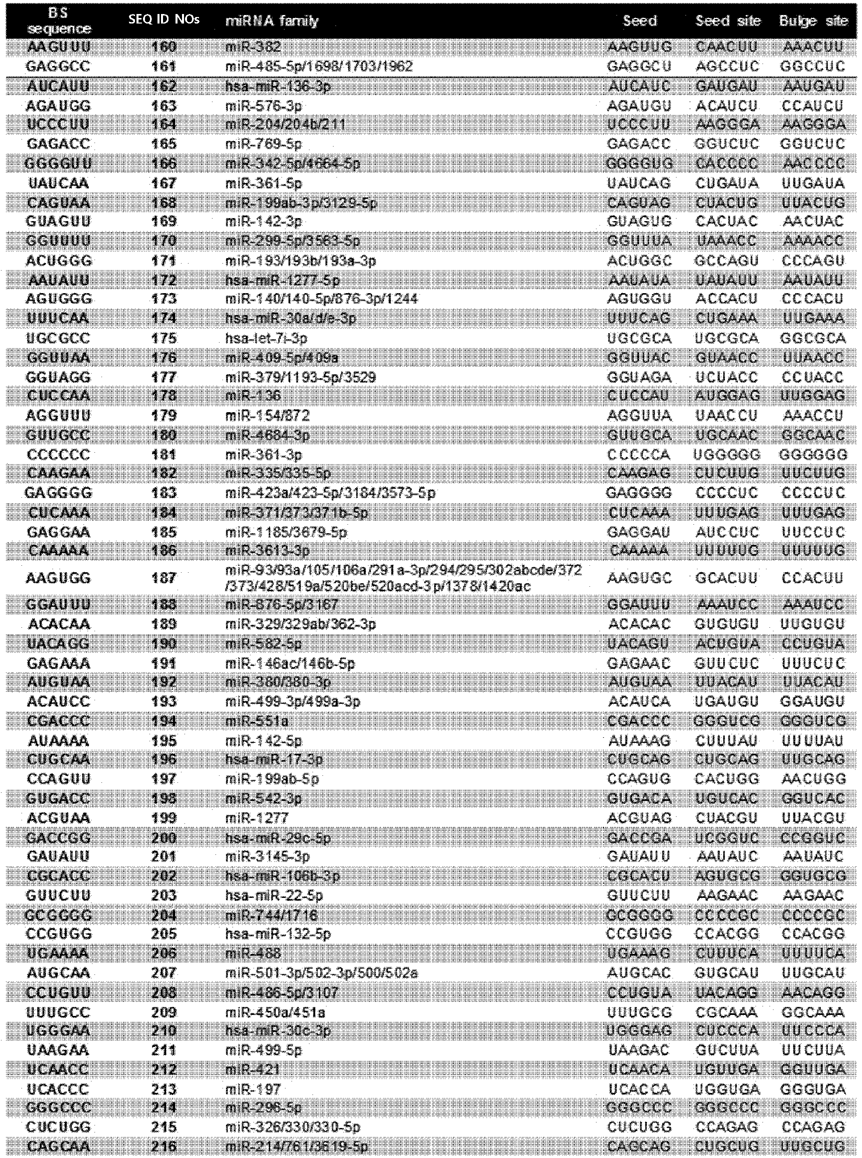

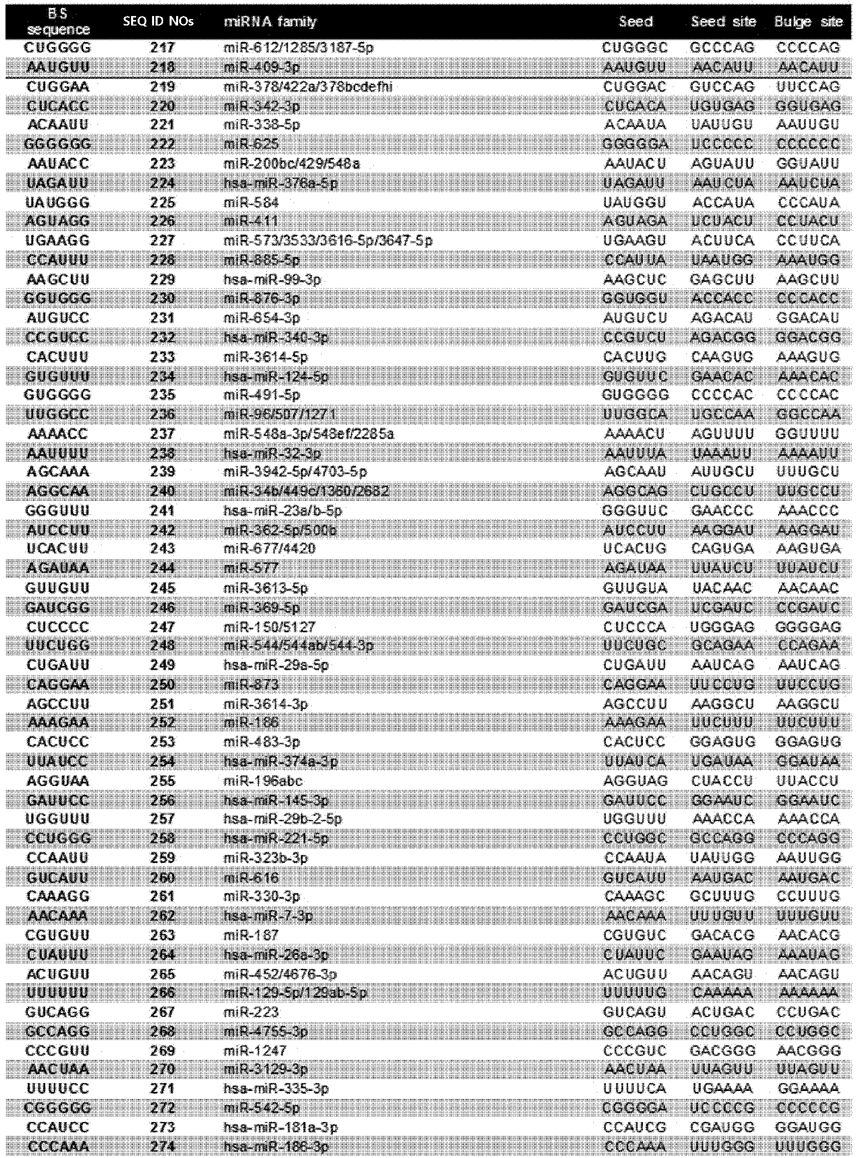

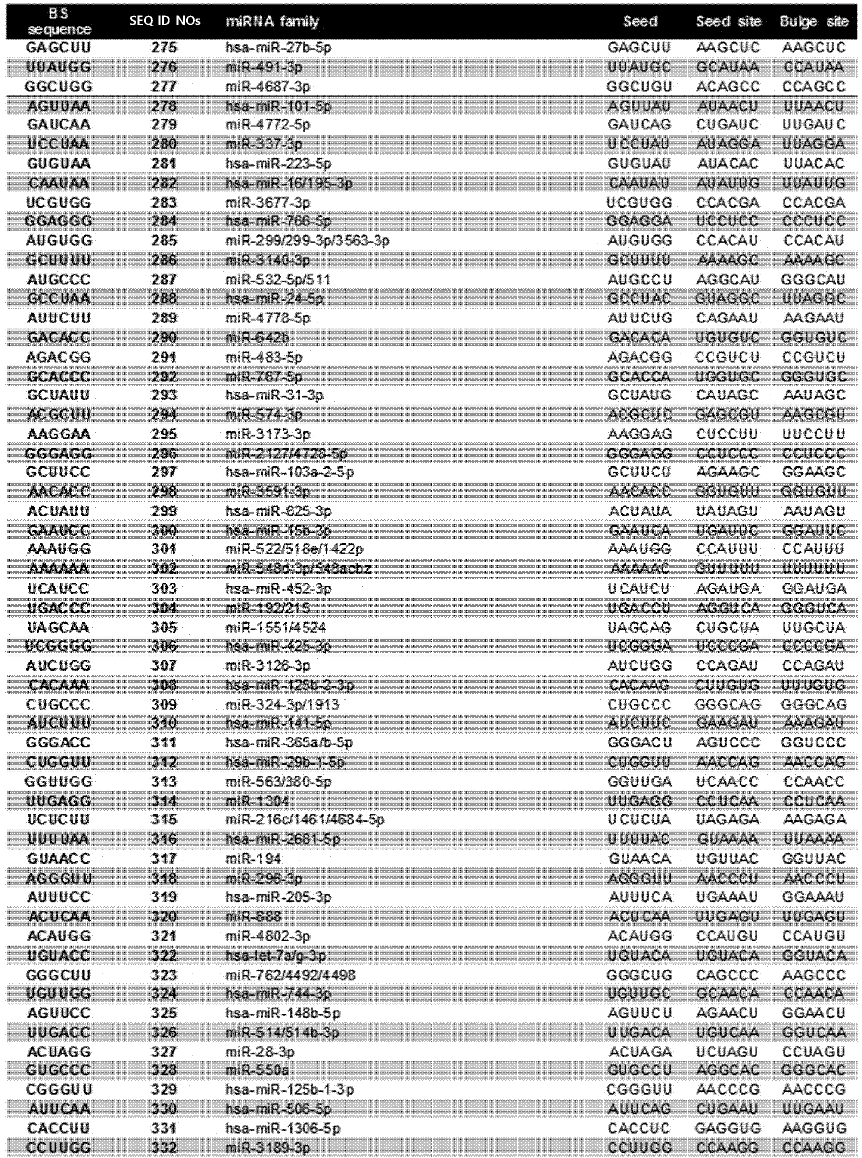

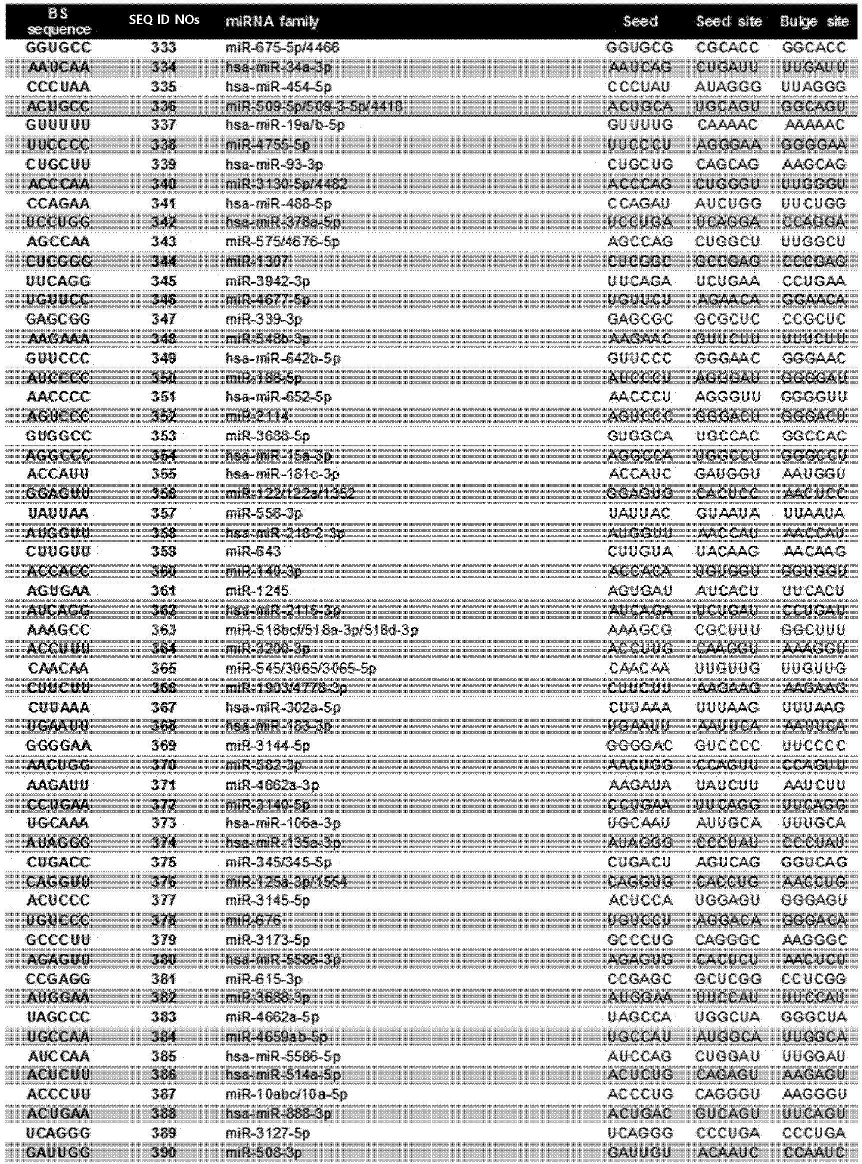

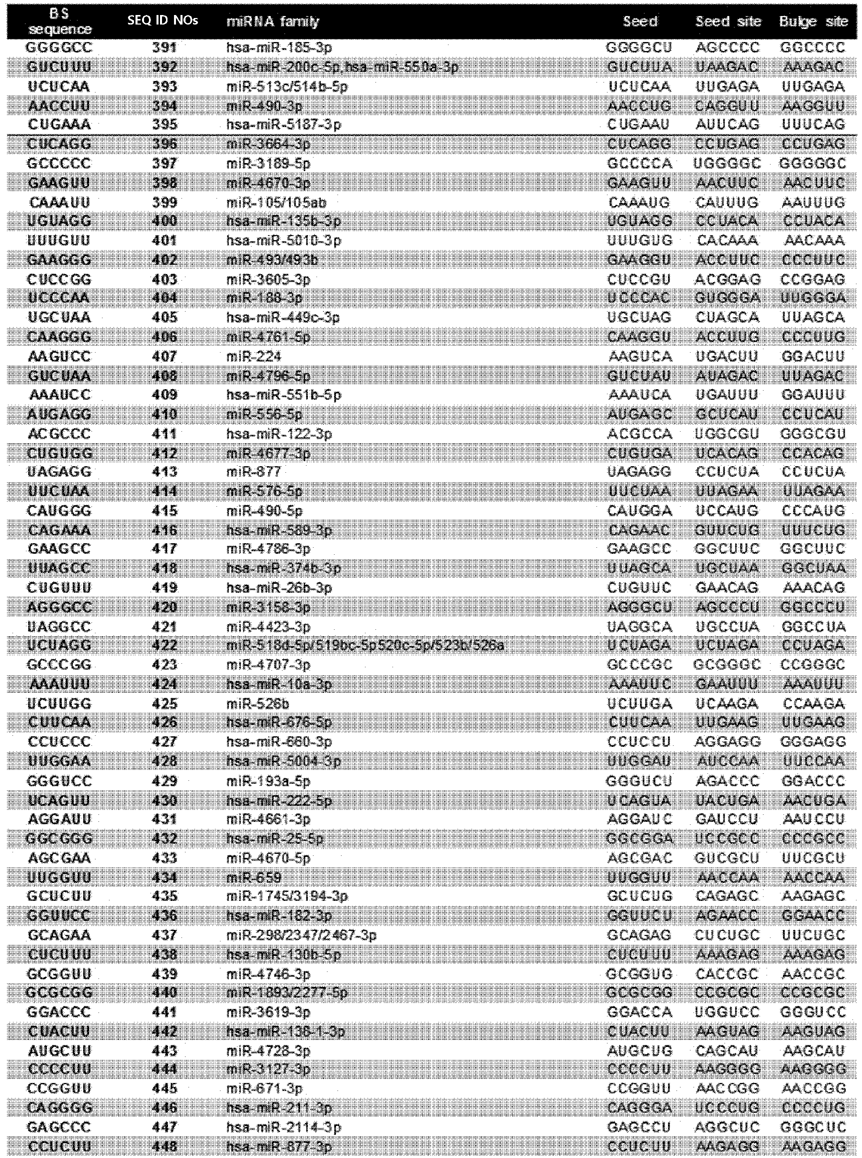

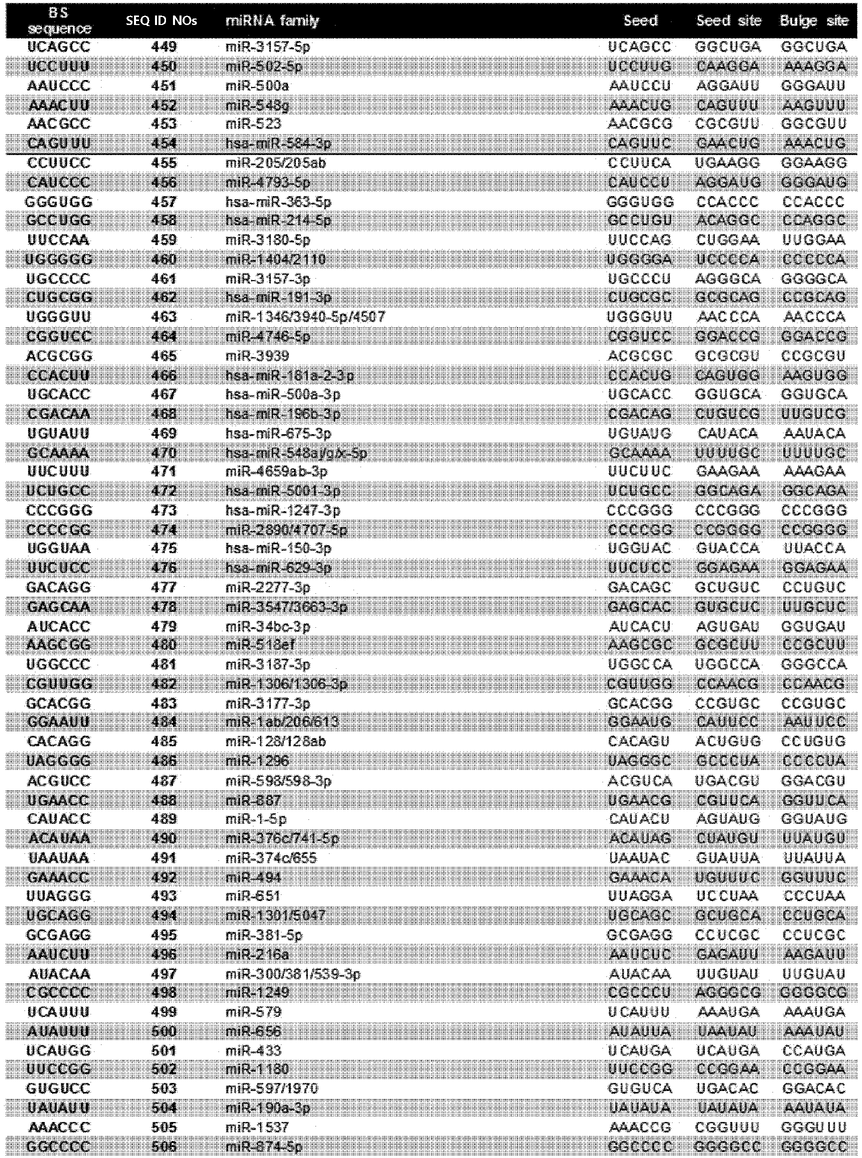

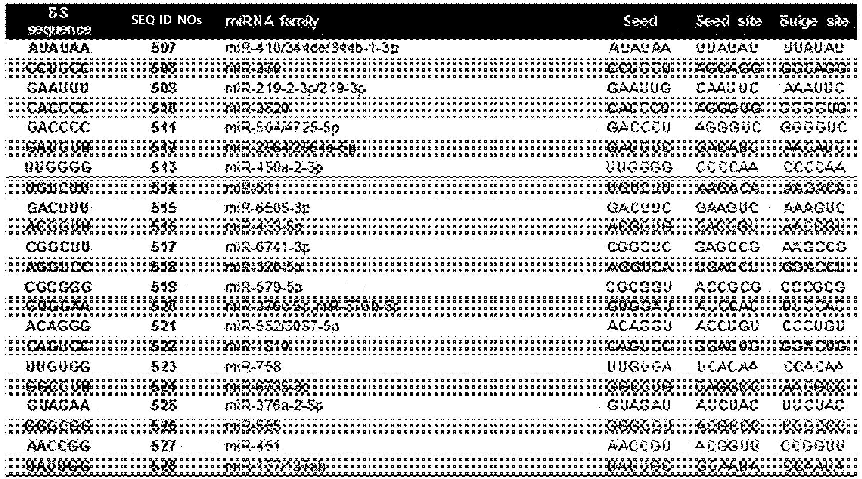

21. A composition for inhibiting the expression of a non-canonical target gene of microRNA (miRNA), comprising the RNA interference-inducing nucleic acid of any one of claims 18 to 20.

22. A method of preparing an RNA interference-inducing nucleic acid, which inhibits the expression of a non-canonical target gene of microRNA (miRNA) by modifying a partial sequence of specific miRNA in one or more single strands of the double strands of a nucleic acid inducing RNA interference, the method comprising the following steps:
constructing an RNA interference-inducing nucleic acid having a sequence of four bases in positions 2 to 5 from the 5' end of the specific miRNA, and bases in positions 6 and 7, which are the same and complementary to a base capable of pairing with the 6^{th} base of the specific miRNA, including all complementary bases including G:Aand G:U wobble pairs, to allow non-canonical target base pairs bound to a bulge generated in the target gene between positions 5 and 6 of the miRNA to be a consecutive base pair by the disappearance of the bulge.

23. An RNA interference-inducing nucleic acid suppressing a non-canonical target gene of microRNA (miRNA) by modifying a partial sequence of specific miRNA in one or more single strands of the double strands of a nucleic acid inducing RNA interference,
wherein the RNA interference-inducing nucleic acid has a modified base sequence in which at least one guanine is substituted with uracil in a base sequence between the second to ninth bases from the 5' end of specific miRNA, and the G:A wobble at the corresponding site becomes the canonical base pair of U:A.

24. The RNA interference-inducing nucleic acid of claim 23, wherein the RNA interference-inducing nucleic acid has a sequence of 6 to 8 consecutive bases, starting from the 2^{nd} base from the 5' end of specific miRNA, and
the base sequence has at least one guanine base substituted with an uracil base.

25. The RNA interference-inducing nucleic acid of claim 23, wherein the RNA interference-inducing nucleic acid selectively suppresses only a non-canonical target gene of miRNA binding in a G:A wobble pair, and does not suppress a canonical target gene of miRNA.

26. The RNA interference-inducing nucleic acid of claim 24, wherein the RNA interference-inducing nucleic acid has the sequence of 6 to 8 consecutive bases, starting from the 2^{nd} base from the 5' end, represented by any one or more of SEQ ID NOs: 529 to 863 shown in the following table.

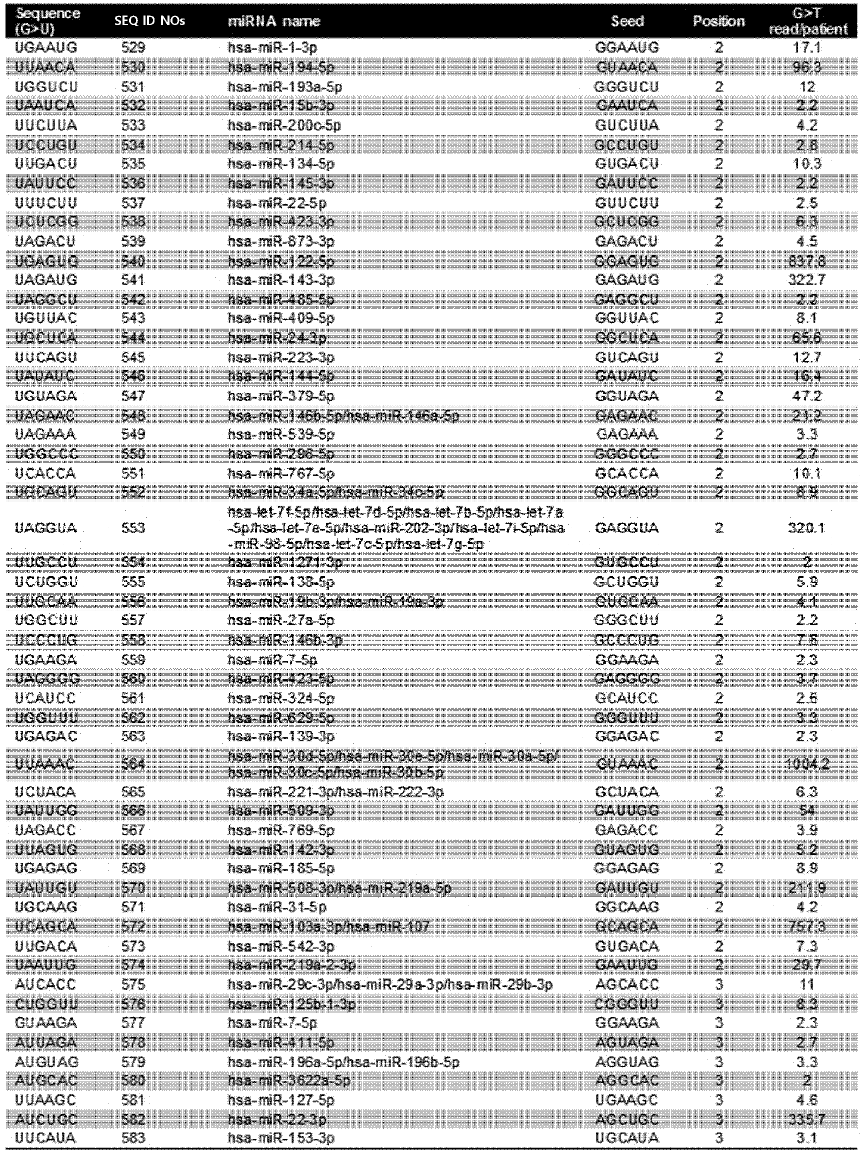

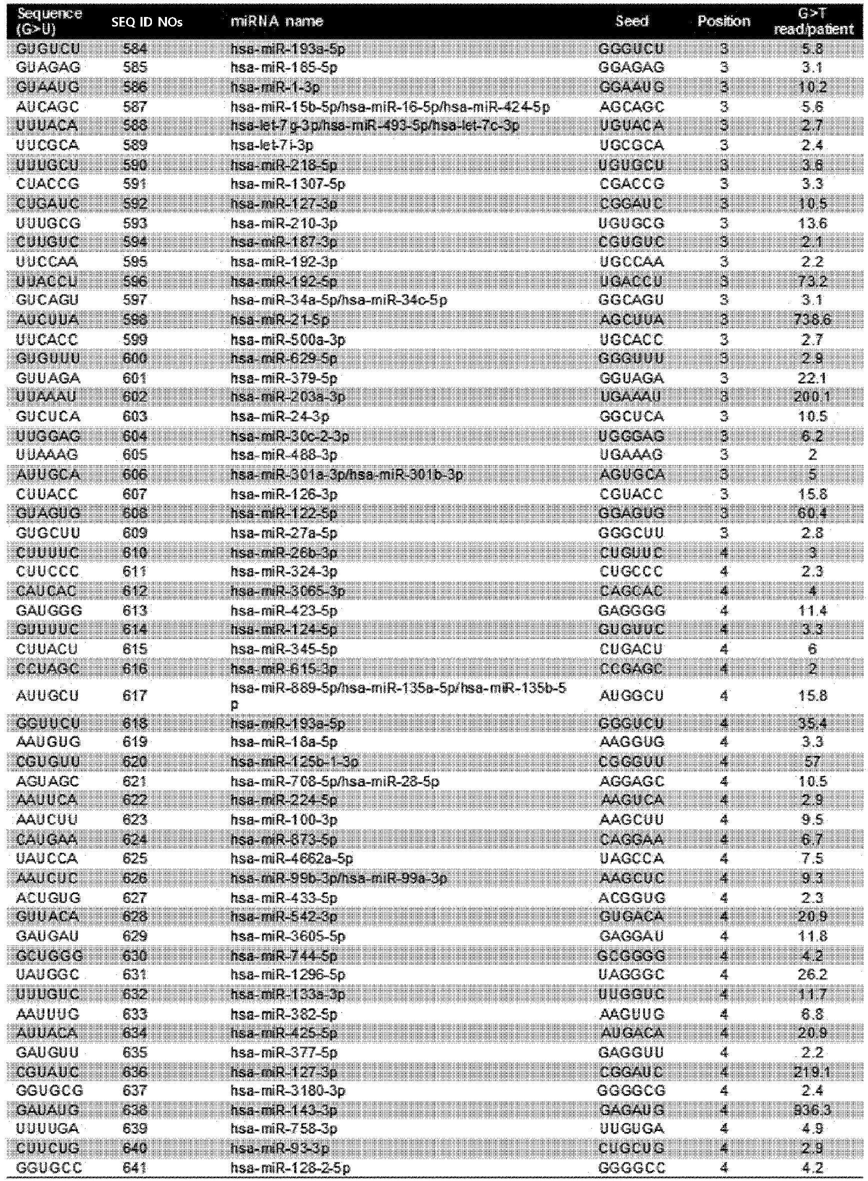

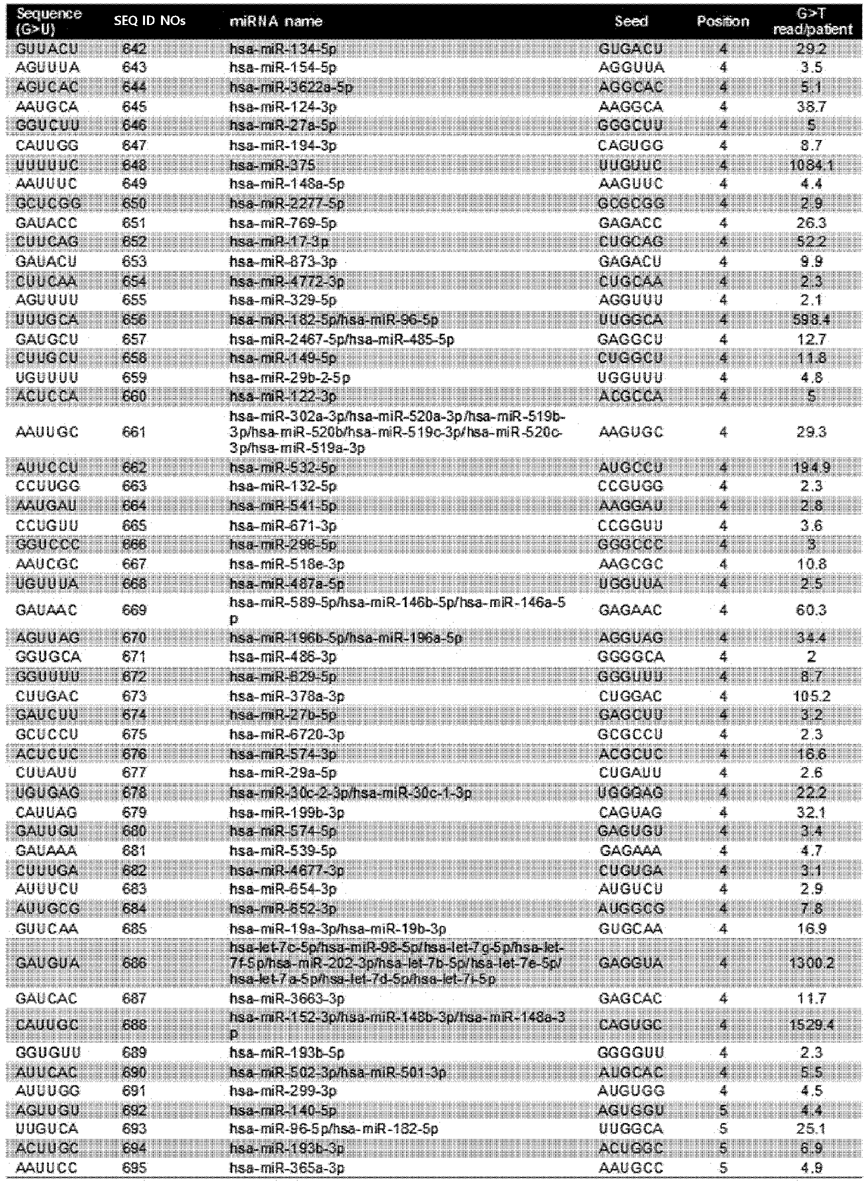

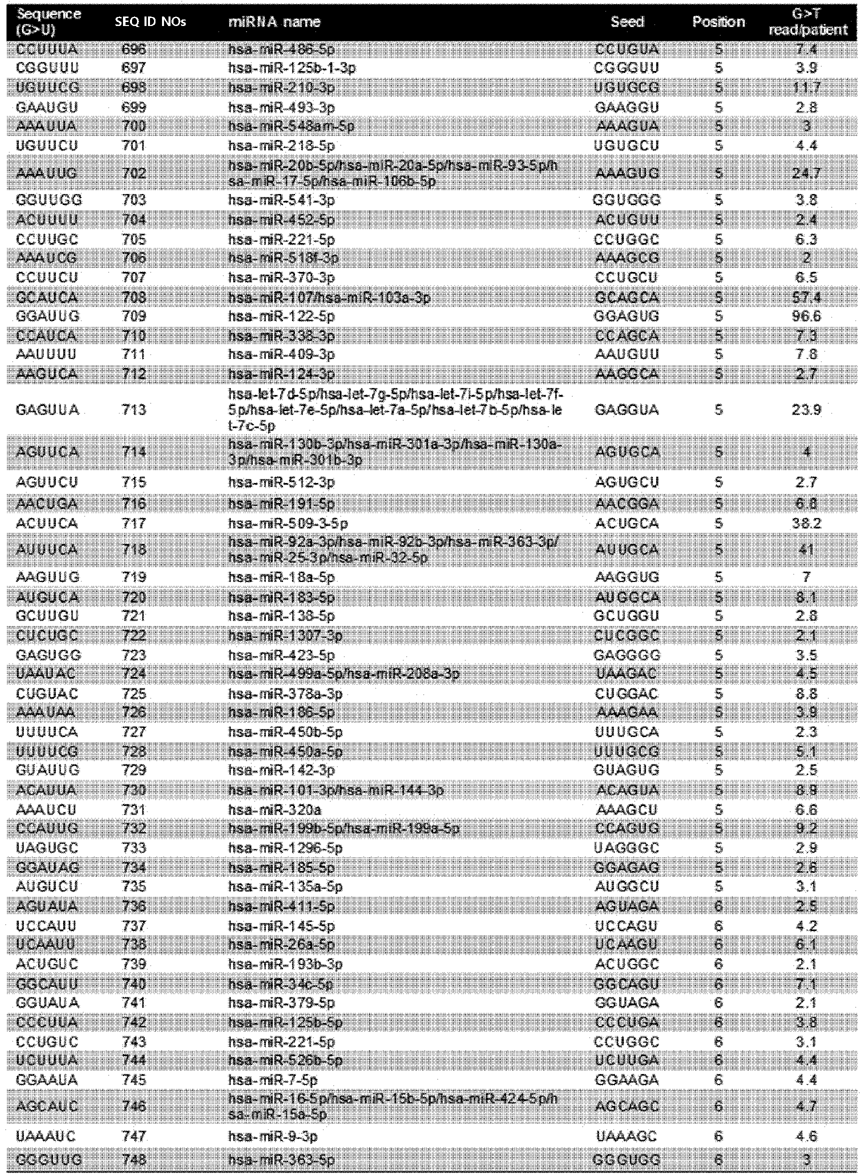

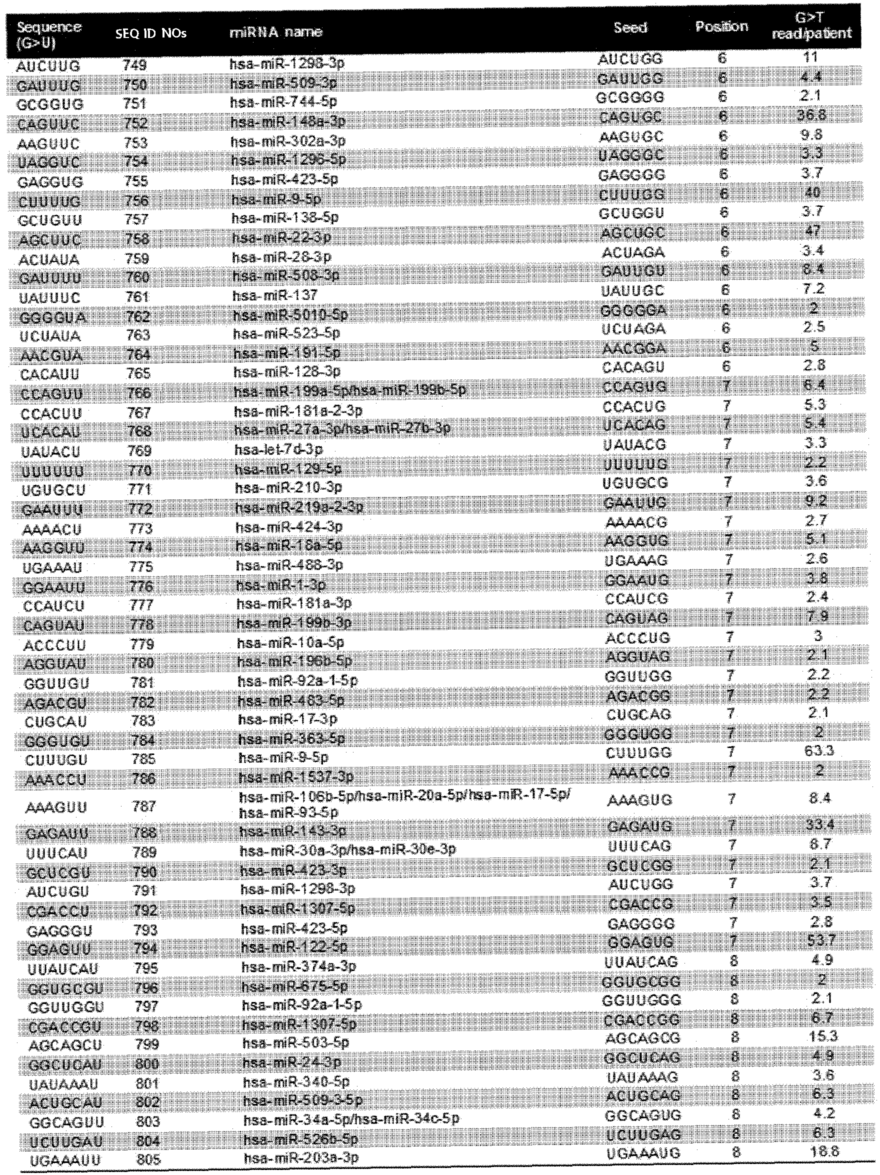

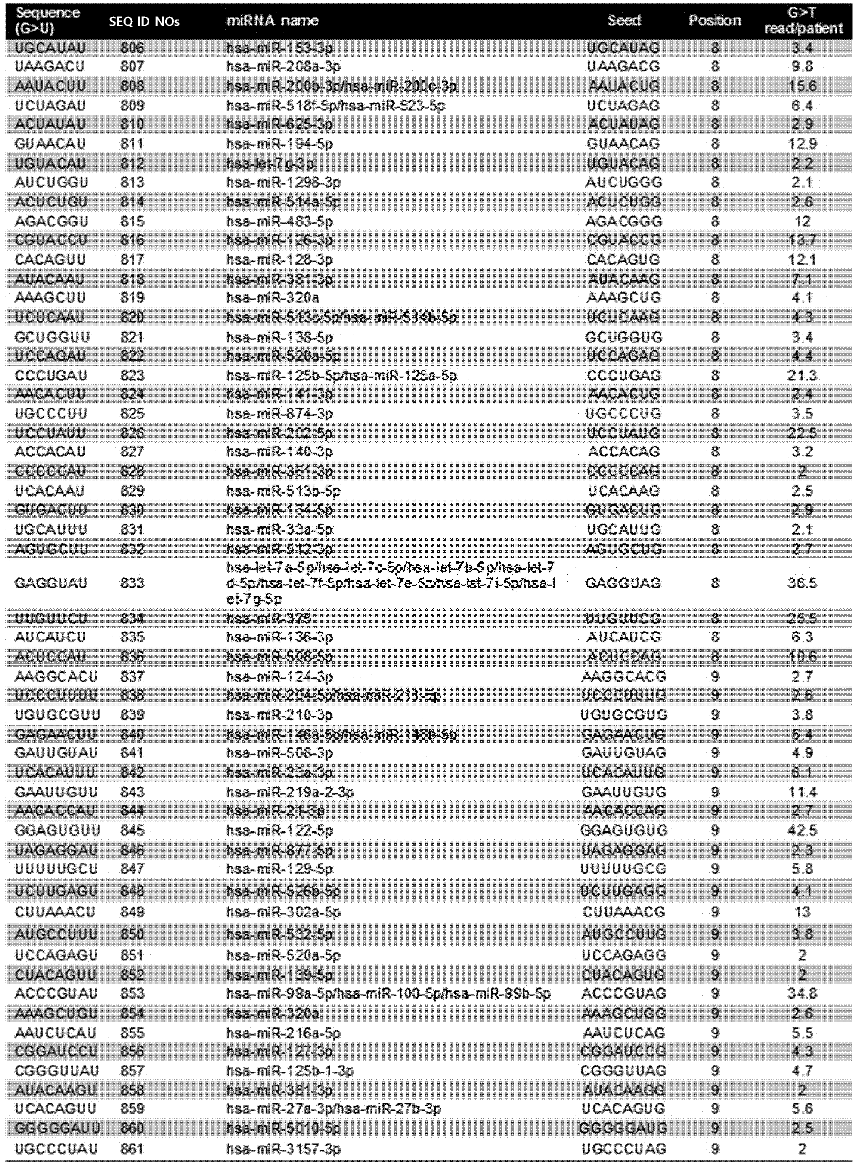

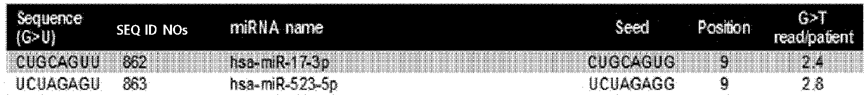

27. A composition for inhibiting the expression of a non-canonical target gene of microRNA (miRNA), comprising: the RNA interference-inducing nucleic acid of any one of claims 23 to 26.

28. A method of preparing an RNA interference-inducing nucleic acid inhibiting the expression of a non-canonical target gene of microRNA (miRNA) by modifying a partial sequence of specific miRNA in one or more single strands of the double strands of a nucleic acid inducing RNA interference, the method comprising the following steps:
constructing an RNA interference-inducing nucleic acid to have a modified base sequence in which at least one guanine base is substituted with an uracil base in a sequence of 6 to 8 consecutive bases, starting from the second base from the 5' end of specific miRNA.

29. A method of inhibiting the expression of a non-canonical target gene of microRNA (miRNA), comprising:
administrating the composition comprising the RNA interference-inducing nucleic acid of any one of claims 1 to 7 into a subject.

30. A use of the RNA interference-inducing nucleic acid of any one of claims 1 to 7 for inhibiting the expression of a non-canonical target gene of microRNA (miRNA).

31. A method of regulating cell cycling, differentiation, dedifferentiation, morphology, migration, division, proliferation or apoptosis, comprising:
administrating the composition containing the RNA interference-inducing nucleic acid of any one of claims 1 to 7 into a subject.

32. A use of the RNA interference-inducing nucleic acid of any one of claims 1 to 7 for regulating cell cycling, differentiation, dedifferentiation, morphology, migration, division, proliferation or apoptosis.

33. A method of inhibiting the expression of a non-canonical target gene of microRNA (miRNA), comprising:
administrating the composition containing the RNA interference-inducing nucleic acid of any one of claims 13 to 15 into a subject.

34. A use of the RNA interference-inducing nucleic acid of any one of claims 13 to 15 for inhibiting the expression of a non-canonical target gene of microRNA (miRNA).

35. A method of inhibiting the expression of a non-canonical target gene of microRNA (miRNA), comprising:
administrating the composition containing the RNA interference-inducing nucleic acid of any one of claims 18 to 20 into a subject.

36. A use of the RNA interference-inducing nucleic acid of any one of claims 18 to 20 for inhibiting the expression of a non-canonical target gene of microRNA (miRNA).

37. A method of inhibiting the expression of a non-canonical target gene of microRNA (miRNA), comprising:
administrating the composition containing the RNA interference-inducing nucleic acid of any one of claims 23 to 26 into a subject.

38. A use of the RNA interference-inducing nucleic acid of any one of claims 23 to 26 for inhibiting the expression of a non-canonical target gene of microRNA (miRNA).


<110>    Korea University Industry and Academy Cooperation Foundation

<120>    RNA interference inducing nucleic acids suppressing noncanonical
         targets of microRNA and use thereof

<130>    MPCT19-045

<150>    KR 10-2018-0063054
<151>    2018-05-31

<150>    KR 10-2018-0063055
<151>    2018-05-31

<150>    KR 10-2019-0064333
<151>    2019-05-31

<150>    KR 10-2019-0064334
<151>    2019-05-31

<150>    KR 10-2019-0064335
<151>    2019-05-31

<150>    KR 10-2019-0064386
<151>    2019-05-31

<160>    863

<170>    KoPatentIn 3.0

<210>    1
<211>    6
<212>    RNA
<213>    Artificial Sequence

<220>
<223>    5′  2~7 of miR-124-BS


<400>    1
aaggcc                                                                   6


<210>    2
<211>    6
<212>    RNA
<213>    Artificial Sequence

<220>
<223>    5′  2~7 of miR-122-BS


<400>    2
aaggcc                                                                   6


<210>    3
<211>    6
<212>    RNA
<213>    Artificial Sequence

<220>
<223>    5′  2~7 of miR-155-BS

<400>    3
uaaugg                                                                    6


<210>    4
<211>    6
<212>    RNA
<213>    Artificial Sequence

<220>
<223>    5′  2˜7 of miR-1-BS


<400>    4
ggaauu                                                                    6


<210>    5
<211>    21
<212>    RNA
<213>    Artificial Sequence

<220>
<223>    miR-124-BS


<400>    5
uaaggccacg cggugaaugc c                                                  21


<210>    6
<211>    21
<212>    RNA
<213>    Artificial Sequence

<220>
<223>    miR-122-BS


<400>    6
uggaguugug acaauggugu u                                                  21


<210>    7
<211>    22
<212>    RNA
<213>    Artificial Sequence

<220>
<223>    miR-155-BS


<400>    7
uuaaugggcu aaucgugaua gg                                                 22


<210>    8
<211>    21
<212>    RNA
<213>    Artificial Sequence

<220>

<223>    miR-1-BS

<400>    8
uggaauugua aagaaguaug u                                                21

<210>    9
<211>    9
<212>    RNA
<213>    Artificial Sequence

<220>
<223>    5′ 1~9 of miR-124-G4U

<400>    9
uaaugcacg                                                               9

<210>    10
<211>    9
<212>    RNA
<213>    Artificial Sequence

<220>
<223>    5′ 1~9 of miR-124-G5U

<400>    10
uaagucacg                                                               9

<210>    11
<211>    9
<212>    RNA
<213>    Artificial Sequence

<220>
<223>    5′ 1~9 of miR-124-G4,5U

<400>    11
uaauucacg                                                               9

<210>    12
<211>    9
<212>    RNA
<213>    Artificial Sequence

<220>
<223>    5′ 1~9 of miR-1-G2U

<400>    12
uugaaugua                                                               9

<210>    13
<211>    9
<212>    RNA
<213>    Artificial Sequence

<220>
<223>    5′ 1~9 of miR-1-G3U


<400>    13
uguaaugua                                                                9


<210>    14
<211>    9
<212>    RNA
<213>    Artificial Sequence

<220>
<223>    5′ 1~9 of miR-1-G7U


<400>    14
uggaauuua                                                                9


<210>    15
<211>    9
<212>    RNA
<213>    Artificial Sequence

<220>
<223>    5′ 1~9 of miR-1-G2,3U


<400>    15
uuuaaugua                                                                9


<210>    16
<211>    9
<212>    RNA
<213>    Artificial Sequence

<220>
<223>    5′ 1~9 of miR-1-G3,7U


<400>    16
uguaauuua                                                                9


<210>    17
<211>    9
<212>    RNA
<213>    Artificial Sequence

<220>
<223>    5′ 1~9 of miR-1-G2,7U


<400>    17
uugaauuua                                                                9


<210>    18
<211>    9

<212>    RNA
<213>    Artificial Sequence

<220>
<223>    5′ 1~9 of miR-1-G2,3,7U


<400>    18
uuuaauuua                                                                9


<210>    19
<211>    9
<212>    RNA
<213>    Artificial Sequence

<220>
<223>    5′ 1~9 of miR-122-G2U


<400>    19
uugagugug                                                                9


<210>    20
<211>    9
<212>    RNA
<213>    Artificial Sequence

<220>
<223>    5′ 1~9 of miR-122-G3U


<400>    20
uguagugug                                                                9


<210>    21
<211>    9
<212>    RNA
<213>    Artificial Sequence

<220>
<223>    5′ 1~9 of miR-122-G5U


<400>    21
uggauugug                                                                9


<210>    22
<211>    9
<212>    RNA
<213>    Artificial Sequence

<220>
<223>    5′ 1~9 of miR-122-G7U


<400>    22
uggaguuug                                                                9

<210>    23
<211>    9
<212>    RNA
<213>    Artificial Sequence

<220>
<223>    5′ 1~9 of miR-122-G9U


<400>    23
uggaguguu                                                                    9


<210>    24
<211>    9
<212>    RNA
<213>    Artificial Sequence

<220>
<223>    5′ 1~9 of miR-122-G2,3U


<400>    24
uuuagugug                                                                    9


<210>    25
<211>    9
<212>    RNA
<213>    Artificial Sequence

<220>
<223>    5′ 1~9 of miR-122-G2,5U


<400>    25
uugauugug                                                                    9


<210>    26
<211>    9
<212>    RNA
<213>    Artificial Sequence

<220>
<223>    5′ 1~9 of miR-122-G2,7U


<400>    26
uugaguuug                                                                    9


<210>    27
<211>    9
<212>    RNA
<213>    Artificial Sequence

<220>
<223>    5′ 1~9 of miR-122-G2,9U


<400>    27
uugaguguu                                                                    9

<210>    28
<211>    9
<212>    RNA
<213>    Artificial Sequence

<220>
<223>    5′ 1˜9 of miR-122-G3,5U


<400>    28
uguauugug                                                                9


<210>    29
<211>    9
<212>    RNA
<213>    Artificial Sequence

<220>
<223>    5′ 1˜9 of miR-122-G3,7U


<400>    29
uguaguuug                                                                9


<210>    30
<211>    9
<212>    RNA
<213>    Artificial Sequence

<220>
<223>    5′ 1˜9 of miR-122-G3,9U


<400>    30
uguaguguu                                                                9


<210>    31
<211>    9
<212>    RNA
<213>    Artificial Sequence

<220>
<223>    5′ 1˜9 of miR-122-G5,7U


<400>    31
uggauuuug                                                                9


<210>    32
<211>    9
<212>    RNA
<213>    Artificial Sequence

<220>
<223>    5′ 1˜9 of miR-122-G5,9U

<400>    32
uggauuguu                                                                    9


<210>    33
<211>    9
<212>    RNA
<213>    Artificial Sequence

<220>
<223>    5′ 1~9 of miR-122-G7,9U


<400>    33
uggaguuuu                                                                    9


<210>    34
<211>    9
<212>    RNA
<213>    Artificial Sequence

<220>
<223>    5′ 1~9 of miR-133-G4U


<400>    34
uuuuguccc                                                                    9


<210>    35
<211>    9
<212>    RNA
<213>    Artificial Sequence

<220>
<223>    5′ 1~9 of miR-133-G5U


<400>    35
uuuguuccc                                                                    9


<210>    36
<211>    9
<212>    RNA
<213>    Artificial Sequence

<220>
<223>    5′ 1~9 of miR-124-G4,5U


<400>    36
uuuuuuccc                                                                    9


<210>    37
<211>    9
<212>    RNA
<213>    Artificial Sequence

<220>
<223>    5′ 1~9 of let-7-G2U

<400>    37
uuagguagu                                                                9

<210>    38
<211>    9
<212>    RNA
<213>    Artificial Sequence

<220>
<223>    5′ 1~9 of let-7-G4U

<400>    38
ugauguagu                                                                9

<210>    39
<211>    9
<212>    RNA
<213>    Artificial Sequence

<220>
<223>    5′ 1~9 of let-7-G5U

<400>    39
ugaguuagu                                                                9

<210>    40
<211>    9
<212>    RNA
<213>    Artificial Sequence

<220>
<223>    5′ 1~9 of let-7-G8U

<400>    40
ugagguauu                                                                9

<210>    41
<211>    9
<212>    RNA
<213>    Artificial Sequence

<220>
<223>    5′ 1~9 of let-7-G2,4U

<400>    41
uuauguagu                                                                9

<210>    42
<211>    9
<212>    RNA
<213>    Artificial Sequence

<220>
<223>    5′ 1~9 of let-7-G2,5U


<400>    42
uuaguuagu                                                                9


<210>    43
<211>    9
<212>    RNA
<213>    Artificial Sequence

<220>
<223>    5′ 1~9 of let-7-G2,8U


<400>    43
uuagguauu                                                                9


<210>    44
<211>    9
<212>    RNA
<213>    Artificial Sequence

<220>
<223>    5′ 1~9 of let-7-G4,5U


<400>    44
ugauuuagu                                                                9


<210>    45
<211>    9
<212>    RNA
<213>    Artificial Sequence

<220>
<223>    5′ 1~9 of let-7-G4,8U


<400>    45
ugauguauu                                                                9


<210>    46
<211>    9
<212>    RNA
<213>    Artificial Sequence

<220>
<223>    5′ 1~9 of let-7-G5,8U


<400>    46
ugaguuauu                                                                9


<210>    47
<211>    9
<212>    RNA

<213>    Artificial Sequence

<220>
<223>    5′ 1~9 of miR-302a-G4U


<400>    47
uaauugcuu                                                                9


<210>    48
<211>    9
<212>    RNA
<213>    Artificial Sequence

<220>
<223>    5′ 1~9 of miR-302a-G6U


<400>    48
uaaguucuu                                                                9


<210>    49
<211>    9
<212>    RNA
<213>    Artificial Sequence

<220>
<223>    5′ 1~9 of miR-302a-G4,6U


<400>    49
uaauuucuu                                                                9


<210>    50
<211>    9
<212>    RNA
<213>    Artificial Sequence

<220>
<223>    5′ 1~9 of miR-372-G4U


<400>    50
aaauugcug                                                                9


<210>    51
<211>    9
<212>    RNA
<213>    Artificial Sequence

<220>
<223>    5′ 1~9 of miR-372-G6U


<400>    51
aaaguucug                                                                9


<210>    52

<211>	9
<212>	RNA
<213>	Artificial Sequence

<220>
<223>	5′ 1˜9 of miR-372-G9U


<400>	52
aaagugcuu	9


<210>	53
<211>	9
<212>	RNA
<213>	Artificial Sequence

<220>
<223>	5′ 1˜9 of miR-372-G4,6U


<400>	53
aaauuucug	9


<210>	54
<211>	9
<212>	RNA
<213>	Artificial Sequence

<220>
<223>	5′ 1˜9 of miR-372-G4,9U


<400>	54
aaauugcuu	9


<210>	55
<211>	9
<212>	RNA
<213>	Artificial Sequence

<220>
<223>	5′ 1˜9 of miR-372-G6,9U


<400>	55
aaaguucuu	9


<210>	56
<211>	22
<212>	RNA
<213>	Artificial Sequence

<220>
<223>	miR-124-G4U


<400>	56
uaaugcacgc ggugaaugcc aa	22

<210>    57
<211>    21
<212>    RNA
<213>    Artificial Sequence

<220>
<223>    miR-124-G5U


<400>    57
uaagucacgc ggugaaugcc a                                                21


<210>    58
<211>    22
<212>    RNA
<213>    Artificial Sequence

<220>
<223>    miR-124-G4,5U


<400>    58
uaauucacgc ggugaaugcc aa                                               22


<210>    59
<211>    22
<212>    RNA
<213>    Artificial Sequence

<220>
<223>    miR-1-G2U


<400>    59
uugaauguaa agaaguaugu au                                               22


<210>    60
<211>    22
<212>    RNA
<213>    Artificial Sequence

<220>
<223>    miR-1-G3U


<400>    60
uguaauguaa agaaguaugu au                                               22


<210>    61
<211>    22
<212>    RNA
<213>    Artificial Sequence

<220>
<223>    miR-1-G7U


<400>    61

uggaauuuaa agaaguaugu au                                              22

<210>    62
<211>    22
<212>    RNA
<213>    Artificial Sequence

<220>
<223>    miR-1-G2,3U

<400>    62
uuuaauguaa agaaguaugu au                                              22

<210>    63
<211>    22
<212>    RNA
<213>    Artificial Sequence

<220>
<223>    miR-1-G3,7U

<400>    63
uguaauuuaa agaaguaugu au                                              22

<210>    64
<211>    22
<212>    RNA
<213>    Artificial Sequence

<220>
<223>    miR-1-G2,7U

<400>    64
uugaauuuaa agaaguaugu au                                              22

<210>    65
<211>    22
<212>    RNA
<213>    Artificial Sequence

<220>
<223>    miR-1-G2,3,7U

<400>    65
uuuaauuuaa agaaguaugu au                                              22

<210>    66
<211>    22
<212>    RNA
<213>    Artificial Sequence

<220>
<223>    miR-122-G2U

<400>    66
   uugaguguga caaugguguu ug                                                22


<210>    67
<211>    22
<212>    RNA
<213>    Artificial Sequence

<220>
<223>    miR-122-G3U


<400>    67
   uguaguguga caaugguguu ug                                                22


<210>    68
<211>    22
<212>    RNA
<213>    Artificial Sequence

<220>
<223>    miR-122-G5U


<400>    68
   uggauuguga caaugguguu ug                                                22


<210>    69
<211>    22
<212>    RNA
<213>    Artificial Sequence

<220>
<223>    miR-122-G7U


<400>    69
   uggaguuuga caaugguguu ug                                                22


<210>    70
<211>    22
<212>    RNA
<213>    Artificial Sequence

<220>
<223>    miR-122-G9U


<400>    70
   uggaguguua caaugguguu ug                                                22


<210>    71
<211>    22
<212>    RNA
<213>    Artificial Sequence

<220>

<223>    miR-122-G2,3U

<400>    71
uuuaguguga caaugguguu ug                                                22

<210>    72
<211>    22
<212>    RNA
<213>    Artificial Sequence

<220>
<223>    miR-122-G2,5U

<400>    72
uugauuguga caaugguguu ug                                                22

<210>    73
<211>    22
<212>    RNA
<213>    Artificial Sequence

<220>
<223>    miR-122-G2,7U

<400>    73
uugaguuuga caaugguguu ug                                                22

<210>    74
<211>    22
<212>    RNA
<213>    Artificial Sequence

<220>
<223>    miR-122-G2,9U

<400>    74
uugaguguua caaugguguu ug                                                22

<210>    75
<211>    22
<212>    RNA
<213>    Artificial Sequence

<220>
<223>    miR-122-G3,5U

<400>    75
uguauuguga caaugguguu ug                                                22

<210>    76
<211>    22
<212>    RNA
<213>    Artificial Sequence

<220>
<223>    miR-122-G3,7U


<400>    76
uguaguuuga caaugguguu ug                                                22


<210>    77
<211>    22
<212>    RNA
<213>    Artificial Sequence

<220>
<223>    miR-122-G3,9U


<400>    77
uguaguguua caaugguguu ug                                                22


<210>    78
<211>    22
<212>    RNA
<213>    Artificial Sequence

<220>
<223>    miR-122-G5,7U


<400>    78
uggauuuuga caaugguguu ug                                                22


<210>    79
<211>    22
<212>    RNA
<213>    Artificial Sequence

<220>
<223>    miR-122-G5,9U


<400>    79
uggauuguua caaugguguu ug                                                22


<210>    80
<211>    22
<212>    RNA
<213>    Artificial Sequence

<220>
<223>    miR-122-G7,9U


<400>    80
uggaguuuua caaugguguu ug                                                22


<210>    81
<211>    22

<212>    RNA
<213>    Artificial Sequence

<220>
<223>    miR-133-G4U


<400>    81
uuuugucccc uucaaccagc ug                                                22


<210>    82
<211>    22
<212>    RNA
<213>    Artificial Sequence

<220>
<223>    miR-133-G5U


<400>    82
uuuguucccc uucaaccagc ug                                                22


<210>    83
<211>    22
<212>    RNA
<213>    Artificial Sequence

<220>
<223>    miR-124-G4,5U


<400>    83
uuuuuucccc uucaaccagc ug                                                22


<210>    84
<211>    22
<212>    RNA
<213>    Artificial Sequence

<220>
<223>    let-7-G2U


<400>    84
uuagguagua gguuguauag uu                                                22


<210>    85
<211>    22
<212>    RNA
<213>    Artificial Sequence

<220>
<223>    let-7-G4U


<400>    85
ugauguagua gguuguauag uu                                                22

<210>    86
<211>    22
<212>    RNA
<213>    Artificial Sequence

<220>
<223>    let-7-G5U


<400>    86
ugaguuagua gguuguauag uu                                                22


<210>    87
<211>    22
<212>    RNA
<213>    Artificial Sequence

<220>
<223>    let-7-G8U


<400>    87
ugagguauua gguuguauag uu                                                22


<210>    88
<211>    22
<212>    RNA
<213>    Artificial Sequence

<220>
<223>    let-7-G2,4U


<400>    88
uuauguagua gguuguauag uu                                                22


<210>    89
<211>    22
<212>    RNA
<213>    Artificial Sequence

<220>
<223>    let-7-G2,5U


<400>    89
uuaguuagua gguuguauag uu                                                22


<210>    90
<211>    22
<212>    RNA
<213>    Artificial Sequence

<220>
<223>    let-7-G2,8U


<400>    90
uuagguauua gguuguauag uu                                                22

<210> 91
<211> 22
<212> RNA
<213> Artificial Sequence

<220>
<223> let-7-G4,5U


<400> 91
ugauuuagua gguuguauag uu 22


<210> 92
<211> 22
<212> RNA
<213> Artificial Sequence

<220>
<223> let-7-G4,8U


<400> 92
ugauguauua gguuguauag uu 22


<210> 93
<211> 22
<212> RNA
<213> Artificial Sequence

<220>
<223> let-7-G5,8U


<400> 93
ugaguuauua gguuguauag uu 22


<210> 94
<211> 23
<212> RNA
<213> Artificial Sequence

<220>
<223> miR-302a-G4U


<400> 94
uaauugcuuc cauguuuugg uga 23


<210> 95
<211> 23
<212> RNA
<213> Artificial Sequence

<220>
<223> miR-302a-G6U

<400>    95
uaaguucuuc cauguuuugg uga                                                23

<210>    96
<211>    23
<212>    RNA
<213>    Artificial Sequence

<220>
<223>    miR-302a-G4,6U

<400>    96
uaauuucuuc cauguuuugg uga                                                23

<210>    97
<211>    23
<212>    RNA
<213>    Artificial Sequence

<220>
<223>    miR-372-G4U

<400>    97
aaauugcugc gacauuugag cgu                                                23

<210>    98
<211>    23
<212>    RNA
<213>    Artificial Sequence

<220>
<223>    miR-372-G6U

<400>    98
aaaguucugc gacauuugag cgu                                                23

<210>    99
<211>    23
<212>    RNA
<213>    Artificial Sequence

<220>
<223>    miR-372-G9U

<400>    99
aaagugcuuc gacauuugag cgu                                                23

<210>    100
<211>    23
<212>    RNA
<213>    Artificial Sequence

<220>
<223>    miR-372-G4,6U

<400>    100
aaauuucugc gacauuugag cgu                                              23


<210>    101
<211>    23
<212>    RNA
<213>    Artificial Sequence

<220>
<223>    miR-372-G4,9U


<400>    101
aaauugcuuc gacauuugag cgu                                              23


<210>    102
<211>    23
<212>    RNA
<213>    Artificial Sequence

<220>
<223>    miR-372-G6,9U


<400>    102
aaaguucuuc gacauuugag cgu                                              23


<210>    103
<211>    6
<212>    RNA
<213>    Artificial Sequence

<220>
<223>    let-7/98/4458/4500 BS seq


<400>    103
gagguu                                                                  6


<210>    104
<211>    6
<212>    RNA
<213>    Artificial Sequence

<220>
<223>    miR-125a-5p/125b-5p/351/670/4319 BS seq


<400>    104
cccugg                                                                  6


<210>    105
<211>    6
<212>    RNA
<213>    Artificial Sequence

<220>
<223>    miR-124/124ab/506 BS seq


<400>    105
aaggcc                                                                    6


<210>    106
<211>    6
<212>    RNA
<213>    Artificial Sequence

<220>
<223>    miR-9/9ab BS seq


<400>    106
cuuugg                                                                    6


<210>    107
<211>    6
<212>    RNA
<213>    Artificial Sequence

<220>
<223>    miR-29abcd BS seq


<400>    107
agcacc                                                                    6


<210>    108
<211>    6
<212>    RNA
<213>    Artificial Sequence

<220>
<223>    miR-103a/107/107ab BS seq


<400>    108
gcagcc                                                                    6


<210>    109
<211>    6
<212>    RNA
<213>    Artificial Sequence

<220>
<223>    miR-221/222/222ab/1928 BS seq


<400>    109
gcuacc                                                                    6


<210>    110
<211>    6
<212>    RNA

<213>    Artificial Sequence

<220>
<223>    miR-26ab/1297/4465 BS seq


<400>    110
ucaagg                                                                    6


<210>    111
<211>    6
<212>    RNA
<213>    Artificial Sequence

<220>
<223>    miR-15abc/16/16abc/195/322/424/497/1907 BS seq


<400>    111
agcagg                                                                    6


<210>    112
<211>    6
<212>    RNA
<213>    Artificial Sequence

<220>
<223>    miR-126-3p BS seq


<400>    112
cguacc                                                                    6


<210>    113
<211>    6
<212>    RNA
<213>    Artificial Sequence

<220>
<223>    miR-30abcdef/30abe-5p/384-5p BS seq


<400>    113
guaaaa                                                                    6


<210>    114
<211>    6
<212>    RNA
<213>    Artificial Sequence

<220>
<223>    miR-33ab/33-5p BS seq


<400>    114
ugcauu                                                                    6


<210>    115

<211>    6
<212>    RNA
<213>    Artificial Sequence

<220>
<223>    miR-34ac/34bc-5p/449abc/449c-5p BS seq


<400>    115
ggcagg                                                                    6


<210>    116
<211>    6
<212>    RNA
<213>    Artificial Sequence

<220>
<223>    miR-19ab BS seq


<400>    116
gugcaa                                                                    6


<210>    117
<211>    6
<212>    RNA
<213>    Artificial Sequence

<220>
<223>    miR-99ab/100 BS seq


<400>    117
acccgg                                                                    6


<210>    118
<211>    6
<212>    RNA
<213>    Artificial Sequence

<220>
<223>    miR-17/17-5p/20ab/20b-5p/93/106ab/427/518a-3p/519d BS seq


<400>    118
aaaguu                                                                    6


<210>    119
<211>    6
<212>    RNA
<213>    Artificial Sequence

<220>
<223>    miR-27abc/27a-3p BS seq


<400>    119
ucacaa                                                                    6

<210>    120
<211>    6
<212>    RNA
<213>    Artificial Sequence

<220>
<223>    miR-218/218a BS seq


<400>    120
ugugcc                                                                    6


<210>    121
<211>    6
<212>    RNA
<213>    Artificial Sequence

<220>
<223>    miR-22/22-3p BS seq


<400>    121
agcugg                                                                    6


<210>    122
<211>    6
<212>    RNA
<213>    Artificial Sequence

<220>
<223>    miR-185/882/3473/4306/4644 BS seq


<400>    122
ggagaa                                                                    6


<210>    123
<211>    6
<212>    RNA
<213>    Artificial Sequence

<220>
<223>    miR-181abcd/4262 BS seq


<400>    123
acauuu                                                                    6


<210>    124
<211>    6
<212>    RNA
<213>    Artificial Sequence

<220>
<223>    miR-338/338-3p BS seq


<400>    124

ccagcc                                                                 6

<210>    125
<211>    6
<212>    RNA
<213>    Artificial Sequence

<220>
<223>    miR-127/127-3p BS seq

<400>    125
cggauu                                                                 6

<210>    126
<211>    6
<212>    RNA
<213>    Artificial Sequence

<220>
<223>    miR-101/101ab BS seq

<400>    126
acaguu                                                                 6

<210>    127
<211>    6
<212>    RNA
<213>    Artificial Sequence

<220>
<223>    miR-149 BS seq

<400>    127
cuggcc                                                                 6

<210>    128
<211>    6
<212>    RNA
<213>    Artificial Sequence

<220>
<223>    miR-324-5p BS seq

<400>    128
gcaucc                                                                 6

<210>    129
<211>    6
<212>    RNA
<213>    Artificial Sequence

<220>
<223>    miR-24/24ab/24-3p BS seq

<400>    129
ggcucc                                                                    6

<210>    130
<211>    6
<212>    RNA
<213>    Artificial Sequence

<220>
<223>    miR-33a-3p/365/365-3p BS seq

<400>    130
aaugcc                                                                    6

<210>    131
<211>    6
<212>    RNA
<213>    Artificial Sequence

<220>
<223>    miR-139-5p BS seq

<400>    131
cuacaa                                                                    6

<210>    132
<211>    6
<212>    RNA
<213>    Artificial Sequence

<220>
<223>    miR-138/138ab BS seq

<400>    132
gcuggg                                                                    6

<210>    133
<211>    6
<212>    RNA
<213>    Artificial Sequence

<220>
<223>    miR-143/1721/4770 BS seq

<400>    133
gagauu                                                                    6

<210>    134
<211>    6
<212>    RNA
<213>    Artificial Sequence

<220>

<223>    miR-25/32/92abc/363/363-3p/367 BS seq

<400>    134
auugcc                                                                    6

<210>    135
<211>    6
<212>    RNA
<213>    Artificial Sequence

<220>
<223>    miR-574-5p BS seq

<400>    135
gagugg                                                                    6

<210>    136
<211>    6
<212>    RNA
<213>    Artificial Sequence

<220>
<223>    miR-7/7ab BS seq

<400>    136
ggaagg                                                                    6

<210>    137
<211>    6
<212>    RNA
<213>    Artificial Sequence

<220>
<223>    miR-145 BS seq

<400>    137
uccagg                                                                    6

<210>    138
<211>    6
<212>    RNA
<213>    Artificial Sequence

<220>
<223>    miR-135ab/135a-5p BS seq

<400>    138
auggcc                                                                    6

<210>    139
<211>    6
<212>    RNA
<213>    Artificial Sequence

<220>
<223>    miR-148ab-3p/152 BS seq


<400>    139
cagugg                                                                    6


<210>    140
<211>    6
<212>    RNA
<213>    Artificial Sequence

<220>
<223>    miR-28-5p/708/1407/1653/3139 BS seq


<400>    140
aggagg                                                                    6


<210>    141
<211>    6
<212>    RNA
<213>    Artificial Sequence

<220>
<223>    miR-130ac/301ab/301b/301b-3p/454/721/4295/3666 BS seq


<400>    141
agugcc                                                                    6


<210>    142
<211>    6
<212>    RNA
<213>    Artificial Sequence

<220>
<223>    miR-3132 BS seq


<400>    142
ggguaa                                                                    6


<210>    143
<211>    6
<212>    RNA
<213>    Artificial Sequence

<220>
<223>    miR-155 BS seq


<400>    143
uaaugg                                                                    6


<210>    144
<211>    6

<212>    RNA
<213>    Artificial Sequence

<220>
<223>    miR-485-3p BS seq


<400>    144
ucauaa                                                                    6


<210>    145
<211>    6
<212>    RNA
<213>    Artificial Sequence

<220>
<223>    miR-132/212/212-3p BS seq


<400>    145
aacagg                                                                    6


<210>    146
<211>    6
<212>    RNA
<213>    Artificial Sequence

<220>
<223>    hsa-miR-9-3p BS seq


<400>    146
uaaagg                                                                    6


<210>    147
<211>    6
<212>    RNA
<213>    Artificial Sequence

<220>
<223>    miR-374ab BS seq


<400>    147
uauaaa                                                                    6


<210>    148
<211>    6
<212>    RNA
<213>    Artificial Sequence

<220>
<223>    miR-129-3p/129ab-3p/129-1-3p/129-2-3p BS seq


<400>    148
agcccc                                                                    6

<210>    149
<211>    6
<212>    RNA
<213>    Artificial Sequence

<220>
<223>    hsa-miR-126-5p BS seq


<400>    149
auuauu                                                                    6


<210>    150
<211>    6
<212>    RNA
<213>    Artificial Sequence

<220>
<223>    miR-425/425-5p/489 BS seq


<400>    150
augacc                                                                    6


<210>    151
<211>    6
<212>    RNA
<213>    Artificial Sequence

<220>
<223>    miR-423-3p BS seq


<400>    151
gcucgg                                                                    6


<210>    152
<211>    6
<212>    RNA
<213>    Artificial Sequence

<220>
<223>    miR-21/590-5p BS seq


<400>    152
agcuuu                                                                    6


<210>    153
<211>    6
<212>    RNA
<213>    Artificial Sequence

<220>
<223>    miR-31 BS seq


<400>    153
ggcaaa                                                                    6

<210>	154
<211>	6
<212>	RNA
<213>	Artificial Sequence

<220>
<223>	hsa-miR-20b-3p BS seq


<400>	154
cuguaa	6


<210>	155
<211>	6
<212>	RNA
<213>	Artificial Sequence

<220>
<223>	hsa-let-7d-3p BS seq


<400>	155
uauacc	6


<210>	156
<211>	6
<212>	RNA
<213>	Artificial Sequence

<220>
<223>	miR-191 BS seq


<400>	156
aacggg	6


<210>	157
<211>	6
<212>	RNA
<213>	Artificial Sequence

<220>
<223>	miR-18ab/4735-3p BS seq


<400>	157
aagguu	6


<210>	158
<211>	6
<212>	RNA
<213>	Artificial Sequence

<220>
<223>	miR-369-3p BS seq

<400>    158
auaauu                                                                    6

<210>    159
<211>    6
<212>    RNA
<213>    Artificial Sequence

<220>
<223>    hsa-miR-5187-5p BS seq

<400>    159
gggauu                                                                    6

<210>    160
<211>    6
<212>    RNA
<213>    Artificial Sequence

<220>
<223>    miR-382 BS seq

<400>    160
aaguuu                                                                    6

<210>    161
<211>    6
<212>    RNA
<213>    Artificial Sequence

<220>
<223>    miR-485-5p/1698/1703/1962 BS seq

<400>    161
gaggcc                                                                    6

<210>    162
<211>    6
<212>    RNA
<213>    Artificial Sequence

<220>
<223>    hsa-miR-136-3p BS seq

<400>    162
aucauu                                                                    6

<210>    163
<211>    6
<212>    RNA
<213>    Artificial Sequence

<220>
<223>    miR-576-3p BS seq

<400>    163
agaugg                                                                    6


<210>    164
<211>    6
<212>    RNA
<213>    Artificial Sequence

<220>
<223>    miR-204/204b/211 BS seq


<400>    164
ucccuu                                                                    6


<210>    165
<211>    6
<212>    RNA
<213>    Artificial Sequence

<220>
<223>    miR-769-5p BS seq


<400>    165
gagacc                                                                    6


<210>    166
<211>    6
<212>    RNA
<213>    Artificial Sequence

<220>
<223>    miR-342-5p/4664-5p BS seq


<400>    166
ggggu u                                                                   6


<210>    167
<211>    6
<212>    RNA
<213>    Artificial Sequence

<220>
<223>    miR-361-5p BS seq


<400>    167
uaucaa                                                                    6


<210>    168
<211>    6
<212>    RNA
<213>    Artificial Sequence

<220>
<223>    miR-199ab-3p/3129-5p BS seq


<400>    168
caguaa                                                                    6


<210>    169
<211>    6
<212>    RNA
<213>    Artificial Sequence

<220>
<223>    miR-142-3p BS seq


<400>    169
guaguu                                                                    6


<210>    170
<211>    6
<212>    RNA
<213>    Artificial Sequence

<220>
<223>    miR-299-5p/3563-5p BS seq


<400>    170
gguuuu                                                                    6


<210>    171
<211>    6
<212>    RNA
<213>    Artificial Sequence

<220>
<223>    miR-193/193b/193a-3p BS seq


<400>    171
acuggg                                                                    6


<210>    172
<211>    6
<212>    RNA
<213>    Artificial Sequence

<220>
<223>    hsa-miR-1277-5p BS seq


<400>    172
aauauu                                                                    6


<210>    173
<211>    6
<212>    RNA

<213>    Artificial Sequence

<220>
<223>    miR-140/140-5p/876-3p/1244 BS seq


<400>    173
agu ggg                                                                    6


<210>    174
<211>    6
<212>    RNA
<213>    Artificial Sequence

<220>
<223>    hsa-miR-30a/d/e-3p BS seq


<400>    174
uuucaa                                                                     6


<210>    175
<211>    6
<212>    RNA
<213>    Artificial Sequence

<220>
<223>    hsa-let-7i-3p BS seq


<400>    175
ugcgcc                                                                     6


<210>    176
<211>    6
<212>    RNA
<213>    Artificial Sequence

<220>
<223>    miR-409-5p/409a BS seq


<400>    176
gguuaa                                                                     6


<210>    177
<211>    6
<212>    RNA
<213>    Artificial Sequence

<220>
<223>    miR-379/1193-5p/3529 BS seq


<400>    177
gguagg                                                                     6


<210>    178

<211>    6
<212>    RNA
<213>    Artificial Sequence

<220>
<223>    miR-136 BS seq


<400>    178
cuccaa                                                                    6


<210>    179
<211>    6
<212>    RNA
<213>    Artificial Sequence

<220>
<223>    miR-154/872 BS seq


<400>    179
agguuu                                                                    6


<210>    180
<211>    6
<212>    RNA
<213>    Artificial Sequence

<220>
<223>    miR-4684-3p BS seq


<400>    180
guugcc                                                                    6


<210>    181
<211>    6
<212>    RNA
<213>    Artificial Sequence

<220>
<223>    miR-361-3p BS seq


<400>    181
cccccc                                                                    6


<210>    182
<211>    6
<212>    RNA
<213>    Artificial Sequence

<220>
<223>    miR-335/335-5p BS seq


<400>    182
caagaa                                                                    6

<210> 183
<211> 6
<212> RNA
<213> Artificial Sequence

<220>
<223> miR-423a/423-5p/3184/3573-5p BS seq

<400> 183
gagggg 6

<210> 184
<211> 6
<212> RNA
<213> Artificial Sequence

<220>
<223> miR-371/373/371b-5p BS seq

<400> 184
cucaaa 6

<210> 185
<211> 6
<212> RNA
<213> Artificial Sequence

<220>
<223> miR-1185/3679-5p BS seq

<400> 185
gaggaa 6

<210> 186
<211> 6
<212> RNA
<213> Artificial Sequence

<220>
<223> miR-3613-3p BS seq

<400> 186
caaaaa 6

<210> 187
<211> 6
<212> RNA
<213> Artificial Sequence

<220>
<223> miR-93/93a/105/106a/291a-3p/294/295/302abcde/372/373/428/519a/520
be/520acd-3p/1378/1420ac BS seq

<400>    187
aagugg                                                                  6


<210>    188
<211>    6
<212>    RNA
<213>    Artificial Sequence

<220>
<223>    miR-876-5p/3167 BS seq


<400>    188
ggauuu                                                                  6


<210>    189
<211>    6
<212>    RNA
<213>    Artificial Sequence

<220>
<223>    miR-329/329ab/362-3p BS seq


<400>    189
acacaa                                                                  6


<210>    190
<211>    6
<212>    RNA
<213>    Artificial Sequence

<220>
<223>    miR-582-5p BS seq


<400>    190
uacagg                                                                  6


<210>    191
<211>    6
<212>    RNA
<213>    Artificial Sequence

<220>
<223>    miR-146ac/146b-5p BS seq


<400>    191
gagaaa                                                                  6


<210>    192
<211>    6
<212>    RNA
<213>    Artificial Sequence

<220>
<223>    miR-380/380-3p BS seq

<400>    192
auguaa                                                                    6

<210>    193
<211>    6
<212>    RNA
<213>    Artificial Sequence

<220>
<223>    miR-499-3p/499a-3p BS seq

<400>    193
acaucc                                                                    6

<210>    194
<211>    6
<212>    RNA
<213>    Artificial Sequence

<220>
<223>    miR-551a BS seq

<400>    194
cgaccc                                                                    6

<210>    195
<211>    6
<212>    RNA
<213>    Artificial Sequence

<220>
<223>    miR-142-5p BS seq

<400>    195
auaaaa                                                                    6

<210>    196
<211>    6
<212>    RNA
<213>    Artificial Sequence

<220>
<223>    hsa-miR-17-3p BS seq

<400>    196
cugcaa                                                                    6

<210>    197
<211>    6
<212>    RNA
<213>    Artificial Sequence

<220>
<223>    miR-199ab-5p BS seq


<400>    197
ccaguu                                                                    6


<210>    198
<211>    6
<212>    RNA
<213>    Artificial Sequence

<220>
<223>    miR-542-3p BS seq


<400>    198
gugacc                                                                    6


<210>    199
<211>    6
<212>    RNA
<213>    Artificial Sequence

<220>
<223>    miR-1277 BS seq


<400>    199
acguaa                                                                    6


<210>    200
<211>    6
<212>    RNA
<213>    Artificial Sequence

<220>
<223>    hsa-miR-29c-5p BS seq


<400>    200
gaccgg                                                                    6


<210>    201
<211>    6
<212>    RNA
<213>    Artificial Sequence

<220>
<223>    miR-3145-3p BS seq


<400>    201
gauauu                                                                    6


<210>    202
<211>    6
<212>    RNA

<213>    Artificial Sequence

<220>
<223>    hsa-miR-106b-3p BS seq


<400>    202
cgcacc                                                                    6


<210>    203
<211>    6
<212>    RNA
<213>    Artificial Sequence

<220>
<223>    hsa-miR-22-5p BS seq


<400>    203
guucuu                                                                    6


<210>    204
<211>    6
<212>    RNA
<213>    Artificial Sequence

<220>
<223>    miR-744/1716 BS seq


<400>    204
gcgggg                                                                    6


<210>    205
<211>    6
<212>    RNA
<213>    Artificial Sequence

<220>
<223>    hsa-miR-132-5p BS seq


<400>    205
ccgugg                                                                    6


<210>    206
<211>    6
<212>    RNA
<213>    Artificial Sequence

<220>
<223>    miR-488 BS seq


<400>    206
ugaaaa                                                                    6


<210>    207

<211>    6
<212>    RNA
<213>    Artificial Sequence

<220>
<223>    miR-501-3p/502-3p/500/502a BS seq


<400>    207
augcaa                                                                    6


<210>    208
<211>    6
<212>    RNA
<213>    Artificial Sequence

<220>
<223>    miR-486-5p/3107 BS seq


<400>    208
ccuguu                                                                    6


<210>    209
<211>    6
<212>    RNA
<213>    Artificial Sequence

<220>
<223>    miR-450a/451a BS seq


<400>    209
uuugcc                                                                    6


<210>    210
<211>    6
<212>    RNA
<213>    Artificial Sequence

<220>
<223>    hsa-miR-30c-3p BS seq


<400>    210
ugggaa                                                                    6


<210>    211
<211>    6
<212>    RNA
<213>    Artificial Sequence

<220>
<223>    miR-499-5p BS seq


<400>    211
uaagaa                                                                    6

<210>    212
<211>    6
<212>    RNA
<213>    Artificial Sequence

<220>
<223>    miR-421 BS seq


<400>    212
ucaacc                                                                    6


<210>    213
<211>    6
<212>    RNA
<213>    Artificial Sequence

<220>
<223>    miR-197 BS seq


<400>    213
ucaccc                                                                    6


<210>    214
<211>    6
<212>    RNA
<213>    Artificial Sequence

<220>
<223>    miR-296-5p BS seq


<400>    214
gggccc                                                                    6


<210>    215
<211>    6
<212>    RNA
<213>    Artificial Sequence

<220>
<223>    miR-326/330/330-5p BS seq


<400>    215
cucugg                                                                    6


<210>    216
<211>    6
<212>    RNA
<213>    Artificial Sequence

<220>
<223>    miR-214/761/3619-5p BS seq


<400>    216

cagcaa                                                                  6


<210>   217
<211>   6
<212>   RNA
<213>   Artificial Sequence

<220>
<223>   miR-612/1285/3187-5p BS seq


<400>   217
cugggg                                                                  6


<210>   218
<211>   6
<212>   RNA
<213>   Artificial Sequence

<220>
<223>   miR-409-3p BS seq


<400>   218
aauguu                                                                  6


<210>   219
<211>   6
<212>   RNA
<213>   Artificial Sequence

<220>
<223>   miR-378/422a/378bcdefhi BS seq


<400>   219
cuggaa                                                                  6


<210>   220
<211>   6
<212>   RNA
<213>   Artificial Sequence

<220>
<223>   miR-342-3p BS seq


<400>   220
cucacc                                                                  6


<210>   221
<211>   6
<212>   RNA
<213>   Artificial Sequence

<220>
<223>   miR-338-5p BS seq

<400>    221
acaauu                                                                    6


<210>    222
<211>    6
<212>    RNA
<213>    Artificial Sequence

<220>
<223>    miR-625 BS seq


<400>    222
gggggg                                                                    6


<210>    223
<211>    6
<212>    RNA
<213>    Artificial Sequence

<220>
<223>    miR-200bc/429/548a BS seq


<400>    223
aauacc                                                                    6


<210>    224
<211>    6
<212>    RNA
<213>    Artificial Sequence

<220>
<223>    hsa-miR-376a-5p BS seq


<400>    224
uagauu                                                                    6


<210>    225
<211>    6
<212>    RNA
<213>    Artificial Sequence

<220>
<223>    miR-584 BS seq


<400>    225
uauggg                                                                    6


<210>    226
<211>    6
<212>    RNA
<213>    Artificial Sequence

<220>

<223>    miR-411 BS seq

<400>    226
aguagg                                                                    6

<210>    227
<211>    6
<212>    RNA
<213>    Artificial Sequence

<220>
<223>    miR-573/3533/3616-5p/3647-5p BS seq

<400>    227
ugaagg                                                                    6

<210>    228
<211>    6
<212>    RNA
<213>    Artificial Sequence

<220>
<223>    miR-885-5p BS seq

<400>    228
ccauuu                                                                    6

<210>    229
<211>    6
<212>    RNA
<213>    Artificial Sequence

<220>
<223>    hsa-miR-99-3p BS seq

<400>    229
aagcuu                                                                    6

<210>    230
<211>    6
<212>    RNA
<213>    Artificial Sequence

<220>
<223>    miR-876-3p BS seq

<400>    230
gguggg                                                                    6

<210>    231
<211>    6
<212>    RNA
<213>    Artificial Sequence

<220>
<223>    miR-654-3p BS seq

<400>    231
augucc                                                                  6

<210>    232
<211>    6
<212>    RNA
<213>    Artificial Sequence

<220>
<223>    hsa-miR-340-3p BS seq

<400>    232
ccgucc                                                                  6

<210>    233
<211>    6
<212>    RNA
<213>    Artificial Sequence

<220>
<223>    miR-3614-5p BS seq

<400>    233
cacuuu                                                                  6

<210>    234
<211>    6
<212>    RNA
<213>    Artificial Sequence

<220>
<223>    hsa-miR-124-5p BS seq

<400>    234
guguuu                                                                  6

<210>    235
<211>    6
<212>    RNA
<213>    Artificial Sequence

<220>
<223>    miR-491-5p BS seq

<400>    235
gugggg                                                                  6

<210>    236
<211>    6

<212>    RNA
<213>    Artificial Sequence

<220>
<223>    miR-96/507/1271 BS seq


<400>    236
uuggcc                                                                    6


<210>    237
<211>    6
<212>    RNA
<213>    Artificial Sequence

<220>
<223>    miR-548a-3p/548ef/2285a BS seq


<400>    237
aaaacc                                                                    6


<210>    238
<211>    6
<212>    RNA
<213>    Artificial Sequence

<220>
<223>    hsa-miR-32-3p BS seq


<400>    238
aauuuu                                                                    6


<210>    239
<211>    6
<212>    RNA
<213>    Artificial Sequence

<220>
<223>    miR-3942-5p/4703-5p BS seq


<400>    239
agcaaa                                                                    6


<210>    240
<211>    6
<212>    RNA
<213>    Artificial Sequence

<220>
<223>    miR-34b/449c/1360/2682 BS seq


<400>    240
aggcaa                                                                    6

<210> 241
<211> 6
<212> RNA
<213> Artificial Sequence

<220>
<223> hsa-miR-23a/b-5p BS seq

<400> 241
ggguuu 6

<210> 242
<211> 6
<212> RNA
<213> Artificial Sequence

<220>
<223> miR-362-5p/500b BS seq

<400> 242
auccuu 6

<210> 243
<211> 6
<212> RNA
<213> Artificial Sequence

<220>
<223> miR-677/4420 BS seq

<400> 243
ucacuu 6

<210> 244
<211> 6
<212> RNA
<213> Artificial Sequence

<220>
<223> miR-577 BS seq

<400> 244
agauaa 6

<210> 245
<211> 6
<212> RNA
<213> Artificial Sequence

<220>
<223> miR-3613-5p BS seq

<400> 245
guuguu 6

<210> 246
<211> 6
<212> RNA
<213> Artificial Sequence

<220>
<223> miR-369-5p BS seq

<400> 246
gaucgg 6

<210> 247
<211> 6
<212> RNA
<213> Artificial Sequence

<220>
<223> miR-150/5127 BS seq

<400> 247
cucccc 6

<210> 248
<211> 6
<212> RNA
<213> Artificial Sequence

<220>
<223> miR-544/544ab/544-3p BS seq

<400> 248
uucugg 6

<210> 249
<211> 6
<212> RNA
<213> Artificial Sequence

<220>
<223> hsa-miR-29a-5p BS seq

<400> 249
cugauu 6

<210> 250
<211> 6
<212> RNA
<213> Artificial Sequence

<220>
<223> miR-873 BS seq

<400>    250
caggaa                                                                    6


<210>    251
<211>    6
<212>    RNA
<213>    Artificial Sequence

<220>
<223>    miR-3614-3p BS seq


<400>    251
agccuu                                                                    6


<210>    252
<211>    6
<212>    RNA
<213>    Artificial Sequence

<220>
<223>    miR-186 BS seq


<400>    252
aaagaa                                                                    6


<210>    253
<211>    6
<212>    RNA
<213>    Artificial Sequence

<220>
<223>    miR-483-3p BS seq


<400>    253
cacucc                                                                    6


<210>    254
<211>    6
<212>    RNA
<213>    Artificial Sequence

<220>
<223>    hsa-miR-374a-3p BS seq


<400>    254
uuaucc                                                                    6


<210>    255
<211>    6
<212>    RNA
<213>    Artificial Sequence

<220>
<223>    miR-196abc BS seq

<400>    255
agguaa                                                                    6

<210>    256
<211>    6
<212>    RNA
<213>    Artificial Sequence

<220>
<223>    hsa-miR-29b-2-5p BS seq

<400>    256
gauucc                                                                    6

<210>    257
<211>    6
<212>    RNA
<213>    Artificial Sequence

<220>
<223>    hsa-miR-29b-2-5p BS seq

<400>    257
ugguuu                                                                    6

<210>    258
<211>    6
<212>    RNA
<213>    Artificial Sequence

<220>
<223>    hsa-miR-221-5p BS seq

<400>    258
ccuggg                                                                    6

<210>    259
<211>    6
<212>    RNA
<213>    Artificial Sequence

<220>
<223>    miR-323b-3p BS seq

<400>    259
ccaauu                                                                    6

<210>    260
<211>    6
<212>    RNA
<213>    Artificial Sequence

<220>
<223>    miR-616 BS seq

<400>    260
gucauu                                                                    6

<210>    261
<211>    6
<212>    RNA
<213>    Artificial Sequence

<220>
<223>    miR-330-3p BS seq

<400>    261
caaagg                                                                    6

<210>    262
<211>    6
<212>    RNA
<213>    Artificial Sequence

<220>
<223>    hsa-miR-7-3p BS seq

<400>    262
aacaaa                                                                    6

<210>    263
<211>    6
<212>    RNA
<213>    Artificial Sequence

<220>
<223>    miR-187 BS seq

<400>    263
cguguu                                                                    6

<210>    264
<211>    6
<212>    RNA
<213>    Artificial Sequence

<220>
<223>    hsa-miR-26a-3p BS seq

<400>    264
cuauuu                                                                    6

<210>    265
<211>    6
<212>    RNA

<213>    Artificial Sequence

<220>
<223>    miR-452/4676-3p BS seq


<400>    265
acuguu                                                                    6


<210>    266
<211>    6
<212>    RNA
<213>    Artificial Sequence

<220>
<223>    miR-129-5p/129ab-5p BS seq


<400>    266
uuuuuu                                                                    6


<210>    267
<211>    6
<212>    RNA
<213>    Artificial Sequence

<220>
<223>    miR-223 BS seq


<400>    267
gucagg                                                                    6


<210>    268
<211>    6
<212>    RNA
<213>    Artificial Sequence

<220>
<223>    miR-4755-3p BS seq


<400>    268
gccagg                                                                    6


<210>    269
<211>    6
<212>    RNA
<213>    Artificial Sequence

<220>
<223>    miR-1247 BS seq


<400>    269
cccguu                                                                    6


<210>    270

<211>    6
<212>    RNA
<213>    Artificial Sequence

<220>
<223>    miR-3129-3p BS seq


<400>    270
aacuaa                                                                    6


<210>    271
<211>    6
<212>    RNA
<213>    Artificial Sequence

<220>
<223>    hsa-miR-335-3p BS seq


<400>    271
uuuucc                                                                    6


<210>    272
<211>    6
<212>    RNA
<213>    Artificial Sequence

<220>
<223>    miR-542-5p BS seq


<400>    272
cggggg                                                                    6


<210>    273
<211>    6
<212>    RNA
<213>    Artificial Sequence

<220>
<223>    hsa-miR-181a-3p BS seq


<400>    273
ccaucc                                                                    6


<210>    274
<211>    6
<212>    RNA
<213>    Artificial Sequence

<220>
<223>    hsa-miR-186-3p BS seq


<400>    274
cccaaa                                                                    6

<210>    275
<211>    6
<212>    RNA
<213>    Artificial Sequence

<220>
<223>    hsa-miR-27b-5p BS seq


<400>    275
gagcuu                                                                    6


<210>    276
<211>    6
<212>    RNA
<213>    Artificial Sequence

<220>
<223>    miR-491-3p BS seq


<400>    276
uuaugg                                                                    6


<210>    277
<211>    6
<212>    RNA
<213>    Artificial Sequence

<220>
<223>    miR-4687-3p BS seq


<400>    277
ggcugg                                                                    6


<210>    278
<211>    6
<212>    RNA
<213>    Artificial Sequence

<220>
<223>    hsa-miR-101-5p BS seq


<400>    278
aguuaa                                                                    6


<210>    279
<211>    6
<212>    RNA
<213>    Artificial Sequence

<220>
<223>    miR-4772-5p BS seq


<400>    279

gaucaa                                                                 6


<210>    280
<211>    6
<212>    RNA
<213>    Artificial Sequence

<220>
<223>    miR-337-3p BS seq


<400>    280
uccuaa                                                                 6


<210>    281
<211>    6
<212>    RNA
<213>    Artificial Sequence

<220>
<223>    hsa-miR-223-5p BS seq


<400>    281
guguaa                                                                 6


<210>    282
<211>    6
<212>    RNA
<213>    Artificial Sequence

<220>
<223>    hsa-miR-16/195-3p BS seq


<400>    282
caauaa                                                                 6


<210>    283
<211>    6
<212>    RNA
<213>    Artificial Sequence

<220>
<223>    miR-3677-3p BS seq


<400>    283
ucgugg                                                                 6


<210>    284
<211>    6
<212>    RNA
<213>    Artificial Sequence

<220>
<223>    hsa-miR-766-5p BS seq

<400>    284
ggaggg                                                                    6


<210>    285
<211>    6
<212>    RNA
<213>    Artificial Sequence

<220>
<223>    miR-299/299-3p/3563-3p BS seq


<400>    285
augugg                                                                    6


<210>    286
<211>    6
<212>    RNA
<213>    Artificial Sequence

<220>
<223>    miR-3140-3p BS seq


<400>    286
gcuuuu                                                                    6


<210>    287
<211>    6
<212>    RNA
<213>    Artificial Sequence

<220>
<223>    miR-532-5p/511 BS seq


<400>    287
augccc                                                                    6


<210>    288
<211>    6
<212>    RNA
<213>    Artificial Sequence

<220>
<223>    hsa-miR-24-5p BS seq


<400>    288
gccuaa                                                                    6


<210>    289
<211>    6
<212>    RNA
<213>    Artificial Sequence

<220>

<223>    miR-4778-5p BS seq

<400>    289
auucuu                                                                    6

<210>    290
<211>    6
<212>    RNA
<213>    Artificial Sequence

<220>
<223>    miR-642b BS seq

<400>    290
gacacc                                                                    6

<210>    291
<211>    6
<212>    RNA
<213>    Artificial Sequence

<220>
<223>    miR-483-5p BS seq

<400>    291
agacgg                                                                    6

<210>    292
<211>    6
<212>    RNA
<213>    Artificial Sequence

<220>
<223>    miR-767-5p BS seq

<400>    292
gcaccc                                                                    6

<210>    293
<211>    6
<212>    RNA
<213>    Artificial Sequence

<220>
<223>    hsa-miR-31-3p BS seq

<400>    293
gcuauu                                                                    6

<210>    294
<211>    6
<212>    RNA
<213>    Artificial Sequence

<220>
<223>    miR-574-3p BS seq

<400>    294
acgcuu                                                                    6

<210>    295
<211>    6
<212>    RNA
<213>    Artificial Sequence

<220>
<223>    miR-3173-3p BS seq

<400>    295
aaggaa                                                                    6

<210>    296
<211>    6
<212>    RNA
<213>    Artificial Sequence

<220>
<223>    miR-2127/4728-5p BS seq

<400>    296
gggagg                                                                    6

<210>    297
<211>    6
<212>    RNA
<213>    Artificial Sequence

<220>
<223>    hsa-miR-103a-2-5p BS seq

<400>    297
gcuucc                                                                    6

<210>    298
<211>    6
<212>    RNA
<213>    Artificial Sequence

<220>
<223>    miR-3591-3p BS seq

<400>    298
aacacc                                                                    6

<210>    299
<211>    6

<212>    RNA
<213>    Artificial Sequence

<220>
<223>    hsa-miR-625-3p BS seq


<400>    299
acuauu                                                                    6


<210>    300
<211>    6
<212>    RNA
<213>    Artificial Sequence

<220>
<223>    hsa-miR-15b-3p BS seq


<400>    300
gaaucc                                                                    6


<210>    301
<211>    6
<212>    RNA
<213>    Artificial Sequence

<220>
<223>    miR-522/518e/1422p BS seq


<400>    301
aaaugg                                                                    6


<210>    302
<211>    6
<212>    RNA
<213>    Artificial Sequence

<220>
<223>    miR-548d-3p/548acbz BS seq


<400>    302
aaaaaa                                                                    6


<210>    303
<211>    6
<212>    RNA
<213>    Artificial Sequence

<220>
<223>    hsa-miR-452-3p BS seq


<400>    303
ucaucc                                                                    6

<210> 304
<211> 6
<212> RNA
<213> Artificial Sequence

<220>
<223> miR-192/215 BS seq

<400> 304
ugaccc 6

<210> 305
<211> 6
<212> RNA
<213> Artificial Sequence

<220>
<223> miR-1551/4524 BS seq

<400> 305
uagcaa 6

<210> 306
<211> 6
<212> RNA
<213> Artificial Sequence

<220>
<223> hsa-miR-425-3p BS seq

<400> 306
ucgggg 6

<210> 307
<211> 6
<212> RNA
<213> Artificial Sequence

<220>
<223> miR-3126-3p BS seq

<400> 307
aucugg 6

<210> 308
<211> 6
<212> RNA
<213> Artificial Sequence

<220>
<223> hsa-miR-125b-2-3p BS seq

<400> 308
cacaaa 6

<210>	309
<211>	6
<212>	RNA
<213>	Artificial Sequence

<220>
<223>	miR-324-3p/1913 BS seq


<400>	309
cugccc	6


<210>	310
<211>	6
<212>	RNA
<213>	Artificial Sequence

<220>
<223>	hsa-miR-141-5p BS seq


<400>	310
aucuuu	6


<210>	311
<211>	6
<212>	RNA
<213>	Artificial Sequence

<220>
<223>	hsa-miR-365a/b-5p BS seq


<400>	311
gggacc	6


<210>	312
<211>	6
<212>	RNA
<213>	Artificial Sequence

<220>
<223>	hsa-miR-29b-1-5p BS seq


<400>	312
cugguu	6


<210>	313
<211>	6
<212>	RNA
<213>	Artificial Sequence

<220>
<223>	miR-563/380-5p BS seq

<400>    313
gguugg                                                                 6


<210>    314
<211>    6
<212>    RNA
<213>    Artificial Sequence

<220>
<223>    miR-1304 BS seq


<400>    314
uugagg                                                                 6


<210>    315
<211>    6
<212>    RNA
<213>    Artificial Sequence

<220>
<223>    miR-216c/1461/4684-5p BS seq


<400>    315
ucucuu                                                                 6


<210>    316
<211>    6
<212>    RNA
<213>    Artificial Sequence

<220>
<223>    hsa-miR-2681-5p BS seq


<400>    316
uuuuaa                                                                 6


<210>    317
<211>    6
<212>    RNA
<213>    Artificial Sequence

<220>
<223>    miR-194 BS seq


<400>    317
guaacc                                                                 6


<210>    318
<211>    6
<212>    RNA
<213>    Artificial Sequence

<220>
<223>    miR-296-3p BS seq

<400>    318
aggguu                                                                 6

<210>    319
<211>    6
<212>    RNA
<213>    Artificial Sequence

<220>
<223>    hsa-miR-205-3p BS seq

<400>    319
auuucc                                                                 6

<210>    320
<211>    6
<212>    RNA
<213>    Artificial Sequence

<220>
<223>    miR-888 BS seq

<400>    320
acucaa                                                                 6

<210>    321
<211>    6
<212>    RNA
<213>    Artificial Sequence

<220>
<223>    miR-4802-3p BS seq

<400>    321
acaugg                                                                 6

<210>    322
<211>    6
<212>    RNA
<213>    Artificial Sequence

<220>
<223>    hsa-let-7a/g-3p BS seq

<400>    322
uguacc                                                                 6

<210>    323
<211>    6
<212>    RNA
<213>    Artificial Sequence

<220>
<223>    miR-762/4492/4498 BS seq

<400>    323
gggcuu                                                                    6

<210>    324
<211>    6
<212>    RNA
<213>    Artificial Sequence

<220>
<223>    hsa-miR-744-3p BS seq

<400>    324
uguugg                                                                    6

<210>    325
<211>    6
<212>    RNA
<213>    Artificial Sequence

<220>
<223>    hsa-miR-148b-5p BS seq

<400>    325
aguucc                                                                    6

<210>    326
<211>    6
<212>    RNA
<213>    Artificial Sequence

<220>
<223>    miR-514/514b-3p BS seq

<400>    326
uugacc                                                                    6

<210>    327
<211>    6
<212>    RNA
<213>    Artificial Sequence

<220>
<223>    miR-28-3p BS seq

<400>    327
acuagg                                                                    6

<210>    328
<211>    6
<212>    RNA

<213>    Artificial Sequence

<220>
<223>    miR-550a BS seq


<400>    328
gugccc                                                                    6


<210>    329
<211>    6
<212>    RNA
<213>    Artificial Sequence

<220>
<223>    hsa-miR-125b-1-3p BS seq


<400>    329
cggguu                                                                    6


<210>    330
<211>    6
<212>    RNA
<213>    Artificial Sequence

<220>
<223>    hsa-miR-506-5p BS seq


<400>    330
auucaa                                                                    6


<210>    331
<211>    6
<212>    RNA
<213>    Artificial Sequence

<220>
<223>    hsa-miR-1306-5p BS seq


<400>    331
caccuu                                                                    6


<210>    332
<211>    6
<212>    RNA
<213>    Artificial Sequence

<220>
<223>    miR-3189-3p BS seq


<400>    332
ccuugg                                                                    6


<210>    333

<211>    6
<212>    RNA
<213>    Artificial Sequence

<220>
<223>    miR-675-5p/4466 BS seq


<400>    333
ggugcc                                                                    6


<210>    334
<211>    6
<212>    RNA
<213>    Artificial Sequence

<220>
<223>    hsa-miR-34a-3p BS seq


<400>    334
aaucaa                                                                    6


<210>    335
<211>    6
<212>    RNA
<213>    Artificial Sequence

<220>
<223>    hsa-miR-454-5p BS seq


<400>    335
cccuaa                                                                    6


<210>    336
<211>    6
<212>    RNA
<213>    Artificial Sequence

<220>
<223>    miR-509-5p/509-3-5p/4418 BS seq


<400>    336
acugcc                                                                    6


<210>    337
<211>    6
<212>    RNA
<213>    Artificial Sequence

<220>
<223>    hsa-miR-19a/b-5p BS seq


<400>    337
guuuuu                                                                    6

<210>    338
<211>    6
<212>    RNA
<213>    Artificial Sequence

<220>
<223>    miR-4755-5p BS seq


<400>    338
uucccc                                                                    6


<210>    339
<211>    6
<212>    RNA
<213>    Artificial Sequence

<220>
<223>    hsa-miR-93-3p BS seq


<400>    339
cugcuu                                                                    6


<210>    340
<211>    6
<212>    RNA
<213>    Artificial Sequence

<220>
<223>    miR-3130-5p/4482 BS seq


<400>    340
acccaa                                                                    6


<210>    341
<211>    6
<212>    RNA
<213>    Artificial Sequence

<220>
<223>    hsa-miR-488-5p BS seq


<400>    341
ccagaa                                                                    6


<210>    342
<211>    6
<212>    RNA
<213>    Artificial Sequence

<220>
<223>    hsa-miR-378a-5p BS seq


<400>    342

uccugg                                                                  6


<210>   343
<211>   6
<212>   RNA
<213>   Artificial Sequence

<220>
<223>   miR-575/4676-5p BS seq


<400>   343
agccaa                                                                  6


<210>   344
<211>   6
<212>   RNA
<213>   Artificial Sequence

<220>
<223>   miR-1307 BS seq


<400>   344
cucggg                                                                  6


<210>   345
<211>   6
<212>   RNA
<213>   Artificial Sequence

<220>
<223>   miR-3942-3p BS seq


<400>   345
uucagg                                                                  6


<210>   346
<211>   6
<212>   RNA
<213>   Artificial Sequence

<220>
<223>   miR-4677-5p BS seq


<400>   346
uguucc                                                                  6


<210>   347
<211>   6
<212>   RNA
<213>   Artificial Sequence

<220>
<223>   miR-339-3p BS seq

<400>    347
gagcgg                                                                    6


<210>    348
<211>    6
<212>    RNA
<213>    Artificial Sequence

<220>
<223>    miR-548b-3p BS seq


<400>    348
aagaaa                                                                    6


<210>    349
<211>    6
<212>    RNA
<213>    Artificial Sequence

<220>
<223>    hsa-miR-642b-5p BS seq


<400>    349
guuccc                                                                    6


<210>    350
<211>    6
<212>    RNA
<213>    Artificial Sequence

<220>
<223>    miR-188-5p BS seq


<400>    350
   aucccc 6


<210>    351
<211>    6
<212>    RNA
<213>    Artificial Sequence

<220>
<223>    hsa-miR-652-5p BS seq


<400>    351
aacccc                                                                    6


<210>    352
<211>    6
<212>    RNA
<213>    Artificial Sequence

<220>

<223>    miR-2114 BS seq

<400>    352
aguccc                                                                    6

<210>    353
<211>    6
<212>    RNA
<213>    Artificial Sequence

<220>
<223>    miR-3688-5p BS seq

<400>    353
guggcc                                                                    6

<210>    354
<211>    6
<212>    RNA
<213>    Artificial Sequence

<220>
<223>    hsa-miR-15a-3p BS seq

<400>    354
aggccc                                                                    6

<210>    355
<211>    6
<212>    RNA
<213>    Artificial Sequence

<220>
<223>    hsa-miR-181c-3p BS seq

<400>    355
accauu                                                                    6

<210>    356
<211>    6
<212>    RNA
<213>    Artificial Sequence

<220>
<223>    miR-122/122a/1352 BS seq

<400>    356
ggaguu                                                                    6

<210>    357
<211>    6
<212>    RNA
<213>    Artificial Sequence

<220>
<223>    miR-556-3p BS seq


<400>    357
uauuaa                                                                    6


<210>    358
<211>    6
<212>    RNA
<213>    Artificial Sequence

<220>
<223>    hsa-miR-218-2-3p BS seq


<400>    358
augguu                                                                    6


<210>    359
<211>    6
<212>    RNA
<213>    Artificial Sequence

<220>
<223>    miR-643 BS seq


<400>    359
cuuguu                                                                    6


<210>    360
<211>    6
<212>    RNA
<213>    Artificial Sequence

<220>
<223>    miR-140-3p BS seq


<400>    360
accacc                                                                    6


<210>    361
<211>    6
<212>    RNA
<213>    Artificial Sequence

<220>
<223>    miR-1245 BS seq


<400>    361
agugaa                                                                    6


<210>    362
<211>    6

<212>    RNA
<213>    Artificial Sequence

<220>
<223>    hsa-miR-2115-3p BS seq


<400>    362
aucagg                                                                    6


<210>    363
<211>    6
<212>    RNA
<213>    Artificial Sequence

<220>
<223>    miR-518bcf/518a-3p/518d-3p BS seq


<400>    363
aaagcc                                                                    6


<210>    364
<211>    6
<212>    RNA
<213>    Artificial Sequence

<220>
<223>    miR-3200-3p BS seq


<400>    364
accuuu                                                                    6


<210>    365
<211>    6
<212>    RNA
<213>    Artificial Sequence

<220>
<223>    miR-545/3065/3065-5p BS seq


<400>    365
caacaa                                                                    6


<210>    366
<211>    6
<212>    RNA
<213>    Artificial Sequence

<220>
<223>    miR-1903/4778-3p BS seq


<400>    366
cuucuu                                                                    6

<210> 367
<211> 6
<212> RNA
<213> Artificial Sequence

<220>
<223> hsa-miR-302a-5p BS seq


<400> 367
cuuaaa 6


<210> 368
<211> 6
<212> RNA
<213> Artificial Sequence

<220>
<223> hsa-miR-183-3p BS seq


<400> 368
ugaauu 6


<210> 369
<211> 6
<212> RNA
<213> Artificial Sequence

<220>
<223> miR-3144-5p BS seq


<400> 369
ggggaa 6


<210> 370
<211> 6
<212> RNA
<213> Artificial Sequence

<220>
<223> miR-582-3p BS seq


<400> 370
aacugg 6


<210> 371
<211> 6
<212> RNA
<213> Artificial Sequence

<220>
<223> miR-4662a-3p BS seq


<400> 371
aagauu 6

<210>    372
<211>    6
<212>    RNA
<213>    Artificial Sequence

<220>
<223>    miR-3140-5p BS seq


<400>    372
ccugaa                                                                    6


<210>    373
<211>    6
<212>    RNA
<213>    Artificial Sequence

<220>
<223>    hsa-miR-106a-3p BS seq


<400>    373
ugcaaa                                                                    6


<210>    374
<211>    6
<212>    RNA
<213>    Artificial Sequence

<220>
<223>    hsa-miR-135a-3p BS seq


<400>    374
auaggg                                                                    6


<210>    375
<211>    6
<212>    RNA
<213>    Artificial Sequence

<220>
<223>    miR-345/345-5p BS seq


<400>    375
cugacc                                                                    6


<210>    376
<211>    6
<212>    RNA
<213>    Artificial Sequence

<220>
<223>    miR-125a-3p/1554 BS seq

<400>    376
cagguu                                                                    6


<210>    377
<211>    6
<212>    RNA
<213>    Artificial Sequence

<220>
<223>    miR-3145-5p BS seq


<400>    377
acuccc                                                                    6


<210>    378
<211>    6
<212>    RNA
<213>    Artificial Sequence

<220>
<223>    miR-676 BS seq


<400>    378
uguccc                                                                    6


<210>    379
<211>    6
<212>    RNA
<213>    Artificial Sequence

<220>
<223>    miR-3173-5p BS seq


<400>    379
gcccuu                                                                    6


<210>    380
<211>    6
<212>    RNA
<213>    Artificial Sequence

<220>
<223>    hsa-miR-5586-3p BS seq


<400>    380
agaguu                                                                    6


<210>    381
<211>    6
<212>    RNA
<213>    Artificial Sequence

<220>
<223>    miR-615-3p BS seq

<400>    381
ccgagg                                                                 6


<210>    382
<211>    6
<212>    RNA
<213>    Artificial Sequence

<220>
<223>    miR-3688-3p BS seq


<400>    382
auggaa                                                                 6


<210>    383
<211>    6
<212>    RNA
<213>    Artificial Sequence

<220>
<223>    miR-4662a-5p BS seq


<400>    383
uagccc                                                                 6


<210>    384
<211>    6
<212>    RNA
<213>    Artificial Sequence

<220>
<223>    miR-4659ab-5p BS seq


<400>    384
ugccaa                                                                 6


<210>    385
<211>    6
<212>    RNA
<213>    Artificial Sequence

<220>
<223>    hsa-miR-5586-5p BS seq


<400>    385
auccaa                                                                 6


<210>    386
<211>    6
<212>    RNA
<213>    Artificial Sequence

<220>
<223>    hsa-miR-514a-5p BS seq

<400>    386
acucuu                                                                    6

<210>    387
<211>    6
<212>    RNA
<213>    Artificial Sequence

<220>
<223>    miR-10abc/10a-5p BS seq

<400>    387
acccuu                                                                    6

<210>    388
<211>    6
<212>    RNA
<213>    Artificial Sequence

<220>
<223>    hsa-miR-888-3p BS seq

<400>    388
acugaa                                                                    6

<210>    389
<211>    6
<212>    RNA
<213>    Artificial Sequence

<220>
<223>    miR-3127-5p BS seq

<400>    389
ucaggg                                                                    6

<210>    390
<211>    6
<212>    RNA
<213>    Artificial Sequence

<220>
<223>    miR-508-3p BS seq

<400>    390
gauugg                                                                    6

<210>    391
<211>    6
<212>    RNA

<213>    Artificial Sequence

<220>
<223>    hsa-miR-185-3p BS seq


<400>    391
ggggcc                                                                    6


<210>    392
<211>    6
<212>    RNA
<213>    Artificial Sequence

<220>
<223>    hsa-miR-200c-5p,hsa-miR-550a-3p BS seq


<400>    392
gucuuu                                                                    6


<210>    393
<211>    6
<212>    RNA
<213>    Artificial Sequence

<220>
<223>    miR-513c/514b-5p BS seq


<400>    393
ucucaa                                                                    6


<210>    394
<211>    6
<212>    RNA
<213>    Artificial Sequence

<220>
<223>    miR-490-3p BS seq


<400>    394
aaccuu                                                                    6


<210>    395
<211>    6
<212>    RNA
<213>    Artificial Sequence

<220>
<223>    hsa-miR-5187-3p BS seq


<400>    395
cugaaa                                                                    6


<210>    396

<211>    6
<212>    RNA
<213>    Artificial Sequence

<220>
<223>    miR-3664-3p BS seq


<400>    396
cucagg                                                                    6


<210>    397
<211>    6
<212>    RNA
<213>    Artificial Sequence

<220>
<223>    miR-3189-5p BS seq


<400>    397
gccccc                                                                    6


<210>    398
<211>    6
<212>    RNA
<213>    Artificial Sequence

<220>
<223>    miR-4670-3p BS seq


<400>    398
gaaguu                                                                    6


<210>    399
<211>    6
<212>    RNA
<213>    Artificial Sequence

<220>
<223>    miR-105/105ab BS seq


<400>    399
caaauu                                                                    6


<210>    400
<211>    6
<212>    RNA
<213>    Artificial Sequence

<220>
<223>    hsa-miR-135b-3p BS seq


<400>    400
uguagg                                                                    6

<210>    401
<211>    6
<212>    RNA
<213>    Artificial Sequence

<220>
<223>    hsa-miR-5010-3p BS seq


<400>    401
uuuguu                                                                    6


<210>    402
<211>    6
<212>    RNA
<213>    Artificial Sequence

<220>
<223>    miR-493/493b BS seq


<400>    402
gaaggg                                                                    6


<210>    403
<211>    6
<212>    RNA
<213>    Artificial Sequence

<220>
<223>    miR-3605-3p BS seq


<400>    403
   cuccgg 6


<210>    404
<211>    6
<212>    RNA
<213>    Artificial Sequence

<220>
<223>    miR-188-3p BS seq


<400>    404
ucccaa                                                                    6


<210>    405
<211>    6
<212>    RNA
<213>    Artificial Sequence

<220>
<223>    hsa-miR-449c-3p BS seq


<400>    405

ugcuaa                                                                  6


<210>    406
<211>    6
<212>    RNA
<213>    Artificial Sequence

<220>
<223>    miR-4761-5p BS seq


<400>    406
caaggg                                                                  6


<210>    407
<211>    6
<212>    RNA
<213>    Artificial Sequence

<220>
<223>    miR-224 BS seq


<400>    407
aagucc                                                                  6


<210>    408
<211>    6
<212>    RNA
<213>    Artificial Sequence

<220>
<223>    miR-4796-5p BS seq


<400>    408
gucuaa                                                                  6


<210>    409
<211>    6
<212>    RNA
<213>    Artificial Sequence

<220>
<223>    hsa-miR-551b-5p BS seq


<400>    409
aaaucc                                                                  6


<210>    410
<211>    6
<212>    RNA
<213>    Artificial Sequence

<220>
<223>    miR-556-5p BS seq

<400>    410
augagg                                                                 6


<210>    411
<211>    6
<212>    RNA
<213>    Artificial Sequence

<220>
<223>    hsa-miR-122-3p BS seq


<400>    411
acgccc                                                                 6


<210>    412
<211>    6
<212>    RNA
<213>    Artificial Sequence

<220>
<223>    miR-4677-3p BS seq


<400>    412
cugugg                                                                 6


<210>    413
<211>    6
<212>    RNA
<213>    Artificial Sequence

<220>
<223>    miR-877 BS seq


<400>    413
uagagg                                                                 6


<210>    414
<211>    6
<212>    RNA
<213>    Artificial Sequence

<220>
<223>    miR-576-5p BS seq


<400>    414
uucuaa                                                                 6


<210>    415
<211>    6
<212>    RNA
<213>    Artificial Sequence

<220>

<223>    miR-490-5p BS seq

<400>    415
cauggg                                                                    6

<210>    416
<211>    6
<212>    RNA
<213>    Artificial Sequence

<220>
<223>    hsa-miR-589-3p BS seq

<400>    416
cagaaa                                                                    6

<210>    417
<211>    6
<212>    RNA
<213>    Artificial Sequence

<220>
<223>    miR-4786-3p BS seq

<400>    417
gaagcc                                                                    6

<210>    418
<211>    6
<212>    RNA
<213>    Artificial Sequence

<220>
<223>    hsa-miR-374b-3p BS seq

<400>    418
uuagcc                                                                    6

<210>    419
<211>    6
<212>    RNA
<213>    Artificial Sequence

<220>
<223>    hsa-miR-26b-3p BS seq

<400>    419
cuguuu                                                                    6

<210>    420
<211>    6
<212>    RNA
<213>    Artificial Sequence

<220>
<223>    miR-3158-3p BS seq


<400>    420
agggcc                                                                    6


<210>    421
<211>    6
<212>    RNA
<213>    Artificial Sequence

<220>
<223>    miR-4423-3p BS seq


<400>    421
uaggcc                                                                    6


<210>    422
<211>    6
<212>    RNA
<213>    Artificial Sequence

<220>
<223>    miR-518d-5p/519bc-5p520c-5p/523b/526a BS seq


<400>    422
ucuagg                                                                    6


<210>    423
<211>    6
<212>    RNA
<213>    Artificial Sequence

<220>
<223>    miR-4707-3p BS seq


<400>    423
gcccgg                                                                    6


<210>    424
<211>    6
<212>    RNA
<213>    Artificial Sequence

<220>
<223>    hsa-miR-10a-3p BS seq


<400>    424
aaauuu                                                                    6


<210>    425
<211>    6

<212>    RNA
<213>    Artificial Sequence

<220>
<223>    miR-526b BS seq


<400>    425
ucuugg                                                                    6


<210>    426
<211>    6
<212>    RNA
<213>    Artificial Sequence

<220>
<223>    hsa-miR-676-5p BS seq


<400>    426
cuucaa                                                                    6


<210>    427
<211>    6
<212>    RNA
<213>    Artificial Sequence

<220>
<223>    hsa-miR-660-3p BS seq


<400>    427
ccuccc                                                                    6


<210>    428
<211>    6
<212>    RNA
<213>    Artificial Sequence

<220>
<223>    hsa-miR-5004-3p BS seq


<400>    428
uuggaa                                                                    6


<210>    429
<211>    6
<212>    RNA
<213>    Artificial Sequence

<220>
<223>    miR-193a-5p BS seq


<400>    429
gggucc                                                                    6

<210>    430
<211>    6
<212>    RNA
<213>    Artificial Sequence

<220>
<223>    hsa-miR-222-5p BS seq


<400>    430
ucaguu                                                                    6


<210>    431
<211>    6
<212>    RNA
<213>    Artificial Sequence

<220>
<223>    miR-4661-3p BS seq


<400>    431
aggauu                                                                    6


<210>    432
<211>    6
<212>    RNA
<213>    Artificial Sequence

<220>
<223>    hsa-miR-25-5p BS seq


<400>    432
ggcggg                                                                    6


<210>    433
<211>    6
<212>    RNA
<213>    Artificial Sequence

<220>
<223>    miR-4670-5p BS seq


<400>    433
agcgaa                                                                    6


<210>    434
<211>    6
<212>    RNA
<213>    Artificial Sequence

<220>
<223>    miR-659 BS seq


<400>    434
uugguu                                                                    6

<210>	435
<211>	6
<212>	RNA
<213>	Artificial Sequence

<220>
<223>	miR-1745/3194-3p BS seq


<400>	435
gcucuu	6


<210>	436
<211>	6
<212>	RNA
<213>	Artificial Sequence

<220>
<223>	hsa-miR-182-3p BS seq


<400>	436
gguucc	6


<210>	437
<211>	6
<212>	RNA
<213>	Artificial Sequence

<220>
<223>	miR-298/2347/2467-3p BS seq


<400>	437
gcagaa	6


<210>	438
<211>	6
<212>	RNA
<213>	Artificial Sequence

<220>
<223>	hsa-miR-130b-5p BS seq


<400>	438
cucuuu	6


<210>	439
<211>	6
<212>	RNA
<213>	Artificial Sequence

<220>
<223>	miR-4746-3p BS seq

<400>    439
gcgguu                                                                    6

<210>    440
<211>    6
<212>    RNA
<213>    Artificial Sequence

<220>
<223>    miR-1893/2277-5p BS seq

<400>    440
gcgcgg                                                                    6

<210>    441
<211>    6
<212>    RNA
<213>    Artificial Sequence

<220>
<223>    miR-3619-3p BS seq

<400>    441
ggaccc                                                                    6

<210>    442
<211>    6
<212>    RNA
<213>    Artificial Sequence

<220>
<223>    hsa-miR-138-1-3p BS seq

<400>    442
cuacuu                                                                    6

<210>    443
<211>    6
<212>    RNA
<213>    Artificial Sequence

<220>
<223>    miR-4728-3p BS seq

<400>    443
augcuu                                                                    6

<210>    444
<211>    6
<212>    RNA
<213>    Artificial Sequence

<220>
<223>    miR-3127-3p BS seq

<400>    444
ccccuu                                                                 6

<210>    445
<211>    6
<212>    RNA
<213>    Artificial Sequence

<220>
<223>    miR-671-3p BS seq

<400>    445
ccgguu                                                                 6

<210>    446
<211>    6
<212>    RNA
<213>    Artificial Sequence

<220>
<223>    hsa-miR-211-3p BS seq

<400>    446
cagggg                                                                 6

<210>    447
<211>    6
<212>    RNA
<213>    Artificial Sequence

<220>
<223>    hsa-miR-2114-3p BS seq

<400>    447
gagccc                                                                 6

<210>    448
<211>    6
<212>    RNA
<213>    Artificial Sequence

<220>
<223>    hsa-miR-877-3p BS seq

<400>    448
ccucuu                                                                 6

<210>    449
<211>    6
<212>    RNA
<213>    Artificial Sequence

<220>
<223>    miR-3157-5p BS seq


<400>    449
ucagcc                                                                    6


<210>    450
<211>    6
<212>    RNA
<213>    Artificial Sequence

<220>
<223>    miR-502-5p BS seq


<400>    450
uccuuu                                                                    6


<210>    451
<211>    6
<212>    RNA
<213>    Artificial Sequence

<220>
<223>    miR-500a BS seq


<400>    451
aauccc                                                                    6


<210>    452
<211>    6
<212>    RNA
<213>    Artificial Sequence

<220>
<223>    miR-548g BS seq


<400>    452
aaacuu                                                                    6


<210>    453
<211>    6
<212>    RNA
<213>    Artificial Sequence

<220>
<223>    miR-523 BS seq


<400>    453
aacgcc                                                                    6


<210>    454
<211>    6
<212>    RNA

<213>    Artificial Sequence

<220>
<223>    hsa-miR-584-3p BS seq


<400>    454
caguuu                                                                    6


<210>    455
<211>    6
<212>    RNA
<213>    Artificial Sequence

<220>
<223>    miR-205/205ab BS seq


<400>    455
ccuucc                                                                    6


<210>    456
<211>    6
<212>    RNA
<213>    Artificial Sequence

<220>
<223>    miR-4793-5p BS seq


<400>    456
cauccc                                                                    6


<210>    457
<211>    6
<212>    RNA
<213>    Artificial Sequence

<220>
<223>    hsa-miR-363-5p BS seq


<400>    457
gggugg                                                                    6


<210>    458
<211>    6
<212>    RNA
<213>    Artificial Sequence

<220>
<223>    hsa-miR-214-5p BS seq


<400>    458
gccugg                                                                    6


<210>    459

<211>    6
<212>    RNA
<213>    Artificial Sequence

<220>
<223>    miR-3180-5p BS seq


<400>    459
uuccaa                                                                    6


<210>    460
<211>    6
<212>    RNA
<213>    Artificial Sequence

<220>
<223>    miR-1404/2110 BS seq


<400>    460
uggggg                                                                    6


<210>    461
<211>    6
<212>    RNA
<213>    Artificial Sequence

<220>
<223>    miR-3157-3p BS seq


<400>    461
ugcccc                                                                    6


<210>    462
<211>    6
<212>    RNA
<213>    Artificial Sequence

<220>
<223>    hsa-miR-191-3p BS seq


<400>    462
cugcgg                                                                    6


<210>    463
<211>    6
<212>    RNA
<213>    Artificial Sequence

<220>
<223>    miR-1346/3940-5p/4507 BS seq


<400>    463
   ugggu u	6

<210> 464
<211> 6
<212> RNA
<213> Artificial Sequence

<220>
<223> miR-4746-5p BS seq

<400> 464
cggucc 6

<210> 465
<211> 6
<212> RNA
<213> Artificial Sequence

<220>
<223> miR-3939 BS seq

<400> 465
acgcgg 6

<210> 466
<211> 6
<212> RNA
<213> Artificial Sequence

<220>
<223> hsa-miR-181a-2-3p BS seq

<400> 466
ccacuu 6

<210> 467
<211> 6
<212> RNA
<213> Artificial Sequence

<220>
<223> hsa-miR-500a-3p BS seq

<400> 467
ugcacc 6

<210> 468
<211> 6
<212> RNA
<213> Artificial Sequence

<220>
<223> hsa-miR-196b-3p BS seq

<400> 468

cgacaa                                                                    6

<210>    469
<211>    6
<212>    RNA
<213>    Artificial Sequence

<220>
<223>    hsa-miR-675-3p BS seq

<400>    469
uguauu                                                                    6

<210>    470
<211>    6
<212>    RNA
<213>    Artificial Sequence

<220>
<223>    hsa-miR-548aj/g/x-5p BS seq

<400>    470
gcaaaa                                                                    6

<210>    471
<211>    6
<212>    RNA
<213>    Artificial Sequence

<220>
<223>    miR-4659ab-3p BS seq

<400>    471
uucuuu                                                                    6

<210>    472
<211>    6
<212>    RNA
<213>    Artificial Sequence

<220>
<223>    hsa-miR-5001-3p BS seq

<400>    472
ucugcc                                                                    6

<210>    473
<211>    6
<212>    RNA
<213>    Artificial Sequence

<220>
<223>    hsa-miR-1247-3p BS seq

<400>    473
cccggg                                                                    6


<210>    474
<211>    6
<212>    RNA
<213>    Artificial Sequence

<220>
<223>    miR-2890/4707-5p BS seq


<400>    474
ccccgg                                                                    6


<210>    475
<211>    6
<212>    RNA
<213>    Artificial Sequence

<220>
<223>    hsa-miR-150-3p BS seq


<400>    475
ugguaa                                                                    6


<210>    476
<211>    6
<212>    RNA
<213>    Artificial Sequence

<220>
<223>    hsa-miR-629-3p BS seq


<400>    476
uucucc                                                                    6


<210>    477
<211>    6
<212>    RNA
<213>    Artificial Sequence

<220>
<223>    miR-2277-3p BS seq


<400>    477
gacagg                                                                    6


<210>    478
<211>    6
<212>    RNA
<213>    Artificial Sequence

<220>

<223>    miR-3547/3663-3p BS seq

<400>    478
gagcaa                                                                    6

<210>    479
<211>    6
<212>    RNA
<213>    Artificial Sequence

<220>
<223>    miR-34bc-3p BS seq

<400>    479
aucacc                                                                    6

<210>    480
<211>    6
<212>    RNA
<213>    Artificial Sequence

<220>
<223>    miR-518ef BS seq

<400>    480
aagcgg                                                                    6

<210>    481
<211>    6
<212>    RNA
<213>    Artificial Sequence

<220>
<223>    miR-3187-3p BS seq

<400>    481
uggccc                                                                    6

<210>    482
<211>    6
<212>    RNA
<213>    Artificial Sequence

<220>
<223>    miR-1306/1306-3p BS seq

<400>    482
cguugg                                                                    6

<210>    483
<211>    6
<212>    RNA
<213>    Artificial Sequence

<220>
<223>    miR-3177-3p BS seq


<400>    483
gcacgg                                                                    6


<210>    484
<211>    6
<212>    RNA
<213>    Artificial Sequence

<220>
<223>    miR-1ab/206/613 BS seq


<400>    484
ggaauu                                                                    6


<210>    485
<211>    6
<212>    RNA
<213>    Artificial Sequence

<220>
<223>    miR-128/128ab BS seq


<400>    485
cacagg                                                                    6


<210>    486
<211>    6
<212>    RNA
<213>    Artificial Sequence

<220>
<223>    miR-1296 BS seq


<400>    486
uagggg                                                                    6


<210>    487
<211>    6
<212>    RNA
<213>    Artificial Sequence

<220>
<223>    miR-598/598-3p BS seq


<400>    487
acgucc                                                                    6


<210>    488
<211>    6

<212>    RNA
<213>    Artificial Sequence

<220>
<223>    miR-887 BS seq


<400>    488
ugaacc                                                                 6


<210>    489
<211>    6
<212>    RNA
<213>    Artificial Sequence

<220>
<223>    miR-1-5p BS seq


<400>    489
cauacc                                                                 6


<210>    490
<211>    6
<212>    RNA
<213>    Artificial Sequence

<220>
<223>    miR-376c/741-5p BS seq


<400>    490
acauaa                                                                 6


<210>    491
<211>    6
<212>    RNA
<213>    Artificial Sequence

<220>
<223>    miR-374c/655 BS seq


<400>    491
uaauaa                                                                 6


<210>    492
<211>    6
<212>    RNA
<213>    Artificial Sequence

<220>
<223>    miR-494 BS seq


<400>    492
gaaacc                                                                 6

<210>	493
<211>	6
<212>	RNA
<213>	Artificial Sequence

<220>
<223>	miR-651 BS seq


<400>	493
uuaggg	6


<210>	494
<211>	6
<212>	RNA
<213>	Artificial Sequence

<220>
<223>	miR-1301/5047 BS seq


<400>	494
ugcagg	6


<210>	495
<211>	6
<212>	RNA
<213>	Artificial Sequence

<220>
<223>	miR-381-5p BS seq


<400>	495
gcgagg	6


<210>	496
<211>	6
<212>	RNA
<213>	Artificial Sequence

<220>
<223>	miR-216a BS seq


<400>	496
aaucuu	6


<210>	497
<211>	6
<212>	RNA
<213>	Artificial Sequence

<220>
<223>	miR-300/381/539-3p BS seq


<400>	497
auacaa	6

<210>    498
<211>    6
<212>    RNA
<213>    Artificial Sequence

<220>
<223>    miR-1249 BS seq


<400>    498
cgcccc                                                                    6


<210>    499
<211>    6
<212>    RNA
<213>    Artificial Sequence

<220>
<223>    miR-579 BS seq


<400>    499
ucauuu                                                                    6


<210>    500
<211>    6
<212>    RNA
<213>    Artificial Sequence

<220>
<223>    miR-656 BS seq


<400>    500
auauuu                                                                    6


<210>    501
<211>    6
<212>    RNA
<213>    Artificial Sequence

<220>
<223>    miR-433 BS seq


<400>    501
ucaugg                                                                    6


<210>    502
<211>    6
<212>    RNA
<213>    Artificial Sequence

<220>
<223>    miR-1180 BS seq

<400>    502
uuccgg                                                                 6

<210>    503
<211>    6
<212>    RNA
<213>    Artificial Sequence

<220>
<223>    miR-597/1970 BS seq

<400>    503
   guguc c                                                                6

<210>    504
<211>    6
<212>    RNA
<213>    Artificial Sequence

<220>
<223>    miR-190a-3p BS seq

<400>    504
uauauu                                                                 6

<210>    505
<211>    6
<212>    RNA
<213>    Artificial Sequence

<220>
<223>    miR-1537 BS seq

<400>    505
aaaccc                                                                 6

<210>    506
<211>    6
<212>    RNA
<213>    Artificial Sequence

<220>
<223>    miR-874-5p BS seq

<400>    506
ggcccc                                                                 6

<210>    507
<211>    6
<212>    RNA
<213>    Artificial Sequence

<220>
<223>    miR-410/344de/344b-1-3p BS seq

<400>    507
auauaa                                                                    6

<210>    508
<211>    6
<212>    RNA
<213>    Artificial Sequence

<220>
<223>    miR-370 BS seq

<400>    508
ccugcc                                                                    6

<210>    509
<211>    6
<212>    RNA
<213>    Artificial Sequence

<220>
<223>    miR-219-2-3p/219-3p BS seq

<400>    509
gaauuu                                                                    6

<210>    510
<211>    6
<212>    RNA
<213>    Artificial Sequence

<220>
<223>    miR-3620 BS seq

<400>    510
cacccc                                                                    6

<210>    511
<211>    6
<212>    RNA
<213>    Artificial Sequence

<220>
<223>    miR-504/4725-5p BS seq

<400>    511
gacccc                                                                    6

<210>    512
<211>    6
<212>    RNA
<213>    Artificial Sequence

<220>
<223>    miR-2964/2964a-5p BS seq


<400>    512
gauguu                                                                    6


<210>    513
<211>    6
<212>    RNA
<213>    Artificial Sequence

<220>
<223>    miR-450a-2-3p BS seq


<400>    513
uugggg                                                                    6


<210>    514
<211>    6
<212>    RNA
<213>    Artificial Sequence

<220>
<223>    miR-511 BS seq


<400>    514
ugucuu                                                                    6


<210>    515
<211>    6
<212>    RNA
<213>    Artificial Sequence

<220>
<223>    miR-6505-3p BS seq


<400>    515
gacuuu                                                                    6


<210>    516
<211>    6
<212>    RNA
<213>    Artificial Sequence

<220>
<223>    miR-433-5p BS seq


<400>    516
acgguu                                                                    6


<210>    517
<211>    6
<212>    RNA

<213>    Artificial Sequence

<220>
<223>    miR-6741-3p BS seq


<400>    517
cggcuu                                                                    6


<210>    518
<211>    6
<212>    RNA
<213>    Artificial Sequence

<220>
<223>    miR-370-5p BS seq


<400>    518
aggucc                                                                    6


<210>    519
<211>    6
<212>    RNA
<213>    Artificial Sequence

<220>
<223>    miR-579-5p BS seq


<400>    519
cgcggg                                                                    6


<210>    520
<211>    6
<212>    RNA
<213>    Artificial Sequence

<220>
<223>    miR-376c-5p,miR-376b-5p BS seq


<400>    520
guggaa                                                                    6


<210>    521
<211>    6
<212>    RNA
<213>    Artificial Sequence

<220>
<223>    miR-552/3097-5p BS seq


<400>    521
acaggg                                                                    6


<210>    522

<211>    6
<212>    RNA
<213>    Artificial Sequence

<220>
<223>    miR-1910 BS seq


<400>    522
cagucc                                                                    6


<210>    523
<211>    6
<212>    RNA
<213>    Artificial Sequence

<220>
<223>    miR-758 BS seq


<400>    523
uugugg                                                                    6


<210>    524
<211>    6
<212>    RNA
<213>    Artificial Sequence

<220>
<223>    miR-6735-3p BS seq


<400>    524
ggccuu                                                                    6


<210>    525
<211>    6
<212>    RNA
<213>    Artificial Sequence

<220>
<223>    miR-376a-2-5p BS seq


<400>    525
guagaa                                                                    6


<210>    526
<211>    6
<212>    RNA
<213>    Artificial Sequence

<220>
<223>    miR-585 BS seq


<400>    526
gggcgg                                                                    6

<210>    527
<211>    6
<212>    RNA
<213>    Artificial Sequence

<220>
<223>    miR-451 BS seq


<400>    527
aaccgg                                                                    6


<210>    528
<211>    6
<212>    RNA
<213>    Artificial Sequence

<220>
<223>    miR-137/137ab BS seq


<400>    528
uauugg                                                                    6


<210>    529
<211>    6
<212>    RNA
<213>    Artificial Sequence

<220>
<223>    hsa-miR-1-3p G2U


<400>    529
ugaaug                                                                    6


<210>    530
<211>    6
<212>    RNA
<213>    Artificial Sequence

<220>
<223>    hsa-miR-194-5p G2U


<400>    530
uuaaca                                                                    6


<210>    531
<211>    6
<212>    RNA
<213>    Artificial Sequence

<220>
<223>    hsa-miR-193a-5p G2U


<400>    531

uggucu                                                                  6


<210>    532
<211>    6
<212>    RNA
<213>    Artificial Sequence

<220>
<223>    hsa-miR-15b-3p G2U


<400>    532
uaauca                                                                  6


<210>    533
<211>    6
<212>    RNA
<213>    Artificial Sequence

<220>
<223>    hsa-miR-200c-5p G2U


<400>    533
uucuua                                                                  6


<210>    534
<211>    6
<212>    RNA
<213>    Artificial Sequence

<220>
<223>    hsa-miR-214-5p G2U


<400>    534
uccugu                                                                  6


<210>    535
<211>    6
<212>    RNA
<213>    Artificial Sequence

<220>
<223>    hsa-miR-134-5p G2U


<400>    535
uugacu                                                                  6


<210>    536
<211>    6
<212>    RNA
<213>    Artificial Sequence

<220>
<223>    hsa-miR-145-3p G2U

<400>    536
uauucc                                                                 6


<210>    537
<211>    6
<212>    RNA
<213>    Artificial Sequence

<220>
<223>    hsa-miR-22-5p G2U


<400>    537
uuucuu                                                                 6


<210>    538
<211>    6
<212>    RNA
<213>    Artificial Sequence

<220>
<223>    hsa-miR-423-3p G2U


<400>    538
ucucgg                                                                 6


<210>    539
<211>    6
<212>    RNA
<213>    Artificial Sequence

<220>
<223>    hsa-miR-873-3p G2U


<400>    539
uagacu                                                                 6


<210>    540
<211>    6
<212>    RNA
<213>    Artificial Sequence

<220>
<223>    hsa-miR-122-5p G2U


<400>    540
ugagug                                                                 6


<210>    541
<211>    6
<212>    RNA
<213>    Artificial Sequence

<220>

<223>    hsa-miR-143-3p G2U

<400>    541
uagaug                                                                 6

<210>    542
<211>    6
<212>    RNA
<213>    Artificial Sequence

<220>
<223>    hsa-miR-485-5p G2U

<400>    542
uaggcu                                                                 6

<210>    543
<211>    6
<212>    RNA
<213>    Artificial Sequence

<220>
<223>    hsa-miR-409-5p G2U

<400>    543
uguuac                                                                 6

<210>    544
<211>    6
<212>    RNA
<213>    Artificial Sequence

<220>
<223>    hsa-miR-24-3p G2U

<400>    544
ugcuca                                                                 6

<210>    545
<211>    6
<212>    RNA
<213>    Artificial Sequence

<220>
<223>    hsa-miR-223-3p G2U

<400>    545
uucagu                                                                 6

<210>    546
<211>    6
<212>    RNA
<213>    Artificial Sequence

<220>
<223>    hsa-miR-144-5p G2U

<400>    546
uauauc                                                                    6

<210>    547
<211>    6
<212>    RNA
<213>    Artificial Sequence

<220>
<223>    hsa-miR-379-5p G2U

<400>    547
uguaga                                                                    6

<210>    548
<211>    6
<212>    RNA
<213>    Artificial Sequence

<220>
<223>    hsa-miR-146b-5p/hsa-miR-146a-5p G2U

<400>    548
uagaac                                                                    6

<210>    549
<211>    6
<212>    RNA
<213>    Artificial Sequence

<220>
<223>    hsa-miR-539-5p G2U

<400>    549
uagaaa                                                                    6

<210>    550
<211>    6
<212>    RNA
<213>    Artificial Sequence

<220>
<223>    hsa-miR-296-5p G2U

<400>    550
uggccc                                                                    6

<210>    551
<211>    6

<212>    RNA
<213>    Artificial Sequence

<220>
<223>    hsa-miR-767-5p G2U


<400>    551
ucacca                                                                    6


<210>    552
<211>    6
<212>    RNA
<213>    Artificial Sequence

<220>
<223>    hsa-miR-34a-5p/hsa-miR-34c-5p G2U


<400>    552
ugcagu                                                                    6


<210>    553
<211>    6
<212>    RNA
<213>    Artificial Sequence

<220>
<223>    hsa-let-7f-5p/hsa-let-7d-5p/hsa-let-7b-5p/hsa-let-7a-5p/hsa-let-7
         e-5p/hsa-miR-202-3p/hsa-let-7i-5p/hsa-miR-98-5p/hsa-let-7c-5p/hsa
         -let-7g-5p G2U


<400>    553
uaggua                                                                    6


<210>    554
<211>    6
<212>    RNA
<213>    Artificial Sequence

<220>
<223>    hsa-miR-1271-3p G2U


<400>    554
uugccu                                                                    6


<210>    555
<211>    6
<212>    RNA
<213>    Artificial Sequence

<220>
<223>    hsa-miR-138-5p G2U


<400>    555
ucuggu                                                                    6

<210>    556
<211>    6
<212>    RNA
<213>    Artificial Sequence

<220>
<223>    hsa-miR-19b-3p/hsa-miR-19a-3p G2U


<400>    556
uugcaa                                                                    6


<210>    557
<211>    6
<212>    RNA
<213>    Artificial Sequence

<220>
<223>    hsa-miR-27a-5p G2U


<400>    557
uggcuu                                                                    6


<210>    558
<211>    6
<212>    RNA
<213>    Artificial Sequence

<220>
<223>    hsa-miR-146b-3p G2U


<400>    558
ucccug                                                                    6


<210>    559
<211>    6
<212>    RNA
<213>    Artificial Sequence

<220>
<223>    hsa-miR-7-5p G2U


<400>    559
ugaaga                                                                    6


<210>    560
<211>    6
<212>    RNA
<213>    Artificial Sequence

<220>
<223>    hsa-miR-423-5p G2U

<400>    560
uagggg                                                                    6


<210>    561
<211>    6
<212>    RNA
<213>    Artificial Sequence

<220>
<223>    hsa-miR-324-5p G2U


<400>    561
ucaucc                                                                    6


<210>    562
<211>    6
<212>    RNA
<213>    Artificial Sequence

<220>
<223>    hsa-miR-629-5p G2U


<400>    562
ugguuu                                                                    6


<210>    563
<211>    6
<212>    RNA
<213>    Artificial Sequence

<220>
<223>    hsa-miR-139-3p G2U


<400>    563
ugagac                                                                    6


<210>    564
<211>    6
<212>    RNA
<213>    Artificial Sequence

<220>
<223>    hsa-miR-30d-5p/hsa-miR-30e-5p/hsa-miR-30a-5p/hsa-miR-30c-5p/hsa-m
         iR-30b-5p G2U


<400>    564
uuaaac                                                                    6


<210>    565
<211>    6
<212>    RNA
<213>    Artificial Sequence

<220>

<223>    hsa-miR-221-3p/hsa-miR-222-3p G2U

<400>    565
ucuaca                                                                    6

<210>    566
<211>    6
<212>    RNA
<213>    Artificial Sequence

<220>
<223>    hsa-miR-509-3p G2U

<400>    566
uauugg                                                                    6

<210>    567
<211>    6
<212>    RNA
<213>    Artificial Sequence

<220>
<223>    hsa-miR-769-5p G2U

<400>    567
uagacc                                                                    6

<210>    568
<211>    6
<212>    RNA
<213>    Artificial Sequence

<220>
<223>    hsa-miR-142-3p G2U

<400>    568
uuagug                                                                    6

<210>    569
<211>    6
<212>    RNA
<213>    Artificial Sequence

<220>
<223>    hsa-miR-185-5p G2U

<400>    569
ugagag                                                                    6

<210>    570
<211>    6
<212>    RNA
<213>    Artificial Sequence

<220>
<223>    hsa-miR-508-3p/hsa-miR-219a-5p G2U


<400>    570
uauugu                                                                    6


<210>    571
<211>    6
<212>    RNA
<213>    Artificial Sequence

<220>
<223>    hsa-miR-31-5p G2U


<400>    571
ugcaag                                                                    6


<210>    572
<211>    6
<212>    RNA
<213>    Artificial Sequence

<220>
<223>    hsa-miR-103a-3p/hsa-miR-107 G2U


<400>    572
ucagca                                                                    6


<210>    573
<211>    6
<212>    RNA
<213>    Artificial Sequence

<220>
<223>    hsa-miR-542-3p G2U


<400>    573
uugaca                                                                    6


<210>    574
<211>    6
<212>    RNA
<213>    Artificial Sequence

<220>
<223>    hsa-miR-219a-2-3p G2U


<400>    574
uaauug                                                                    6


<210>    575
<211>    6

<212>    RNA
<213>    Artificial Sequence

<220>
<223>    hsa-miR-29c-3p/hsa-miR-29a-3p/hsa-miR-29b-3p G3U


<400>    575
aucacc                                                                    6


<210>    576
<211>    6
<212>    RNA
<213>    Artificial Sequence

<220>
<223>    hsa-miR-125b-1-3p G3U


<400>    576
cugguu                                                                    6


<210>    577
<211>    6
<212>    RNA
<213>    Artificial Sequence

<220>
<223>    hsa-miR-7-5p G3U


<400>    577
guaaga                                                                    6


<210>    578
<211>    6
<212>    RNA
<213>    Artificial Sequence

<220>
<223>    hsa-miR-411-5p G3U


<400>    578
auuaga                                                                    6


<210>    579
<211>    6
<212>    RNA
<213>    Artificial Sequence

<220>
<223>    hsa-miR-196a-5p/hsa-miR-196b-5p G3U


<400>    579
auguag                                                                    6

<210> 580
<211> 6
<212> RNA
<213> Artificial Sequence

<220>
<223> hsa-miR-3622a-5p G3U

<400> 580
augcac 6

<210> 581
<211> 6
<212> RNA
<213> Artificial Sequence

<220>
<223> hsa-miR-127-5p G3U

<400> 581
uuaagc 6

<210> 582
<211> 6
<212> RNA
<213> Artificial Sequence

<220>
<223> hsa-miR-22-3p G3U

<400> 582
aucugc 6

<210> 583
<211> 6
<212> RNA
<213> Artificial Sequence

<220>
<223> hsa-miR-153-3p G3U

<400> 583
uucaua 6

<210> 584
<211> 6
<212> RNA
<213> Artificial Sequence

<220>
<223> hsa-miR-193a-5p G3U

<400> 584
gugucu 6

<210>	585
<211>	6
<212>	RNA
<213>	Artificial Sequence

<220>
<223>	hsa-miR-185-5p G3U


<400>	585
guagag	6


<210>	586
<211>	6
<212>	RNA
<213>	Artificial Sequence

<220>
<223>	hsa-miR-1-3p G3U


<400>	586
guaaug	6


<210>	587
<211>	6
<212>	RNA
<213>	Artificial Sequence

<220>
<223>	hsa-miR-15b-5p/hsa-miR-16-5p/hsa-miR-424-5p G3U


<400>	587
aucagc	6


<210>	588
<211>	6
<212>	RNA
<213>	Artificial Sequence

<220>
<223>	hsa-let-7g-3p/hsa-miR-493-5p/hsa-let-7c-3p G3U


<400>	588
uuuaca	6


<210>	589
<211>	6
<212>	RNA
<213>	Artificial Sequence

<220>
<223>	hsa-let-7i-3p G3U

<400>    589
uucgca                                                                    6


<210>    590
<211>    6
<212>    RNA
<213>    Artificial Sequence

<220>
<223>    hsa-miR-218-5p G3U


<400>    590
uuugcu                                                                    6


<210>    591
<211>    6
<212>    RNA
<213>    Artificial Sequence

<220>
<223>    hsa-miR-1307-5p G3U


<400>    591
cuaccg                                                                    6


<210>    592
<211>    6
<212>    RNA
<213>    Artificial Sequence

<220>
<223>    hsa-miR-127-3p G3U


<400>    592
cugauc                                                                    6


<210>    593
<211>    6
<212>    RNA
<213>    Artificial Sequence

<220>
<223>    hsa-miR-210-3p G3U


<400>    593
uuugcg                                                                    6


<210>    594
<211>    6
<212>    RNA
<213>    Artificial Sequence

<220>
<223>    hsa-miR-187-3p G3U

<400>    594
cuuguc                                                                    6

<210>    595
<211>    6
<212>    RNA
<213>    Artificial Sequence

<220>
<223>    hsa-miR-192-3p G3U

<400>    595
uuccaa                                                                    6

<210>    596
<211>    6
<212>    RNA
<213>    Artificial Sequence

<220>
<223>    hsa-miR-192-5p G3U

<400>    596
uuaccu                                                                    6

<210>    597
<211>    6
<212>    RNA
<213>    Artificial Sequence

<220>
<223>    hsa-miR-34a-5p/hsa-miR-34c-5p G3U

<400>    597
gucagu                                                                    6

<210>    598
<211>    6
<212>    RNA
<213>    Artificial Sequence

<220>
<223>    hsa-miR-21-5p G3U

<400>    598
aucuua                                                                    6

<210>    599
<211>    6
<212>    RNA
<213>    Artificial Sequence

<220>
<223>    hsa-miR-500a-3p G3U


<400>    599
uucacc                                                                    6


<210>    600
<211>    6
<212>    RNA
<213>    Artificial Sequence

<220>
<223>    hsa-miR-629-5p G3U


<400>    600
guguuu                                                                    6


<210>    601
<211>    6
<212>    RNA
<213>    Artificial Sequence

<220>
<223>    hsa-miR-379-5p G3U


<400>    601
guuaga                                                                    6


<210>    602
<211>    6
<212>    RNA
<213>    Artificial Sequence

<220>
<223>    hsa-miR-203a-3p G3U


<400>    602
uuaaau                                                                    6


<210>    603
<211>    6
<212>    RNA
<213>    Artificial Sequence

<220>
<223>    hsa-miR-24-3p G3U


<400>    603
gucuca                                                                    6


<210>    604
<211>    6
<212>    RNA

<213>    Artificial Sequence

<220>
<223>    hsa-miR-30c-2-3p G3U


<400>    604
uuggag                                                                    6


<210>    605
<211>    6
<212>    RNA
<213>    Artificial Sequence

<220>
<223>    hsa-miR-488-3p G3U


<400>    605
uuaaag                                                                    6


<210>    606
<211>    6
<212>    RNA
<213>    Artificial Sequence

<220>
<223>    hsa-miR-301a-3p/hsa-miR-301b-3p G3U


<400>    606
auugca                                                                    6


<210>    607
<211>    6
<212>    RNA
<213>    Artificial Sequence

<220>
<223>    hsa-miR-126-3p G3U


<400>    607
cuuacc                                                                    6


<210>    608
<211>    6
<212>    RNA
<213>    Artificial Sequence

<220>
<223>    hsa-miR-122-5p G3U


<400>    608
guagug                                                                    6


<210>    609

<211>    6
<212>    RNA
<213>    Artificial Sequence

<220>
<223>    hsa-miR-27a-5p G3U


<400>    609
gugcuu                                                                    6


<210>    610
<211>    6
<212>    RNA
<213>    Artificial Sequence

<220>
<223>    hsa-miR-26b-3p G4U


<400>    610
cuuuuc                                                                    6


<210>    611
<211>    6
<212>    RNA
<213>    Artificial Sequence

<220>
<223>    hsa-miR-324-3p G4U


<400>    611
cuuccc                                                                    6


<210>    612
<211>    6
<212>    RNA
<213>    Artificial Sequence

<220>
<223>    hsa-miR-3065-3p G4U


<400>    612
caucac                                                                    6


<210>    613
<211>    6
<212>    RNA
<213>    Artificial Sequence

<220>
<223>    hsa-miR-423-5p G4U


<400>    613
gauggg                                                                    6

<210>    614
<211>    6
<212>    RNA
<213>    Artificial Sequence

<220>
<223>    hsa-miR-124-5p G4U


<400>    614
guuuuc                                                                    6


<210>    615
<211>    6
<212>    RNA
<213>    Artificial Sequence

<220>
<223>    hsa-miR-345-5p G4U


<400>    615
cuuacu                                                                    6


<210>    616
<211>    6
<212>    RNA
<213>    Artificial Sequence

<220>
<223>    hsa-miR-615-3p G4U


<400>    616
ccuagc                                                                    6


<210>    617
<211>    6
<212>    RNA
<213>    Artificial Sequence

<220>
<223>    hsa-miR-889-5p/hsa-miR-135a-5p/hsa-miR-135b-5p G4U


<400>    617
auugcu                                                                    6


<210>    618
<211>    6
<212>    RNA
<213>    Artificial Sequence

<220>
<223>    hsa-miR-193a-5p G4U


<400>    618

gguucu                                                                  6

<210>    619
<211>    6
<212>    RNA
<213>    Artificial Sequence

<220>
<223>    hsa-miR-18a-5p G4U

<400>    619
aaugug                                                                  6

<210>    620
<211>    6
<212>    RNA
<213>    Artificial Sequence

<220>
<223>    hsa-miR-125b-1-3p G4U

<400>    620
cguguu                                                                  6

<210>    621
<211>    6
<212>    RNA
<213>    Artificial Sequence

<220>
<223>    hsa-miR-708-5p/hsa-miR-28-5p G4U

<400>    621
aguagc                                                                  6

<210>    622
<211>    6
<212>    RNA
<213>    Artificial Sequence

<220>
<223>    hsa-miR-224-5p G4U

<400>    622
aauuca                                                                  6

<210>    623
<211>    6
<212>    RNA
<213>    Artificial Sequence

<220>
<223>    hsa-miR-100-3p G4U

<400>    623
aaucuu                                                                    6


<210>    624
<211>    6
<212>    RNA
<213>    Artificial Sequence

<220>
<223>    hsa-miR-873-5p G4U


<400>    624
caugaa                                                                    6


<210>    625
<211>    6
<212>    RNA
<213>    Artificial Sequence

<220>
<223>    hsa-miR-4662a-5p G4U


<400>    625
uaucca                                                                    6


<210>    626
<211>    6
<212>    RNA
<213>    Artificial Sequence

<220>
<223>    hsa-miR-99b-3p/hsa-miR-99a-3p G4U


<400>    626
aaucuc                                                                    6


<210>    627
<211>    6
<212>    RNA
<213>    Artificial Sequence

<220>
<223>    hsa-miR-433-5p G4U


<400>    627
acugug                                                                    6


<210>    628
<211>    6
<212>    RNA
<213>    Artificial Sequence

<220>

<223>    hsa-miR-542-3p G4U

<400>    628
guuaca                                                                    6

<210>    629
<211>    6
<212>    RNA
<213>    Artificial Sequence

<220>
<223>    hsa-miR-3605-5p G4U

<400>    629
gaugau                                                                    6

<210>    630
<211>    6
<212>    RNA
<213>    Artificial Sequence

<220>
<223>    hsa-miR-744-5p G4U

<400>    630
gcuggg                                                                    6

<210>    631
<211>    6
<212>    RNA
<213>    Artificial Sequence

<220>
<223>    hsa-miR-1296-5p G4U

<400>    631
uauggc                                                                    6

<210>    632
<211>    6
<212>    RNA
<213>    Artificial Sequence

<220>
<223>    hsa-miR-133a-3p G4U

<400>    632
uuuguc                                                                    6

<210>    633
<211>    6
<212>    RNA
<213>    Artificial Sequence

<220>
<223>    hsa-miR-382-5p G4U


<400>    633
aauuug                                                                    6


<210>    634
<211>    6
<212>    RNA
<213>    Artificial Sequence

<220>
<223>    hsa-miR-425-5p G4U


<400>    634
auuaca                                                                    6


<210>    635
<211>    6
<212>    RNA
<213>    Artificial Sequence

<220>
<223>    hsa-miR-377-5p G4U


<400>    635
gauguu                                                                    6


<210>    636
<211>    6
<212>    RNA
<213>    Artificial Sequence

<220>
<223>    hsa-miR-127-3p G4U


<400>    636
cguauc                                                                    6


<210>    637
<211>    6
<212>    RNA
<213>    Artificial Sequence

<220>
<223>    hsa-miR-3180-3p G4U


<400>    637
ggugcg                                                                    6


<210>    638
<211>    6

<212>    RNA
<213>    Artificial Sequence

<220>
<223>    hsa-miR-143-3p G4U


<400>    638
gauaug                                                                    6


<210>    639
<211>    6
<212>    RNA
<213>    Artificial Sequence

<220>
<223>    hsa-miR-758-3p G4U


<400>    639
uuuuga                                                                    6


<210>    640
<211>    6
<212>    RNA
<213>    Artificial Sequence

<220>
<223>    hsa-miR-93-3p G4U


<400>    640
cuucug                                                                    6


<210>    641
<211>    6
<212>    RNA
<213>    Artificial Sequence

<220>
<223>    hsa-miR-128-2-5p G4U


<400>    641
ggugcc                                                                    6


<210>    642
<211>    6
<212>    RNA
<213>    Artificial Sequence

<220>
<223>    hsa-miR-134-5p G4U


<400>    642
guuacu                                                                    6

<210>    643
<211>    6
<212>    RNA
<213>    Artificial Sequence

<220>
<223>    hsa-miR-154-5p G4U


<400>    643
aguuua                                                                    6


<210>    644
<211>    6
<212>    RNA
<213>    Artificial Sequence

<220>
<223>    hsa-miR-3622a-5p G4U


<400>    644
agucac                                                                    6


<210>    645
<211>    6
<212>    RNA
<213>    Artificial Sequence

<220>
<223>    hsa-miR-124-3p G4U


<400>    645
aaugca                                                                    6


<210>    646
<211>    6
<212>    RNA
<213>    Artificial Sequence

<220>
<223>    hsa-miR-27a-5p G4U


<400>    646
ggucuu                                                                    6


<210>    647
<211>    6
<212>    RNA
<213>    Artificial Sequence

<220>
<223>    hsa-miR-194-3p G4U


<400>    647
cauugg                                                                    6

<210>    648
<211>    6
<212>    RNA
<213>    Artificial Sequence

<220>
<223>    hsa-miR-375 G4U


<400>    648
uuuuuc                                                                    6


<210>    649
<211>    6
<212>    RNA
<213>    Artificial Sequence

<220>
<223>    hsa-miR-148a-5p G4U


<400>    649
aauuuc                                                                    6


<210>    650
<211>    6
<212>    RNA
<213>    Artificial Sequence

<220>
<223>    hsa-miR-2277-5p G4U


<400>    650
gcucgg                                                                    6


<210>    651
<211>    6
<212>    RNA
<213>    Artificial Sequence

<220>
<223>    hsa-miR-769-5p G4U


<400>    651
gauacc                                                                    6


<210>    652
<211>    6
<212>    RNA
<213>    Artificial Sequence

<220>
<223>    hsa-miR-17-3p G4U

<400>    652
cuucag                                                                    6


<210>    653
<211>    6
<212>    RNA
<213>    Artificial Sequence

<220>
<223>    hsa-miR-873-3p G4U


<400>    653
gauacu                                                                    6


<210>    654
<211>    6
<212>    RNA
<213>    Artificial Sequence

<220>
<223>    hsa-miR-4772-3p G4U


<400>    654
cuucaa                                                                    6


<210>    655
<211>    6
<212>    RNA
<213>    Artificial Sequence

<220>
<223>    hsa-miR-329-5p G4U


<400>    655
aguuuu                                                                    6


<210>    656
<211>    6
<212>    RNA
<213>    Artificial Sequence

<220>
<223>    hsa-miR-182-5p/hsa-miR-96-5p G4U


<400>    656
uuugca                                                                    6


<210>    657
<211>    6
<212>    RNA
<213>    Artificial Sequence

<220>
<223>    hsa-miR-2467-5p/hsa-miR-485-5p G4U

<400>    657
gaugcu                                                                    6


<210>    658
<211>    6
<212>    RNA
<213>    Artificial Sequence

<220>
<223>    hsa-miR-149-5p G4U


<400>    658
cuugcu                                                                    6


<210>    659
<211>    6
<212>    RNA
<213>    Artificial Sequence

<220>
<223>    hsa-miR-29b-2-5p G4U


<400>    659
uguuuu                                                                    6


<210>    660
<211>    6
<212>    RNA
<213>    Artificial Sequence

<220>
<223>    hsa-miR-122-3p G4U


<400>    660
acucca                                                                    6


<210>    661
<211>    6
<212>    RNA
<213>    Artificial Sequence

<220>
<223>    hsa-miR-302a-3p/hsa-miR-520a-3p/hsa-miR-519b-3p/hsa-miR-520b/hsa-
         miR-519c-3p/hsa-miR-520c-3p/hsa-miR-519a-3p G4U


<400>    661
aauugc                                                                    6


<210>    662
<211>    6
<212>    RNA
<213>    Artificial Sequence

<220>
<223>    hsa-miR-532-5p G4U


<400>    662
auuccu                                                                    6


<210>    663
<211>    6
<212>    RNA
<213>    Artificial Sequence

<220>
<223>    hsa-miR-132-5p G4U


<400>    663
ccuugg                                                                    6


<210>    664
<211>    6
<212>    RNA
<213>    Artificial Sequence

<220>
<223>    hsa-miR-541-5p G4U


<400>    664
aaugau                                                                    6


<210>    665
<211>    6
<212>    RNA
<213>    Artificial Sequence

<220>
<223>    hsa-miR-671-3p G4U


<400>    665
ccuguu                                                                    6


<210>    666
<211>    6
<212>    RNA
<213>    Artificial Sequence

<220>
<223>    hsa-miR-296-5p G4U


<400>    666
   gguccc                                                                    6


<210>    667
<211>    6

<212>    RNA
<213>    Artificial Sequence

<220>
<223>    hsa-miR-518e-3p G4U


<400>    667
aaucgc                                                                    6


<210>    668
<211>    6
<212>    RNA
<213>    Artificial Sequence

<220>
<223>    hsa-miR-487a-5p G4U


<400>    668
uguuua                                                                    6


<210>    669
<211>    6
<212>    RNA
<213>    Artificial Sequence

<220>
<223>    hsa-miR-589-5p/hsa-miR-146b-5p/hsa-miR-146a-5p G4U


<400>    669
gauaac                                                                    6


<210>    670
<211>    6
<212>    RNA
<213>    Artificial Sequence

<220>
<223>    hsa-miR-196b-5p/hsa-miR-196a-5p G4U


<400>    670
aguuag                                                                    6


<210>    671
<211>    6
<212>    RNA
<213>    Artificial Sequence

<220>
<223>    hsa-miR-486-3p G4U


<400>    671
ggugca                                                                    6

<210>	672
<211>	6
<212>	RNA
<213>	Artificial Sequence

<220>
<223>	hsa-miR-629-5p G4U


<400>	672
gguuuu	6


<210>	673
<211>	6
<212>	RNA
<213>	Artificial Sequence

<220>
<223>	hsa-miR-378a-3p G4U


<400>	673
cuugac	6


<210>	674
<211>	6
<212>	RNA
<213>	Artificial Sequence

<220>
<223>	hsa-miR-27b-5p G4U


<400>	674
gaucuu	6


<210>	675
<211>	6
<212>	RNA
<213>	Artificial Sequence

<220>
<223>	hsa-miR-6720-3p G4U


<400>	675
gcuccu	6


<210>	676
<211>	6
<212>	RNA
<213>	Artificial Sequence

<220>
<223>	hsa-miR-574-3p G4U


<400>	676
acucuc	6

<210>    677
<211>    6
<212>    RNA
<213>    Artificial Sequence

<220>
<223>    hsa-miR-29a-5p G4U


<400>    677
cuuauu                                                                    6


<210>    678
<211>    6
<212>    RNA
<213>    Artificial Sequence

<220>
<223>    hsa-miR-30c-2-3p/hsa-miR-30c-1-3p G4U


<400>    678
ugugag                                                                    6


<210>    679
<211>    6
<212>    RNA
<213>    Artificial Sequence

<220>
<223>    hsa-miR-199b-3p G4U


<400>    679
cauuag                                                                    6


<210>    680
<211>    6
<212>    RNA
<213>    Artificial Sequence

<220>
<223>    hsa-miR-574-5p G4U


<400>    680
gauugu                                                                    6


<210>    681
<211>    6
<212>    RNA
<213>    Artificial Sequence

<220>
<223>    hsa-miR-539-5p G4U

<400>    681
gauaaa                                                                  6


<210>    682
<211>    6
<212>    RNA
<213>    Artificial Sequence

<220>
<223>    hsa-miR-4677-3p G4U


<400>    682
cuuuga                                                                  6


<210>    683
<211>    6
<212>    RNA
<213>    Artificial Sequence

<220>
<223>    hsa-miR-654-3p G4U


<400>    683
auuucu                                                                  6


<210>    684
<211>    6
<212>    RNA
<213>    Artificial Sequence

<220>
<223>    hsa-miR-652-3p G4U


<400>    684
auugcg                                                                  6


<210>    685
<211>    6
<212>    RNA
<213>    Artificial Sequence

<220>
<223>    hsa-miR-19a-3p/hsa-miR-19b-3p G4U


<400>    685
guucaa                                                                  6


<210>    686
<211>    6
<212>    RNA
<213>    Artificial Sequence

<220>
<223>    hsa-let-7c-5p/hsa-miR-98-5p/hsa-let-7g-5p/hsa-let-7f-5p/hsa-miR-2

02-3p/hsa-let-7b-5p/hsa-let-7e-5p/hsa-let-7a-5p/hsa-let-7d-5p/hsa
-let-7i-5p G4U

<400> 686
gaugua 6

<210> 687
<211> 6
<212> RNA
<213> Artificial Sequence

<220>
<223> hsa-miR-3663-3p G4U

<400> 687
gaucac 6

<210> 688
<211> 6
<212> RNA
<213> Artificial Sequence

<220>
<223> hsa-miR-152-3p/hsa-miR-148b-3p/hsa-miR-148a-3p G4U

<400> 688
cauugc 6

<210> 689
<211> 6
<212> RNA
<213> Artificial Sequence

<220>
<223> hsa-miR-193b-5p G4U

<400> 689
gguguu 6

<210> 690
<211> 6
<212> RNA
<213> Artificial Sequence

<220>
<223> hsa-miR-502-3p/hsa-miR-501-3p G4U

<400> 690
auucac 6

<210> 691
<211> 6
<212> RNA

<213>    Artificial Sequence

<220>
<223>    hsa-miR-299-3p G4U


<400>    691
auuugg                                                                    6


<210>    692
<211>    6
<212>    RNA
<213>    Artificial Sequence

<220>
<223>    hsa-miR-140-5p G5U


<400>    692
aguugu                                                                    6


<210>    693
<211>    6
<212>    RNA
<213>    Artificial Sequence

<220>
<223>    hsa-miR-96-5p/hsa-miR-182-5p G5U


<400>    693
uuguca                                                                    6


<210>    694
<211>    6
<212>    RNA
<213>    Artificial Sequence

<220>
<223>    hsa-miR-193b-3p G5U


<400>    694
acuugc                                                                    6


<210>    695
<211>    6
<212>    RNA
<213>    Artificial Sequence

<220>
<223>    hsa-miR-365a-3p G5U


<400>    695
aauucc                                                                    6


<210>    696

<211>	6
<212>	RNA
<213>	Artificial Sequence

<220>
<223>	hsa-miR-486-5p G5U


<400>	696
ccuuua	6


<210>	697
<211>	6
<212>	RNA
<213>	Artificial Sequence

<220>
<223>	hsa-miR-125b-1-3p G5U


<400>	697
cgguuu	6


<210>	698
<211>	6
<212>	RNA
<213>	Artificial Sequence

<220>
<223>	hsa-miR-210-3p G5U


<400>	698
uguucg	6


<210>	699
<211>	6
<212>	RNA
<213>	Artificial Sequence

<220>
<223>	hsa-miR-493-3p G5U


<400>	699
gaaugu	6


<210>	700
<211>	6
<212>	RNA
<213>	Artificial Sequence

<220>
<223>	hsa-miR-548am-5p G5U


<400>	700
aaauua	6

<210>    701
<211>    6
<212>    RNA
<213>    Artificial Sequence

<220>
<223>    hsa-miR-218-5p G5U


<400>    701
uguucu                                                                    6


<210>    702
<211>    6
<212>    RNA
<213>    Artificial Sequence

<220>
<223>    hsa-miR-20b-5p/hsa-miR-20a-5p/hsa-miR-93-5p/hsa-miR-17-5p/hsa-miR
         -106b-5p G5U


<400>    702
aaauug                                                                    6


<210>    703
<211>    6
<212>    RNA
<213>    Artificial Sequence

<220>
<223>    hsa-miR-541-3p G5U


<400>    703
gguugg                                                                    6


<210>    704
<211>    6
<212>    RNA
<213>    Artificial Sequence

<220>
<223>    hsa-miR-452-5p G5U


<400>    704
acuuuu                                                                    6


<210>    705
<211>    6
<212>    RNA
<213>    Artificial Sequence

<220>
<223>    hsa-miR-221-5p G5U

<400>    705
ccuugc                                                                 6

<210>    706
<211>    6
<212>    RNA
<213>    Artificial Sequence

<220>
<223>    hsa-miR-518f-3p G5U

<400>    706
aaaucg                                                                 6

<210>    707
<211>    6
<212>    RNA
<213>    Artificial Sequence

<220>
<223>    hsa-miR-370-3p G5U

<400>    707
ccuucu                                                                 6

<210>    708
<211>    6
<212>    RNA
<213>    Artificial Sequence

<220>
<223>    hsa-miR-107/hsa-miR-103a-3p G5U

<400>    708
gcauca                                                                 6

<210>    709
<211>    6
<212>    RNA
<213>    Artificial Sequence

<220>
<223>    hsa-miR-122-5p G5U

<400>    709
ggauug                                                                 6

<210>    710
<211>    6
<212>    RNA
<213>    Artificial Sequence

<220>
<223>    hsa-miR-338-3p G5U

<400>    710
ccauca                                                                    6


<210>    711
<211>    6
<212>    RNA
<213>    Artificial Sequence

<220>
<223>    hsa-miR-409-3p G5U


<400>    711
aauuuu                                                                    6


<210>    712
<211>    6
<212>    RNA
<213>    Artificial Sequence

<220>
<223>    hsa-miR-124-3p G5U


<400>    712
aaguca                                                                    6


<210>    713
<211>    6
<212>    RNA
<213>    Artificial Sequence

<220>
<223>    hsa-let-7d-5p/hsa-let-7g-5p/hsa-let-7i-5p/hsa-let-7f-5p/hsa-let-7
         e-5p/hsa-let-7a-5p/hsa-let-7b-5p/hsa-let-7c-5p G5U


<400>    713
gaguua                                                                    6


<210>    714
<211>    6
<212>    RNA
<213>    Artificial Sequence

<220>
<223>    hsa-miR-130b-3p/hsa-miR-301a-3p/hsa-miR-130a-3p/hsa-miR-301b-3p
         G5U


<400>    714
aguuca                                                                    6


<210>    715
<211>    6
<212>    RNA

<213>    Artificial Sequence

<220>
<223>    hsa-miR-512-3p G5U


<400>    715
aguucu                                                                    6


<210>    716
<211>    6
<212>    RNA
<213>    Artificial Sequence

<220>
<223>    hsa-miR-191-5p G5U


<400>    716
aacuga                                                                    6


<210>    717
<211>    6
<212>    RNA
<213>    Artificial Sequence

<220>
<223>    hsa-miR-509-3-5p G5U


<400>    717
acuuca                                                                    6


<210>    718
<211>    6
<212>    RNA
<213>    Artificial Sequence

<220>
<223>    hsa-miR-92a-3p/hsa-miR-92b-3p/hsa-miR-363-3p/hsa-miR-25-3p/hsa-mi
         R-32-5p G5U


<400>    718
auuuca                                                                    6


<210>    719
<211>    6
<212>    RNA
<213>    Artificial Sequence

<220>
<223>    hsa-miR-18a-5p G5U


<400>    719
aaguug                                                                    6

<210>	720
<211>	6
<212>	RNA
<213>	Artificial Sequence

<220>
<223>	hsa-miR-183-5p G5U


<400>	720
auguca	6


<210>	721
<211>	6
<212>	RNA
<213>	Artificial Sequence

<220>
<223>	hsa-miR-138-5p G5U


<400>	721
gcuugu	6


<210>	722
<211>	6
<212>	RNA
<213>	Artificial Sequence

<220>
<223>	hsa-miR-1307-3p G5U


<400>	722
cucugc	6


<210>	723
<211>	6
<212>	RNA
<213>	Artificial Sequence

<220>
<223>	hsa-miR-423-5p G5U


<400>	723
gagugg	6


<210>	724
<211>	6
<212>	RNA
<213>	Artificial Sequence

<220>
<223>	hsa-miR-499a-5p/hsa-miR-208a-3p G5U


<400>	724
uaauac	6

<210>    725
<211>    6
<212>    RNA
<213>    Artificial Sequence

<220>
<223>    hsa-miR-378a-3p G5U


<400>    725
cuguac                                                                    6


<210>    726
<211>    6
<212>    RNA
<213>    Artificial Sequence

<220>
<223>    hsa-miR-186-5p G5U


<400>    726
aaauaa                                                                    6


<210>    727
<211>    6
<212>    RNA
<213>    Artificial Sequence

<220>
<223>    hsa-miR-450b-5p G5U


<400>    727
uuuuca                                                                    6


<210>    728
<211>    6
<212>    RNA
<213>    Artificial Sequence

<220>
<223>    hsa-miR-450a-5p G5U


<400>    728
uuuucg                                                                    6


<210>    729
<211>    6
<212>    RNA
<213>    Artificial Sequence

<220>
<223>    hsa-miR-142-3p G5U

<400>    729
guauug                                                                 6


<210>    730
<211>    6
<212>    RNA
<213>    Artificial Sequence

<220>
<223>    hsa-miR-101-3p/hsa-miR-144-3p G5U


<400>    730
acauua                                                                 6


<210>    731
<211>    6
<212>    RNA
<213>    Artificial Sequence

<220>
<223>    hsa-miR-320a G5U


<400>    731
aaaucu                                                                 6


<210>    732
<211>    6
<212>    RNA
<213>    Artificial Sequence

<220>
<223>    hsa-miR-199b-5p/hsa-miR-199a-5p G5U


<400>    732
ccauug                                                                 6


<210>    733
<211>    6
<212>    RNA
<213>    Artificial Sequence

<220>
<223>    hsa-miR-1296-5p G5U


<400>    733
uagugc                                                                 6


<210>    734
<211>    6
<212>    RNA
<213>    Artificial Sequence

<220>
<223>    hsa-miR-185-5p G5U

<400>    734
ggauag                                                                 6

<210>    735
<211>    6
<212>    RNA
<213>    Artificial Sequence

<220>
<223>    hsa-miR-135a-5p G5U

<400>    735
augucu                                                                 6

<210>    736
<211>    6
<212>    RNA
<213>    Artificial Sequence

<220>
<223>    hsa-miR-411-5p G6U

<400>    736
aguaua                                                                 6

<210>    737
<211>    6
<212>    RNA
<213>    Artificial Sequence

<220>
<223>    hsa-miR-145-5p G6U

<400>    737
uccauu                                                                 6

<210>    738
<211>    6
<212>    RNA
<213>    Artificial Sequence

<220>
<223>    hsa-miR-26a-5p G6U

<400>    738
ucaauu                                                                 6

<210>    739
<211>    6
<212>    RNA
<213>    Artificial Sequence

<220>
<223>    hsa-miR-193b-3p G6U


<400>    739
acuguc                                                                    6


<210>    740
<211>    6
<212>    RNA
<213>    Artificial Sequence

<220>
<223>    hsa-miR-34c-5p G6U


<400>    740
ggcauu                                                                    6


<210>    741
<211>    6
<212>    RNA
<213>    Artificial Sequence

<220>
<223>    hsa-miR-379-5p G6U


<400>    741
gguaua                                                                    6


<210>    742
<211>    6
<212>    RNA
<213>    Artificial Sequence

<220>
<223>    hsa-miR-125b-5p G6U


<400>    742
cccuua                                                                    6


<210>    743
<211>    6
<212>    RNA
<213>    Artificial Sequence

<220>
<223>    hsa-miR-221-5p G6U


<400>    743
ccuguc                                                                    6


<210>    744
<211>    6
<212>    RNA

<213>    Artificial Sequence

<220>
<223>    hsa-miR-526b-5p G6U


<400>    744
ucuuua                                                                    6


<210>    745
<211>    6
<212>    RNA
<213>    Artificial Sequence

<220>
<223>    hsa-miR-7-5p G6U


<400>    745
ggaaua                                                                    6


<210>    746
<211>    6
<212>    RNA
<213>    Artificial Sequence

<220>
<223>    hsa-miR-16-5p/hsa-miR-15b-5p/hsa-miR-424-5p/hsa-miR-15a-5p G6U


<400>    746
agcauc                                                                    6


<210>    747
<211>    6
<212>    RNA
<213>    Artificial Sequence

<220>
<223>    hsa-miR-9-3p G6U


<400>    747
uaaauc                                                                    6


<210>    748
<211>    6
<212>    RNA
<213>    Artificial Sequence

<220>
<223>    hsa-miR-363-5p G6U


<400>    748
ggguug                                                                    6


<210>    749

<211>    6
<212>    RNA
<213>    Artificial Sequence

<220>
<223>    hsa-miR-1298-3p G6U


<400>    749
aucuug                                                                    6


<210>    750
<211>    6
<212>    RNA
<213>    Artificial Sequence

<220>
<223>    hsa-miR-509-3p G6U


<400>    750
gauuug                                                                    6


<210>    751
<211>    6
<212>    RNA
<213>    Artificial Sequence

<220>
<223>    hsa-miR-744-5p G6U


<400>    751
gcggug                                                                    6


<210>    752
<211>    6
<212>    RNA
<213>    Artificial Sequence

<220>
<223>    hsa-miR-148a-3p G6U


<400>    752
caguuc                                                                    6


<210>    753
<211>    6
<212>    RNA
<213>    Artificial Sequence

<220>
<223>    hsa-miR-302a-3p G6U


<400>    753
aaguuc                                                                    6

<210>    754
<211>    6
<212>    RNA
<213>    Artificial Sequence

<220>
<223>    hsa-miR-1296-5p G6U


<400>    754
uagguc                                                                    6


<210>    755
<211>    6
<212>    RNA
<213>    Artificial Sequence

<220>
<223>    hsa-miR-423-5p G6U


<400>    755
gaggug                                                                    6


<210>    756
<211>    6
<212>    RNA
<213>    Artificial Sequence

<220>
<223>    hsa-miR-9-5p G6U


<400>    756
cuuuug                                                                    6


<210>    757
<211>    6
<212>    RNA
<213>    Artificial Sequence

<220>
<223>    hsa-miR-138-5p G6U


<400>    757
gcuguu                                                                    6


<210>    758
<211>    6
<212>    RNA
<213>    Artificial Sequence

<220>
<223>    hsa-miR-22-3p G6U


<400>    758

agcuuc                                                                  6

<210>    759
<211>    6
<212>    RNA
<213>    Artificial Sequence

<220>
<223>    hsa-miR-28-3p G6U

<400>    759
acuaua                                                                  6

<210>    760
<211>    6
<212>    RNA
<213>    Artificial Sequence

<220>
<223>    hsa-miR-508-3p G6U

<400>    760
gauuuu                                                                  6

<210>    761
<211>    6
<212>    RNA
<213>    Artificial Sequence

<220>
<223>    hsa-miR-137 G6U

<400>    761
uauuuc                                                                  6

<210>    762
<211>    6
<212>    RNA
<213>    Artificial Sequence

<220>
<223>    hsa-miR-5010-5p G6U

<400>    762
ggggua                                                                  6

<210>    763
<211>    6
<212>    RNA
<213>    Artificial Sequence

<220>
<223>    hsa-miR-523-5p G6U

<400>    763
ucuaua                                                                    6


<210>    764
<211>    6
<212>    RNA
<213>    Artificial Sequence

<220>
<223>    hsa-miR-191-5p G6U


<400>    764
aacgua                                                                    6


<210>    765
<211>    6
<212>    RNA
<213>    Artificial Sequence

<220>
<223>    hsa-miR-128-3p G6U


<400>    765
cacauu                                                                    6


<210>    766
<211>    6
<212>    RNA
<213>    Artificial Sequence

<220>
<223>    hsa-miR-199a-5p/hsa-miR-199b-5p G7U


<400>    766
ccaguu                                                                    6


<210>    767
<211>    6
<212>    RNA
<213>    Artificial Sequence

<220>
<223>    hsa-miR-181a-2-3p G7U


<400>    767
ccacuu                                                                    6


<210>    768
<211>    6
<212>    RNA
<213>    Artificial Sequence

<220>

<223>     hsa-miR-27a-3p/hsa-miR-27b-3p G7U

<400>     768
ucacau                                                                    6

<210>     769
<211>     6
<212>     RNA
<213>     Artificial Sequence

<220>
<223>     hsa-let-7d-3p G7U

<400>     769
uauacu                                                                    6

<210>     770
<211>     6
<212>     RNA
<213>     Artificial Sequence

<220>
<223>     hsa-miR-129-5p G7U

<400>     770
uuuuuu                                                                    6

<210>     771
<211>     6
<212>     RNA
<213>     Artificial Sequence

<220>
<223>     hsa-miR-210-3p G7U

<400>     771
ugugcu                                                                    6

<210>     772
<211>     6
<212>     RNA
<213>     Artificial Sequence

<220>
<223>     hsa-miR-219a-2-3p G7U

<400>     772
gaauuu                                                                    6

<210>     773
<211>     6
<212>     RNA
<213>     Artificial Sequence

<220>
<223>    hsa-miR-424-3p G7U


<400>    773
aaaacu                                                                    6


<210>    774
<211>    6
<212>    RNA
<213>    Artificial Sequence

<220>
<223>    hsa-miR-18a-5p G7U


<400>    774
aagguu                                                                    6


<210>    775
<211>    6
<212>    RNA
<213>    Artificial Sequence

<220>
<223>    hsa-miR-488-3p G7U


<400>    775
ugaaau                                                                    6


<210>    776
<211>    6
<212>    RNA
<213>    Artificial Sequence

<220>
<223>    hsa-miR-1-3p G7U


<400>    776
ggaauu                                                                    6


<210>    777
<211>    6
<212>    RNA
<213>    Artificial Sequence

<220>
<223>    hsa-miR-181a-3p G7U


<400>    777
ccaucu                                                                    6


<210>    778
<211>    6

<212>    RNA
<213>    Artificial Sequence

<220>
<223>    hsa-miR-199b-3p G7U


<400>    778
caguau                                                                    6


<210>    779
<211>    6
<212>    RNA
<213>    Artificial Sequence

<220>
<223>    hsa-miR-10a-5p G7U


<400>    779
acccuu                                                                    6


<210>    780
<211>    6
<212>    RNA
<213>    Artificial Sequence

<220>
<223>    hsa-miR-196b-5p G7U


<400>    780
agguau                                                                    6


<210>    781
<211>    6
<212>    RNA
<213>    Artificial Sequence

<220>
<223>    hsa-miR-92a-1-5p G7U


<400>    781
gguugu                                                                    6


<210>    782
<211>    6
<212>    RNA
<213>    Artificial Sequence

<220>
<223>    hsa-miR-483-5p G7U


<400>    782
agacgu                                                                    6

<210>    783
<211>    6
<212>    RNA
<213>    Artificial Sequence

<220>
<223>    hsa-miR-17-3p G7U


<400>    783
cugcau                                                                    6


<210>    784
<211>    6
<212>    RNA
<213>    Artificial Sequence

<220>
<223>    hsa-miR-363-5p G7U


<400>    784
gggugu                                                                    6


<210>    785
<211>    6
<212>    RNA
<213>    Artificial Sequence

<220>
<223>    hsa-miR-9-5p G7U


<400>    785
cuuugu                                                                    6


<210>    786
<211>    6
<212>    RNA
<213>    Artificial Sequence

<220>
<223>    hsa-miR-1537-3p G7U


<400>    786
aaaccu                                                                    6


<210>    787
<211>    6
<212>    RNA
<213>    Artificial Sequence

<220>
<223>    hsa-miR-106b-5p/hsa-miR-20a-5p/hsa-miR-17-5p/hsa-miR-93-5p G7U


<400>    787
aaaguu                                                                    6

<210>    788
<211>    6
<212>    RNA
<213>    Artificial Sequence

<220>
<223>    hsa-miR-143-3p G7U


<400>    788
gagauu                                                                    6


<210>    789
<211>    6
<212>    RNA
<213>    Artificial Sequence

<220>
<223>    hsa-miR-30a-3p/hsa-miR-30e-3p G7U


<400>    789
uuucau                                                                    6


<210>    790
<211>    6
<212>    RNA
<213>    Artificial Sequence

<220>
<223>    hsa-miR-423-3p G7U


<400>    790
gcucgu                                                                    6


<210>    791
<211>    6
<212>    RNA
<213>    Artificial Sequence

<220>
<223>    hsa-miR-1298-3p G7U


<400>    791
aucugu                                                                    6


<210>    792
<211>    6
<212>    RNA
<213>    Artificial Sequence

<220>
<223>    hsa-miR-1307-5p G7U

<400>    792
cgaccu                                                                 6


<210>    793
<211>    6
<212>    RNA
<213>    Artificial Sequence

<220>
<223>    hsa-miR-423-5p G7U


<400>    793
gagggu                                                                 6


<210>    794
<211>    6
<212>    RNA
<213>    Artificial Sequence

<220>
<223>    hsa-miR-122-5p G7U


<400>    794
ggaguu                                                                 6


<210>    795
<211>    7
<212>    RNA
<213>    Artificial Sequence

<220>
<223>    hsa-miR-374a-3p G8U


<400>    795
uuaucau                                                                7


<210>    796
<211>    7
<212>    RNA
<213>    Artificial Sequence

<220>
<223>    hsa-miR-675-5p G8U


<400>    796
ggugcgu                                                                7


<210>    797
<211>    7
<212>    RNA
<213>    Artificial Sequence

<220>
<223>    hsa-miR-92a-1-5p G8U

<400>    797
gguuggu                                                                 7

<210>    798
<211>    7
<212>    RNA
<213>    Artificial Sequence

<220>
<223>    hsa-miR-1307-5p G8U

<400>    798
cgaccgu                                                                 7

<210>    799
<211>    7
<212>    RNA
<213>    Artificial Sequence

<220>
<223>    hsa-miR-503-5p G8U

<400>    799
agcagcu                                                                 7

<210>    800
<211>    7
<212>    RNA
<213>    Artificial Sequence

<220>
<223>    hsa-miR-24-3p G8U

<400>    800
ggcucau                                                                 7

<210>    801
<211>    7
<212>    RNA
<213>    Artificial Sequence

<220>
<223>    hsa-miR-340-5p G8U

<400>    801
uauaaau                                                                 7

<210>    802
<211>    7
<212>    RNA
<213>    Artificial Sequence

<220>
<223>    hsa-miR-509-3-5p G8U


<400>    802
acugcau                                                                    7


<210>    803
<211>    7
<212>    RNA
<213>    Artificial Sequence

<220>
<223>    hsa-miR-34a-5p/hsa-miR-34c-5p G8U


<400>    803
ggcaguu                                                                    7


<210>    804
<211>    7
<212>    RNA
<213>    Artificial Sequence

<220>
<223>    hsa-miR-526b-5p G8U


<400>    804
ucuugau                                                                    7


<210>    805
<211>    7
<212>    RNA
<213>    Artificial Sequence

<220>
<223>    hsa-miR-203a-3p G8U


<400>    805
ugaaauu                                                                    7


<210>    806
<211>    7
<212>    RNA
<213>    Artificial Sequence

<220>
<223>    hsa-miR-153-3p G8U


<400>    806
ugcauau                                                                    7


<210>    807
<211>    7
<212>    RNA

<213>    Artificial Sequence

<220>
<223>    hsa-miR-208a-3p G8U


<400>    807
uaagacu                                                                 7


<210>    808
<211>    7
<212>    RNA
<213>    Artificial Sequence

<220>
<223>    hsa-miR-200b-3p/hsa-miR-200c-3p G8U


<400>    808
aauacuu                                                                 7


<210>    809
<211>    7
<212>    RNA
<213>    Artificial Sequence

<220>
<223>    hsa-miR-518f-5p/hsa-miR-523-5p G8U


<400>    809
ucuagau                                                                 7


<210>    810
<211>    7
<212>    RNA
<213>    Artificial Sequence

<220>
<223>    hsa-miR-625-3p G8U


<400>    810
acuauau                                                                 7


<210>    811
<211>    7
<212>    RNA
<213>    Artificial Sequence

<220>
<223>    hsa-miR-194-5p G8U


<400>    811
guaacau                                                                 7


<210>    812

<211>    7
<212>    RNA
<213>    Artificial Sequence

<220>
<223>    hsa-let-7g-3p G8U


<400>    812
uguacau                                                                  7


<210>    813
<211>    7
<212>    RNA
<213>    Artificial Sequence

<220>
<223>    hsa-miR-1298-3p G8U


<400>    813
aucuggu                                                                  7


<210>    814
<211>    7
<212>    RNA
<213>    Artificial Sequence

<220>
<223>    hsa-miR-514a-5p G8U


<400>    814
acucugu                                                                  7


<210>    815
<211>    7
<212>    RNA
<213>    Artificial Sequence

<220>
<223>    hsa-miR-483-5p G8U


<400>    815
agacggu                                                                  7


<210>    816
<211>    7
<212>    RNA
<213>    Artificial Sequence

<220>
<223>    hsa-miR-126-3p G8U


<400>    816
cguaccu                                                                  7

<210>    817
<211>    7
<212>    RNA
<213>    Artificial Sequence

<220>
<223>    hsa-miR-128-3p G8U


<400>    817
cacaguu                                                                    7


<210>    818
<211>    7
<212>    RNA
<213>    Artificial Sequence

<220>
<223>    hsa-miR-381-3p G8U


<400>    818
auacaau                                                                    7


<210>    819
<211>    7
<212>    RNA
<213>    Artificial Sequence

<220>
<223>    hsa-miR-320a G8U


<400>    819
aaagcuu                                                                    7


<210>    820
<211>    7
<212>    RNA
<213>    Artificial Sequence

<220>
<223>    hsa-miR-513c-5p/hsa-miR-514b-5p G8U


<400>    820
ucucaau                                                                    7


<210>    821
<211>    7
<212>    RNA
<213>    Artificial Sequence

<220>
<223>    hsa-miR-138-5p G8U


<400>    821

gcugguu                                                                 7

<210>    822
<211>    7
<212>    RNA
<213>    Artificial Sequence

<220>
<223>    hsa-miR-520a-5p G8U

<400>    822
uccagau                                                                 7

<210>    823
<211>    7
<212>    RNA
<213>    Artificial Sequence

<220>
<223>    hsa-miR-125b-5p/hsa-miR-125a-5p G8U

<400>    823
cccugau                                                                 7

<210>    824
<211>    7
<212>    RNA
<213>    Artificial Sequence

<220>
<223>    hsa-miR-141-3p G8U

<400>    824
aacacuu                                                                 7

<210>    825
<211>    7
<212>    RNA
<213>    Artificial Sequence

<220>
<223>    hsa-miR-874-3p G8U

<400>    825
ugcccuu                                                                 7

<210>    826
<211>    7
<212>    RNA
<213>    Artificial Sequence

<220>
<223>    hsa-miR-202-5p G8U

<400>    826
uccuauu                                                                  7

<210>    827
<211>    7
<212>    RNA
<213>    Artificial Sequence

<220>
<223>    hsa-miR-140-3p G8U

<400>    827
accacau                                                                  7

<210>    828
<211>    7
<212>    RNA
<213>    Artificial Sequence

<220>
<223>    hsa-miR-361-3p G8U

<400>    828
cccccau                                                                  7

<210>    829
<211>    7
<212>    RNA
<213>    Artificial Sequence

<220>
<223>    hsa-miR-513b-5p G8U

<400>    829
ucacaau                                                                  7

<210>    830
<211>    7
<212>    RNA
<213>    Artificial Sequence

<220>
<223>    hsa-miR-134-5p G8U

<400>    830
gugacuu                                                                  7

<210>    831
<211>    7
<212>    RNA
<213>    Artificial Sequence

<220>

<223>    hsa-miR-33a-5p G8U

<400>    831
ugcauuu                                                                 7

<210>    832
<211>    7
<212>    RNA
<213>    Artificial Sequence

<220>
<223>    hsa-miR-512-3p G8U

<400>    832
agugcuu                                                                 7

<210>    833
<211>    7
<212>    RNA
<213>    Artificial Sequence

<220>
<223>    hsa-let-7a-5p/hsa-let-7c-5p/hsa-let-7b-5p/hsa-let-7d-5p/hsa-let-7
         f-5p/hsa-let-7e-5p/hsa-let-7i-5p/hsa-let-7g-5p G8U

<400>    833
gagguau                                                                 7

<210>    834
<211>    7
<212>    RNA
<213>    Artificial Sequence

<220>
<223>    hsa-miR-375 G8U

<400>    834
uuguucu                                                                 7

<210>    835
<211>    7
<212>    RNA
<213>    Artificial Sequence

<220>
<223>    hsa-miR-136-3p G8U

<400>    835
aucaucu                                                                 7

<210>    836
<211>    7
<212>    RNA

<213>    Artificial Sequence

<220>
<223>    hsa-miR-508-5p G8U


<400>    836
acuccau                                                                  7


<210>    837
<211>    8
<212>    RNA
<213>    Artificial Sequence

<220>
<223>    hsa-miR-124-3p G9U


<400>    837
aaggcacu                                                                 8


<210>    838
<211>    8
<212>    RNA
<213>    Artificial Sequence

<220>
<223>    hsa-miR-204-5p/hsa-miR-211-5p G9U


<400>    838
ucccuuuu                                                                 8


<210>    839
<211>    8
<212>    RNA
<213>    Artificial Sequence

<220>
<223>    hsa-miR-210-3p G9U


<400>    839
ugugcguu                                                                 8


<210>    840
<211>    8
<212>    RNA
<213>    Artificial Sequence

<220>
<223>    hsa-miR-146a-5p/hsa-miR-146b-5p G9U


<400>    840
gagaacuu                                                                 8


<210>    841

<211>    8
<212>    RNA
<213>    Artificial Sequence

<220>
<223>    hsa-miR-508-3p G9U


<400>    841
gauuguau                                                                 8


<210>    842
<211>    8
<212>    RNA
<213>    Artificial Sequence

<220>
<223>    hsa-miR-23a-3p G9U


<400>    842
ucacauuu                                                                 8


<210>    843
<211>    8
<212>    RNA
<213>    Artificial Sequence

<220>
<223>    hsa-miR-219a-2-3p G9U


<400>    843
gaauuguu                                                                 8


<210>    844
<211>    8
<212>    RNA
<213>    Artificial Sequence

<220>
<223>    hsa-miR-21-3p G9U


<400>    844
aacaccau                                                                 8


<210>    845
<211>    8
<212>    RNA
<213>    Artificial Sequence

<220>
<223>    hsa-miR-122-5p G9U


<400>    845
ggaguguu                                                                 8

<210>    846
<211>    8
<212>    RNA
<213>    Artificial Sequence

<220>
<223>    hsa-miR-877-5p G9U


<400>    846
uagaggau                                                                    8


<210>    847
<211>    8
<212>    RNA
<213>    Artificial Sequence

<220>
<223>    hsa-miR-129-5p G9U


<400>    847
uuuuugcu                                                                    8


<210>    848
<211>    8
<212>    RNA
<213>    Artificial Sequence

<220>
<223>    hsa-miR-526b-5p G9U


<400>    848
ucuugagu                                                                    8


<210>    849
<211>    8
<212>    RNA
<213>    Artificial Sequence

<220>
<223>    hsa-miR-302a-5p G9U


<400>    849
cuuaaacu                                                                    8


<210>    850
<211>    8
<212>    RNA
<213>    Artificial Sequence

<220>
<223>    hsa-miR-532-5p G9U


<400>    850

augccuuu                                                                 8


<210>    851
<211>    8
<212>    RNA
<213>    Artificial Sequence

<220>
<223>    hsa-miR-520a-5p G9U


<400>    851
uccagagu                                                                 8


<210>    852
<211>    8
<212>    RNA
<213>    Artificial Sequence

<220>
<223>    hsa-miR-139-5p G9U


<400>    852
cuacaguu                                                                 8


<210>    853
<211>    8
<212>    RNA
<213>    Artificial Sequence

<220>
<223>    hsa-miR-99a-5p/hsa-miR-100-5p/hsa-miR-99b-5p G9U


<400>    853
acccguau                                                                 8


<210>    854
<211>    8
<212>    RNA
<213>    Artificial Sequence

<220>
<223>    hsa-miR-320a G9U


<400>    854
aaagcugu                                                                 8


<210>    855
<211>    8
<212>    RNA
<213>    Artificial Sequence

<220>
<223>    hsa-miR-216a-5p G9U

<400>    855
aaucucau                                                                 8


<210>    856
<211>    8
<212>    RNA
<213>    Artificial Sequence

<220>
<223>    hsa-miR-127-3p G9U


<400>    856
cggauccu                                                                 8


<210>    857
<211>    8
<212>    RNA
<213>    Artificial Sequence

<220>
<223>    hsa-miR-125b-1-3p G9U


<400>    857
cggguuau                                                                 8


<210>    858
<211>    8
<212>    RNA
<213>    Artificial Sequence

<220>
<223>    hsa-miR-381-3p G9U


<400>    858
auacaagu                                                                 8


<210>    859
<211>    8
<212>    RNA
<213>    Artificial Sequence

<220>
<223>    hsa-miR-27a-3p/hsa-miR-27b-3p G9U


<400>    859
ucacaguu                                                                 8


<210>    860
<211>    8
<212>    RNA
<213>    Artificial Sequence

<220>

<223>    hsa-miR-5010-5p G9U

<400>    860
gggggauu                                                                 8

<210>    861
<211>    8
<212>    RNA
<213>    Artificial Sequence

<220>
<223>    hsa-miR-3157-3p G9U

<400>    861
ugcccuau                                                                 8

<210>    862
<211>    8
<212>    RNA
<213>    Artificial Sequence

<220>
<223>    hsa-miR-17-3p G9U

<400>    862
cugcaguu                                                                 8

<210>    863
<211>    8
<212>    RNA
<213>    Artificial Sequence

<220>
<223>    hsa-miR-523-5p G9U

<400>    863
ucuagagu                                                                 8